# EUROPEAN PATENT APPLICATION

(11) **EP 1 452 526 A1**
(43) Date of publication of application: **01.09.2004**
(21) Application number: 02777995.8
(22) Date of filing: 29.10.2002
(51) Int. Cl.: C07D 209/42, C07D 401/12, C07D 401/14, C07D 403/12, C07D 405/12, C07D 409/12, C07D 413/12, C07D 417/12, C07D 487/04, C07D 495/04, C07D 409/04, C07D 409/14, A61K 31/404, A61K 31/407, A61K 31/41, A61K 31/4178, A61K 31/4188, A61K 31/4196

(54) **INDOLE COMPOUND AND MEDICINAL USE THEREOF**

(30) Priority: 29.10.2001 JP 2001331501
(71) Applicant: Japan Tobacco Inc., Tokyo 105-8422 (JP)
(72) Inventor: NAKAMURA, Takeshi Central Pharmaceutical Research, Takatsuki-shi, Osaka 569-1125 (JP); TAKAGI, Masaki Central Pharmaceutical Research, Takatsuki-shi, Osaka 569-1125 (JP); UEDA, Nobuhisa Central Pharmaceutical Research, Takatsuki-shi, Osaka 569-1125 (JP)
(74) Representative: von Kreisler, Alek, Dipl.-Chem.
(86) International application number: PCT/JP2002/011234
(87) International publication number: WO 2003/037864

(57) **Abstract**

An indole compound represented by the formula (1) 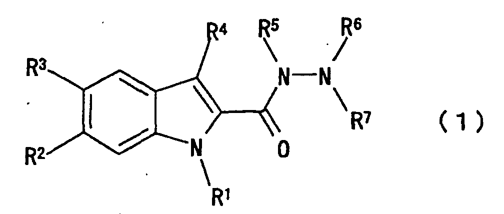 wherein each symbol is as defined in the specification, a pharmaceutically acceptable salt thereof or a prodrug thereof is useful as a therapeutic agents for diabetes having an HLGPa inhibitory activity.

## Description

### Technical Field

The present invention relates to a novel indole compound having an HLGPa (Human Liver Glycogen Phosphorylase a) inhibitory activity and use thereof as a pharmaceutical agent. More particularly, the present invention relates to a therapeutic agent for diabetes, which comprises an indole compound, a pharmaceutically acceptable salt thereof or a hydrate thereof.

### Background Art

Diabetes is a chronic disease caused by abnormal metabolism of sugar, lipid and amino acid due to shortage of insulin action. When maintained without treatment, it shows hyperglycemia and urine sugar. Diabetes is divided into insulin-dependent type and non-insulin-dependent type, and about 90% of diabetic patients suffer from non-insulin-dependent diabetes mellitus.

In insulin dependent diabetes, since insulin secretory capacity has disappeared, ketonemia and acidosis easily occur, and when left as is, diabetic coma follows. Food restriction and oral hypoglycemic agent provide no therapeutic effect but only insulin does.

On the other hand, non-insulin-dependent diabetes mellitus (NIDDM) shows an insulin action lower than normal, but shows little propensity toward ketonemia and acidosis, and its treatment does not always require insulin.

Hypoglycemic agents currently in use for correcting hyperglycemia include insulin preparations, sulfonylurea agents (e.g., glibenclamide, torbutamide), biguanides (e.g., metformin), insulin sensitizers (e.g., troglitazone) and α-glucosidase inhibitors (e.g., acarbose).

Insulin preparations are pharmaceutical agents used for insulin dependent diabetes, which certainly decrease blood glucose but require administration by injection and are associated with the risk of causing hypoglycemia.

Sulfonylurea agents stimulate beta cell of pancreas and accelerate endogenous insulin secretion. However, the timing and amount of insulin secretion have nothing to do with blood glucose level, and vary depending on the timing and dose of drug administration. As side effects, therefore, hypoglycemia due to the duration of action of pharmaceutical agent often occurs. In addition, symptoms in digestive organs such as anorexia and the like are developed. They are prohibited to patients with severe ketosis or liver or renal dysfunction.

Biguanides are free of stimulation of beta cell of pancreas, their single administration does not cause hypoglycemia in healthy individuals and diabetes patients. Predictable action mechanism includes increase in the use of sugar due to anaerobic glycolysis action, suppression of gluconeogenesis, suppression of absorption of sugar from intestinal tract and the like. As a side effect, they easily develop comparatively severe lactic acidic acidosis.

As insulin sensitizers, thiazolidine derivatives can be mentioned. The thiazolidine derivatives do not have an insulin secretion promoting action, enhance insulin action, and show an insulin receptor kinase activation, peripheral tissue glucose uptake promoting action, improvement of liver glucose production promotion and the like. As side effects, symptoms of digestive organs, edema and the like are developed, and decrease of red count, hematocrit and hemoglobin as well as increase in LDH are known to occur.

While α-glucosidase inhibitors delay digestion and absorption of carbohydrate from the gastrointestinal tract and suppress postprandial elevation of glycemia, they pose problems of side effects such as abdominal distension, borborygmus, diarrhea and the like (JOSLIN'S DIABETES MELLITUS 13Th Edition 521-522).

As mentioned above, the use of these pharmaceutical agents is limited due to the presence of side effects and ineffective patients, and a hypoglycemic agent based on a new action mechanism has been desired.

It has been reported in recent years that NIDDM patients .show increased amounts of glucose released from the liver during fasting as compared to healthy individuals. This sugar release from the liver suggests possibility as a treatment target of pharmaceutical agent for NIDDM.

The supply source of sugar in sugar release from the liver is glucose produced by gluconeogenesis and glycogenolysis. It has been reported that, in diabetic patients, the glycogenolysis is deeply involved in the glucose release from the liver, and glycogenolysis rate increases by 75% during fasted state as compared to healthy individuals. In NIDDM patients, glucose release from the liver transitionally increases after glucose loading, and involvement of glycogenolysis in this increase has been suggested. In type IV glycogenosis (liver glycogen phosphorylase deficiency), moreover, it is known that hypoglycemia is developed during fasted state.

These reports suggest that glycogenolysis plays a major role in glucose release from the liver.

It is known that the glycogen decomposition is catalyzed by HLGPa, and glucose 1-phosphoric acid (G1-P) and glycogen (glucose units in the number of n-1) are produced by the glycogenolysis (glucose units in the number of n) by superphosphoric acid.

Therefore, a therapeutic agent for diabetes having an HLGPa inhibitory action deeply involved in this glycogen decomposition, which is based on a new mechanism has been. developed. As the situation stands, however, no agent having satisfactory activity has been found.

Incidentally, as compounds having a similar structure as that of the present invention, the following compounds are known.

For example, JP-A-5-39253 discloses the following compounds useful as harmful animal repellents:

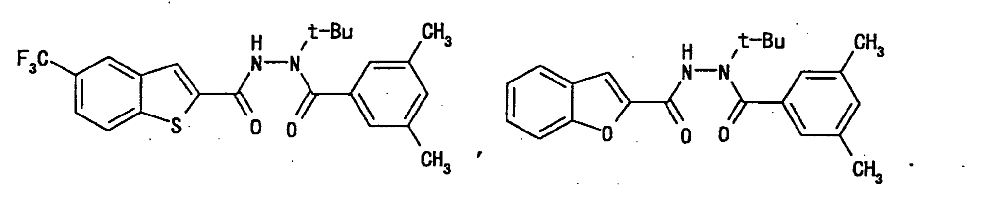

In this publication, no indole compound as in the present application is disclosed and use thereof is also completely different.

Tetrahedron Letter, vol. 23, pp. 2333-2335 (1972) discloses the following indole compound:

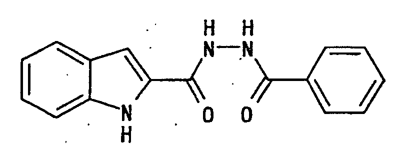

However, this reference does not disclose at all that an indole compound as in the present application has an HLGPa inhibitory activity and that the compound is effective as a therapeutic agent for diabetes.

Yaoxue Xuebao, 19(10), 737-741 (1984) discloses

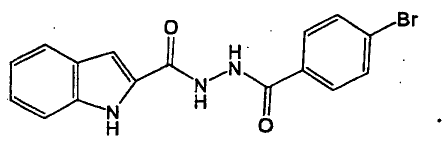

However, this reference does not disclose at all that an indole compound as in the present application has an HLGPa inhibitory activity and that the compound is effective as a therapeutic agent for diabetes.

In addition, WO96/39384 (JP-T-10-511687) discloses, as a compound having an indole structure and a glycogen phosphorylase suppressive activity similar to the action of the present invention, the following formula: 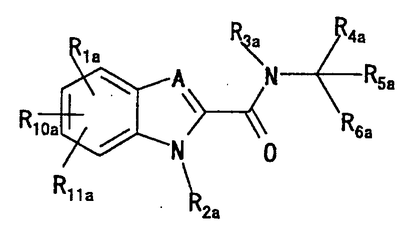 wherein R₄ₐ is a phenylalkyl group etc., R₅ₐ is a hydrogen atom etc., R₆ₐ is an alkoxycarbonyl group etc., R₂ₐ is a hydrogen atom, R₁ₐ, R₁₀ₐ and R₁₁ₐ are each independently a hydrogen atom, a halogen atom etc., R₃ₐ is a hydrogen atom etc. and A is -N= etc. As a specific example, the following compound has been disclosed:

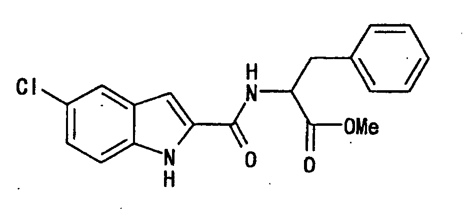

However, the compound of our invention is characterized in that it has an indole ring and a hydrazine and that the hydrazine is further bonded to a ring, but this compound does not essentially require a hydrazide structure.

While the development of a therapeutic agent for diabetes, which is based on inhibition of HLGPa is currently ongoing, a therapeutic agent having satisfactory activity has not been found as yet. Accordingly, there is a strong demand for the development of a superior HLGPa inhibitor having a stronger activity and free of side effects as compared to conventional therapeutic agents for diabetes.

### Disclosure of the Invention

In view of the above-mentioned problems, the present inventors have conducted intensive studies in an attempt to provide a therapeutic agent for diabetes having a useful HLGPa inhibitory activity and found that an indole compound represented by the following formula (1) has a stronger and remarkable HLGPa inhibitory activity as compared to conventional therapeutic agents for diabetes, which resulted in the completion of the present invention.

Accordingly, the present invention provides the following.
1. An indole compound represented by the formula (1) 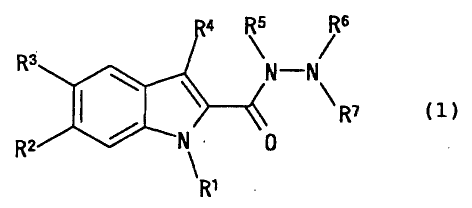 wherein
   R¹ is a hydrogen atom, a C₁₋₆ alkyl group or an acyl group;
   R² is a hydrogen atom or a halogen atom;
   R³ is a halogen atom, a C₁₋₆ alkyl group, a C₁₋₆ alkoxy group, a nitro group, an amino group, a hydroxyl group, a cyano group, an acyl group, an aralkyloxy group or a thiazolyl group
      wherein the thiazolyl group is optionally substituted by a C₁₋₆ alkyl group or an amino group;
   R⁴ is a hydrogen atom or a C₁₋₆ alkyl group;
   R⁵ is a hydrogen atom, a C₁₋₆ alkyl group or a C₂₋₇₋ alkoxycarbonyl group;
   R⁶ is a hydrogen atom, a C₁₋₆ alkyl group or an aralkyl group
      wherein the aralkyl group is optionally substituted by a halogen atom;
   R⁷ is 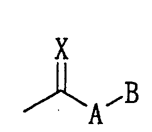 wherein X is =O, =S or =NH;
   A is -N(R⁸)- wherein R⁸ is a hydrogen atom, a C₁₋₆ alkyl group or an aryl group optionally having substituents, -C(R⁹)(R¹⁰)- wherein R⁹ and R¹⁰ are the same or different and each is independently a hydrogen atom, a hydroxyl group, an amino group, a C₁₋₆ alkyl group, a C₁₋₆ hydroxyalkyl group, a C₂₋₇ alkoxycarbonylamino group or an acylamino group, or R⁹ and R¹⁰ may form a C₃₋₇ cycloalkyl group together with the adjacent carbon atom, -(CH₂)ₘ-NH- wherein m is an integer of 1 to 4, -CO-, -S- or a single bond; and
   B is 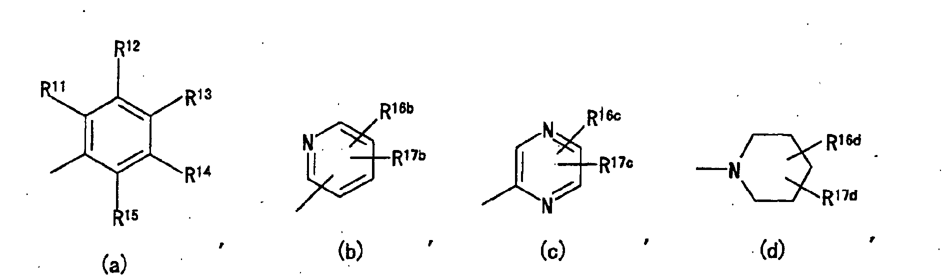 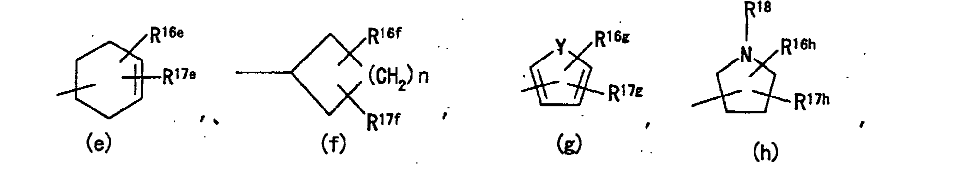 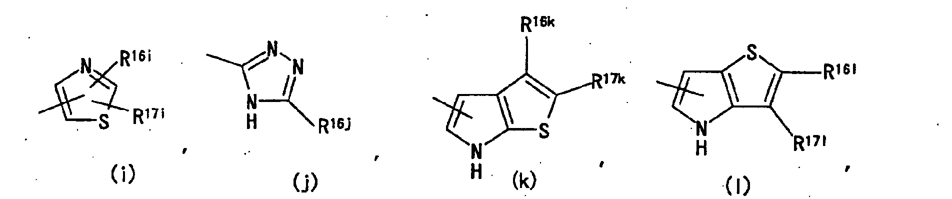 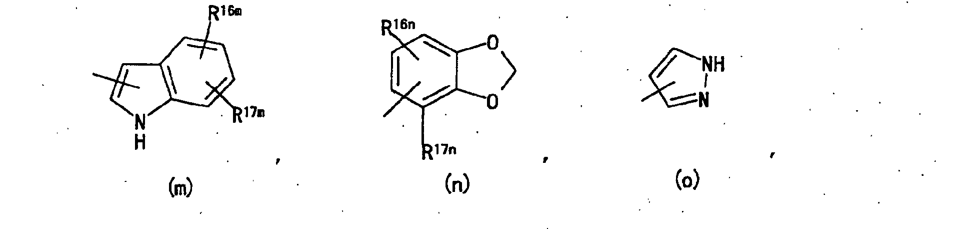 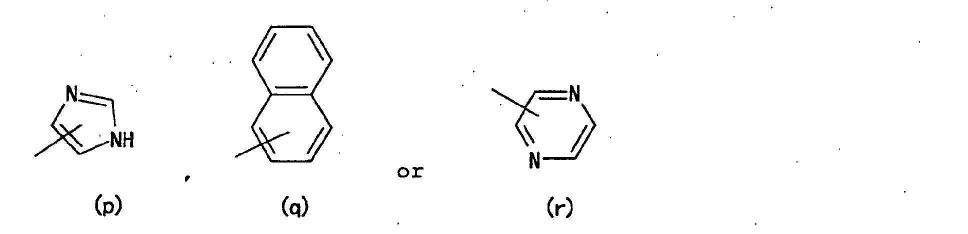 wherein R¹¹, R¹², R¹³, R¹⁴ and R¹⁵ are the same or different and each is independently a hydrogen atom, a halogen atom, a C₁₋₆ alkyl group, a C₁₋₆ alkoxy group, a nitro group, a hydroxyl group, a cyano group, a haloalkyl group, an aralkyl group, an aryl group optionally having substituents, an aryloxy group, a tetrazolyl group, a triazolyl group, -(CH₂)ₚ-CO-R¹⁹ wherein p is 0 or an integer of 1 to 4 and R¹⁹ is an aryl group optionally having substituents, a hydroxyl group, a C₁₋₆ alkoxy group or -N(R²⁰)(R²¹) wherein R²⁰ and R²¹ are the same or different and each is independently a hydrogen atom, a C₁₋₆ alkyl group, an aralkyl group or a C₃₋₁₃ alkoxycarbonylalkyl group, or R²⁰ and R²¹ may form, together with the adjacent nitrogen atom, 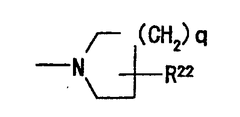 wherein q is an integer of 1 to 3 and R²² is a hydrogen atom, a hydroxyl group, a C₁₋₆ alkoxy group, an amino group, a C₂₋₁₂ dialkylamino group or a C₂₋₇ alkoxycarbonylamino group, -O-(CH₂)ᵣ-R²³ wherein r is an integer of 1 to 4 and R²³ is a hydroxyl group, an amino group, a C₂₋₇ alkylcarbonyloxy group or -CO-R²⁴ wherein R²⁴ is a hydroxyl group, a C₁₋₆ alkoxy group or -N(R²⁵)(R²⁶) wherein R²⁵ and R²⁶ are the same or different and each is a hydrogen atom, a C₁₋₆ alkyl group or an aralkyl group, or R²⁵ and R²⁶ may form, together with the adj acent' nitrogen atom, 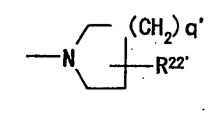 or 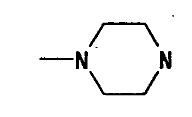 wherein q' and R^{22'} are as defined for q and R²², respectively, -O-CO-R²⁷ wherein R²⁷ is a C₁₋₆ alkylamino group or a C₂₋₁₂ dialkylamino group, or -N(R²⁸)(R²⁹) wherein R²⁸ and R²⁹ are the same or different and each is a hydrogen atom, a C₁₋₆ alkyl group, an aryl group optionally having substituents, an acyl group, -(CH₂)_{p'}-COO-R³⁰ wherein p' is as defined for p and R³⁰ is a hydrogen atom, an aryl group optionally having substituents or a C₁₋₆ alkyl group wherein the C₁₋₆ alkyl group is optionally substituted by a hydroxyl group, a trifluoromethyl group, an aryl group optionally having substituents, a morpholino group or a carboxyl group, -CON(R³¹)(R³²) wherein R³¹ and R³² are the same or different and each is a hydrogen atom, a C₁₋₆ alkyl group or an aryl group optionally having substituents, -CO-R³³ wherein R³³ is a C₁₋₆ alkyl group or an aryl group optionally having substituents or -CO-(CH₂)_{r'}-R³⁴ wherein r' is as defined for r and R³⁹ is a C₁₋₆ alkylamino group, a C₂₋₁₂ dialkylamino group, a C₁₋₆ alkoxy group or a C₂₋₇ alkylcarbonyloxy group, R^{16b}-R¹⁶ⁿ and R^{17b}-R¹⁷ⁿ are the same or different and each is a hydrogen atom, a halogen atom, a C₁₋₆ alkyl group, an amino group, a hydroxyl group, a C₁₋₆ alkoxy group or -CON(R^{31'})(R^{32'}) wherein R^{31'} and R^{32'} are as defined for R³¹ and R³²,
   R¹⁸ is a hydrogen atom or a C₂₋₇ alkoxycarbonyl group,
   Y is -S-, -O- or -N(R³⁵)- wherein R³⁵ is a hydrogen atom or a C₁₋₆ alkyl group, and
   n is 0 or an integer of 1 to 4, or
   R⁶ and R⁷ may form, together with the adjacent nitrogen atom, 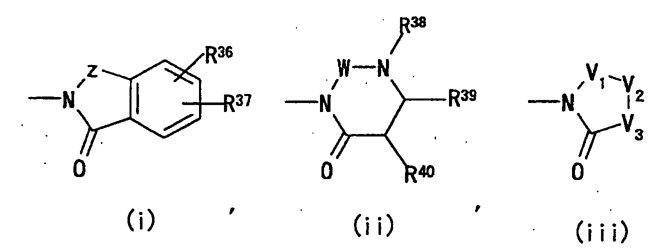 or 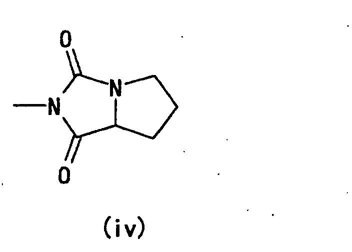 wherein R³⁶ and R³⁷ are the same or different and each is a hydrogen atom, a halogen atom, a C₁₋₆ alkyl group, a C₁₋₆ alkoxy group, an amino group, a nitro group, a hydroxyl group, a C₂₋₇ alkoxycarbonyl group, a carboxyl group, a C₂₋₇ haloalkylcarbonylamino group or -O-CO-R⁴¹ wherein R⁴¹ is a C₁₋₆ alkyl group, a C₁₋₆ alkylamino group or a C₂₋₁₂ dialkylamino group;
   Z is -CH₂-CH₂-, -C(R⁴²)=CH-, -C(R^{42'})=N-, -N=N-, -CO-, -CO-O-, -CO-CH₂-O-, -CH₂-CO-NH-, -C(R^{42''})(R⁴³)-N(R⁴⁴)- wherein R⁴², R^{42'}, R^{42''} and R⁴³ are the same or different and each is a hydrogen atom, a C₁₋₆ alkyl group or an aryl group optionally having substituents and R⁴⁴ is a hydrogen atom, a C₂₋₇ alkoxycarbonyl group or a C₁₋₆ alkyl group wherein the C₁₋₆ alkyl group is optionally substituted by a carboxyl group or a C₂₋₇ alkoxycarbonyl group, or -C(U)-N(R^{44'})- wherein U is =O or =S and R^{44'} is as defined for R⁴⁴ wherein an atom adjacent to the nitrogen atom on the fused ring in the formula (i) is described on the left end of each group;
   R³⁸ is a hydrogen atom, an aryl group optionally having substituents or a heteroaryl group;
   R³⁹ and R⁴⁰ are the same or different and each is a hydrogen atom, a C₁₋₆ alkyl group, a C₁₋₆ alkoxy group or a C₂₋₇ alkoxycarbonyl group, or R³⁹ and R⁴⁰ may form, together with the adjacent carbon atom, 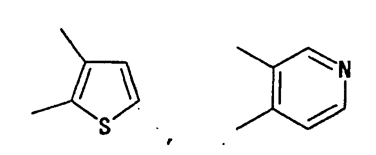 or 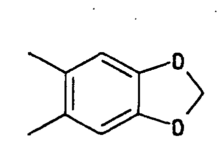
   W is -CO-, -CS- or -CH₂-;
   V₁ is -CO-, -CS- or -CH₂-;
   V₂ is -O-, -CH₂- or -N(R⁴⁵)- wherein R⁴⁵ is a hydrogen atom, a C₁₋₆ alkyl group or an aryl group optionally having substituents; and
   V₃ is -CH(R⁴⁶)- or -N(R^{46'})- wherein R⁴⁶ and R^{46'} are each a hydrogen atom, an aralkyl group, a heteroaryl group or an aryl group optionally having substituents,
      a pharmaceutically acceptable salt thereof or a prodrug thereof (hereinafter sometimes to be abbreviated as the compound (1) of the present invention.
2. The indole compound of 1 above,
   wherein
   R⁶ is a hydrogen atom, a C₁₋₆ alkyl group or an aralkyl group wherein the aralkyl group is optionally substituted by a halogen atom;
   R⁷ is 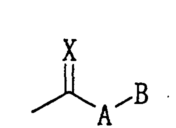 wherein X is =O, =S or =NH;
   A is -N(R^{8'})- wherein R^{8'} is a hydrogen atom, a C₁₋₆ alkyl group or a phenyl group optionally having substituents, -C(R^{9'})(R^{10'})- wherein R^{9'} and R^{10'} are the same or different and each is a hydrogen atom, a hydroxyl group, an amino group, a C₁₋₆ alkyl group, a C₁₋₆ hydroxyalkyl group, a C₂₋₇ alkoxycarbonylamino group or an acylamino group, or R^{9'} and R^{10'} may form, together with the adjacent carbon atom, a C₃₋₇ cycloalkyl group, -(CH₂)ₘ-NH- wherein m is an integer of 1 to 4, -CO-, -S- or a single bond; and
   B is 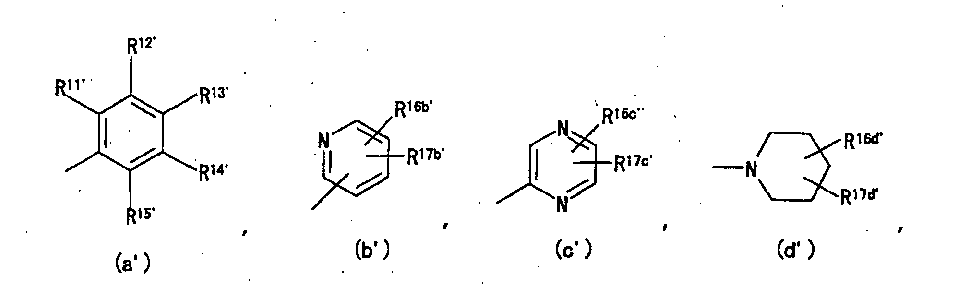 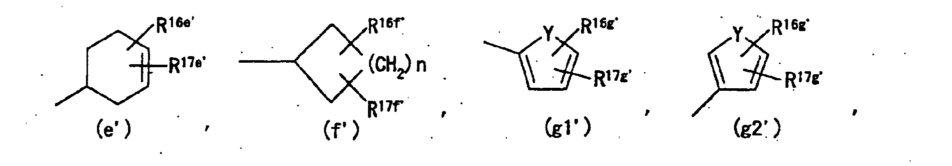 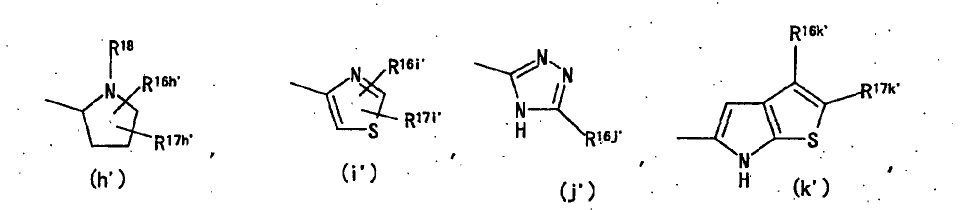 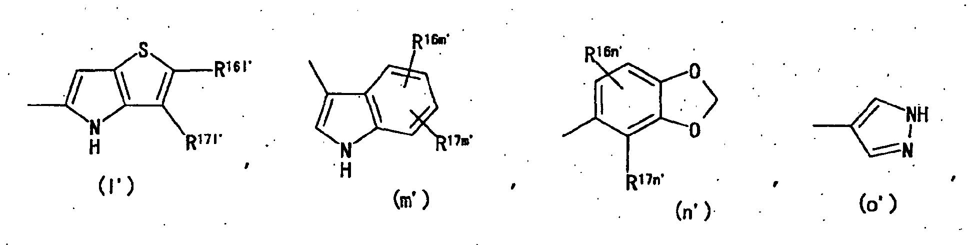 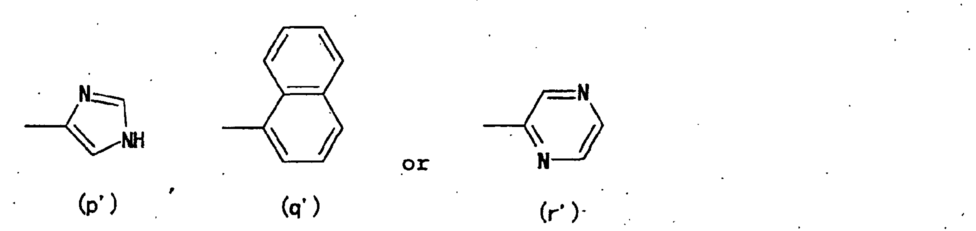 wherein R^{11'}, R^{12'}, R^{13'}, R^{14'} and R^{15'} are the same or different and each is a hydrogen atom, a halogen atom, a C₁₋₆ alkyl group, a C₁₋₆ alkoxy group, a nitro group, a hydroxyl group, a cyano group, a haloalkyl group, an aralkyl group, a phenyl group optionally having substituents, an aryloxy group, a tetrazolyl group, a triazolyl group, -(CH₂)ₚ-CO-R^{19'} wherein p is 0 or an integer of 1 to 4 and R^{19'} is a phenyl group optionally having substituents, a hydroxyl group, a C₁₋₆ alkoxy group or -N(R²⁰)(R²¹)- wherein R²⁰ and R²¹ are as defined in the above-mentioned 1, -O-(CH₂)ᵣ-R²³ wherein r and R²³ are as defined in the above-mentioned 1, -O-CO-R²⁷ wherein R²⁷ is as defined in the above-mentioned 1, or -N(R^{28'})(R^{29'}) wherein R^{28'} and R^{29'} are the same or different and each is a hydrogen atom, a C₁₋₆ alkyl group, a phenyl group optionally having substituents, an acyl group, -(CH₂)_{p'}-COO-R^{30'} wherein p' is as defined in the above-mentioned 1 and R^{30'} is a hydrogen atom, a phenyl group optionally having substituents or a C₁₋₆ alkyl group wherein the C₁₋₆ alkyl group is optionally substituted by a hydroxyl group, a trifluoromethyl group, a phenyl group optionally having substituents, a morpholino group or a carboxyl group, -CON(R^{31''})(R^{32''}) wherein R^{31''} and R^{32''} are the same or different and each is a hydrogen atom, a C₁₋₆ alkyl group or a phenyl group optionally having substituents, -CO-R^{33'} wherein R^{33'} is a C₁₋₆ alkyl group or a phenyl group optionally having substituents or -CO-(CH₂)_{r'}-R³⁴ wherein r' and R³⁴ are as defined in the above-mentioned 1,
   R^{16b'}-R^{16n'} and R^{17b'}-R^{17n'} are the same or different'and each is a hydrogen atom, a halogen atom, a C₁₋₆ alkyl group, an amino group, a hydroxyl group, a C₁₋₆ alkoxy group or -CON(R^{31'''})(R^{32'''}) wherein R^{31'''} and R^{32'''} are as defined for R^{31''} and R^{32''}, and
   R¹⁸, Y and n are as defined in the above-mentioned 1, or R⁶ and R⁷ may form, together with the adjacent nitrogen atom, 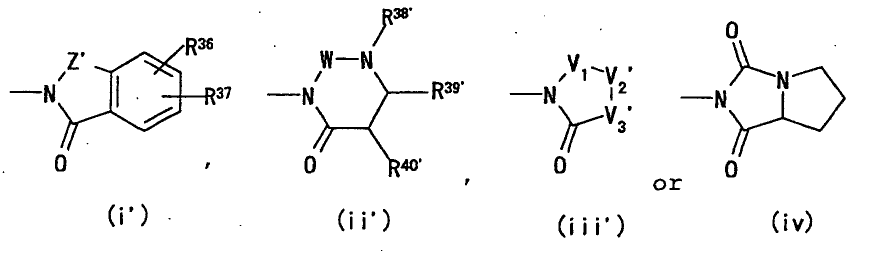 wherein R³⁶ and R³⁷ are as defined in the above-mentioned 1;
   Z' is -CH₂-CH₂-, -C(R⁴²)=CH-, -C(R^{42'})=N-, -N=N-, -CO-, -CO-O-, -CO-CH₂-O-, -CH₂-CO-NH-, -C(R^{42''})(R⁴³)-N(R⁴⁴)- wherein R⁴², R^{42'}, R^{42''} and R⁴³ are the same or different and each is a hydrogen atom, a C₁₋₆ alkyl group or a phenyl group optionally having substituents and R⁴⁴ are as defined in the above-mentioned 1 or -C(U)-N(R^{44'})- wherein U and R^{44'} are as defined in the above-mentioned 1;
   R^{38'} is a hydrogen atom, a phenyl group optionally substituted by a halogen atom or a C₁₋₆ alkyl group, or a pyridyl group;
   R^{39'} and R^{40'} are both hydrogen atoms, or R^{39'} and R^{40'} may form, together with the adjacent carbon atom, 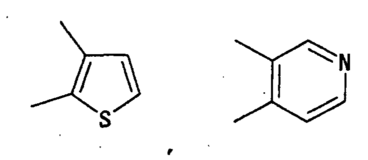 or 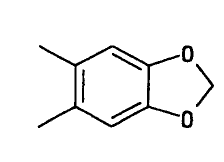
   W and V₁ are as defined in the above-mentioned 1;
   V₂' is -O-, -CH₂- or -N(R⁴⁵)- wherein R⁴⁵ is a hydrogen atom, a C₁₋₆ alkyl group, a phenyl group optionally substituted by a halogen atom; and
   V₃' is -CH(R⁴⁶)- or -N(R^{46'})- wherein R⁴⁶ and R^{46'} are each a hydrogen atom, a benzyl group, a thienyl group, or a phenyl group optionally substituted by a halogen atom, a hydroxy group or a C₁₋₆ alkoxy group,
      a pharmaceutically acceptable salt thereof or a prodrug thereof.
3. The indole compound of 1 above,
   wherein
   R⁶ is a hydrogen atom, a C₁₋₆ alkyl group or an aralkyl group
      wherein the aralkyl group is optionally substituted by a halogen atom;
   R⁷ is 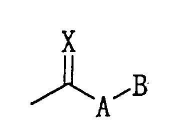 wherein X is as defined in the above-mentioned 1;
   A is -N(R^{8''})- wherein R^{8''} is a hydrogen atom, a C₁₋₆ alkyl group or an aryl group optionally having substituents, -C(R^{9''})(R^{10''})- wherein R^{9''} and R^{10''} are the same or different and each is a hydrogen atom, a hydroxyl group, an amino group, a C₁₋₆ alkyl group, a C₁₋₆ hydroxyalkyl group or a C₂₋₇ alkoxycarbonylamino group, or R^{9''} and R^{10''} may form, together with the adjacent carbon atom, a C₃₋₇ cycloalkyl group, -(CH₂)ₘ-NH- wherein m is as defined in the above-mentioned 1, -CO- or a single bond; and
   B is 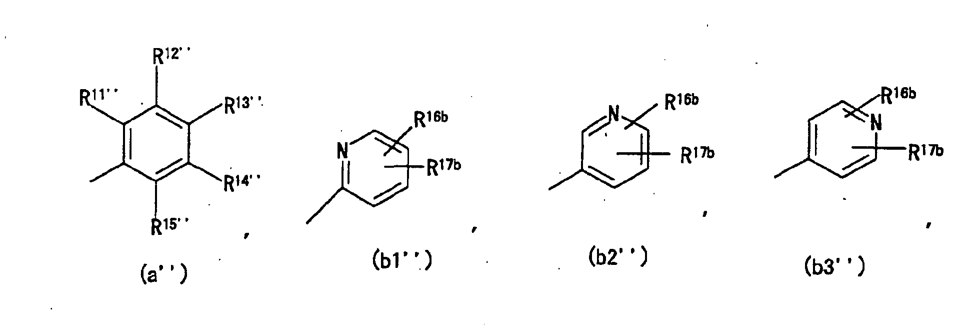 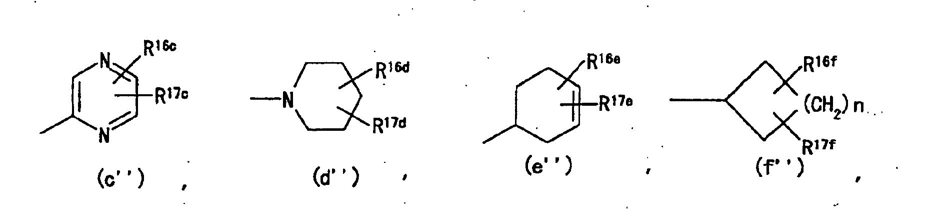 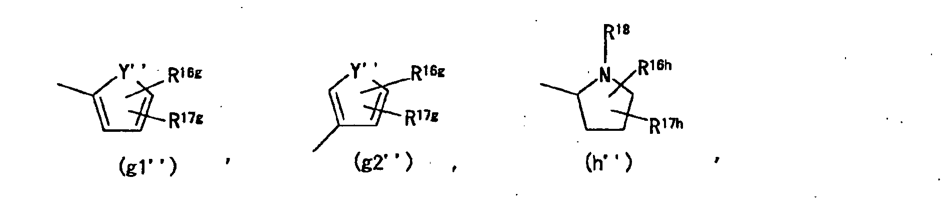 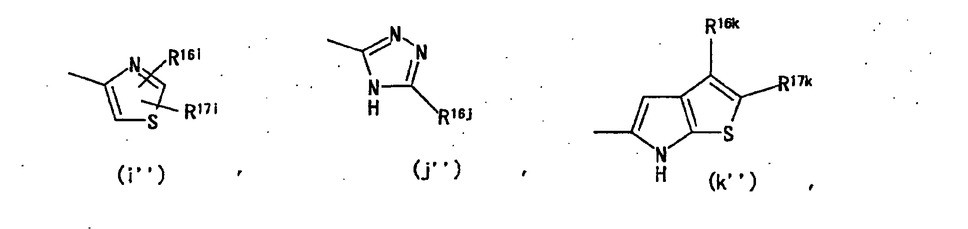 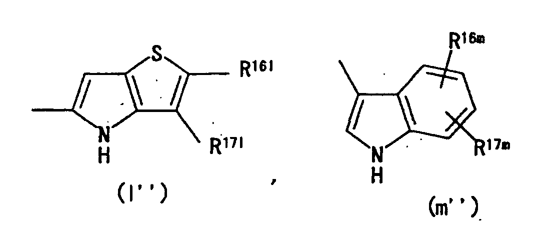 or 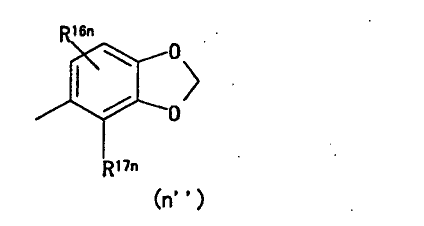 wherein R^{11''}, R^{12''}, R^{13''}, R^{14''} and R^{15''} are the same or different and each is a hydrogen atom, a halogen atom, a C₁₋₆ alkyl group, a C₁₋₆ alkoxy group, a nitro group, a hydroxyl group, a cyano group, a haloalkyl group; an aralkyl group, an aryl group optionally having substituents, an aryloxy group, a tetrazolyl group, a triazolyl group, -(CH₂)ₚ-CO-R¹⁹ wherein p and R¹⁹ are as defined in the above-mentioned 1, -O-(CH₂)ᵣ-R²³ wherein r and R²³ are as defined in the above-mentioned 1, -O-CO-R²⁷ wherein R²⁷ is as defined in the above-mentioned 1 or -N(R^{28''})(R^{29''}) wherein R^{28''} and R^{29''} are the same or different and each is a hydrogen atom, a C₁₋₆ alkyl group, an aryl group optionally having substituents, -(CH₂)_{p'}-COO-R^{30''} wherein p' is as defined for p and R^{30''} is a hydrogen atom or a C₁₋₆ alkyl group wherein the C₁₋₆ alkyl group is optionally substituted by a hydroxyl group, a trifluoromethyl group or a carboxyl group, -CON(R³¹)(R³²) wherein R³¹ and R³² are as defined in the above-mentioned 1, -CO-R³³ wherein R³³ is as defined in the above-mentioned 1 or -CO-(CH₂)_{r'}-R³⁴ wherein r' and R³⁴ are as defined in the above-mentioned 1,
   R^{16b}-R¹⁶ⁿ and R^{17b}-R¹⁷ⁿ are as defined in the above-mentioned 1,
   R¹⁸ is as defined in the above-mentioned 1,
   Y'' is -S- or -N(R³⁵)- wherein R³⁵ is as defined in the above-mentioned 1, and
   n is as defined in the above-mentioned 1, or
   R⁶ and R⁷ may form, together with the adjacent nitrogen atom, 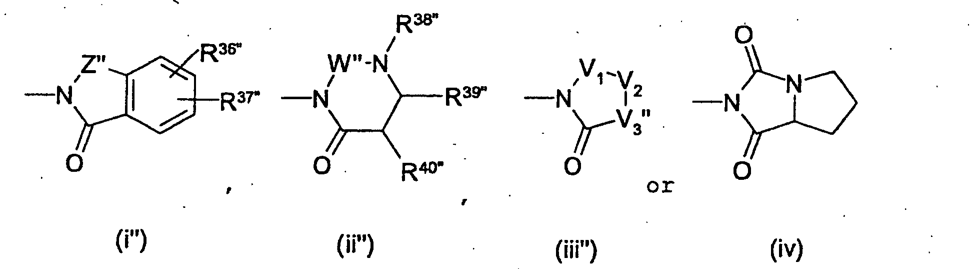 wherein R^{36''} and R^{37''} are the same or different and each is a hydrogen atom, a halogen atom, a C₁₋₆ alkyl group, a C₁₋₆ alkoxy group, an amino group, a hydroxyl group or -O-CO-R⁴¹ wherein R⁴¹ are as defined in the above-mentioned 1;
   Z'' is, -CH₂-CH₂-, -C(R⁴²)=CH- , -N=N- , -CO- , -CO-O-, -CO-CH₂-O-, -CH₂-CO-NH-, -C(R^{42''})(R⁴³)-N(R^{44''})- wherein R⁴², R^{42''} and R⁴³ are as defined in the above-mentioned 1 and R^{44''} is a hydrogen atom, a C₁₋₆ alkyl group or a C₂₋₇ alkoxycarbonyl group or -C(U)-N(R^{44'''})- wherein U is =O or =S and R^{44'''} is as defined for R^{44''};
   R^{38''} is a hydrogen atom or an aryl group optionally having substituents;
   R^{39''} and R^{40''} are the same or different and each is a hydrogen atom, a C₁₋₆ alkyl group or a C₂₋₇ alkoxycarbonyl group, or R^{39''} and R^{40''} may form, together with the adjacent carbon atom, 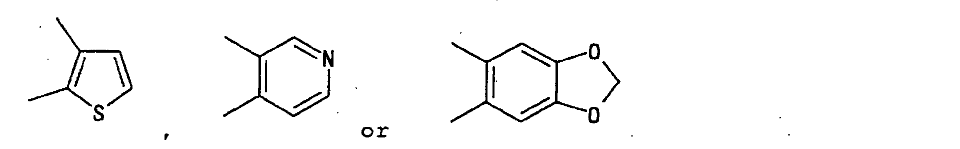
   W'' is -CO- or -CH₂-;
   V₁ and V₂ are as defined in the above-mentioned 1; and
   V₃'' is -CH(R^{46''})- or -N(R^{46'''})- wherein R^{46''} and R^{46'''} are the same or different and each is a hydrogen atom or an aryl group optionally having substituents,
      a pharmaceutically acceptable salt thereof or a prodrug thereof.
4. The indole compound of 1 above, wherein R¹, R², R⁴, R⁵ and R⁶ are each a hydrogen atom, a pharmaceutically acceptable salt thereof or a prodrug thereof.
5. The indole compound of 4 above, wherein R³ is a halogen atom or a C₁₋₆ alkyl group, a pharmaceutically acceptable salt thereof or a prodrug thereof.
6. The indole compound of 4 above, wherein X=O, A is a single bond and B is 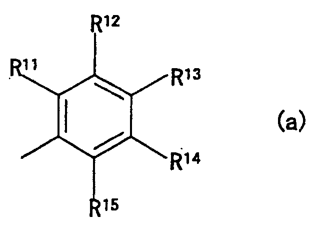 a pharmaceutically acceptable salt thereof or a prodrug thereof.
7. The indole compound of 4 above, wherein X=NH, A is a single bond and B is 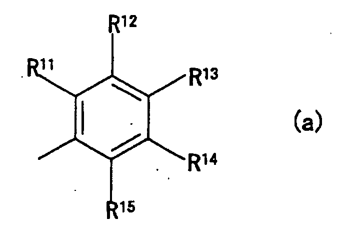 a pharmaceutically acceptable salt thereof or a prodrug thereof.
8. The indole compound of 1 above, wherein R⁶ and R⁷ may form, together with the adjacent nitrogen atom, 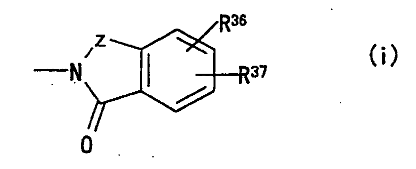 a pharmaceutically acceptable salt thereof or a prodrug thereof.
9. The indole compound of 1 above, which is selected from the group consisting of
   benzoic acid 2-(5-chloro-1H-indole-2-carbonyl)hydrazide,
   2-aminobenzoic acid 2-(5-chloro-1H-indole-2-carbonyl)hydrazide,
   2-hydroxybenzoic acid 2-(5-chloro-1H-indole-2-carbonyl)hydrazide,
   3-(2-(2-(5-chloro-1H-indole-2-carbonyl)hydrazinocarbonyl)phenylcarbamoyloxy)-2,2-dimethylpropionic acid,
   benzoic acid 2-(5-chloro-1H-indole-2-carbonyl)-1-methylhydrazide,
   benzoic acid 2-(1-acetyl-5-chloro-1H-indole-2-carbonyl)hydrazide,
   5-chloro-1H-indole-2-carboxylic acid 2-(imino-phenyl-methyl)hydrazide,
   5-aminothiazole-4-carboxylic acid 2-(5-chloro-1H-indole-2-carbonyl)hydrazide,
   benzoic acid 2-(5-fluoro-1H-indole-2-carbonyl)hydrazide,
   cyclohexanecarboxylic acid 2-(5-fluoro-1H-indole-2-carbonyl)hydrazide,
   thiophene-2-carboxylic acid 2-(5-fluoro-1H-indole-2-carbonyl)hydrazide,
   4-nitrobenzoic acid 2-(5-chloro-1H-indole-2-carbonyl)hydrazide,
   2-methylbenzoic acid 2-(5-chloro-1H-indole-2-carbonyl)hydrazide,
   4-methylbenzoic acid 2-(5-chloro-1H-indole-2-carbonyl)hydrazide,
   2-methoxybenzoic acid 2-(5-chloro-1H-indole-2-carbonyl)hydrazide,
   3-methoxybenzoic acid 2-(5-chloro-1H-indole-2-carbonyl)hydrazide,
   4-methoxybenzoic acid 2-(5-chloro-1H-indole-2-carbonyl)hydrazide,
   3-methylbenzoic acid 2-(5-chloro-1H-indole-2-carbonyl)hydrazide,
   2-chlorobenzoic acid 2-(5-chloro-1H-indole-2-carbonyl)hydrazide,
   3-chlorobenzoic acid 2-(5-chloro-1H-indole-2-carbonyl)hydrazide,
   4-chlorobenzoic acid 2-(5-chloro-1H-indole-2-carbonyl)hydrazide,
   4-(2-(5-chloro-1H-indole-2-carbonyl)hydrazinocarbonyl)benzoic acid methyl ester,
   cyclohexanecarboxylic acid 2-(5-chloro-1H-indole-2-carbonyl)hydrazide,
   2,4-dichlorobenzoic acid 2-(5-chloro-1H-indole-2-carbonyl)hydrazide,
   2,6-dichlorobenzoic acid 2-(5-chloro-1H-indole-2-carbonyl)hydrazide,
   2,4-difluorobenzoic acid 2-(5-chloro-1H-indole-2-carbonyl)hydrazide,
   biphenyl-2-carboxylic acid 2-(5-chloro-1H-indole-2-carbonyl)hydrazide,
   3-fluorobenzoic acid 2-(5-chloro-1H-indole-2-carbonyl)hydrazide,
   4-fluorobenzoic acid 2-(5-chloro-1H-indole-2-carbonyl)hydrazide,
   3-trifluoromethylbenzoic acid 2-(5-chloro-1H-indole-2-carbonyl)hydrazide,
   4-trifluoromethylbenzoic acid 2-(5-chloro-1H-indole-2-carbonyl)hydrazide,
   2-trifluoromethylbenzoic acid 2-(5-chloro-1H-indole-2-carbonyl)hydrazide,
   benzoic acid 2-(5-methyl-lH-indole-2-carbonyl)hydrazide,
   benzoic acid 2-(5-chloro-3-methyl-1H-indole-2-carbonyl)hydrazide,
   benzoic acid 2-(5,7-dichloro-1H-indole-2-carbonyl)hydrazide,
   2-aminobenzoic acid 2-(5-isopropyl-1H-indole-2-carbonyl)hydrazide,
   2-amino-4-fluorobenzoic acid 2-(5-isopropyl-1H-indole-2-carbonyl)hydrazide,
   2-aminobenzoic acid 2-(5-fluoro-1H-indole-2-carbonyl)hydrazide,
   2-aminobenzoic acid 2-(6-chloro-1H-indole-2-carbonyl)hydrazide,
   3-amino-4-(2-(5-chloro-1H-indole-2-carbonyl)hydrazinocarbonyl)benzoic acid methyl ester,
   3-aminoisonicotinic acid 2-(5-chloro-1H-indole-2-carbonyl)hydrazide,
   isonicotinic acid 2-(5-chloro-1H-indole-2-carbonyl)hydrazide,
   nicotinic acid 2-(5-chloro-1H-indole-2-carbonyl)hydrazide,
   pyridine-2-carboxylic acid 2-(5-chloro-1H-indole-2-carbonyl)hydrazide,
   3-(dimethylamino)benzoic acid 2-(5-chloro-1H-indole-2-carbonyl)hydrazide,
   N-(3-(2-(5-chloro-1H-indole-2-carbonyl)hydrazinocarbonyl)phenyl)acetamide,
   N-(2-(2-(5-chloro-1H-indole-2-carbonyl)hydrazinocarbonyl)phenyl)acetamide,
   4-fluorobenzoic acid 2-(5-chloro-1H-indole-2-carbonyl)-2-methylhydrazide,
   2-(2-(2-(5-chloro-1H-indole-2-carbonyl)hydrazinocarbonyl)phenoxy)acetic acid methyl ester,
   2-(2-(2-(5-chloro-1H-indole-2-carbonyl)hydrazinocarbonyl)phenoxy)acetic acid,
   2-(dimethylamino)benzoic acid 2-(5-chloro-1H-indole-2-carbonyl)hydrazide,
   2-(2-(5-chloro-1H-indole-2-carbonyl)hydrazinocarbonyl)phenoxy-N,N-dimethylacetamide,
   2-methylaminobenzoic acid 2-(5-chloro-1H-indole-2-carbonyl)hydrazide,
   2-amino-4-chlorobenzoic acid 2-(5-chloro-1H-indole-2-carbonyl)hydrazide,
   2-amino-6-chlorobenzoic acid 2-(5-chloro-1H-indole-2-carbonyl)hydrazide,
   2-amino-3-chlorobenzoic acid 2-(5-chloro-1H-indole-2-carbonyl)hydrazide,
   2-amino-5-chlorobenzoic acid 2-(5-chloro-1H-indole-2-carbonyl)hydrazide,
   4-cyanobenzoic acid 2-(5-chloro-1H-indole-2-carbonyl)hydrazide,
   4-(1H-tetrazol-5-yl)benzoic acid 2-(5-chloro-1H-indole-2-carbonyl)hydrazide,
   3-(1H-tetrazol-5-yl)benzoic acid 2-(5-chloro-1H-indole-2-carbonyl)hydrazide,
   2-(2-(2-(5-chloro-1H-indole-2-carbonyl)hydrazinocarbonyl)anilino)acetic acid,
   2-aminobenzoic acid 2-(5-methyl-1H-indole-2-carbonyl)hydrazide,
   2-amino-4-fluorobenzoic acid 2-(5-methyl-1H-indole-2-carbonyl)hydrazide,
   methyl (2-(2-(5-methyl-1H-indole-2-carbonyl)hydrazinocarbonyl)phenyl)carbamate,
   2-(2-(5-chloro-1H-indole-2-carbonyl)hydrazinocarbonyl)phenyl dimethylcarbamate,
   2-aminobenzoic acid 2-(5-ethyl-1H-indole-2-carbonyl)hydrazide,
   2-amino-4,5-difluorobenzoic acid 2-(5-methyl-1H-indole-2-carbonyl)hydrazide,
   2-(2-hydroxyethoxy)benzoic acid 2-(5-chloro-1H-indole-2-carbonyl)hydrazide,
   2-(3-(2-(5-chloro-1H-indole-2-carbonyl)hydrazinocarbonyl)phenoxy)acetic acid methyl ester,
   2-(3-(2-(5-chloro-1H-indole-2-carbonyl)hydrazinocarbonyl)phenoxy)acetic acid,
   2-(3-(2-(5-chloro-1H-indole-2-carbonyl)hydrazinocarbonyl)phenoxy)-N,N-dimethylacetamide,
   2-methylthiazole-4-carboxylic acid 2-(5-chloro-1H-indole-2-carbonyl)hydrazide,
   4-(2H-[1,2,4]triazol-3-yl)benzoic acid 2-(5-chloro-1H-indole-2-carbonyl) hydrazide,
   3-(2H-[1,2,4]triazol-3-yl)benzoic acid 2-(5-chloro-1H-indole-2-carbonyl)hydrazide,
   1,3-dihydroxy-2-propyl (2-(2-(5-chloro-1H-indole-2-carbonyl)hydrazinocarbonyl)phenyl)carbamate,
   3-((2-(2-(5-chloro-1H-indole-2-carbonyl)hydrazinocarbonyl)phenyl)carbamoyloxy)-2,2-dimethylpropionic acid,
   thiophene-2-carboxylic acid 2-(5-chloro-1H-indole-2-carbonyl)hydrazide,
   furan-2-carboxylic acid 2-(5-chloro-1H-indole-2-carbonyl)hydrazide,
   2,6-dichloronicotinic acid 2-(5-chloro-1H-indole-2-carbonyl)hydrazide,
   1H-pyrrole-2-carboxylic acid 2-(5-chloro-1H-indole-2-carbonyl)hydrazide,
   1H-imidazole-4-carboxylic acid 2-(5-chloro-1H-indole-2-carbonyl)hydrazide,
   pyrazine-2-carboxylic acid 2-(5-chloro-1H-indole-2-carbonyl)hydrazide,
   thiophene-3-carboxylic acid 2-(5-chloro-1H-indole-2-carbonyl)hydrazide,
   furan-3-carboxylic acid 2-(5-chloro-1H-indole-2-carbonyl)hydrazide,
   5-chlorothiophene-2-carboxylic acid 2-(5-chloro-1H-indole-2-carbonyl)hydrazide,
   3-chlorothiophene-2-carboxylic acid 2-(5-chloro-1H-indole-2-carbonyl)hydrazide,
   1-methyl-1H-pyrrole-2-carboxylic acid 2-(5-chloro-1H-indole-2-carbonyl)hydrazide,
   5-methylthiophene-2-carboxylic acid 2-(5-chloro-1H-indole-2-carbonyl)hydrazide,
   3-methylthiophene-2-carboxylic acid 2-(5-chloro-1H-indole-2-carbonyl)hydrazide,
   2,6-difluorobenzoic acid 2-(5-chloro-1H-indole-2-carbonyl)hydrazide,
   2,3-difluorobenzoic acid 2-(5-chloro-1H-indole-2-carbonyl)hydrazide,
   5-chloro-1H-indole-2-carboxylic acid 2-(naphthalene-1-carbonyl)hydrazide,
   3,4,5-trifluorobenzoic acid 2-(5-chloro-1H-indole-2-carbonyl)hydrazide,
   2,3,4,5-tetrafluorobenzoic acid 2-(5-chloro-1H-indole-2-carbonyl)hydrazide,
   2-amino-4-methylbenzoic acid 2-(5-chloro-1H-indole-2-carbonyl)hydrazide,
   2-amino-4-fluorobenzoic acid 2-(5-chloro-1H-indole-2-carbonyl)hydrazide,
   2-amino-5-methylbenzoic acid 2-(5-chloro-1H-indole-2-carbonyl)hydrazide,
   2-amino-6-methylbenzoic acid 2-(5-chloro-1H-indole-2-carbonyl)hydrazide,
   2-amino-3-methylbenzoic acid 2-(5-chloro-1H-indole-2-carbonyl)hydrazide,
   2-amino-4,5-difluorobenzoic acid 2-(5-chloro-1H-indole-2-carbonyl)hydrazide,
   3-aminothiophene-2-carboxylic acid 2-(5-chloro-1H-indole-2-carbonyl)hydrazide,
   2-aminobenzoic acid 2-(5-bromo-1H-indole-2-carbonyl)hydrazide,
   2-amino-4-fluorobenzoic acid 2-(5-bromo-1H-indole-2-carbonyl)hydrazide,
   1H-pyrazole-4-carboxylic acid 2-(5-chloro-1H-indole-2-carbonyl)hydrazide,
   methyl (2-(2-(5-fluoro-1H-indole-2-carbonyl)hydrazinocarbonyl)phenyl)carbamate,
   1-methyl-1H-pyrrole-2-carboxylic acid 2-(5-methyl-1H-indole-2-carbonyl)hydrazide,
   thiophene-3-carboxylic acid 2-(5-methyl-1H-indole-2-carbonyl)hydrazide,
   4H-thieno[3,2-b]pyrrole-5-carboxylic acid 2-(5-chloro-1H-indole-2-carbonyl)hydrazide,
   phenyl (2-(2-(5-chloro-1H-indole-2-carbonyl)hydrazinocarbonyl)phenyl)carbamate,
   benzyl (2-(2-(5-chloro-1H-indole-2-carbonyl)hydrazinocarbonyl)phenyl)carbamate,
   2-hydroxyethyl (2-(2-(5-chloro-1H-indole-2-carbonyl)hydrazinocarbonyl)phenyl)carbamate,
   3-hydroxypropyl (2-(2-(5-chloro-1H-indole-2-carbonyl)hydrazinocarbonyl)phenyl)carbamate,
   2-((2-(2-(5-chloro-1H-indole-2-carbonyl)hydrazinocarbonyl)phenyl)carbamoyloxy)acetic acid,
   2-((2-(2-(5-chloro-1H-indole-2-carbonyl)hydrazinocarbonyl)phenyl)carbamoyloxymethyl)-2-methylmalonic acid,
   methyl 2-(2-(5-chloro-1H-indole-2-carbonyl)hydrazinocarbonyl)phenylcarbamate,
   cyclohexanecarboxylic acid 2-(5-chloro-1H-indole-2-carbonyl)-1-methylhydrazide,
   thiophene-2-carboxylic acid 2-(5-chloro-1H-indole-2-carbonyl)-1-methylhydrazide,
   benzoic acid 2-(1H-indole-2-carbonyl)hydrazide,
   benzoic acid 2-(5-chloro-1-methyl-1H-indole-2-carbonyl)hydrazide,
   benzoic acid 2-(5-methoxy-1H-indole-2-carbonyl)hydrazide,
   benzoic acid 2-(5-isopropyl-1H-indole-2-carbonyl)hydrazide,
   benzoic acid 2-(5-nitro-1H-indole-2-carbonyl)hydrazide,
   benzoic acid 2-(5-benzyloxy-1H-indole-2-carbonyl)hydrazide,
   benzoic acid 2-(6-chloro-1H-indole-2-carbonyl)hydrazide,
   6H-thieno[2,3-b]pyrrole-5-carboxylic acid 2-(5-chloro-1H-indole-2-carbonyl)hydrazide,
   5-chloro-1H-indole-2-carboxylic acid 2-((2-fluorophenyl)-imino-methyl)hydrazide,
   5-chloro-1H-indole-2-carboxylic acid 2-((3-fluorophenyl)-imino-methyl)hydrazide,
   5-chloro-1H-indole-2-carboxylic acid 2-((4-fluorophenyl)-imino-methyl)hydrazide,
   5-chloro-1H-indole-2-carboxylic acid 2-(imino-(p-tolyl)-methyl) hydrazide,
   5-chloro-1H-indole-2-carboxylic acid 2-((4-chlorophenyl)-imino-methyl)hydrazide,
   5-chloro-1H-indole-2-carboxylic acid 2-((3-chlorophenyl)-imino-methyl)hydrazide,
   5-chloro-1H-indole-2-carboxylic acid 2-((3-fluorophenyl)-imino-methyl)hydrazide,
   5-chloro-1H-indole-2-carboxylic acid 2-(imino-(o-tolyl)-methyl)hydrazide,
   5-chloro-1H-indole-2-carboxylic acid 2-(imino-(m-tolyl)-methyl)hydrazide,
   5-chloro-1H-indole-2-carboxylic acid 2-(imino-(thiophen-2-yl)-methyl)hydrazide,
   5-chloro-1H-indole-2-carboxylic acid 2-(imino-(pyridin-2-yl)-methyl)hydrazide,
   5-chloro-1H-indole-2-carboxylic acid 2-((furan-2-yl)-imino-methyl)hydrazide,
   5-chloro-1H-indole-2-carboxylic acid 2-((2-chloro-6-fluorophenyl)-imino-methyl)hydrazide,
   5-chloro-1H-indole-2-carboxylic acid 2-(imino-(2-trifluoromethylphenyl)-methyl)hydrazide,
   5-chloro-1H-indole-2-carboxylic acid 2-(imino-(pyrazin-2-yl)-methyl)hydrazide,
   3-aminobenzoic acid 2-(5-chloro-1H-indole-2-carbonyl)hydrazide, 3-methoxybenzoic acid 2-(5-chloro-1H-indole-2-carbonyl)hydrazide,
   5-amino-2-methylthiazole-4-carboxylic acid 2-(5-chloro-1H-indole-2-carbonyl)hydrazide,
   5-chloro-1H-indole-2-carboxylic acid (1,2,3,4-tetrahydro-2,4-dioxoquinazolin-3-yl)amide,
   5-chloro-1H-indole-2-carboxylic acid (2,3-dihydro-2,4-dioxo-4H-benzo[e][1,3]oxazin-3-yl)amide,
   5-chloro-1H-indole-2-carboxylic acid (1,2,3,4-tetrahydro-4-oxo-2-thioxoquinazolin-3-yl)amide,
   5-chloro-1H-indole-2-carboxylic acid (3,4-dihydro-2-methyl-4-oxoquinazolin-3-yl)amide,
   5-chloro-1H-indole-2-carboxylic acid (3,4-dihydro-4-oxoquinazolin-3-yl)amide,
   5-chloro-1H-indole-2-carboxylic acid (1,2,3,4-tetrahydro-4-oxoquinazolin-3-yl)amide,
   5-chloro-1H-indole-2-carboxylic acid (1,2,3,4-tetrahydro-2,5-dioxo-5H-benzo[e][1,4]diazepin-4-yl)amide,
   5-chloro-1H-indole-2-carboxylic acid (2,3,4,5-tetrahydro-3,5-dioxo-benzo[f][1,4]oxazepin-4-yl)amide,
   5-isopropyl-1H-indole-2-carboxylic acid (1,2,3,4-tetrahydro-2,4-dioxoquinazolin-3-yl)amide,
   5-isopropyl-1H-indole-2-carboxylic acid (7-fluoro-1,2,3,4-tetrahydro-2,4-dioxoquinazolin-3-yl)amide,
   5-fluoro-1H-indole-2-carboxylic acid (1,2,3,4-tetrahydro-2,4-dioxoquinazolin-3-yl)amide,
   6-chloro-1H-indole-2-carboxylic acid (1,2,3,4-tetrahydro-2,4-dioxoquinazolin-3-yl)amide,
   3-((5-chloro-1H-indole-2-carbonyl)amino)-1,2,3,4-tetrahydro-2,4-dioxoquinazoline-7-carboxylic acid methyl ester,
   3-((5-chloro-1H-indole-2-carbonyl)amino)-1,2,3,4-tetrahydro-2,4-dioxoquinazoline-7-carboxylic acid,
   5-chloro-1H-indole-2-carboxylic acid (1,2,3,4-tetrahydro-2,4-dioxo-6-(trifluoroacetylamino)quinazolin-3-yl)amide,
   5-chloro-1H-indole-2-carboxylic acid (6-amino-1,2,3,4-tetrahydro-2,4-dioxoquinazolin-3-yl)amide,
   5-chloro-1H-indole-2-carboxylic acid (5-chloro-1,2,3,4-tetrahydro-2,4-dioxoquinazolin-3-yl)amide,
   5-chloro-1H-indole-2-carboxylic acid (6-chloro-1,2,3,4-tetrahydro-2,4-dioxoquinazolin-3-yl)amide,
   5-chloro-1H-indole-2-carboxylic acid (7-chloro-1,2,3,4-tetrahydro-2,4-dioxoquinazolin-3-yl)amide,
   5-chloro-1H-indole-2-carboxylic acid (8-chloro-1,2,3,4-tetrahydro-2,4-dioxoquinazolin-3-yl)amide,
   2-(3-((5-chloro-1H-indole-2-carbonyl)amino)-1,2,3,4-tetrahydro-2,4-dioxoquinazolin-1-yl)acetic acid,
   2-(3-((5-chloro-1H-indole-2-carbonyl)amino)-1,2,3,4-tetrahydro-2,4-dioxoquinazolin-1-yl)acetic acid methyl ester,
   5-methyl-1H-indole-2-carboxylic acid (1,2,3,4-tetrahydro-2,4-dioxoquinazolin-3-yl)amide,
   5-methyl-1H-indole-2-carboxylic acid (7-fluoro-1,2,3,4-tetrahydro-2,4-dioxoquinazolin-3-yl)amide,
   5-ethyl-1H-indole-2-carboxylic acid (1,2,3,4-tetrahydro-2,4-dioxoquinazolin-3-yl)amide,
   5-methyl-1H-indole-2-carboxylic acid (6,7-difluoro-1,2,3,4-tetrahydro-2,4-dioxoquinazolin-3-yl)amide,
   5-methyl-1H-indole-2-carboxylic acid (1,2,3,4-tetrahydro-6-methoxy-2,4-dioxoquinazolin-3-yl)amide,
   5-methyl-1H-indole-2-carboxylic acid (1,2,3,4-tetrahydro-6-hydroxy-2,4-dioxoquinazolin-3-yl)amide,
   acetic acid 3-((5-methyl-1H-indole-2-carbonyl)amino)-1,2,3,4-tetrahydro-2,4-dioxoquinazolin-6-yl ester,
   5-chloro-1H-indole-2-carboxylic acid (1,2,3,4-tetrahydro-2,4-dioxo-1-propylquinazolin-3-yl)amide,
   5-chloro-1H-indole-2-carboxylic acid (1,2,3,4-tetrahydro-1-methyl-2,4-dioxoquinazolin-3-yl)amide,
   N-(1,2,3,4-tetrahydro-7-nitro-2,4-dioxoquinazolin-3-yl)-5-chloro-1H-indole-2-carboxylic acid amide,
   5-chloro-1H-indole-2-carboxylic acid (2,4-dioxoperhydropyrimidin-3-yl)amide,
   5-chloro-1H-indole-2-carboxylic acid (4-oxo-2-thioxoperhydropyrimidin-3-yl)amide,
   5-chloro-1H-indole-2-carboxylic acid (2,4-dioxo-1-phenylperhydropyrimidin-3-yl)amide,
   5-chloro-1H-indole-2-carboxylic acid (4-oxo-1-phenylperhydropyrimidin-3-yl)amide,
   5-chloro-1H-indole-2-carboxylic acid (1-(4-fluorophenyl)-2,4-dioxoperhydropyrimidin-3-yl)amide,
   5-chloro-1H-indole-2-carboxylic acid (2,4-dioxo-1-(pyridin-2-yl)perhydropyrimidin-3-yl)amide,
   5-chloro-1H-indole-2-carboxylic acid (1-(3-fluorophenyl)-2,4-dioxoperhydropyrimidin-3-yl)amide,
   5-chloro-1H-indole-2-carboxylic acid (1-(2-fluorophenyl)-2,4-dioxoperhydropyrimidin-3-yl)amide,
   5-fluoro-1H-indole-2-carboxylic acid (2,4-dioxo-1-phenyl-perhydropyrimidin-3-yl)amide,
   5-methyl-1H-indole-2-carboxylic acid (2,4-dioxo-1-phenyl-perhydropyrimidin-3-yl)amide,
   5-chloro-1H-indole-2-carboxylic acid (1-(3-chlorophenyl)-2,4-dioxoperhydropyrimidin-3-yl)amide,
   5-chloro-1H-indole-2-carboxylic acid (2,4-dioxo-1-(m-tolyl)perhydropyrimidin-3-yl)amide,
   5-chloro-1H-indole-2-carboxylic acid (2,4-dioxo-1-(p-tolyl)perhydropyrimidin-3-yl)amide,
   5-chloro-1H-indole-2-carboxylic acid (1-(4-chlorophenyl)-2,4-dioxoperhydropyrimidin-3-yl)amide,
   5-chloro-1H-indole-2-carboxylic acid (2,4-dioxo-1-(o-tolyl)perhydropyrimidin-3-yl)amide,
   5-chloro-1H-indole-2-carboxylic acid ((4S)-2,5-dioxo-4-phenylimidazolidin-1-yl)amide,
   5-chloro-1H-indole-2-carboxylic acid (2,4-dioxo-1-phenylimidazolidin-3-yl)amide,
   5-chloro-1H-indole-2-carboxylic acid (4-oxo-1-phenyl-2-thioxoimidazolidin-3-yl)amide,
   5-chloro-1H-indole-2-carboxylic acid (4-oxo-1-phenylimidazolidin-3-yl)amide,
   5-chloro-1H-indole-2-carboxylic acid (2-oxo-1-phenylimidazolidin-3-yl)amide,
   5-chloro-1H-indole-2-carboxylic acid ((4R)-2,5-dioxo-4-phenylimidazolidin-1-yl)amide,
   5-chloro-1H-indole-2-carboxylic acid ((4S)-1,3-dioxo-perhydropyrrolo[1,2-c]imidazol-2-yl)amide,
   5-chloro-1H-indole-2-carboxylic acid ((4R)-1,3-dioxo-perhydropyrrolo[1,2-c]imidazol-2-yl)amide,
   5-chloro-1H-indole-2-carboxylic acid ((4S)-4-benzyl-2,5-dioxoimidazolidin-1-yl)amide,
   5-chloro-1H-indole-2-carboxylic acid ((4R)-4-benzyl-2,5-dioxoimidazolidin-1-yl)amide,
   5-chloro-1H-indole-2-carboxylic acid (2,4-dioxoimidazolidin-3-yl)amide,
   5-chloro-1H-indole-2-carboxylic acid (1-methyl-2,5-dioxo-4-phenylimidazolidin-1-yl)amide,
   5-chloro-1H-indole-2-carboxylic acid (2,4-dioxo-1-(4-fluorophenyl)imidazolidin-3-yl)amide,
   5-chloro-1H-indole-2-carboxylic acid (2,5-dioxo-4-(2-fluorophenyl)imidazolidin-1-yl)amide,
   5-chloro-1H-indole-2-carboxylic acid (2,5-dioxo-4-(2-thienyl)imidazolidin-1-yl)amide,
   5-chloro-1H-indole-2-carboxylic acid (2,5-dioxo-4-(4-fluorophenyl)imidazolidin-1-yl)amide,
   5-chloro-1H-indole-2-carboxylic acid (2,5-dioxo-4-(4-chlorophenyl)imidazolidin-1-yl)amide,
   5-chloro-1H-indole-2-carboxylic acid ((4S)-2,5-dioxo-4-(4-hydroxyphenyl)imidazolidin-1-yl)amide,
   5-chloro-1H-indole-2-carboxylic acid ((4S)-2,5-dioxo-4-(4-methoxyphenyl)imidazolidin-1-yl)amide,
   5-chloro-1H-indole-2-carboxylic acid ((4R)-2,5-dioxo-4-(4-methoxyphenyl)imidazolidin-1-yl)amide,
   5-chloro-1H-indole-2-carboxylic acid 2-(anilinocarbonyl)hydrazide,
   5-chloro-1H-indole-2-carboxylic acid 2-(phenylthiocarbonyl)hydrazide,
   5-chloro-1H-indole-2-carboxylic acid 2-(2-phenylacetyl)hydrazide,
   5-chloro-1H-indole-2-carboxylic acid 2-(2-oxo-2-phenylacetyl)hydrazide,
   5-chloro-1H-indole-2-carboxylic acid 2-((2-fluorophenyl)aminocarbonyl)hydrazide,
   5-chloro-1H-indole-2-carboxylic acid 2-((3-fluorophenyl)aminocarbonyl)hydrazide,
   5-chloro-1H-indole-2-carboxylic acid 2-((4-fluorophenyl)aminocarbonyl)hydrazide,
   5-chloro-1H-indole-2-carboxylic acid 2-(anilinocarbonyl)-2-methylhydrazide,
   5-chloro-1H-indole-2-carboxylic acid 2-((2-chloroanilino)carbonyl)hydrazide,
   5-chloro-1H-indole-2-carboxylic acid 2-((3-chloroanilino)carbonyl)hydrazide,
   5-chloro-1H-indole-2-carboxylic acid 2-((4-chloroanilino)carbonyl)hydrazide,
   5-chloro-1H-indole-2-carboxylic acid 2-((1-phenylcyclopropane)carbonyl)hydrazide,
   5-chloro-1H-indole-2-carboxylic acid 2-((1-phenylcyclopentane)carbonyl)hydrazide,
   5-chloro-1H-indole-2-carboxylic acid 2-((1-phenylcyclohexane)carbonyl)hydrazide,
   5-chloro-1H-indole-2-carboxylic acid 2-(2-phenylpropanoyl)hydrazide,
   5-chloro-1H-indole-2-carboxylic acid 2-(3-hydroxy-2-phenylpropanoyl)hydrazide,
   5-chloro-1H-indole-2-carboxylic acid 2-(2-methyl-2-phenylpropanoyl)hydrazide,
   5-chloro-1H-indole-2-carboxylic acid 2-((2S)-2-amino-2-phenylacetyl)hydrazide,
   N-(2-(2-(5-chloro-1H-indole-2-carbonyl)hydrazino)-2-oxo-1-phenylethyl)acetamide,
   2-morpholinoethyl (2-((2-(5-chloro-1H-indole-2-carbonyl)hydrazino)carbonyl)phenyl)carbamate p-toluenesulfonate,
   2-amino-4,5-difluorobenzoic acid 2-(5-methyl-1H-indole-2-carbonyl)hydrazide benzenesulfonate,
   2-amino-4-fluorobenzoic acid 2-(5-chloro-1H-indole-2-carbonyl)hydrazide benzenesulfonate,
   3-(dimethylamino)benzoic acid 2-(5-chloro-1H-indole-2-carbonyl)hydrazide methanesulfonate,
   2-amino-4-fluorobenzoic acid 2-(5-chloro-1H-indole-2-carbonyl)hydrazide hydrochloride,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-aminobenzoic acid 2-(5-methyl-1H-indole-2-carbonyl)hydrazide p-toluenesulfonate,
   2-aminobenzoic acid 2-(5-methyl-1H-indole-2-carbonyl)hydrazide benzenesulfonate,
   2-amino-4-fluorobenzoic acid 2-(5-methyl-1H-indole-2-carbonyl)hydrazide benzenesulfonate,
   3-aminobenzoic acid 2-(5-chloro-1H-indole-2-carbonyl)hydrazide p-toluenesulfonate,
   5-chloro-1H-indole-2-carboxylic acid 2-((2-chlorophenyl)-imino-methyl)hydrazide methanesulfonate,
   5-chloro-1H-indole-2-carboxylic acid 2-((2-chlorophenyl)-imino-methyl)hydrazide p-toluenesulfonate,
   5-chloro-1H-indole-2-carboxylic acid 2-((2-chlorophenyl)-imino-methyl)hydrazide hydrochloride,
   5-chloro-1H-indole-2-carboxylic acid 2-(imino-phenyl-methyl)hydrazide methanesulfonate,
   5-chloro-1H-indole-2-carboxylic acid 2-((2-chlorophenyl)-imino-methyl)hydrazide butenedioic acid salt,
   5-chloro-1H-indole-2-carboxylic acid 2-((2-fluorophenyl)-imino-methyl)hydrazide hydrochloride,
   5-chloro-1H-indole-2-carboxylic acid 2-((2-fluorophenyl)-imino-methyl)hydrazide methanesulfonate,
   5-chloro-1H-indole-2-carboxylic acid 2-((1-imino-2-phenylethyl)hydrazide methanesulfonate,
   5-chloro-1H-indole-2-carboxylic acid 2-((3-fluorophenyl)-imino-methyl)hydrazide hydrochloride,
   5-chloro-1H-indole-2-carboxylic acid 2-((3,4-difluorophenyl)-imino-methyl)hydrazide methanesulfonate,
   5-chloro-1H-indole-2-carboxylic acid 2-(imino-(2-methoxyphenyl)-methyl)hydrazide methanesulfonate,
   5-chloro-1H-indole-2-carboxylic acid 2-((2,6-difluorophenyl)-imino-methyl)hydrazide methanesulfonate,
   5-chloro-1H-indole-2-carboxylic acid 2-((2,4-difluorophenyl)-imino-methyl)hydrazide methanesulfonate,
   5-chloro-1H-indole-2-carboxylic acid 2-((1,2-dimethyl-1H-pyrrol-5-yl)-imino-methyl)hydrazide methanesulfonate,
   2-aminobenzoic acid 2-(5-methyl-1H-indole-2-carbonyl)hydrazide p-toluenesulfonate,
   2-aminobenzoic acid 2-(5-methyl-1H-indole-2-carbonyl)hydrazide benzenesulfonate,
   2-amino-4-fluorobenzoic acid 2-(5-methyl-1H-indole-2-carbonyl)hydrazide benzenesulfonate,
   2-(dimethylamino)benzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide methanesulfonate,
   2-aminobenzoic acid 2-(5-bromo-1H-indole-2-carbonyl)hydrazide methanesulfonate,
   2-amino-4-fluorobenzoic acid 2-(5-methyl-1H-indole-2-carbonyl)hydrazide p-toluenesulfonate, and
   2-amino-4-fluorobenzoic acid 2-(5-methyl-1H-indole-2-carbonyl)hydrazide methanesulfonate,
   a pharmaceutically acceptable salt thereof or a prodrug thereof.
10. The indole compound of 1 above, which is selected from the group consisting of
   benzoic acid 2-(5-chloro-1H-indole-2-carbonyl)hydrazide,
   2-aminobenzoic acid 2-(5-chloro-1H-indole-2-carbonyl)hydrazide,
   2-hydroxybenzoic acid 2-(5-chloro-1H-indole-2-carbonyl)hydrazide,
   3-(2-(2-(5-chloro-1H-indole-2-carbonyl)hydrazinocarbonyl)phenylcarbamoyloxy)-2,2-dimethylpropionic acid,
   benzoic acid 2-(5-chloro-1H-indole-2-carbonyl)-1-methylhydrazide,
   benzoic acid 2-(1-acetyl-5-chloro-1H-indole-2-carbonyl)hydrazide,
   5-chloro-1H-indole-2-carboxylic acid 2-(imino-phenyl-methyl)hydrazide,
   5-aminothiazole-4-carboxylic acid 2-(5-chloro-1H-indole-2-carbonyl)hydrazide,
   benzoic acid 2-(5-fluoro-1H-indole-2-carbonyl)hydrazide,
   cyclohexanecarboxylic acid 2-(5-fluoro-1H-indole-2-carbonyl)hydrazide,
   thiophene-2-carboxylic acid 2-(5-fluoro-1H-indole-2-carbonyl)hydrazide,
   4-nitrobenzoic acid 2-(5-chloro-1H-indole-2-carbonyl)hydrazide,
   2-methylbenzoic acid 2-(5-chloro-1H-indole-2-carbonyl)hydrazide,
   4-methylbenzoic acid 2-(5-chloro-1H-indole-2-carbonyl)hydrazide,
   2-methoxybenzoic acid 2-(5-chloro-1H-indole-2-carbonyl)hydrazide,
   3-methoxybenzoic acid 2-(5-chloro-1H-indole-2-carbonyl)hydrazide,
   4-methoxybenzoic acid 2-(5-chloro-1H-indole-2-carbonyl)hydrazide,
   3-methylbenzoic acid 2-(5-chloro-1H-indole-2-carbonyl)hydrazide,
   2-chlorobenzoic acid 2-(5-chloro-1H-indole-2-carbonyl)hydrazide,
   3-chlorobenzoic acid 2-(5-chloro-1H-indole-2-carbonyl)hydrazide,
   4-chlorobenzoic acid 2-(5-chloro-1H-indole-2-carbonyl)hydrazide,
   4-(2-(5-chloro-1H-indole-2-carbonyl)hydrazinocarbonyl)benzoic acid methyl ester,
   cyclohexanecarboxylic acid 2-(5-chloro-1H-indole-2-carbonyl)hydrazide,
   2,4-dichlorobenzoic acid 2-(5-chloro-1H-indole-2-carbonyl)hydrazide,
   2,6-dichlorobenzoic acid 2-(5-chloro-1H-indole-2-carbonyl)hydrazide,
   2,4-difluorobenzoic acid 2-(5-chloro-1H-indole-2-carbonyl)hydrazide,
   biphenyl-2-carboxylic acid 2-(5-chloro-1H-indole-2-carbonyl)hydrazide,
   3-fluorobenzoic acid 2-(5-chloro-1H-indole-2-carbonyl)hydrazide,
   4-fluorobenzoic acid 2-(5-chloro-1H-indole-2-carbonyl)hydrazide,
   3-trifluoromethylbenzoic acid 2-(5-chloro-1H-indole-2-carbonyl)hydrazide,
   4-trifluoromethylbenzoic acid 2-(5-chloro-1H-indole-2-carbonyl)hydrazide,
   2-trifluoromethylbenzoic acid 2-(5-chloro-1H-indole-2-carbonyl)hydrazide,
   benzoic acid 2-(5-methyl-1H-indole-2-carbonyl)hydrazide,
   benzoic acid 2-(5-chloro-3-methyl-1H-indole-2-carbonyl)hydrazide,
   benzoic acid 2-(5,7-dichloro-1H-indole-2-carbonyl)hydrazide,
   2-aminobenzoic acid 2-(5-isopropyl-1H-indole-2-carbonyl)hydrazide,
   2-amino-4-fluorobenzoic acid 2-(5-isopropyl-1H-indole-2-carbonyl)hydrazide,
   2-aminobenzoic acid 2-(5-fluoro-1H-indole-2-carbonyl)hydrazide,
   2-aminobenzoic acid 2-(6-chloro-1H-indole-2-carbonyl)hydrazide,
   3-amino-4-(2-(5-chloro-1H-indole-2-carbonyl)hydrazinocarbonyl)benzoic acid methyl ester,
   3-aminoisonicotinic acid 2-(5-chloro-1H-indole-2-carbonyl)hydrazide,
   isonicotinic acid 2-(5-chloro-lH-indole-2-carbonyl)hydrazide,
   nicotinic acid 2-(5-chloro-1H-indole-2-carbonyl)hydrazide,
   pyridine-2-carboxylic acid 2-(5-chloro-1H-indole-2-carbonyl)hydrazide,
   3-(dimethylamino)benzoic acid 2-(5-chloro-1H-indole-2-carbonyl)hydrazide,
   N-(3-(2-(5-chloro-1H-indole-2-carbonyl)hydrazinocarbonyl)phenyl)acetamide,
   N-(2-(2-(5-chloro-1H-indole-2-carbonyl)hydrazinocarbonyl)phenyl)acetamide,
   4-fluorobenzoic acid 2-(5-chloro-1H-indole-2-carbonyl)-2-methylhydrazide,
   2-(2-(2-(5-chloro-1H-indole-2-carbonyl)hydrazinocarbonyl)phenoxy)acetic acid methyl ester,
   2-(2-(2-(5-chloro-1H-indole-2-carbonyl)hydrazinocarbonyl)phenoxy)acetic acid,
   2-(dimethylamino)benzoic acid 2-(5-chloro-1H-indole-2-carbonyl)hydrazide,
   2-(2-(5-chloro-1H-indole-2-carbonyl)hydrazinocarbonyl)phenoxy-N,N-dimethylacetamide,
   2-methylaminobenzoic acid 2-(5-chloro-1H-indole-2-carbonyl)hydrazide,
   2-amino-4-chlorobenzoic acid 2-(5-chloro-1H-indole-2-carbonyl)hydrazide,
   2-amino-6-chlorobenzoic acid 2-(5-chloro-1H-indole-2-carbonyl)hydrazide,
   2-amino-3-chlorobenzoic acid 2-(5-chloro-1H-indole-2-carbonyl)hydrazide,
   2-amino-5-chlorobenzoic acid 2-(5-chloro-1H-indole-2-carbonyl)hydrazide,
   4-cyanobenzoic acid 2-(5-chloro-1H-indole-2-carbonyl)hydrazide,
   4-(1H-tetrazol-5-yl)benzoic acid 2-(5-chloro-1H-indole-2-carbonyl)hydrazide,
   3-(1H-tetrazol-5-yl)benzoic acid 2-(5-chloro-1H-indole-2-carbohyl)hydrazide,
   2-(2-(2-(5-chloro-1H-indole-2-carbonyl)hydrazinocarbonyl)anilino)acetic acid,
   2-aminobenzoic acid 2-(5-methyl-1H-indole-2-carbonyl)hydrazide,
   2-amino-4-fluorobenzoic acid 2-(5-methyl-1H-indole-2-carbonyl)hydrazide,
   methyl (2-(2-(5-methyl-1H-indole-2-carbonyl)hydrazinocarbonyl)phenyl)carbamate,
   2-(2-(5-chloro-1H-indole-2-carbonyl)hydrazinocarbonyl)phenyl dimethylcarbamate,
   2-aminobenzoic acid 2-(5-ethyl-1H-indole-2-carbonyl)hydrazide,
   2-amino-4,5-difluorobenzoic acid 2-(5-methyl-1H-indole-2-carbonyl)hydrazide,
   2-(2-hydroxyethoxy)benzoic acid 2-(5-chloro-1H-indole-2-carbonyl)hydrazide,
   2-(3-(2-(5-chloro-1H-indole-2-carbonyl)hydrazinocarbonyl)phenoxy)acetic acid methyl ester,
   2-(3-(2-(5-chloro-1H-indole-2-carbonyl)hydrazinocarbonyl)phenoxy)acetic acid,
   2-(3-(2-(5-chloro-1H-indole-2-carbonyl)hydrazinocarbonyl)phenoxy)-N,N-dimethylacetamide,
   2-methylthiazole-4-carboxylic acid 2-(5-chloro-1H-indole-2-carbonyl)hydrazide,
   4-(2H-[1,2,4]triazol-3-yl)benzoic acid 2-(5-chloro-1H-indole-2-carbonyl)hydrazide,
   3-(2H-[1,2,4]triazol-3-yl)benzoic acid 2-(5-chloro-1H-indole-2-carbonyl)hydrazide,
   1,3-dihydroxy-2-propyl (2-(2-(5-chloro-1H-indole-2-carbonyl)hydrazinocarbonyl)phenyl)carbamate,
   3-((2-(2-(5-chloro-1H-indole-2-carbonyl)hydrazinocarbonyl)phenyl)carbamoyloxy)-2,2-dimethylpropionic acid,
   thiophene-2-carboxylic acid 2-(5-chloro-1H-indole-2-carbonyl)hydrazide,
   furan-2-carboxylic acid 2-(5-chloro-1H-indole-2-carbonyl)hydrazide,
   2,6-dichloronicotinic acid 2-(5-chloro-1H-indole-2-carbonyl)hydrazide,
   1H-pyrrole-2-carboxylic acid 2-(5-chloro-1H-indole-2-carbonyl)hydrazide,
   1H-imidazole-4-carboxylic acid 2-(5-chloro-1H-indole-2-carbonyl)hydrazide,
   pyrazine-2-carboxylic acid 2-(5-chloro-1H-indole-2-carbonyl)hydrazide,
   thiophene-3-carboxylic acid 2-(5-chloro-1H-indole-2-carbonyl)hydrazide,
   furan-3-carboxylic acid 2-(5-chloro-1H-indole-2-carbonyl)hydrazide,
   5-chlorothiophene-2-carboxylic acid 2-(5-chloro-1H-indole-2-carbonyl)hydrazide,
   3-chlorothiophene-2-carboxylic acid 2-(5-chloro-1H-indole-2-carbonyl)hydrazide,
   1-methyl-1H-pyrrole-2-carboxylic acid 2-(5-chloro-1H-indole-2-carbonyl)hydrazide,
   5-methylthiophene-2-carboxylic acid 2-(5-chloro-1H-indole-2-carbonyl)hydrazide,
   3-methylthiophene-2-carboxylic acid 2-(5-chloro-1H-indole-2-carbonyl)hydrazide,
   2,6-difluorobenzoic acid 2-(5-chloro-1H-indole-2-carbonyl)hydrazide,
   2,3-difluorobenzoic acid 2-(5-chloro-1H-indole-2-carbonyl)hydrazide,
   5-chloro-1H-indole-2-carboxylic acid 2-(naphthalene-1-carbonyl)hydrazide,
   3,4,5-trifluorobenzoic acid 2-(5-chloro-1H-indole-2-carbonyl)hydrazide,
   2,3,4,5-tetrafluorobenzoic acid 2-(5-chloro-1H-indole-2-carbonyl)hydrazide,
   2-amino-4-methylbenzoic acid 2-(5-chloro-1H-indole-2-carbonyl)hydrazide,
   2-amino-4-fluorobenzoic acid 2-(5-chloro-1H-indole-2-carbonyl)hydrazide,
   2-amino-5-methylbenzoic acid 2-(5-chloro-1H-indole-2-carbonyl)hydrazide,
   2-amino-6-methylbenzoic acid 2-(5-chloro-1H-indole-2-carbonyl)hydrazide,
   2-amino-3-methylbenzoic acid 2-(5-chloro-1H-indole-2-carbonyl)hydrazide,
   2-amino-4,5-difluorobenzoic acid 2-(5-chloro-1H-indole-2-carbonyl)hydrazide,
   3-aminothiophene-2-carboxylic acid 2-(5-chloro-1H-indole-2-carbonyl)hydrazide,
   2-aminobenzoic acid 2-(5-bromo-1H-indole-2-carbonyl)hydrazide,
   2-amino-4-fluorobenzoic acid 2-(5-bromo-1H-indole-2-carbonyl)hydrazide,
   1H-pyrazole-4-carboxylic acid 2-(5-chloro-1H-indole-2-carbonyl)hydrazide,
   methyl (2-(2-(5-fluoro-1H-indole-2-carbonyl)hydrazinocarbonyl)phenyl)carbamate,
   1-methyl-1H-pyrrole-2-carboxylic acid 2-(5-methyl-1H-indole-2-carbonyl)hydrazide,
   thiophene-3-carboxylic acid 2-(5-methyl-1H-indole-2-carbonyl)hydrazide,
   4H-thieno[3,2-b]pyrrole-5-carboxylic acid 2-(5-chloro-1H-indole-2-carbonyl)hydrazide,
   phenyl (2-(2-(5-chloro-1H-indole-2-carbonyl)hydrazinocarbonyl)phenyl)carbamate,
   benzyl (2-(2-(5-chloro-1H-indole-2-carbonyl)hydrazinocarbonyl)phenyl)carbamate,
   2-hydroxyethyl (2-(2-(5-chloro-1H-indole-2-carbonyl)hydrazinocarbonyl)phenyl)carbamate,
   3-hydroxypropyl (2-(2-(5-chloro-1H-indole-2-carbonyl)hydrazinocarbonyl)phenyl)carbamate,
   2-((2-(2-(5-chloro-1H-indole-2-carbonyl)hydrazinocarbonyl)phenyl)carbamoyloxy)acetic acid,
   2-((2-(2-(5-chloro-1H-indole-2-carbonyl)hydrazinocarbonyl)phenyl)carbamoyloxymethyl)-2-methylmalonic acid,
   methyl 2-(2-(5-chloro-1H-indole-2-carbonyl)hydrazinocarbonyl)phenylcarbamate,
   cyclohexanecarboxylic acid 2-(5-chloro-1H-indole-2-carbonyl)-1-methylhydrazide,
   thiophene-2-carboxylic acid 2-(5-chloro-1H-indole-2-carbonyl)-1-methylhydrazide,
   benzoic acid 2-(1H-indole-2-carbonyl)hydrazide,
   benzoic acid 2-(5-chloro-1-methyl-1H-indole-2-carbonyl) hydrazide,
   benzoic acid 2-(5-methoxy-1H-indole-2-carbonyl)hydrazide,
   benzoic acid 2-(5-isopropyl-1H-indole-2-carbonyl)hydrazide,
   benzoic acid 2-(5-nitro-1H-indole-2-carbonyl)hydrazide,
   benzoic acid 2-(5-benzyloxy-1H-indole-2-carbonyl)hydrazide,
   benzoic acid 2-(6-chloro-1H-indole-2-carbonyl)hydrazide,
   6H-thieno[2,3-b]pyrrole-5-carboxylic acid 2-(5-chloro-1H-indole-2-carbonyl)hydrazide,
   5-chloro-1H-indole-2-carboxylic acid 2-((2-fluorophenyl)-imino-methyl)hydrazide,
   5-chloro-1H-indole-2-carboxylic acid 2-((3-fluorophenyl)-imino-methyl)hydrazide,
   5-chloro-1H-indole-2-carboxylic acid 2-((4-fluorophenyl)-imino-methyl)hydrazide,
   5-chloro-1H-indole-2-carboxylic acid 2-(imino-(p-tolyl)-methyl)hydrazide,
   5-chloro-1H-indole-2-carboxylic acid 2-((4-chlorophenyl)-imino-methyl)hydrazide,
   5-chloro-1H-indole-2-carboxylic acid 2-((3-chlorophenyl)-imino-methyl)hydrazide,
   5-chloro-1H-indole-2-carboxylic acid 2-((3-fluorophenyl)-imino-methyl)hydrazide,
   5-chloro-1H-indole-2-carboxylic acid 2-(imino-(o-tolyl)-methyl)hydrazide,
   5-chloro-1H-indole-2-carboxylic acid 2-(imino-(m-tolyl)-methyl)hydrazide,
   5-chloro-1H-indole-2-carboxylic acid 2-(imino-(thiophen-2-yl)-methyl)hydrazide,
   5-chloro-1H-indole-2-carboxylic acid 2-(imino-(pyridin-2-yl)-methyl)hydrazide,
   5-chloro-1H-indole-2-carboxylic acid 2-((furan-2-yl)-imino-methyl)hydrazide,
   5-chloro-1H-indole-2-carboxylic acid 2-((2-chloro-6-fluorophenyl)-imino-methyl)hydrazide,
   5-chloro-1H-indole-2-carboxylic acid 2-(imino-(2-trifluoromethylphenyl)-methyl)hydrazide,
   5-chloro-1H-indole-2-carboxylic acid 2-(imino-(pyrazin-2-yl)-methyl) hydrazide,
   3-aminobenzoic acid 2-(5-chloro-1H-indole-2-carbonyl)hydrazide,
   3-methoxybenzoic acid 2-(5-chloro-1H-indole-2-carbonyl)hydrazide,
   5-amino-2-methylthiazole-4-carboxylic acid 2-(5-chloro-1H-indole-2-carbonyl)hydrazide,
   2-morpholinoethyl (2-((2-(5-chloro-1H-indole-2-carbonyl)hydrazino)carbonyl)phenyl)carbamate p-toluenesulfonate,
   2-amino-4,5-difluorobenzoic acid 2-(5-methyl-1H-indole-2-carbonyl)hydrazide benzenesulfonate,
   2-amino-4-fluorobenzoic acid 2-(5-chloro-1H-indole-2-carbonyl)hydrazide benzenesulfonate,
   3-(dimethylamino)benzoic acid 2-(5-chloro-1H-indole-2-carbonyl)hydrazide methanesulfonate,
   2-amino-4-fluorobenzoic acid 2-(5-chloro-1H-indole-2-carbonyl)hydrazide hydrochloride,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-aminobenzoic acid 2-(5-methyl-1H-indole-2-carbonyl)hydrazide p-toluenesulfonate,
   2-aminobenzoic acid 2-(5-methyl-1H-indole-2-carbonyl)hydrazide benzenesulfonate,
   2-amino-4-fluorobenzoic acid 2-(5-methyl-1H-indole-2-carbonyl)hydrazide benzenesulfonate,
   3-aminobenzoic acid 2-(5-chloro-1H-indole-2-carbonyl)hydrazide p-toluenesulfonate,
   5-chloro-1H-indole-2-carboxylic acid 2-((2-chlorophenyl)-imino-methyl)hydrazide methanesulfonate,
   5-chloro-1H-indole-2-carboxylic acid 2-((2-chlorophenyl)-imino-methyl)hydrazide p-toluenesulfonate,
   5-chloro-1H-indole-2-carboxylic acid 2-((2-chlorophenyl)-imino-methyl)hydrazide hydrochloride,
   5-chloro-1H-indole-2-carboxylic acid 2-(imino-phenyl-methyl)hydrazide methanesulfonate,
   5-chloro-1H-indole-2-carboxylic acid 2-((2-chlorophenyl)-imino-methyl)hydrazide butenedioic acid salt,
   5-chloro-1H-indole-2-carboxylic acid 2-((2-fluorophenyl)-imino-methyl)hydrazide hydrochloride,
   5-chloro-1H-indole-2-carboxylic acid 2-((2-fluorophenyl)-imino-methyl)hydrazide methanesulfonate,
   5-chloro-1H-indole-2-carboxylic acid 2-((1-imino-2-phenylethyl)hydrazide methanesulfonate,
   5-chloro-1H-indole-2-carboxylic acid 2-((3-fluorophenyl)-imino-methyl)hydrazide hydrochloride,
   5-chloro-1H-indole-2-carboxylic acid 2-((3,4-difluorophenyl)-imino-methyl)hydrazide methanesulfonate,
   5-chloro-1H-indole-2-carboxylic acid 2-(imino-(2-methoxyphenyl)-methyl)hydrazide methanesulfonate,
   5-chloro-1H-indole-2-carboxylic acid 2-((2,6-difluorophenyl)-imino-methyl)hydrazide methanesulfonate,
   5-chloro-1H-indole-2-carboxylic acid 2-((2,4-difluorophenyl)-imino-methyl)hydrazide methanesulfonate,
   5-chloro-1H-indole-2-carboxylic acid 2-((1,2-dimethyl-1H-pyrrol-5-yl)-imino-methyl)hydrazide methanesulfonate,
   2-aminobenzoic acid 2-(5-methyl-1H-indole-2-carbonyl)hydrazide p-toluenesulfonate,
   2-aminobenzoic acid 2-(5-methyl-1H-indole-2-carbonyl)hydrazide benzenesulfonate,
   2-amino-4-fluorobenzoic acid 2-(5-methyl-1H-indole-2-carbonyl)hydrazide benzenesulfonate,
   2-(dimethylamino)benzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide methanesulfonate,
   2-aminobenzoic acid 2-(5-bromo-1H-indole-2-carbonyl)hydrazide methanesulfonate,
   2-amino-4-fluorobenzoic acid 2-(5-methyl-1H-indole-2-carbonyl)hydrazide p-toluenesulfonate, and
   2-amino-4-fluorobenzoic acid 2-(5-methyl-1H-indole-2-carbonyl)hydrazide methanesulfonate
   a pharmaceutically acceptable salt thereof or a prodrug thereof.
11. The indole compound of 1 above, which is selected from the group consisting of
   2-morpholinoethyl (2-((2-(5-chloro-1H-indole-2-carbonyl)hydrazino)carbonyl)phenyl)carbamate p-toluenesulfonate,
   2-amino-4,5-difluorobenzoic acid 2-(5-methyl-1H-indole-2-carbonyl)hydrazide benzenesulfonate,
   2-amino-4-fluorobenzoic acid 2-(5-chloro-1H-indole-2-. carbonyl)hydrazide benzenesulfonate,
   3-(dimethylamino)benzoic acid 2-(5-chloro-1H-indole-2-carbonyl)hydrazide methanesulfonate,
   2-amino-4-fluorobenzoic acid 2-(5-chloro-1H-indole-2-carbonyl)hydrazide hydrochloride,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide p-toluenesulfonate,
   2-aminobenzoic acid 2-(5-methyl-1H-indole-2-carbonyl)hydrazide p-toluenesulfonate,
   2-aminobenzoic acid 2-(5-methyl-1H-indole-2-carbonyl)hydrazide benzenesulfonate,
   2-amino-4-fluorobenzoic acid 2-(5-methyl-1H-indole-2-carbonyl)hydrazide benzenesulfonate,
   3-aminobenzoic acid 2-(5-chloro-1H-indole-2-carbonyl)hydrazide p-toluenesulfonate,
   5-chloro-1H-indole-2-carboxylic acid 2-((2-chlorophenyl)-imino-methyl)hydrazide methanesulfonate,
   5-chloro-1H-indole-2-carboxylic acid 2-((2-chlorophenyl)-imino-methyl)hydrazide p-toluenesulfonate,
   5-chloro-1H-indole-2-carboxylic acid 2-((2-chlorophenyl)-imino-methyl)hydrazide hydrochloride,
   5-chloro-1H-indole-2-carboxylic acid 2-(imino-phenyl-methyl)hydrazide methanesulfonate,
   5-chloro-1H-indole-2-carboxylic acid 2-((2-chlorophenyl)-imino-methyl)hydrazide butenedioic acid salt ,
   5-chloro-1H-indole-2-carboxylic acid 2-((2-fluorophenyl)-imino-methyl)hydrazide hydrochloride,
   5-chloro-1H-indole-2-carboxylic acid 2-((2-fluorophenyl)-imino-methyl)hydrazide methanesulfonate,
   5-chloro-1H-indole-2-carboxylic acid 2-((1-imino-2-phenylethyl)hydrazide methanesulfonate,
   5-chloro-1H-indole-2-carboxylic acid 2-((3-fluorophenyl)-imino-methyl)hydrazide hydrochloride,
   5-chloro-1H-indole-2-carboxylic acid 2-((3,4-difluorophenyl)-imino-methyl)hydrazide methanesulfonate,
   5-chloro-1H-indole-2-carboxylic acid 2-(imino-(2-methoxyphenyl)-methyl)hydrazide methanesulfonate,
   5-chloro-1H-indole-2-carboxylic acid 2-((2,6-difluorophenyl)-imino-methyl)hydrazide methanesulfonate,
   5-chloro-1H-indole-2-carboxylic acid 2-((2,4-difluorophenyl)-imino-methyl)hydrazide methanesulfonate,
   5-chloro-1H-indole-2-carboxylic acid 2-((1,2-dimethyl-1H-pyrrol-5-yl)-imino-methyl)hydrazide methanesulfonate,
   2-aminobenzoic acid 2-(5-methyl-1H-indole-2-carbonyl)hydrazide p-toluenesulfonate,
   2-aminobenzoic acid 2-(5-methyl-1H-indole-2-carbonyl)hydrazide benzenesulfonate,
   2-amino-4-fluorobenzoic acid 2-(5-methyl-1H-indole-2-carbonyl)hydrazide benzenesulfonate,
   2-(dimethylamino)benzoic acid 2-(5-chloro-1H-indole-2-carbonyl)hydrazide p-toluenesulfonate,
   2-amino-4-fluorobenzoic acid 2-(5-chloro-1H-indole-2-carbonyl)hydrazide methanesulfonate,
   2-aminobenzoic acid 2-(5-bromo-1H-indole-2-carbonyl)hydrazide methanesulfonate,
   2-amino-4-fluorobenzoic acid 2-(5-methyl-1H-indole-2-carbonyl)hydrazide p-toluenesulfonate, and
   2-amino-4-fluorobenzoic acid 2-(5-methyl-1H-indole-2-carbonyl)hydrazide methanesulfonate
   a pharmaceutically acceptable salt thereof or a prodrug thereof.
12. A pharmaceutical composition comprising an indole compound of any of the above-mentioned 1 to 11, a pharmaceutically acceptable salt thereof or a prodrug thereof, and a pharmaceutically acceptable carrier.
13. An HLGPa inhibitor comprising an indole compound of any of the above-mentioned 1 to 11, a pharmaceutically acceptable salt thereof or a prodrug thereof, and a pharmaceutically acceptable carrier.
14. A therapeutic agents for diabetes, which comprises an indole compound of any of the above-mentioned 1 to 11, a pharmaceutically acceptable salt thereof or a prodrug thereof, and a pharmaceutically acceptable carrier.
15. The pharmaceutical composition of the above-mentioned 14, which is used together with a therapeutic agent for hyperlipidemia.
16. The pharmaceutical composition of the above-mentioned 15, wherein the therapeutic agent for hyperlipidemia is a statin pharmaceutical agent.
17. The pharmaceutical composition of the above-mentioned 16, wherein the statin pharmaceutical agent is lovastatin, simvastatin, pravastatin, fluvastatin, atorvastatin or cerivastatin.
18. A pharmaceutical composition for the treatment or prophylaxis of diabetes, which comprises a therapeutic agent for diabetes selected from the group consisting of insulin preparations, sulfonylurea agents, insulin secretagogues, sulfonamides, biguanides, α-glucosidase inhibitors and insulin sensitizers, and an HLGPa inhibitor in combination.
19. The pharmaceutical composition of claim 18, wherein the therapeutic agent for diabetes is selected from the group consisting of insulin, glibenclamide, torbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.
20. The therapeutic agent for diabetes of the above-mentioned 18 or 19, wherein the HLGPa inhibitor is an indole compound of any of the above-mentioned 1 to 11, a pharmaceutically acceptable salt thereof or a prodrug thereof.
21. A method for treating or preventing diabetes, which comprises administering an indole compound of any of the above-mentioned 1 to 11, a pharmaceutically acceptable salt thereof or a prodrug thereof.
22. The method of the above-mentioned 21, which comprises using a therapeutic agent for hyperlipidemia in combination.
23. The method of the above-mentioned 22, wherein the therapeutic agent for hyperlipidemia is a statin pharmaceutical agent.
24. The method of the above-mentioned 23, wherein the statin pharmaceutical agent is lovastatin, simvastatin, pravastatin, fluvastatin, atorvastatin or cerivastatin.
25. A method for treating or preventing diabetes, which comprises administering a pharmaceutical composition for the treatment or prophylaxis of diabetes comprising a therapeutic agent for diabetes selected from the group consisting of insulin preparations, sulfonylurea agents, insulin secretagogues, sulfonamides, biguanides, α-glucosidase inhibitors and insulin sensitizers, and an HLGPa inhibitor in combination.
26. The method of the above-mentioned 25, wherein the therapeutic agent for diabetes is selected from the group consisting of insulin, glibenclamide, torbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.
27. The method of the above-mentioned 25 or 26, wherein the HLGPa inhibitor is an indole compound of any of the above-mentioned 1 to 11, a pharmaceutically acceptable salt thereof or a prodrug thereof.
28. Use of an indole compound of any of the above-mentioned 1 to 11, a pharmaceutically acceptable salt thereof or a prodrug thereof for the production of a therapeutic agent for diabetes.
29. The use of the above-mentioned 28, which comprises use of a therapeutic agent for hyperlipidemia in combination.
30. The use of the above-mentioned 29, wherein the therapeutic agent for hyperlipidemia is a statin pharmaceutical agent.
31. The use of the above-mentioned 30, wherein the statin pharmaceutical agent is lovastatin, simvastatin, pravastatin, fluvastatin, atorvastatin or cerivastatin.
32. Use of a therapeutic agent for diabetes selected from the group consisting of selected from the group consisting of insulin preparations, sulfonylurea agents, insulin secretagogues, sulfonamides, biguanides, α-glucosidase inhibitors and insulin sensitizers and an HLGPa inhibitor for the production of a pharmaceutical composition for the treatment or prophylaxis of diabetes.
33. The use of the above-mentioned 32, wherein the therapeutic agent for diabetes is selected from the group consisting of insulin, glibenclamide, torbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.
34. The use of the above-mentioned 32 or 33, wherein the HLGPa inhibitor is an indole compound of any of the above-mentioned 1 to 11, a pharmaceutically acceptable salt thereof or a prodrug thereof.

### Embodiment of the Invention

The definition of each substituent used in the present specification is as follows.

The "halogen atom" is a chlorine atom, a bromine atom, a fluorine atom and the like. For R², it is preferably a chlorine atom, for R³, it is preferably a chlorine atom, a bromine atom or a fluorine atom, for R¹¹, R¹², R¹³, R¹⁴ or R¹⁵, it is preferably a chlorine atom or a fluorine atom, for R^{16b}-R¹⁶ⁿ or R^{17b}-R¹⁷ⁿ, it is preferably a chlorine atom, and for R³⁶ or R³⁷, it is preferably a chlorine atom or a fluorine atom.

The "C₁₋₆ alkyl group" is a straight chain or branched chain alkyl group having 1 to 6 carbon atoms, such as methyl group, ethyl group, propyl group, isopropyl group, butyl group, isobutyl group, sec-butyl group, tert-butyl group, pentyl group, isopentyl group, neopentyl group, tert-pentyl group, hexyl group and the like. Preferred is a straight chain or branched chain alkyl group having 1 to 4 carbon atoms, and particularly preferred are methyl group, ethyl group and isopropyl group. For R¹, it is preferably a methyl group, for R³, it is preferably a methyl group, an ethyl group or an isopropyl group, for R⁴, it is preferably a methyl group, for R⁵, it is preferably a methyl group, for R⁶, it is preferably a methyl group, for R⁸, it is preferably a methyl group, for R⁹ or R¹⁰, it is preferably a methyl group, for R¹¹, R¹², R¹³, R¹⁴ or R¹⁵, it is preferably a methyl group, for R^{16b}-R¹⁶ⁿ or R^{17b}-R¹⁷ⁿ, it is preferably a methyl group, for R²⁰ or R²¹, it is preferably a methyl group or an ethyl group, for R²⁵ or R²⁶, it is preferably a methyl group or an ethyl group, for R²⁸ or R²⁹, it is preferably a methyl group or an ethyl group, for R³⁰, it is preferably a methyl group, an ethyl group, a propyl group, an isopropyl group, a butyl group, an isobutyl group, a sec-butyl group, a tert-butyl group, a pentyl group, an isopentyl group, a neopentyl group or a tert-pentyl group, for R³¹, R^{31'}, R³² or R^{32'}, it is preferably a methyl group or an ethyl group, for R³³, it is preferably a methyl group, for R³⁵, it is preferably a methyl group, for R³⁶ or R³⁷, it is preferably a methyl group, for R³⁹ or R⁴⁰, it is preferably a methyl group, for R⁴¹, it is preferably a methyl group, for R⁴², R^{42'}, R^{42''} or R⁴³, it is preferably a methyl group, for R⁴⁴ or R^{44'}, it is preferably a methyl group, an ethyl group, a propyl group or an isopropyl group, and for R⁴⁵, it is preferably a methyl group.

The "C₁₋₆ alkyl group" for R³⁰ may be substituted by a hydroxyl group, a trifluoromethyl group, an aryl group optionally having substituents (hereinafter as defined for "aryl group", preferably phenyl group), morpholino group or carboxyl group, wherein the position of substitution is not particularly limited as long as substitution is possible. As the C₁₋₆ alkyl group substituted by hydroxyl group, trifluoromethyl group, aryl group optionally having substituents, morpholino group or carboxyl group, for example, 2-hydroxyethyl group, 3-hydroxypropyl group, 4-hydroxybutyl group, 2,3-dihydroxylpropyl group; 2-carboxypropyl group, 2,2-dicarboxypropyl group; 2,2,2-trifluoroethyl group; benzyl group; morpholinomethyl group and the like can be mentioned, with preference given to 2,3-dihydroxypropyl group and 2,2-dicarboxypropyl group.

The "C₁₋₆ alkyl group" for R⁴⁴ and R^{44'} may be substituted by a carboxyl group or a C₂₋₇ alkoxycarbonyl group (as defined below), wherein the position of substitution is not particularly limited as long as substitution is possible. The C₁₋₆ alkyl group substituted by a carboxyl group or a C₂₋₇ alkoxycarbonyl group is preferably a carboxymethyl group or a methoxycarbonylmethyl group.

The "C₁₋₆ alkoxy group" is a straight chain or branched chain alkoxy group having 1 to 6 carbon atoms, such as methoxy group, ethoxy group, propoxy group, isopropoxy group, butoxy group, tert-butoxy group, pentyloxy group, tert-pentyloxy group and hexyloxy group. Preferred is a straight chain or branched chain alkoxy group having 1 to 4 carbon atoms, such as methoxy group, ethoxy group, isopropoxy group, butoxy group, tert-butoxy group, and particularly preferred are methoxy group and ethoxy group. For R³, it is preferably a methoxy group, for R¹¹, R¹², R¹³, R¹⁴ or R¹⁵, it is preferably a methoxy group or an ethoxy group, for R^{16b}-R¹⁶ⁿ or R^{17b}-R¹⁷ⁿ, it is preferably a methoxy group, for R¹⁹, it is preferably a methoxy group, for R²² or R^{22'}, it is preferably a methoxy group, for R²⁴, it is preferably a methoxy group, an ethoxy group, a propoxy group or an isopropoxy group, for R³⁴, it is preferably a methoxy group, for R³⁶ or R³⁷, it is preferably a methoxy group, and for R³⁹ or R⁴⁰, it is preferably a methoxy group.

The "C₂₋₇ alkoxycarbonyl group" is a linear or branched chain alkoxycarbonyl group wherein the alkyl moiety has 1 to 6 (preferably 1 to 4) carbon atoms. Examples thereof include methoxycarbonyl group, ethoxycarbonyl group, propoxycarbonyl group, isopropoxycarbonyl group, butoxycarbonyl group, isobutoxycarbonyl group, tert-butoxycarbonyl group, pentyloxycarbonyl group, hexyloxycarbonyl group and the like. Preferred are methoxycarbonyl group, ethoxycarbonyl group and tert-butoxycarbonyl group. For R⁵, it is preferably a methoxycarbonyl group, for R¹⁸, it is preferably a methoxycarbonyl group or a tert-butoxycarbonyl group, for R³⁶ or R³⁷, it is preferably a methoxycarbonyl group, for R³⁹ or R⁴⁰, it is preferably a methoxycarbonyl group, and for R⁴⁴ or R^{44'}, it is preferably a methoxycarbonyl group.

The "C₂₋₇ alkoxycarbonylamino group" is a linear or branched chain alkoxycarbonylamino group wherein the alkyl moiety has 1 to 6 (preferably 1 to 4) carbon atoms. Examples thereof include methoxycarbonylamino group, ethoxycarbonylamino group, propoxycarbonylamino group, isopropoxycarbonylamino group, butoxycarbonylamino group, isobutoxycarbonylamino group, tert-butoxycarbonylamino group, pentyloxycarbonylamino group, hexyloxycarbonylamino and the like. Preferred are methoxycarbonylamino group, ethoxycarbonylamino group and tert-butoxycarbonylamino group. For R⁹ or R¹⁰, it is preferably a methoxycarbonyl group or a tert-butoxycarbonylamino group, and for R²² or R^{22'}, it is preferably a tert-butoxycarbonylamino group.

The "C₃₋₁₃ alkoxycarbonylalkyl group" is a linear or branched chain alkoxycarbonylalkyl group wherein the both alkyl moieties (alkoxy moiety and alkyl moiety) have 1 to 6 (preferably 1 to 4) carbon atoms. Examples thereof include methoxycarbonylmethyl group, methoxycarbonylethyl group, ethoxycarbonylmethyl group, ethoxycarbonylethyl group, propoxycarbonylmethyl group, isopropoxycarbonylmethyl group, butoxycarbonylmethyl group, isobutoxycarbonylmethyl group, tert-butoxycarbonylmethyl group, pentyloxycarbonylmethyl group, hexyloxycarbonylmethyl and the like. Preferred are methoxycarbonylmethyl group, ethoxycarbonylmethyl group and tert-butoxycarbonylmethyl group. For R²⁰ and R²¹, it is preferably a methoxycarbonylmethyl group.

The "C₂₋₇ alkylcarbonyloxy group" is a linear or branched chain alkylcarbonyloxy group wherein the alkyl moiety has 1 to 6 (preferably 1 to 4) carbon atoms. Examples thereof include methylcarbonyloxy group, ethylcarbonyloxy group, propylcarbonyloxy group, isopropylcarbonyloxy group, butylcarbonyloxy group, isobutylcarbonyloxy group, tert-butylcarbonyloxy group, pentylcarbonyloxy group, hexylcarbonyloxy and the like. Preferred are methylcarbonyloxy group, ethylcarbonyloxy group and tert-butylcarbonyloxy group. For R²³, it is preferably a methylcarbonyloxy group, and for R³⁴, it is preferably a methylcarbonyloxy group.

The "C₁₋₆ hydroxyalkyl group" is a straight chain or branched chain alkyl group having 1 to 6 (preferably 1 to 4) carbon atoms, which is substituted by one or more hydroxyl groups, wherein the position of substitution of the hydroxyl group is not particularly limited. For example, hydroxymethyl group; 1- or 2-hydroxyethyl group; 1-, 2- or 3-hydroxypropyl group; 1-, 2-, 3- or 4-hydroxybutyl group; 1-, 2-, 3-, 4- or 5-hydroxypentyl group; 1-, 2-, 3-, 4-, 5- or 6-hydroxyhexyl group; 2-hydroxy-2-methylethyl group; 1,2-dihydroxyethyl group and the like can be mentioned, with preference given to 2-hydroxyethyl group and 1,2-dihydroxyethyl group. For R⁹ or R¹⁰, it is preferably a hydroxymethyl group.

The "C₁₋₆ haloalkyl group" is a straight chain or branched chain alkyl group having 1 to 6 (preferably 1 to 4) carbon atoms, which is substituted by one or more halogen atoms (as defined above), wherein the position of substitution of the hydroxyl group is not particularly limited. For example, trifluoromethyl group, 1- or 2-chloroethyl group, 1- or 2-bromomethyl group, 1- or 2-fluoroethyl group, 1-, 2- or 3-chloropropyl group, 1-, 2- or 3-bromopropyl group, 1-, 2- or 3-fluoropropyl group, 1-, 2-, 3- or 4-chlorobutyl group, 1-, 2-, 3- or 4-bromobutyl group, 1-, 2-, 3- or 4-fluorobutyl group and the like can be mentioned, with preference given to trifluoromethyl group. For R¹¹, R¹², R¹³, R¹⁴ or R¹⁵, it is preferably a trifluoromethyl group.

The "C₁₋₆ alkylamino group" is an amino group monosubstituted by a straight chain or branched chain alkyl group having 1 to 6 (preferably 1 to 4) carbon atoms, such as methylamino group, ethylamino group, propylamino group, butylamino group, pentylamino group, hexylamino group and the like. Preferred are methylamino group and ethylamino group. For R²⁷, it is preferably a methylamino group or an ethylamino group, for R³⁴, it is preferably a methylamino group, and for R⁴¹, it is preferably a methylamino group or an ethylamino group.

The "C₂₋₁₂ dialkylamino group" is an amino group disubstituted by straight chain or branched chain alkyl groups having 1 to 6 (preferably 1 to 4) carbon atoms, wherein the alkyl moiety may be the same or different. For example, dimethylamino group, diethylamino group, dipropylamino group, dibutylamino group, dipentylamino group, dihexylamino group and the like can be mentioned, with preference given to dimethylamino group and diethylamino group. For R²² or R^{22'}, it is preferably a dimethylamino group, for R²⁷, it is preferably a dimethylamino group, for R³⁴, it is preferably a dimethylamino group, and for R⁴¹, it is preferably a dimethylamino group.

The "C₃₋₇ cycloalkyl group" is a cycloalkyl group having 3 to 7 (preferably 3 to 6) carbon atoms. Specific examples include cyclopropyl group, cyclobutyl group, cyclopentyl group, cyclohexyl group, cycloheptyl group and the like. Preferred are cycloalkyl group having 3 to 6 carbon atoms. Specific examples include cyclopropyl group, cyclobutyl group, cyclopentyl group and cyclohexyl group. Particularly preferred are cyclopropyl group and cyclohexyl group. For R⁹ or R¹⁰, it is preferably a cyclopropyl group, a cyclopentyl group or a cyclohexyl group.

The "acyl group" is an alkylcarbonyl group such as acetyl group, propionyl group, butyryl group, pivaloyl group and the like wherein the alkyl moiety preferably has 1 to 6, more preferably 1 to 4 carbon atoms and is a linear or branched chain; an arylcarbonyl group such as benzoyl group, naphthoyl and the like wherein the aryl moiety preferably has 6 to 12, more preferably 6 to 10 carbon atoms; and the like, with preference given to acetyl group. For R¹, it is preferably an acetyl group, for R³, it is preferably an acetyl group, and for R²⁸ or R²⁹, it is preferably an acetyl group.

The "aryl group" preferably has 6 to 12, more preferably 6 to 10 carbon atoms, such as phenyl group, naphthyl group and the like, with preference given to phenyl group. The aryl group may be substituted by 1 to 6 the same or different substituents selected from phenyl group, haloalkyl group (those similar to the above-mentioned "C₁₋₆ haloalkyl group" can be mentioned), halogen atom (as defined above), C₁₋₆ alkyl group (as defined above), C₁₋₆ alkoxy group (as defined above), C₂₋₇ alkoxycarbonyl group (as defined above), nitro group, cyano group, carboxyl group, hydroxyl group, amino group, C₁₋₆ alkylamino group (as defined above) and diC₁₋₆ alkylamino group (as defined for the above-mentioned "C₂₋₁₂ dialkylamino group"), wherein the position of substituent may be any and is not particularly limited. The phenyl group in the above-mentioned substituents may be further substituted by 1 to 6 the same or different substituents selected from the above-mentioned substituent group except phenyl group. To be concrete, phenyl group substituted by the following same or different 1 to 3 substituents is preferable. Examples of the substituents: haloalkyl groups such as trifluoromethyl group etc.; halogen atoms such as chlorine atom, bromine atom, fluorine atom etc.; C₁₋₆ alkyl groups such as methyl group, ethyl group, propyl group, isopropyl group, butyl group, tert-butyl group etc.; C₁₋₆ alkoxy groups such as methoxy group, ethoxy group, propoxy group, isopropoxy group, butoxy group, isobutoxy group, sec-butoxy group, tert-butoxy group etc.; C₂₋₇ alkoxycarbonyl groups such as methoxycarbonyl group, ethoxycarbonyl group, propoxycarbonyl group etc.; nitro group; cyano group; carboxyl group; hydroxyl group; amino group; C₁₋₆ alkylamino groups such as methylamino group, ethylamino group, propylamino group, butylamino group etc.; di-C₁₋₆ alkylamino groups such as dimethylamino group, diethylamino group, dipropylamino group etc.; and the like. For R⁸, it is preferably a phenyl group, for R¹¹, R¹², R¹³, R¹⁴ or R¹⁵, it is preferably a phenyl group, for R¹⁹, it is preferably a phenyl group, for R²⁸ or R²⁹, it is preferably a phenyl group, for R³¹, R³⁰, R^{31'}, R³² or R^{32'}, it is preferably a phenyl group, for R³³, it is preferably a phenyl group, for R³⁸, it is preferably phenyl group optionally substituted by a halogen atom or C₁₋₆ alkyl group (particularly, phenyl group, chlorophenyl group, fluorophenyl group, tolyl group), for R⁴², R^{42'}, R^{42''} or R⁴³, it is preferably a phenyl group, for R⁴⁵, it is preferably phenyl group optionally substituted by a halogen atom (particularly, phenyl group, fluorophenyl group), and for R⁴⁶ or R^{46'}, it is preferably phenyl group optionally substituted by a halogen atom, a hydroxy group or C₁₋₆ alkoxy group (particularly, phenyl group, fluorophenyl group, chlorophenyl group, hydroxyphenyl group, methoxyphenyl group).

The "aryloxy group" preferably has 6 to 12, more preferably 6 to 10, carbon atoms, such as phenoxy group, naphthyloxy group and the like. Preferred is phenoxy group. The aryl group of the aryloxy group may be substituted by 1 to 6 the same or different substituents selected from phenyl group, haloalkyl group (those similar to the above-mentioned "C₁₋₆ haloalkyl group" can be mentioned), halogen atom (as defined above), C₁₋₆ alkyl group (as defined above), C₁₋₆ alkoxy group (as defined above), C₂₋₇ alkoxycarbonyl group (as defined above), nitro group, cyano group, carboxyl group, hydroxyl group, amino group, C₁₋₆ alkylamino group (as defined above) and diC₁₋₆ alkylamino group (as defined for the above-mentioned "C₂₋₁₂ dialkylamino group"). The phenyl group in the above-mentioned substituents may be further substituted by 1 to 6 the same or different substituents selected from the above-mentioned substituent group except phenyl group. For R¹¹, R¹², R¹³, R¹⁴ or R¹⁵, it is preferably a phenoxy group.

The "aralkyl group" is an arylalkyl group wherein the aryl moiety is phenyl group and the alkyl moiety is a straight chain or branched chain alkyl group having 1 to 6 (preferably 1 to 4) carbon atoms, for example, benzyl group, phenylpropyl group, phenylbutyl group, phenylhexyl group and the like. The phenyl group may be further substituted by 1 to 6 the same or different substituents selected from haloalkyl group (those similar to the above-mentioned "C₁₋₆ haloalkyl group" can be mentioned), halogen atom (as defined above), C₁₋₆ alkyl group (as defined above), C₁₋₆ alkoxy group (as defined above), C₂₋₇ alkoxycarbonyl group (as defined above), nitro group, cyano group, carboxyl group, hydroxyl group, amino group, C₁₋₆ alkylamino group (as defined above) and di-C₁₋₆ alkylamino group (as defined for the above-mentioned "C₂₋₁₂ dialkylamino group"). For R⁶, it is preferably a benzyl group or a 4-fluorobenzyl group, for R¹¹, R¹², R¹³, R¹⁴ or R¹⁵, it is preferably a benzyl group or a phenylpropyl group, for R²⁰ or R²¹, it is preferably a benzyl group, for R²⁵ or R²⁶, it is preferably a benzyl group, and for R⁴⁶ or R^{46'}, it is preferably a benzyl group.

The "aralkyloxy group" is an arylalkoxy group wherein the aryl moiety is phenyl group and alkoxy moiety is a linear or branched chain alkoxy group having 1 to 6 (preferably 1 to 4) carbon atoms, such as benzyloxy group, phenylpropoxy group, phenylbutoxy group, phenylhexyloxy group and the like. The phenyl group may be further substituted by 1 to 6 the same or different substituents selected from haloalkyl group (those similar to the above-mentioned "C₁₋₆ haloalkyl group" can be mentioned), halogen atom (as defined above), C₁₋₆ alkyl group (as defined above), C₁₋₆ alkoxy group (as defined above), C₂₋₇ alkoxycarbonyl group (as defined above), nitro group, cyano group, carboxyl group, hydroxyl group, amino group, C₁₋₆ alkylamino group (as defined above) and diC₁₋₆ alkylamino group (as defined for the above-mentioned "C₂₋₁₂ dialkylamino group"). For R³, it is preferably a benzyloxy group.

The "acylamino group" has 2 to 13, preferably 2 to 11, carbon atoms, and is exemplified by alkylcarbonylamino having 2 to 7 carbon atoms (e.g., acetylamino, propionylamino, butyrylamino, pivaloylamino and the like) and the like, with preference given to acetylamino.

The "C₂₋₇ haloalkylcarbonylamino group" is a haloalkylcarbonylamino group having preferably 2 to 5 carbon atoms, wherein the haloalkyl moiety is as defined for the above-mentioned "C₁₋₆ haloalkyl group", with preference given to trifluoromethylcarbonylamino.

The "heteroaryl group" is a heteroaryl group having, besides carbon atom, one or more, preferably 1 to 3, heteroatoms such as nitrogen atom, sulfur atom, oxygen atom and the like, which is preferably a 4- to 7-membered ring, more preferably a 5- or 6-membered ring. Specific examples include thienyl, furyl, imidazolyl, pyrrolyl, oxazolyl, isoxazolyl, thiazolyl, isothiazolyl, pyrazolyl, thioxazolyl, diazolyl, pyridyl, pyridazinyl, pyrimidinyl, pyrazinyl, pyrrolinyl, pyrrolidinyl, piperidinyl, piperazinyl, morpholinyl, azepinyloxepinyl and the like. R³⁸ is preferably pyridyl, and R⁴⁶ and R^{46'} are preferably thienyl.

The thiazolyl group for R³ is optionally substituted by a C₁₋₆ alkyl group (as defined above) or amino group.

The aralkyl group for R⁶ is optionally substituted by a halogen atom (as defined above).

The triazolyl group encompasses both a 1,2,3-form and a 1,2,4-form.

The position of bonding of tetrazolyl group and triazolyl group is not particularly limited as long as the bonding is possible.

The "pharmaceutically acceptable salt" includes, for example, various inorganic acid addition salts such as hydrochloride, hydrobromide, sulfonate, phosphate, nitrate and the like; various organic acid addition salts such as acetate, propionate, succinate, glycolate, lactate, malate, oxalate, tartrate, citrate, maleate, fumarate, methanesulfonate, benzenesulfonate, p-toluenesulfonate, ascorbate and the like; salts with various amino acids such as aspartate, glutamate and the like. Preferred are hydrochloride, methanesulfonate, benzenesulfonate and p-toluenesulfonate, and particularly preferred is p-toluenesulfonate. In some cases, it may be a water-containing salt, a hydrate or a solvate.

A preferable embodiment of the compound (1) of the present invention is, for example, an embodiment wherein
R⁶ is a hydrogen atom, a C₁₋₆ alkyl group or an aralkyl group wherein the aralkyl group is optionally substituted by a halogen atom;
R⁷ is 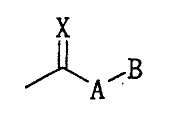 wherein X is =O, =S or =NH;
A is -N(R^{8'})- wherein R^{8'} is a hydrogen atom, a C₁₋₆ alkyl group or a phenyl group optionally having substituents, -C(R^{9'})(R^{10'})- wherein R^{9'} and R^{10'} are the same or different and each is a hydrogen atom, a hydroxyl group, an amino group, a C₁₋₆ alkyl group, a C₁₋₆ hydroxyalkyl group, a C₂₋₇ alkoxycarbonylamino group or an acylamino group, or R^{9'} and R^{10'} may form, together with the adjacent carbon atom, a C₃₋₇ cycloalkyl group, -(CH₂)ₘ-NH- wherein m is an integer of 1 to 4), -CO-, -S- or a single bond; and
B is 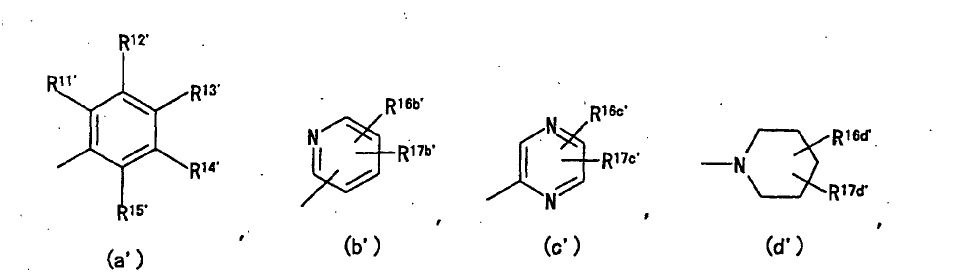 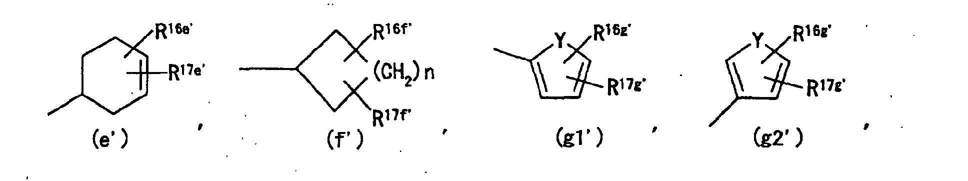 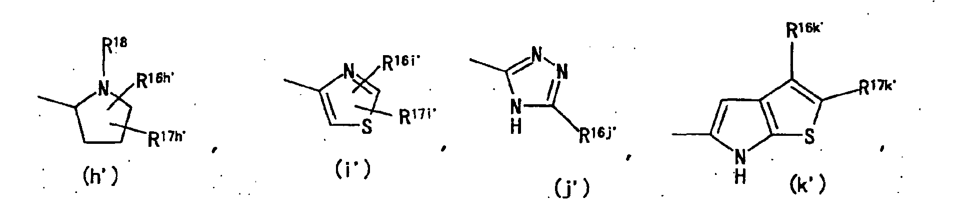 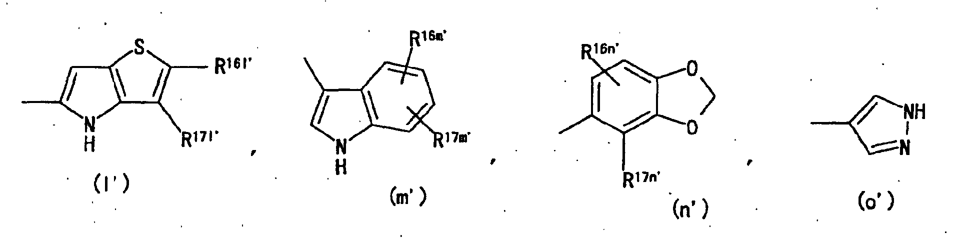 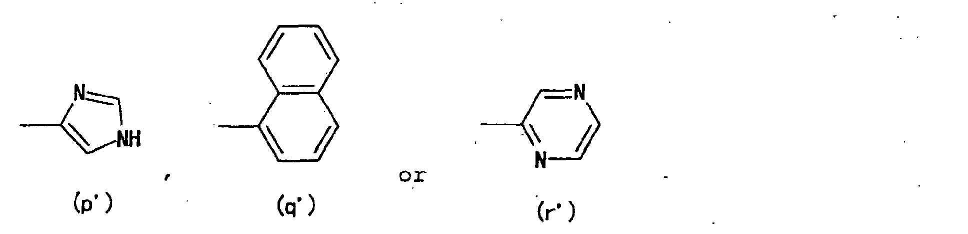 wherein R^{11'}, R^{12'}, R^{13'}, R^{14'} and R^{15'} are the same or different and each is a hydrogen atom, a halogen atom, a C₁₋₆ alkyl group, a C₁₋₆ alkoxy group, a nitro group, a hydroxyl group, a cyano group, a haloalkyl group, an aralkyl group, a phenyl group optionally having substituents, an aryloxy group, a tetrazolyl group, a triazolyl group, -(CH₂)ₚ-CO-R^{19'} wherein p is 0 or an integer of 1 to 4 and R^{19'} is a phenyl group optionally having substituents, a hydroxyl group, a C₁₋₆ alkoxy group or -N(R²⁰)(R²¹)- wherein R²⁰ and R²¹ are as defined in the compound (1) of the present invention, -O-(CH₂)ᵣ-R²³ wherein r and R²³ are as defined in the compound (1) of the present invention, -O-CO-R²⁷ wherein R²⁷ is as defined in the compound (1) of the present invention, or -N(R^{28'})(R^{29'}) wherein R^{28'} and R^{29'} are the same or different and each is a hydrogen atom, a C₁₋₆ alkyl group, a phenyl group optionally having substituents, an acyl group, -(CH₂)_{p'}-COO-R^{30'} wherein p' is as defined in the compound (1) of the present invention and R^{30'} is a hydrogen atom, a phenyl group optionally having substituents or a C₁₋₆ alkyl group wherein the C₁₋₆ alkyl group is optionally substituted by a hydroxyl group, a trifluoromethyl group, a phenyl group optionally having substituents, a morpholino group or a carboxyl group, -CON(R^{31''})(R^{32''}) wherein R^{31''} and R^{32''} are the same or different and each is a hydrogen atom, a C₁₋₆ alkyl group or a phenyl group optionally having substituents, -CO-R^{33'} wherein R^{33'} is a C₁₋₆ alkyl group or a phenyl group optionally having substituents or -CO-(CH₂)_{r'}-R³⁴ wherein r' and R³⁴ are as defined in the compound (1) of the present invention, R^{16b'}-R^{16n'} and R^{17b'}-R^{17n'} are the same or different and each is a hydrogen atom, a halogen atom, a C₁₋₆ alkyl group, an amino group, a hydroxyl group, a C₁₋₆ alkoxy group or -CON(R^{31'''})(R^{32'''}) wherein R^{31'''} and R^{32'''} are as defined for R^{31''} and R^{32''}, and
R¹⁸, Y and n are as defined in the compound (1) of the present invention, or
R⁶ and R⁷ may form, together with the adjacent nitrogen atom, 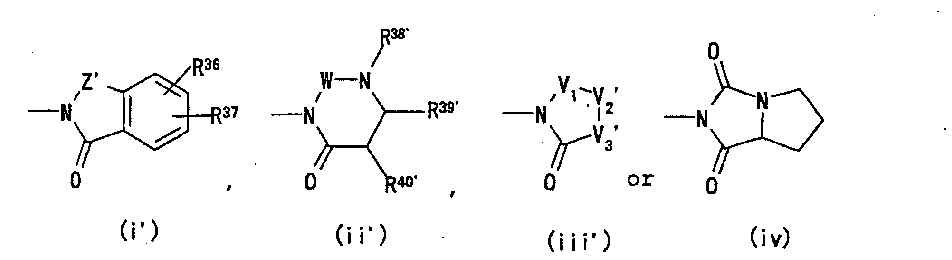 wherein R³⁶ and R³⁷ are as defined in the compound (1) of the present invention;
Z' is -CH₂-CH₂-, -C(R⁴²)=CH-, -C(R^{42'})=N-, -N=N-, -CO-, -CO-O-, -CO-CH₂-O-, -CH₂-CO-NH-, -C(R^{42''})(R⁴³)-N(R⁴⁴)- wherein R⁴², R^{42'}, R^{42''} and R⁴³ are the same or different and each is a hydrogen atom, a C₁₋₆ alkyl group or a phenyl group optionally having substituents and R⁴⁴ is as defined in the compound (1) of the present invention or -C(U)-N(R^{44'})- wherein U and R^{44'} are as defined in the compound (1) of the present invention;
R^{38'} is a hydrogen atom, a phenyl group optionally substituted by a halogen atom or a C₁₋₆ alkyl group, or a pyridyl group;
R^{39'} and R^{40'} are both hydrogen atoms, or R^{39'} and R^{40'} may form, together with the adjacent carbon atom, 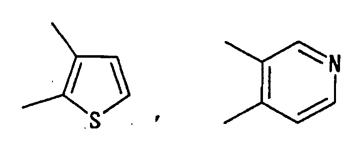 or 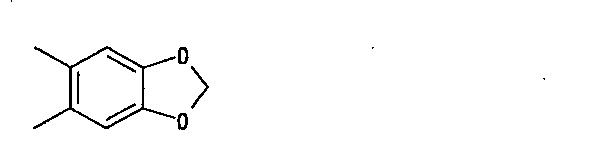
W and V₁ are as defined in the compound (1) of the present invention;
V₂' is -O-, -CH₂- or -N(R⁴⁵)- wherein R⁴⁵ is a hydrogen atom, a C₁₋₆ alkyl group or a phenyl group optionally substituted by a halogen atom; and
V₃' is -CH(R⁴⁶)- or -N(R⁴⁶')- wherein R⁴⁶ and R^{46'} are each a hydrogen atom, a benzyl group, a thienyl group, or a phenyl group optionally substituted by a halogen atom, a hydroxy group or a C₁₋₆ alkoxy group; and
   an embodiment wherein
R⁶ is a hydrogen atom, a C₁₋₆ alkyl group or an aralkyl group wherein the aralkyl group is optionally substituted by a halogen atom;
R⁷ is 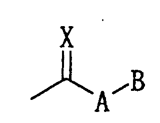 wherein X is as defined in the compound (1) of the present invention;
A is -N(R^{8''})- wherein R^{8''} is a hydrogen atom, a C₁₋₆ alkyl group or an aryl group optionally having substituents, -C(R^{9''})(R^{10''})- wherein R^{9''} and R^{10''} are the same or different and each is a hydrogen atom, a hydroxyl group, an amino group, a C₁₋₆ alkyl group, a C₁₋₆ hydroxyalkyl group or a C₂₋₇ alkoxycarbonylamino group, or R^{9''} and R^{10''} may form, together with the adjacent carbon atom, a C₃₋₇ cycloalkyl group, -(CH₂)ₘ-NH- wherein m are as defined in the compound (1) of the present invention, -CO- or a single bond; and
B is 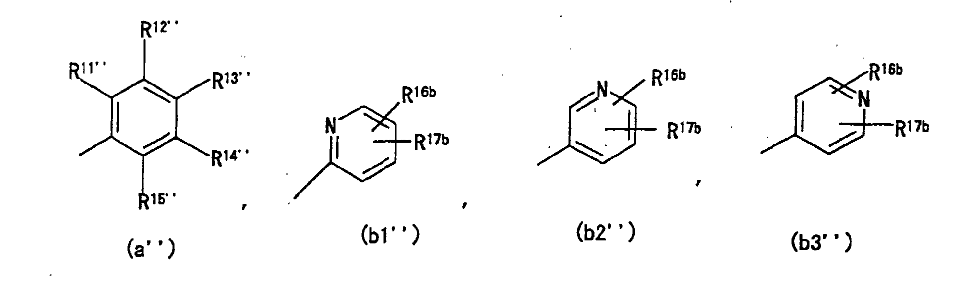 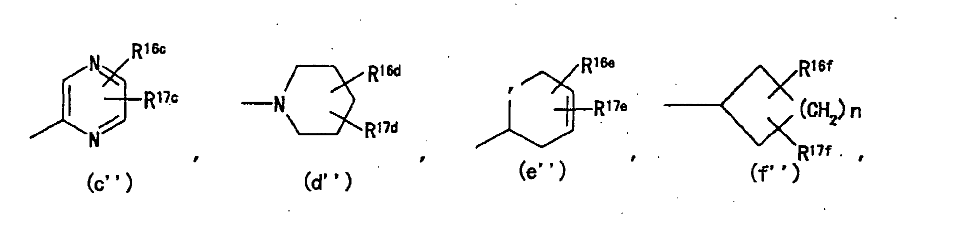 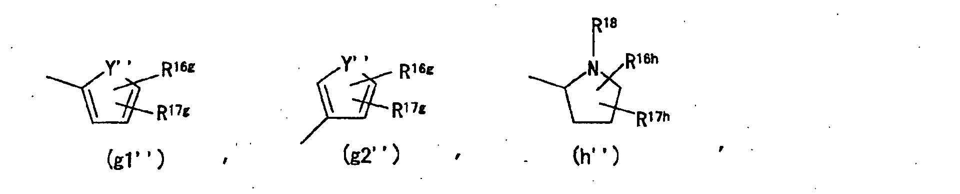 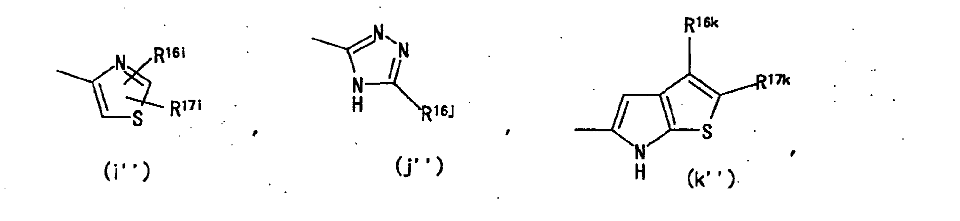 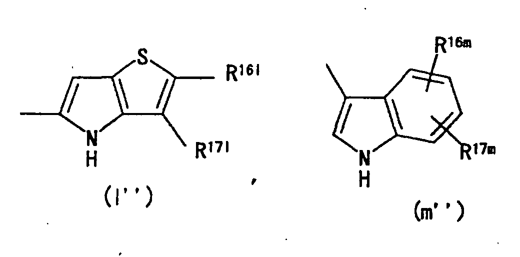 or 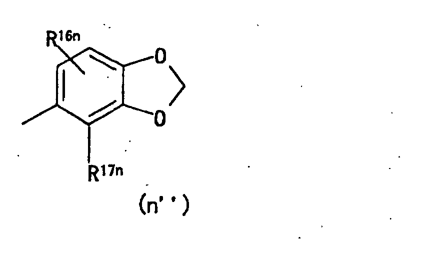 wherein R^{11''}, R^{12''}, R^{13''}, R^{14''} and R^{15''} are the same or different and each is a hydrogen atom, a halogen atom, a C₁₋₆ alkyl group, a C₁₋₆ alkoxy group, a nitro group, a hydroxyl group, a cyano group, a haloalkyl group, an aralkyl group, an aryl group optionally having substituents, an aryloxy group, a tetrazolyl group, a triazolyl group, -(CH₂)ₚ-CO-R¹⁹ wherein p and R¹⁹ are as defined in the compound (1) of the present invention, -O-(CH₂)ᵣ-R²³ wherein r and R²³ are as defined in the compound (1) of the present invention, -O-CO-R²⁷ wherein R²⁷ is as defined in the compound (1) of the present invention or -N(R^{28''}) (R^{29''}) wherein R^{28''} and R^{29''} are the same or different and each is a hydrogen atom, a C₁₋₆ alkyl group, an aryl group optionally having substituents, -(CH₂)_{p'}-COO-R^{30''} wherein p' is as defined for p and R^{30''} is a hydrogen atom or a C₁₋₆ alkyl group wherein the C₁₋₆ alkyl group is optionally substituted by a hydroxyl group, a trifluoromethyl group or a carboxyl group, -CON(R³¹)(R³²) wherein R³¹ and R³² are as defined in the compound (1) of the present invention, -CO-R³³ wherein R³³ is as defined in the compound (1) of the present invention or -CO-(CH₂)_{r'}-R³⁴ wherein r' and R³⁴ are as defined in the compound (1) of the present invention,
R^{16b}-R¹⁶ⁿ and R^{17b}-R¹⁷ⁿ are as defined in the compound (1) of the present invention,
R¹⁸ is as defined in the compound (1) of the present invention,
Y'' is -S- or -N(R³⁵)- wherein R³⁵ is as defined in the compound (1) of the present invention, and
n is as defined in the compound (1) of the present invention, or
R⁶ and R⁷ may form, together with the adjacent nitrogen atom, 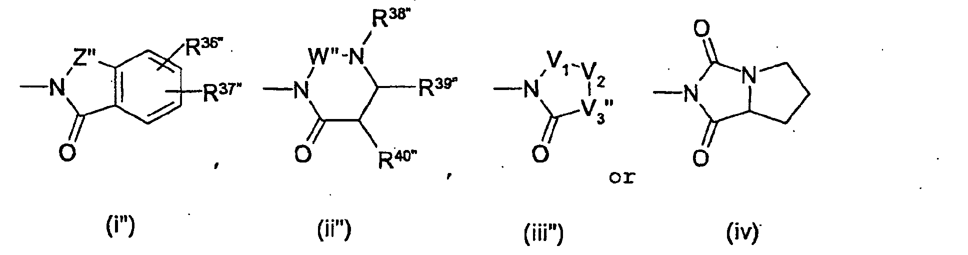 wherein R^{36''} and R^{37''} are the same or different and each is a hydrogen atom, a halogen atom, a C₁₋₆ alkyl group, a C₁₋₆ alkoxy group, an amino group, a hydroxyl group or -O-CO-R⁴¹ wherein R⁴¹ is as defined in the compound (1) of the present invention;
Z'' is -CH₂-CH₂-, -C(R⁴²)=CH-, -N=N-, -CO-, -CO-O-, -CO-CH₂-O-, -CH₂-CO-NH-, -C(R^{42''})(R⁴³)-N(R^{44''})- wherein R⁴², R^{42''} and R⁴³ are as defined in the compound (1) of the present invention and R^{44''} is a hydrogen atom, a C₁₋₆ alkyl group or a C₂₋₇ alkoxycarbonyl group or -C(U)-N(R^{44'''})- wherein U is =O or =S and R^{44'''} is as defined for R^{44''};
R^{38''} is a hydrogen atom or an aryl group optionally having substituents;
R^{39''} and R^{40''} are the same or different and each is a hydrogen atom, a C₁₋₆ alkyl group or a C₂₋₇ alkoxycarbonyl group, or R^{39''} and R^{40''} may form, together with the adjacent carbon atom, 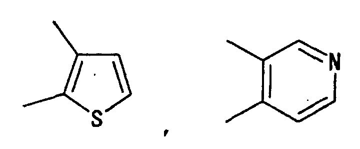 or 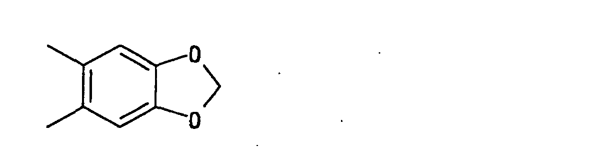
W'' is -CO- or -CH₂-;
V₁ and V₂ are as defined in the compound (1) of the present invention; and
V₃'' is -CH(R^{46''})- or -N(R^{46'''})- wherein R^{46''} and R^{46'''} are the same or different and each is a hydrogen atom or an aryl group optionally having substituents.

Now, various substituents or positions of substitution are described in more detail in the following.

For R¹, it is preferably a hydrogen atom.

For R², it is preferably a hydrogen atom.

For R³, it is preferably a C₁₋₆ alkyl group or a halogen atom, particularly preferably a chlorine atom.

For R⁴, it is preferably a hydrogen atom.

For R⁵, it is preferably a hydrogen atom.

For R⁷, X is preferably =O or -NH, A is a single bond and B forms 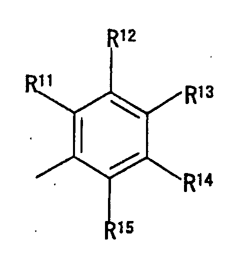 wherein each symbol is as defined above.

For R⁶, it is preferably a hydrogen atom, or R⁶ and R⁷ may form, together with the adjacent nitrogen atom, 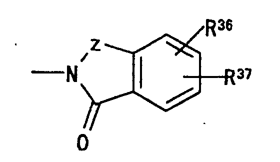 wherein each symbol is as defined above.

Preferable specific examples of the compound (1) of the present invention are shown in the following.
1-1. benzoic acid 2-(5-chloro-1H-indole-2-carbonyl)hydrazide,
1-2. 2-aminobenzoic acid 2-(5-chloro-1H-indole-2-carbonyl)hydrazide,
1-3. 2-hydroxybenzoic acid 2-(5-chloro-1H-indole-2-carbonyl)hydrazide,
1-4. 3-(2-(2-(5-chloro-1H-indole-2-carbonyl)hydrazinocarbonyl)phenylcarbamoyloxy)-2,2-dimethylpropionic acid,
1-5. benzoic acid 2-(5-chloro-1H-indole-2-carbonyl)-1-methylhydrazide,
1-6. benzoic acid 2-(1-acetyl-5-chloro-1H-indole-2-carbonyl)hydrazide,
1-7. 5-chloro-1H-indole-2-carboxylic acid 2-(imino-phenyl-methyl)hydrazide,
1-8. 5-aminothiazole-4-carboxylic acid 2-(5-chloro-1H-indole-2-carbonyl)hydrazide,
1-9. benzoic acid 2-(5-fluoro-1H-indole-2-carbonyl)hydrazide,
1-10.cyclohexanecarboxylic acid 2-(5-fluoro-1H-indole-2-carbonyl)hydrazide,
1-11.thiophene-2-carboxylic acid 2-(5-fluoro-1H-indole-2-carbonyl)hydrazide,
1-12. 4-nitrobenzoic acid 2-(5-chloro-1H-indole-2-carbonyl)hydrazide,
1-13.2-methylbenzoic acid 2-(5-chloro-1H-indole-2-carbonyl)hydrazide,
1-14.4-methylbenzoic acid 2-(5-chloro-1H-indole-2-carbonyl)hydrazide,
1-15.2-methoxybenzoic acid 2-(5-chloro-1H-indole-2-carbonyl)hydrazide,
1-16.3-methoxybenzoic acid 2-(5-chloro-1H-indole-2-carbonyl)hydrazide,
1-17.4-methoxybenzoic acid 2-(5-chloro-1H-indole-2-carbonyl)hydrazide,
1-18.3-methylbenzoic acid 2-(5-chloro-1H-indole-2-carbonyl)hydrazide,
1-19.2-chlorobenzoic acid 2-(5-chloro-1H-indole-2-carbonyl)hydrazide,
1-20.3-chlorobenzoic acid 2-(5-chloro-1H-indole-2-carbonyl)hydrazide,
1-21.4-chlorobenzoic acid 2-(5-chloro-1H-indole-2-carbonyl)hydrazide,
1-22.4-(2-(5-chloro-1H-indole-2-carbonyl)hydrazinocarbonyl)benzoic acid methyl ester,
1-23.cyclohexanecarboxylic acid 2-(5-chloro-1H-indole-2-carbonyl)hydrazide,
1-24.2,4-dichlorobenzoic acid 2-(5-chloro-1H-indole-2-carbonyl)hydrazide,
1-25.2,6-dichlorobenzoic acid 2-(5-chloro-1H-indole-2-carbonyl)hydrazide,
1-26.2,4-difluorobenzoic acid 2-(5-chloro-1H-indole-2-carbonyl)hydrazide,
1-27.biphenyl-2-carboxylic acid 2-(5-chloro-1H-indole-2-carbonyl)hydrazide,
1-28.3-fluorobenzoic acid 2-(5-chloro-1H-indole-2-carbonyl)hydrazide,
1-29.4-fluorobenzoic acid 2-(5-chloro-1H-indole-2-carbonyl)hydrazide,
1-30.3-trifluoromethylbenzoic acid 2-(5-chloro-1H-indole-2-carbonyl)hydrazide,
1-31.4-trifluoromethylbenzoic acid 2-(5-chloro-1H-indole-2-carbonyl)hydrazide,
1-32.2-trifluoromethylbenzoic acid 2-(5-chloro-1H-indole-2-carbonyl)hydrazide,
1-33.benzoic acid 2-(5-methyl-1H-indole-2-carbonyl)hydrazide,
1-34.benzoic acid 2-(5-chloro-3-methyl-1H-indole-2-carbonyl)hydrazide,
1-35.benzoic acid 2-(5,7-dichloro-1H-indole-2-carbonyl)hydrazide,
1-36.2-aminobenzoic acid 2-(5-isopropyl-1H-indole-2-carbonyl)hydrazide,
1-37.2-amino-4-fluorobenzoic acid 2-(5-isopropyl-1H-indole-2-carbonyl)hydrazide,
1-38.2-aminobenzoic acid 2-(5-fluoro-1H-indole-2-carbonyl)hydrazide,
1-39.2-aminobenzoic acid 2-(5-chloro-1H-indole-2-carbonyl)hydrazide,
1-40.3-amino-4-(2-(5-chloro-1H-indole-2-carbonyl)hydrazinocarbonyl)benzoic acid methyl ester,
1-41.3-aminoisonicotinic acid 2-(5-chloro-1H-indole-2-carbonyl)hydrazide,
1-42.isonicotinic acid 2-(5-chloro-1H-indole-2-carbonyl)hydrazide,
1-43.nicotinic acid 2-(5-chloro-1H-indole-2-carbonyl)hydrazide,
1-44. pyridine-2-carboxylic acid 2-(5-chloro-1H-indole-2-carbonyl)hydrazide,
1-45.3-(dimethylamino)benzoic acid 2-(5-chloro-1H-indole-2-carbonyl)hydrazide,
1-46.N*-*(3-(2-(5-chloro-1H-indole-2-carbonyl)hydrazinocarbonyl)phenyl)acetamide,
1-47.N-(2-(2-(5-chloro-1H-indole-2-carbonyl)hydrazinocarbonyl)phenyl)acetamide,
1-48.4-fluorobenzoic acid 2-(5-chloro-1H-indole-2-carbonyl)-2-methylhydrazide,
1-49.2-(2-(2-(5-chloro-1H-indole-2-carbonyl)hydrazinocarbonyl)phenoxy)acetic acid methyl ester,
1-50.2-(2-(2-(5-chloro-1H-indole-2-carbonyl)hydrazinocarbonyl)phenoxy)acetic acid,
1-51.2-(dimethylamino)benzoic acid 2-(5-chloro-1H-indole-2-carbonyl)hydrazide,
1-52.2-(2-(5-chloro-1H-indole-2-carbonyl)hydrazinocarbonyl)phenoxy-N,N-dimethylacetamide,
1-53.2-methylaminobenzoic acid 2-(5-chloro-1H-indole-2-carbonyl)hydrazide,
1-54.2-amino-4-chlorobenzoic acid 2-(5-chloro-1H-indole-2-carbonyl)hydrazide,
1-55.2-amino-6-chlorobenzoic acid 2-(5-chloro-1H-indole-2-carbonyl)hydrazide,
1-56.2-amino-3-chlorobenzoic acid 2-(5-chloro-1H-indole-2-carbonyl)hydrazide,
1-57.2-amino-5-chlorobenzoic acid 2-(5-chloro-1H-indole-2-carbonyl)hydrazide,
1-58.4-cyanobenzoic acid 2-(5-chloro-1H-indole-2-carbonyl)hydrazide,
1-59.4-(1H-tetrazol-5-yl)benzoic acid 2-(5-chloro-1H-indole-2-carbonyl)hydrazide,
1-60.3-(1H-tetrazol-5-yl)benzoic acid 2-(5-chloro-1H-indole-2-carbonyl)hydrazide,
1-61.2-(2-(2-(5-chloro-1H-indole-2-carbonyl)hydrazinocarbonyl)anilino)acetic acid,
1-62.2-aminobenzoic acid 2-(5-methyl-1H-indole-2-carbonyl)hydrazide,
1-63.2-amino-4-fluorobenzoic acid 2-(5-methyl-1H-indole-2-carbonyl)hydrazide,
1-64.methyl (2-(2-(5-methyl-1H-indole-2-carbonyl)hydrazinocarbonyl)phenyl)carbamate,
1-65.2-(2-(5-chloro-1H-indole-2-carbonyl)hydrazinocarbonyl)phenyl dimethylcarbamate,
1-66.2-aminobenzoic acid 2-(5-ethyl-1H-indole-2-carbonyl)hydrazide,
1-67.2-amino-4,5-difluorobenzoic acid 2-(5-methyl-1H-indole-2-carbonyl)hydrazide,
1-68.2-(2-hydroxyethoxy)benzoic acid 2-(5-chloro-1H-indole-2-carbonyl)hydrazide,
1-69.2-(3-(2-(5-chloro-1H-indole-2-carbonyl)hydrazinocarbonyl)phenoxy)acetic acid methyl ester,
1-70.2-(3-(2-(5-chloro-1H-indole-2-carbonyl)hydrazinocarbonyl)phenoxy)acetic acid,
1-71.2-(3-(2-(5-chloro-1H-indole-2-carbonyl)hydrazinocarbonyl)phenoxy)-N,N-dimethylacetamide,
1-72. 2-methylthiazole-4-carboxylic acid 2-(5-chloro-1H-indole-2-carbonyl)hydrazide,
1-73. 4-(2H-[1,2,4]triazol-3-yl)benzoic acid 2-(5-chloro-1H-indole-2-carbonyl)hydrazide,
1-74.3-(2H-[1,2,4]triazol-3-yl)benzoic acid 2-(5-chloro-1H-indole-2-carbonyl)hydrazide,
1-75.1,3-dihydroxy-2-propyl (2-(2-(5-chloro-1H-indole-2-carbonyl)hydrazinocarbonyl)phenyl)carbamate,
1-76.3-((2-(2-(5-chloro-1H-indole-2-carbonyl)hydrazinocarbonyl)phenyl)carbamoyloxy)-2,2-dimethylpropionic acid,
1-77.thiophene-2-carboxylic acid 2-(5-chloro-1H-indole-2-carbonyl)hydrazide,
1-78.furan-2-carboxylic acid 2-(5-chloro-1H-indole-2-carbonyl)hydrazide,
1-79.2,6-dichloronicotinic acid 2-(5-chloro-1H-indole-2-carbonyl)hydrazide,
1-80.1H-pyrrole-2-carboxylic acid 2-(5-chloro-1H-indole-2-carbonyl)hydrazide,
1-81.1H-imidazole-4-carboxylic acid 2-(5-chloro-1H-indole-2-carbonyl)hydrazide,
1-82.pyrazine-2-carboxylic acid 2-(5-chloro-1H-indole-2-carbonyl)hydrazide,
1-83.thiophene-3-carboxylic acid 2-(5-chloro-1H-indole-2-carbonyl)hydrazide,
1-84.furan-3-carboxylic acid 2-(5-chloro-1H-indole-2-carbonyl)hydrazide,
1-85.5-chlorothiophene-2-carboxylic acid 2-(5-chloro-1H-indole-2-carbonyl)hydrazide,
1-86.3-chlorothiophene-2-carboxylic acid 2-(5-chloro-1H-indole-2-carbonyl)hydrazide,
1-87.1-methyl-1H-pyrrole-2-carboxylic acid 2-(5-chloro-1H-indole-2-carbonyl)hydrazide,
1-88.5-methylthiophene-2-carboxylic acid 2-(5-chloro-1H-indole-2-carbonyl)hydrazide,
1-89.3-methylthiophene-2-carboxylic acid 2-(5-chloro-1H-indole-2-carbonyl)hydrazide,
1-90.2,6-difluorobenzoic acid 2-(5-chloro-1H-indole-2-carbonyl)hydrazide,
1-91.2,3-difluorobenzoic acid 2-(5-chloro-1H-indole-2-carbonyl)hydrazide,
1-92.5-chloro-1H-indole-2-carboxylic acid 2-(naphthalene-1-carbonyl)hydrazide,
1-93.3,4,5-trifluorobenzoic acid 2-(5-chloro-1H-indole-2-carbonyl)hydrazide,
1-94.2,3,4,5-tetrafluorobenzoic acid 2-(5-chloro-1H-indole-2-carbonyl)hydrazide,
1-95.2-amino-4-methylbenzoic acid 2-(5-chloro-1H-indole-2-carbonyl)hydrazide,
1-96.2-amino-4-fluorobenzoic acid 2-(5-chloro-1H-indole-2-carbonyl)hydrazide,
1-97.2-amino-5-methylbenzoic acid 2-(5-chloro-1H-indole-2-carbonyl)hydrazide,
1-98.2-amino-6-methylbenzoic acid 2-(5-chloro-1H-indole-2-carbonyl)hydrazide,
1-99.2-amino-3-methylbenzoic acid 2-(5-chloro-1H-indole-2-carbonyl)hydrazide,
1-100. 2-amino-4,5-difluorobenzoic acid 2-(5-chloro-1H-indole-2-carbonyl)hydrazide,
1-101. 3-aminothiophene-2-carboxylic acid 2-(5-chloro-1H-indole-2-carbonyl)hydrazide,
1-102. 2-aminobenzoic acid 2-(5-bromo-1H-indole-2-carbonyl)hydrazide,
1-103. 2-amino-4-fluorobenzoic acid 2-(5-bromo-1H-indole-2-carbonyl)hydrazide,
1-104. 1H-pyrazole-4-carboxylic acid 2-(5-chloro-1H-indole-2-carbonyl)hydrazide,
1-105. methyl (2-(2-(5-fluoro-1H-indole-2-carbonyl)hydrazinocarbonyl)phenyl)carbamate,
1-106. 1-methyl-1H-pyrrole-2-carboxylic acid 2-(5-methyl-1H-indole-2-carbonyl)hydrazide,
1-107. thiophene-3-carboxylic acid 2-(5-methyl-1H-indole-2-carbonyl)hydrazide,
1-108. 4H-thieno[3,2-b]pyrrole-5-carboxylic acid 2-(5-chloro-1H-indole-2-carbonyl)hydrazide,
1-109. phenyl (2-(2-(5-chloro-1H-indole-2-carbonyl)hydrazinocarbonyl)phenyl)carbamate,
1-110. benzyl (2-(2-(5-chloro-1H-indole-2-carbonyl)hydrazinocarbonyl)phenyl)carbamate,
1-111. 2-hydroxyethyl (2-(2-(5-chloro-1H-indole-2-carbonyl)hydrazinocarbonyl)phenyl)carbamate,
1-112. 3-hydroxypropyl (2-(2-(5-chloro-1H-indole-2-carbonyl)hydrazinocarbonyl)phenyl)carbamate,
1-113. 2-((2-(2-(5-chloro-1H-indole-2-carbonyl)hydrazinocarbonyl)phenyl)carbamoyloxy)acetic acid,
1-114. 2-((2-(2-(5-chloro-1H-indole-2-carbonyl)hydrazinocarbonyl)phenyl)carbamoyloxymethyl)-2-methylmalonic acid,
1-115. methyl 2-(2-(5-chloro-1H-indole-2-carbonyl)hydrazinocarbonyl)phenylcarbamate,
1-116. cyclohexanecarboxylic acid 2-(5-chloro-1H-indole-2-carbonyl)-1-methylhydrazide,
1-117. thiophene-2-carboxylic acid 2-(5-chloro-1H-indole-2-carbonyl)-1-methylhydrazide,
1-118. benzoic acid 2-(1H-indole-2-carbonyl)hydrazide,
1-119. benzoic acid 2-(5-chloro-1-methyl-1H-indole-2-carbonyl)hydrazide,
1-120. benzoic acid 2-(5-methoxy-1H-indole-2-carbonyl)hydrazide,
1-121. benzoic acid 2-(5-isopropyl-1H-indole-2-carbonyl)hydrazide,
1-122. benzoic acid 2-(5-nitro-1H-indole-2-carbonyl)hydrazide,
1-123. benzoic acid 2-(5-benzyloxy-1H-indole-2-carbonyl)hydrazide,
1-124. benzoic acid 2-(6-chloro-1H-indole-2-carbonyl)hydrazide,
1-125. 6H-thieno[2,3-b]pyrrole-5-carboxylic acid 2-(5-chloro-1H-indole-2-carbonyl)hydrazide,
1-126. 5-chloro-1H-indole-2-carboxylic acid 2-((2-fluorophenyl)-imino-methyl)hydrazide,
1-127. 5-chloro-1H-indole-2-carboxylic acid 2-((3-fluorophenyl)-imino-methyl)hydrazide,
1-128. 5-chloro-1H-indole-2-carboxylic acid 2-((4-fluorophenyl)-imino-methyl)hydrazide,
1-129. 5-chloro-1H-indole-2-carboxylic acid 2-(imino-(p-tolyl)-methyl)hydrazide,
1-130. 5-chloro-1H-indole-2-carboxylic acid 2-((4-chlorophenyl)-imino-methyl)hydrazide,
1-131. 5-chloro-1H-indole-2-carboxylic acid 2-((3-chlorophenyl)-imino-methyl)hydrazide,
1-132. 5-chloro-1H-indole-2-carboxylic acid 2-((3-fluorophenyl)-imino-methyl)hydrazide,
1-133. 5-chloro-1H-indole-2-carboxylic acid 2-(imino-(o-tolyl)-methyl)hydrazide,
1-134. 5-chloro-1H-indole-2-carboxylic acid 2-(imino-(m-tolyl)-methyl)hydrazide,
1-135. 5-chloro-1H-indole-2-carboxylic acid 2-(imino-(thiophen-2-yl)-methyl)hydrazide,
1-136. 5-chloro-1H-indole-2-carboxylic acid 2-(imino-(pyridin-2-yl)-methyl)hydrazide,
1-137. 5-chloro-1H-indole-2-carboxylic acid 2-((furan-2-yl)-imino-methyl)hydrazide,
1-138. 5-chloro-1H-indole-2-carboxylic acid 2-((2-chloro-6-fluorophenyl)-imino-methyl)hydrazide,
1-139. 5-chloro-1H-indole-2-carboxylic acid 2-(imino-(2-trifluoromethylphenyl)-methyl)hydrazide,
1-140. 5-chloro-1H-indole-2-carboxylic acid 2-(imino-(pyrazin-2-yl)-methyl)hydrazide,
1-141. 3-aminobenzoic acid 2-(5-chloro-1H-indole-2-carbonyl)hydrazide,
1-142. 3-methoxybenzoic acid 2-(5-chloro-lH-indole-2-carbonyl)hydrazide,
1-143. 5-amino-2-methylthiazole-4-carboxylic acid 2-(5-chloro-1H-indple-2-carbonyl)hydrazide,
2-1. 5-chloro-1H-indole-2-carboxylic acid (1,2,3,4-tetrahydro-2,4-dioxoquinazolin-3-yl)amide,
2-2. 5-chloro-1H-indole-2-carboxylic acid (2,3-dihydro-2,4-dioxo-4H-benzo[e][1,3]oxazin-3-yl)amide,
2-3. 5-chloro-1H-indole-2-carboxylic acid (1,2,3,4-tetrahydro-4-oxo-2-thioxoquinazolin-3-yl)amide,
2-4. 5-chloro-1H-indole-2-carboxylic acid (3,4-dihydro-2-methyl-4-oxoquinazolin-3-yl)amide,
2-5. 5-chloro-1H-indole-2-carboxylic acid (3,4-dihydro-4-oxoquinazolin-3-yl)amide,
2-6. 5-chloro-1H-indole-2-carboxylic acid (1,2,3,4-tetrahydro-4-oxoquinazolin-3-yl) amide,
2-7. 5-chloro-1H-indole-2-carboxylic acid (1,2,3,4-tetrahydro-2,5-dioxo-5H-benzo[e][1,4]diazepin-4-yl)amide,
2-8. 5-chloro-1H-indole-2-carboxylic acid (2,3,4,5-tetrahydro-3,5-dioxo-benzo[f][1,4]oxazepin-4-yl)amide,
2-9. 5-isopropyl-1H-indole-2-carboxylic acid (1,2,3,4-tetrahydro-2,4-dioxoquinazolin-3-yl)amide,
2-10.5-isopropyl-1H-indole-2-carboxylic acid (7-fluoro-1,2,3,4-tetrahydro-2,4-dioxoquinazolin-3-yl)amide,
2-11.5-fluoro-1H-indole-2-carboxylic acid (1,2,3,4-tetrahydro-2,4-dioxoquinazolin-3-yl)amide,
2-12.6-chloro-1H-indole-2-carboxylic acid (1,2,3,4-tetrahydro-2,4-dioxoquinazolin-3-yl)amide,
2-13.3-((5-chloro-1H-indole-2-carbonyl)amino)-1,2,3,4-tetrahydro-2,4-dioxoquinazoline-7-carboxylic acid methyl ester,
2-14.3-((5-chloro-1H-indole-2-carbonyl)amino)-1,2,3,4-tetrahydro-2,4-dioxoquinazoline-7-carboxylic acid,
2-15.5-chloro-1H-indole-2-carboxylic acid (1,2,3,4-tetrahydro-2,4-dioxo-6-(trifluoroacetylamino)quinazolin-3-yl)amide,
2-16.5-chloro-1H-indole-2-carboxylic acid (6-amino-1,2,3,4-tetrahydro-2,4-dioxoquinazolin-3-yl)amide,
2-17.5-chloro-1H-indole-2-carboxylic acid (5-chloro-1,2,3,4-tetrahydro-2,4-dioxoquinazolin-3-yl)amide,
2-18.5-chloro-1H-indole-2-carboxylic acid (6-chloro-1,2,3,4-tetrahydro-2,4-dioxoquinazolin-3-yl)amide,
2-19.5-chloro-1H-indole-2-carboxylic acid (7-chloro-1,2,3,4-tetrahydro-2,4-dioxoquinazolin-3-yl)amide,
2-20.5-chloro-1H-indole-2-carboxylic acid (8-chloro-1,2,3,4-tetrahydro-2,4-dioxoquinazolin-3-yl)amide,
2-21.2-(3-((5-chloro-1H-indole-2-carbonyl)ainino)-1,2,3,4-tetrahydro-2,4-dioxoquinazolin-1-yl)acetic acid,
2-22. 2-(3-((5-chloro-1H-indole-2-carbonyl)amino)-1,2,3,4-tetrahydro-2,4-dioxoquinazolin-1-yl)acetic acid methyl ester,
2-23.5-methyl-1H-indole-2-carboxylic acid (1,2,3,4-tetrahydro-2,4-dioxoquinazolin-3-yl)amide,
2-24.5-methyl-1H-indole-2-carboxylic acid (7-fluoro-1,2,3,4-tetrahydro-2,4-dioxoquinazolin-3-yl)amide,
2-25.5-ethyl-1H-indole-2-carboxylic acid (1,2,3,4-tetrahydro-2,4-dioxoquinazolin-3-yl)amide,
2-26.5-methyl-1H-indole-2-carboxylic acid (6,7-difluoro-1,2,3,4-tetrahydro-2,4-dioxoquinazolin-3-yl)amide,
2-27.5-methyl-1H-indole-2-carboxylic acid (1,2,3,4-tetrahydro-6-methoxy-2,4-dioxoquinazolin-3-yl)amide,
2-28.5-methyl-1H-indole-2-carboxylic acid (1,2,3,4-tetrahydro-6-hydroxy-2,4-dioxoquinazolin-3-yl)amide,
2-29.acetic acid 3-((5-methyl-1H-indole-2-carbonyl)amino)-1,2,3,4-tetrahydro-2,4-dioxoquinazolin-6-yl ester,
2-30. 5-chloro-1H-indole-2-carboxylic acid (1,2,3,4-tetrahydro-2,4-dioxo-1-propylquinazolin-3-yl)amide,
2-31.5-chloro-1H-indole-2-carboxylic acid (1,2,3,4-tetrahydro-1-methyl-2,4-dioxoquinazolin-3-yl)amide,
2-32.N-(1,2,3,4-tetrahydro-7-nitro-2,4-dioxoquinazolin-3-yl)-5-chloro-1H-indole-2-carboxylic acid amide,
3-1. 5-chloro-1H-indole-2-carboxylic acid (2,4-dioxoperhydropyrimidin-3-yl)amide,
3-2. 5-chloro-1H-indole-2-carboxylic acid (4-oxo-2-thioxoperhydropyrimidin-3-yl)amide,
3-3. 5-chloro-1H-indole-2-carboxylic acid (2,4-dioxo-1-phenylperhydropyrimidin-3-yl)amide,
3-4. 5-chloro-1H-indole-2-carboxylic acid (4-oxo-1-phenylperhydropyrimidin-3-yl)amide,
3-5. 5-chloro-1H-indole-2-carboxylic acid (1-(4-fluorophenyl)-2,4-dioxoperhydropyrimidin-3-yl)amide,
3-6. 5-chloro-1H-indole-2-carboxylic acid (2,4-dioxo-1-(pyridin-2-yl)perhydropyrimidin-3-yl)amide,
3-7. 5-chloro-1H-indole-2-carboxylic acid (1-(3-fluorophenyl)-2,4-dioxoperhydropyrimidin-3-yl)amide,
3-8. 5-chloro-1H-indole-2-carboxylic acid (1-(2-fluorophenyl)-2,4-dioxoperhydropyrimidin-3-yl)amide,
3-9. 5-fluoro-1H-indole-2-carboxylic acid (2,4-dioxo-1-phenyl-perhydropyrimidin-3-yl)amide,
3-10.5-methyl-1H-indole-2-carboxylic acid (2,4-dioxo-1-phenyl-perhydropyrimidin-3-yl)amide,
3-11.5-chloro-1H-indole-2-carboxylic acid (1-(3-chlorophenyl)-2,4-dioxoperhydropyrimidin-3-yl)amide,
3-12. 5-chloro-1H-indole-2-carboxylic acid (2,4-dioxo-1-(m-tolyl)perhydropyrimidin-3-yl)amide,
3-13.5-chloro-1H-indole-2-carboxylic acid (2,4-dioxo-1-(p-tolyl)perhydropyrimidin-3-yl)amide,
3-14.5-chloro-1H-indole-2-carboxylic acid (1-(4-chlorophenyl)-2,4-dioxoperhydropyrimidin-3-yl)amide,
3-15. 5-chloro-1H-indole-2-carboxylic acid (2,4-dioxo-1-(o-tolyl)perhydropyrimidin-3-yl)amide,
4-1. 5-chloro-1H-indole-2-carboxylic acid ((4S)-2,5-dioxo-4-phenylimidazolidin-1-yl) amide,
4-2. 5-chloro-1H-indole-2-carboxylic acid (2,4-dioxo-1-phenylimidazolidin-3-yl) amide,
4-3. 5-chloro-1H-indole-2-carboxylic acid (4-oxo-1-phenyl-2-thioxoimidazolidin-3-yl) amide,
4-4. 5-chloro-1H-indole-2-carboxylic acid (4-oxo-1-phenylimidazolidin-3-yl) amide,
4-5. 5-chloro-1H-indole-2-carboxylic acid (2-oxo-1-phenylimidazolidin-3-yl) amide,
4-6. 5-chloro-1H-indole-2-carboxylic acid ((4R)-2,5-dioxo-4-phenylimidazolidin-1-yl)amide,
4-7. 5-chloro-1H-indole-2-carboxylic acid ((4S)-1,3-dioxo-perhydropyrrolo[1,2-c]imidazol-2-yl)amide,
4-8. 5-chloro-1H-indole-2-carboxylic acid ((4R)-1,3-dioxo-perhydropyrrolo[1,2-c]imidazol-2-yl)amide,
4-9. 5-chloro-1H-indole-2-carboxylic acid ((4S)-4-benzyl-2,5-dioxoimidazolidin-1-yl)amide,
4-10. 5-chloro-1H-indole-2-carboxylic acid ((4R)-4-benzyl-2,5-dioxoimidazolidin-1-yl)amide,
4-11. 5-chloro-1H-indole-2-carboxylic acid (2,4-dioxoimidazolidin-3-yl)amide,
4-12. 5-chloro-1H-indole-2-carboxylic acid (1-methyl-2,5-dioxo-4-phenylimidazolidin-1-yl)amide,
4-13. 5-chloro-1H-indole-2-carboxylic acid (2,4-dioxo-1-(4-fluorophenyl)imidazolidin-3-yl)amide,
4-14. 5-chloro-1H-indole-2-carboxylic acid (2,5-dioxo-4-(2-fluorophenyl)imidazolidin-1-yl)amide,
4-15. 5-chloro-1H-indole-2-carboxylic acid (2,5-dioxo-4-(2-thienyl)imidazolidin-1-yl)amide,
4-16. 5-chloro-1H-indole-2-carboxylic acid (2,5-dioxo-4-(4-fluorophenyl)imidazolidin-1-yl)amide,
4-17. 5-chloro-1H-indole-2-carboxylic acid (2,5-dioxo-4-(4-chlorophenyl)imidazolidin-1-yl)amide,
4-18. 5-chloro-1H-indole-2-carboxylic acid ((4S)-2,5-dioxo-4-(4-hydroxyphenyl)imidazolidin-1-yl)amide,
4-19. 5-chloro-1H-indole-2-carboxylic acid ((4S)-2,5-dioxo-4-(4-methoxyphenyl)imidazolidin-1-yl)amide,
4-20. 5-chloro-1H-indole-2-carboxylic acid ((4R)-2,5-dioxo-4-(4-methoxyphenyl)imidazolidin-1-yl)amide,
5-1. 5-chloro-1H-indole-2-carboxylic acid 2-(anilinocarbonyl)hydrazide,
5-2. 5-chloro-1H-indole-2-carboxylic acid 2-(phenylthiocarbonyl)hydrazide,
5-3. 5-chloro-1H-indole-2-carboxylic acid 2-(2-phenylacetyl)hydrazide,
5-4. 5-chloro-1H-indole-2-carboxylic acid 2-(2-oxo-2-phenylacetyl)hydrazide,
5-5. 5-chloro-1H-indole-2-carboxylic acid 2-((2-fluorophenyl)aminocarbonyl)hydrazide,
5-6. 5-chloro-1H-indble-2-carboxylic acid 2-((3-fluorophenyl)aminocarbonyl)hydrazide,
5-7. 5-chloro-1H-indole-2-carboxylic acid 2-((4-fluorophenyl)aminocarbonyl)hydrazide,
5-8. 5-chloro-1H-indole-2-carboxylic acid 2-(anilinocarbonyl)-2-methylhydrazide,
5-9. 5-chloro-1H-indole-2-carboxylic acid 2-((2-chloroanilino)carbonyl)hydrazide,
5-10. 5-chloro-1H-indole-2-carboxylic acid 2-((3-chloroanilino)carbonyl)hydrazide,
5-11. 5-chloro-1H-indole-2-carboxylic acid 2-((4-chloroanilino)carbonyl)hydrazide,
5-12. 5-chloro-1H-indole-2-carboxylic acid 2-((1-phenylcyclopropane)carbonyl)hydrazide,
5-13. 5-chloro-1H-indole-2-carboxylic acid 2-((1-phenylcyclopentane)carbonyl)hydrazide,
5-14. 5-chloro-1H-indole-2-carboxylic acid 2-((1-phenylcyclohexane) carbonyl) hydrazide,
5-15. 5-chloro-1H-indole-2-carboxylic acid 2-(2-phenylpropanoyl)hydrazide,
5-16. 5-chloro-1H-indole-2-carboxylic acid 2-(3-hydroxy-2-phenylpropanoyl)hydrazide,
5-17. 5-chloro-1H-indole-2-carboxylic acid 2-(2-methyl-2-phenylpropanoyl)hydrazide,
5-18. 5-chloro-1H-indole-2-carboxylic acid 2-((2S)-2-amino-2-phenylacetyl)hydrazide,
5-19. N-(2-(2-(5-chloro-1H-indole-2-carbonyl)hydrazino)-2-oxo-1-phenylethyl)acetamide,
6-1. 2-morpholinoethyl (2-((2-(5-chloro-1H-indole-2-carbonyl)hydrazino)carbonyl)phenyl)carbamate p-toluenesulfonate,
6-2. 2-amino-4,5-difluorobenzoic acid 2-(5-methyl-1H-indole-2-carbonyl)hydrazide benzenesulfonate,
6-3. 2-amino-4-fluorobenzoic acid 2-(5-chloro-1H-indole-2-carbonyl)hydrazide benzenesulfonate,
6-4. 3-(dimethylamino)benzoic acid 2-(5-chloro-1H-indole-2-carbonyl)hydrazide methanesulfonate,
6-5. 2-amino-4-fluorobenzoic acid 2-(5-chloro-1H-indole-2-carbonyl)hydrazide hydrochloride,
6-6. 2-amino-4-fluorobenzoic acid 2-(5-chloro-1H-indole-2-carbonyl)hydrazide p-toluenesulfonate,
6-7. 2-amino-4-fluorobenzoic acid 2-(5-chloro-1H-indole-2-carbonyl)hydrazide p-toluenesulfonate,
6-8. 2-amino-4-fluorobenzoic acid 2-(5-chloro-1H-indole-2-carbonyl)hydrazide p-toluenesulfonate,
6-9. 2-amino-4-fluorobenzoic acid 2-(5-chloro-1H-indole-2-carbonyl)hydrazide p-toluenesulfonate,
6-10. 2-aminobenzoic acid 2-(5-methyl-1H-indole-2-carbonyl)hydrazide p-toluenesulfonate,
6-11. 2-aminobenzoic acid 2-(5-methyl-1H-indole-2-carbonyl)hydrazide benzenesulfonate,
6-12. 2-amino-4-fluorobenzoic acid 2-(5-methyl-lH-indole-2-carbonyl)hydrazide benzenesulfonate,
6-13. 3-aminobenzoic acid 2-(5-chloro-1H-indole-2-carbonyl)hydrazide p-toluenesulfonate,
6-14. 5-chloro-1H-indole-2-carboxylic acid 2-((2-chlorophenyl)-imino-methyl)hydrazide methanesulfonate,
6-15. 5-chloro-1H-indole-2-carboxylic acid 2-((2-chlorophenyl)-imino-methyl)hydrazide p-toluenesulfonate,
6-16. 5-chloro-1H-indole-2-carboxylic acid 2-((2-chlorophenyl)-imino-methyl)hydrazide hydrochloride,
6-17. 5-chloro-1H-indole-2-carboxylic acid 2-(imino-phenyl-methyl)hydrazide methanesulfonate,
6-18. 5-chloro-1H-indole-2-carboxylic acid 2-((2-chlorophenyl)-imino-methyl)hydrazide butenedioic acid salt,
6-19. 5-chloro-lH-indole-2-carboxylic acid 2-((2-fluorophenyl)-imino-methyl)hydrazide hydrochloride,
6-20. 5-chloro-1H-indole-2-carboxylic acid 2-((2-fluorophenyl)-imino-methyl)hydrazide methanesulfonate,
6-21. 5-chloro-1H-indole-2-carboxylic acid 2-((1-imino-2-phenylethyl)hydrazide methanesulfonate,
6-22. 5-chloro-1H-indole-2-carboxylic acid 2-((3-fluorophenyl)-imino-methyl)hydrazide hydrochloride,
6-23. 5-chloro-1H-indole-2-carboxylic acid 2-((3,4-difluorophenyl)-imino-methyl)hydrazide methanesulfonate,
6-24. 5-chloro-1H-indole-2-carboxylic acid 2-(imino-(2-methoxyphenyl)-methyl)hydrazide methanesulfonate,
6-25. 5-chloro-1H-indole-2-carboxylic acid 2-((2,6-difluorophenyl)-imino-methyl)hydrazide methanesulfonate,
6-26. 5-chloro-1H-indole-2-carboxylic acid 2-((2,4-difluorophenyl)-imino-methyl)hydrazide methanesulfonate,
6-27. 5-chloro-1H-indole-2-carboxylic acid 2-((1,2-dimethyl-1H-pyrrol-5-yl)-imino-methyl)hydrazide methanesulfonate,
6-28. 2-aminobenzoic acid 2-(5-methyl-1H-indole-2-carbonyl)hydrazide p-toluenesulfonate,
6-29. 2-aminobenzoic acid 2-(5-methyl-1H-indole-2-carbonyl)hydrazide benzenesulfonate,
6-30. 2-amino-4-fluorobenzoic acid 2-(5-methyl-1H-indole-2-carbonyl)hydrazide benzenesulfonate,
6-31. 2-(dimethylamino)benzoic acid 2-(5-chloro-1H-indole-2-carbonyl)hydrazide p-toluenesulfonate,
6-32. 2-amino-4-fluorobenzoic acid 2-(5-chloro-1H-indole-2-carbonyl)hydrazide methanesulfonate,
6-33. 2-aminobenzoic acid 2-(5-bromo-1H-indole-2-carbonyl)hydrazide methanesulfonate,
6-34. 2-amino-4-fluorobenzoic acid 2-(5-methyl-1H-indole-2-carbonyl)hydrazide p-toluenesulfonate, and
6-35. 2-amino-4-fluorobenzoic acid 2-(5-methyl-1H-indole-2-carbonyl)hydrazide methanesulfonate.

The number placed on the left end corresponds to Example No. (1-1, 2-1, 3-1, 4-1, 5-1 and 6-1 correspond to Example 1, 2, 3, 4, 5 and 6, respectively). Of these, preferred are the compounds of the above-mentioned 1-1 - 1-143 and 6-1 - 6-35, and more preferred are the compounds of the above-mentioned 6-1 - 6-35.

Here, the compound (1) of the present invention may be a hydrate or a solvate, and encompasses a prodrug thereof and a metabolite thereof.

A "prodrug" of a compound is a derivative of the compound (1) of the present invention, which has a chemically or metabolically decomposable group, and is decomposed by hydrolysis or solvolysis, or under physiological conditions to show pharmaceutical activity. For example, a compound wherein a hydroxyl group is substituted by -CO-alkyl, -CO₂-alkyl, -CONH-alkyl, -CO-alkenyl, -CO₂-alkenyl, -CONH-alkenyl, -CO-aryl, -CO₂-aryl, -CONH-aryl, -CO-heterocycle, -CO₂-heterocycle, -CONH-heterocycle wherein alkyl, alkenyl, aryl and heterocycle are optionally substituted by halogen atom, alkyl group, hydroxyl group, alkoxy group, carboxy group, amino group, amino acid residue, -PO₃H₂, -SO₃H, -OPO₃H₂, -OSO₃H and the like, -CO-polyethylene glycol residue, -CO₂-polyethylene glycol residue, -CO-polyethylene glycol monoalkyl ether residue, -CO₂-polyethylene glycol monoalkyl ether residue, -PO₃H₂ and the like,
a compound wherein an amino group is substituted by -CO-alkyl, -CO₂-alkyl, -CO-alkenyl, -CO₂-alkenyl, -CO₂-aryl, -CO-aryl, -CO-heterocycle, -CO₂-heterocycle wherein alkyl, alkenyl, aryl and heterocycle are optionally substituted by halogen atom, alkyl group, hydroxyl group, alkoxy group, carboxy group, amino group, amino acid residue, -PO₃H₂, -SO₃H, -OPO₃H₂, -OSO₃H and the like, -CO-polyethylene glycol residue, -CO₂-polyethylene glycol residue, -CO-polyethylene glycol monoalkyl ether residue, -CO₂-polyethylene glycol monoalkyl ether residue, -PO₃H₂ and the like, and
a compound wherein a carboxy group is substituted by alkoxy group, aryloxy group wherein alkoxy group and aryloxy group are optionally substituted by halogen atom, alkyl group, hydroxyl group, alkoxy group, carboxy group, amino group, amino acid residue, -PO₃H₂, -SO₃H, -OPO₃H₂, -OSO₃H and the like, polyethylene glycol residue, polyethylene glycol monoalkyl ether residue and the like can be mentioned.

When the compound of the present invention is to be used as a therapeutic agent for diabetes, it is administered systemically or topically, and orally or parenterally. The dose varies depending on the age, body weight, symptom, treatment effect and the like, but it is generally administered in the range of 10 mg to 1 g per dose for an adult once to several times a day.

To give a preparation of a solid composition and liquid composition for oral administration or injectable solution and the like for parenteral administration, the compound (1) of the present invention can be mixed with a suitable diluent, dispersant, adsorbent, solubilizer and the like.

The compound (1) of the present invention can be used for the treatment or prophylaxis of diabetes in human as well as animals besides human, such as mammals.

The compound (1) of the present invention can be used together with one or more other pharmaceutical agents by a conventional method generally used for pharmaceutical agents. While there are various pharmaceutical agents that can be used together with the compound (1) of the present invention, therapeutic agents for hyperlipidemia and therapeutic agents for diabetes are particularly preferable.

As the therapeutic agents for hyperlipidemia that can be used concurrently, statin agents can be mentioned, which are specifically lovastatin, simvastatin, pravastatin, fluvastatin, atorvastatin, cerivastatin and the like.

Similarly, as the therapeutic agents for diabetes that can be used concurrently, insulin preparations, sulfonylurea agents, insulin secretagogues, sulfonamides, biguanides, α-glucosidase inhibitors, insulin sensitizers and the like can be mentioned, which are as follows. As the insulin preparation, for example, insulin and the like, as the sulfonylurea agent, glibenclamide, torbutamide, glyclopyramide, acetohexamide, glimepiride, tolazamide, gliclazide, nateglinide and the like, as the sulfonamide, glybuzole and the like, as the biguanide, metformin hydrochloride, buformin hydrochloride and the like, as the α-glucosidase inhibitor, voglibose, acarbose and the like, and as the insulin sensitizer, pioglitazone hydrochloride and the like can be mentioned.

The present inventors have also found that a combined use with an HLGPa inhibitor that has not been used concurrently with the therapeutic agents for diabetes affords a synergistic therapeutic or prophylaxis effect on diabetes, in comparison with a single use of each pharmaceutical agent. In other words, a combined use of the compound (1) of the present invention with a therapeutic agent for diabetes is useful from the aspect of effect.

Now, one example of the production method of an indole compound represented by compound (1) is shown below. However, the production method of the present invention is not limited to the example.

When the reaction to be mentioned below is conducted, functional groups other than the concerned moiety may be previously protected as necessary and deprotected at a suitable stage.

The amount of the solvent to be used for each step is not particularly limited as long as the reaction mixture can be stirred.

The reagent to be used for each step may be in the form of a hydrate thereof, a salt thereof and the like, as long as the objective reaction is not inhibited.

The reaction in each step may be carried out by a conventional method and for isolation and purification, conventional methods such as crystallization, recrystallization, column chromatography, preparative HPLC and the like are appropriately selected, or used in combination.

### Production Method 1

A production method of a compound represented by the formula (1) wherein R⁷ is -C(=O)- 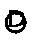 or -C(=NH)- 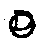 wherein 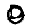 is a group selected from the groups represented by the formulas (a)-(s) is exemplarily shown in the following.

### Production Method

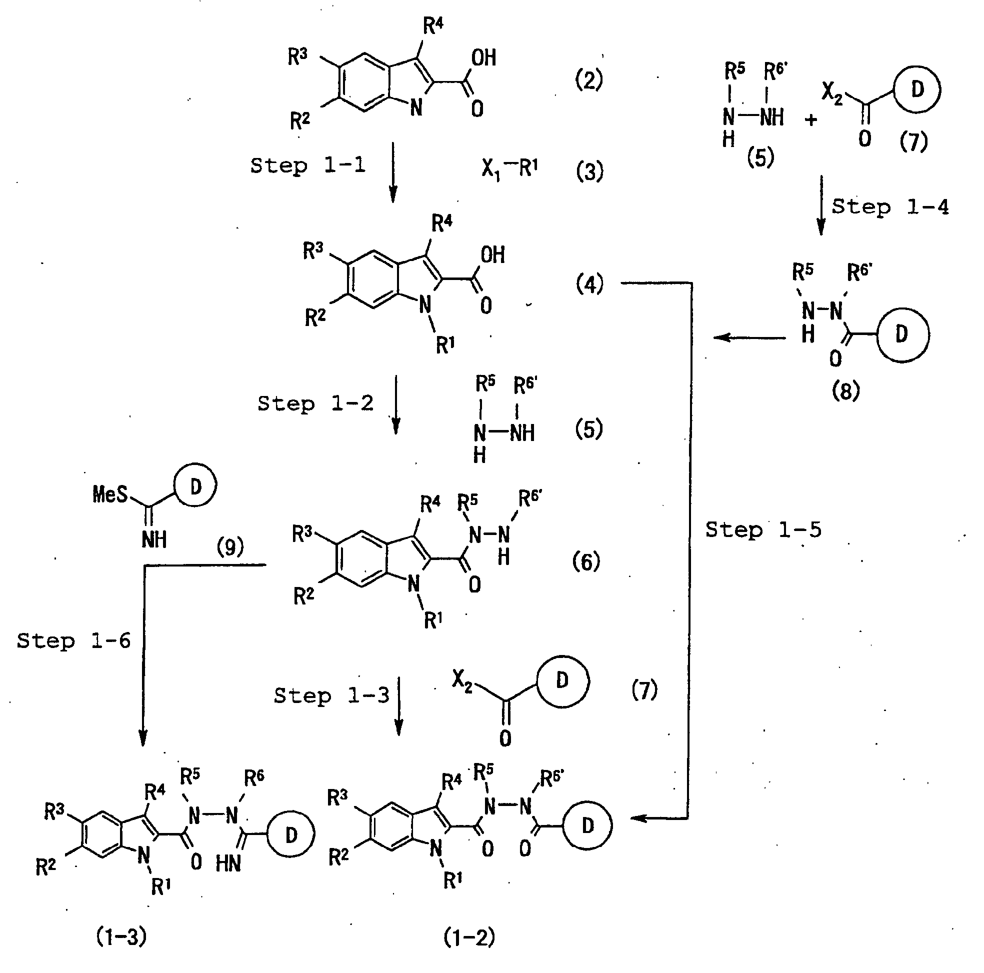

wherein R¹, R², R³, R⁴ and R⁵ are as defined above, R^{6'} is a hydrogen atom, a C₁₋₆ alkyl group or an aralkyl group (aralkyl group is optionally substituted by a halogen atom), X₁ is a halogen atom, X₂ is a halogen atom or a hydroxyl group, and 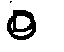 (or also referred to as "ring D") is a group selected from the groups represented by the formulas (a)-(s).

### Step 1-1

A compound represented by the formula (4) can be obtained by reacting a compound represented by the formula (2) with a compound represented by the formula (3) in a solvent in the presence of a base.

As the base to be used in the reaction, for example, alkali metal hydrides such as sodium hydride, potassium hydride etc.; alkali metal alkoxides such as sodium ethoxide, sodium methoxide, potassium tert-butoxide etc.; alkyllithiums such as n-butyllithium, sec-butyllithium etc.; alkali metal azides such as lithium diisopropylamide, sodium amide, lithium bistrimethylsilylazide etc.; alkali metal carbonates such as sodium carbonate, potassium carbonate etc.; alkali metal hydrogen carbonate such as sodium hydrogen carbonate, potassium hydrogen carbonate etc.; alkali metal hydroxides such as lithium hydroxide, sodium hydroxide, potassium hydroxide etc.; alkali metal carboxylates such as sodium acetate, potassium acetate etc.; alkali metal phosphates such as sodium phosphate, potassium phosphate etc.; organic bases such as triethylamine, pyridine, N-methylmorpholine etc.; can be mentioned. Preferred is sodium hydride.

As the solvent, for example, ether solvents such as diethyl ether, tetrahydrofuran (THF), dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide etc.; and the like can be mentioned. These may be used alone or in combination. Preferable solvent for this reaction is N,N-dimethylformamide.

The reaction temperature is generally -50°C to 50°C, preferably -20°C to room temperature.

The reaction time is generally 1-10 hr, preferably 1-5 hr, more preferably 2-5 hr.

### Step 1-2

A compound represented by the formula (6) can be obtained by reacting a compound represented by the formula (4) with a compound represented by the formula (5) in a solvent in the presence of condensing agent.

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvent such as acetone, N,N-dimethylformamide, dimethyl sulfoxide etc.; and the like can be mentioned, These may be used alone or in combination. Preferable solvent for this reaction is N,N-dimethylformamide.

The condensing agent may be any as long as it is used for general methods of peptide condensation (e.g., acid chloride method, mixed acid anhydride method etc.). Of these, a combination of 1-hydroxybenzotriazole monohydrate and 1-(3-(dimethylamino)propyl)-3-ethylcarbodiimide hydrochloride is preferable.

The reaction temperature is generally -20°C to 50°C, preferably 0°C to room temperature.

The reaction time is generally 1-48 hr, preferably 2-24 hr.

### Step 1-3

A compound represented by the formula (1-2), which is one of the object compounds, can be obtained by reacting a compound represented by the formula (6) with a compound represented by the formula (7) in a solvent by using a condensing agent or a base. For smooth progress of this reaction, a base can be used. For example, for the formula (7) when X₂ is a hydroxyl group, a condensing agent is used, in which a base may be used for a smooth reaction. When X₂ is a halogen atom, moreover, the reaction can be carried out smoothly by the use of base, even if a condensing agent is not used.

As the solvent to be used in the reaction, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide etc.; and the like can be mentioned, These may be used alone or in combination. Preferable solvent for this reaction is N,N-dimethylformamide and THF.

The condensing agent may be any as long as it is used for general methods of peptide condensation (e.g., acid chloride method, mixed acid anhydride method etc.). Of these, a combination of 1-hydroxybenzotriazole monohydrate and 1-(3-(dimethylamino)propyl)-3-ethylcarbodiimide hydrochloride is preferable.

As the base, for example, organic bases such as triethylamine, N-methylmorpholine etc. and the like can be mentioned. Preferred is triethylamine.

The reaction temperature is generally -10°C to 60°C, preferably 0°C to room temperature.

The reaction time is generally not less than 10 min., preferably 1-24 hr, more preferably 3-15 hr, further preferably 3-12 hr.

### Step 1-4

A compound represented by the formula (8) can be obtained by subjecting a compound represented by the formula (5) to condensation reaction with a compound represented by the formula (7) in a solvent using a condensing agent.

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide etc.; and the like can be mentioned, These may be used alone or in combination. Preferable solvent for this reaction is N,N-dimethylformamide.

The condensing agent may be any as long as it is used for general methods of peptide condensation (e.g., acid chloride method, mixed acid anhydride method etc.). Of these, a combination of 1-hydroxybenzotriazole monohydrate and 1-(3-(dimethylamino)propyl)-3-ethylcarbodiimide hydrochloride is preferable.

The reaction temperature is generally -30°C to 50°C, preferably -20°C to room temperature.

The reaction time is generally 1-48 hr, preferably 12-24 hr.

### Step 1-5

A compound represented by the formula (1-2), which is one of the object compounds, can be obtained by subjecting a compound represented by the formula (8) to condensation reaction with a compound represented by the formula (4) in a solvent using a condensing agent.

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide etc.; and the like can be mentioned. These may be used alone or in combination. Preferable solvent for this reaction is N,N-dimethylformamide.

The condensing agent may be any as long as it is used for general methods of peptide condensation (e.g., acid chloride method, mixed acid anhydride method etc.). Of these, a combination of 1-hydroxybenzotriazole monohydrate and 1-(3-(dimethylamino)propyl)-3-ethylcarbodiimide hydrochloride is preferable.

The reaction temperature is generally -30°C to 50°C, preferably -20°C to room temperature.
The reaction time is generally 1-48 hr, preferably 1-24 hr.

### Step 1-6

A compound represented by the formula (1-3), which is one of the object compounds, can be obtained by reacting a compound represented by the formula (6) with a compound represented by the formula (9) or a regent equivalent thereto (e.g., methyl benzimidate hydrochloride etc.) in a solvent.

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide etc.; and the like can be mentioned, These may be used alone or in combination. Preferable solvent for this reaction is, methanol.

The reaction temperature is generally -30°C to 50°C, preferably 0°C to room temperature.
The reaction time is generally 3-48 hr, preferably 12-24 hr.

### Production method 2

A compound represented by the formula (1), wherein R⁶ and R⁷ show, together with the nitrogen atom bonded thereto, 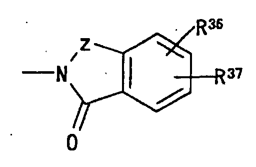 wherein Z, R³⁶ and R³⁷ are as defined above, is exemplarily shown in the following.

### Production Method 2

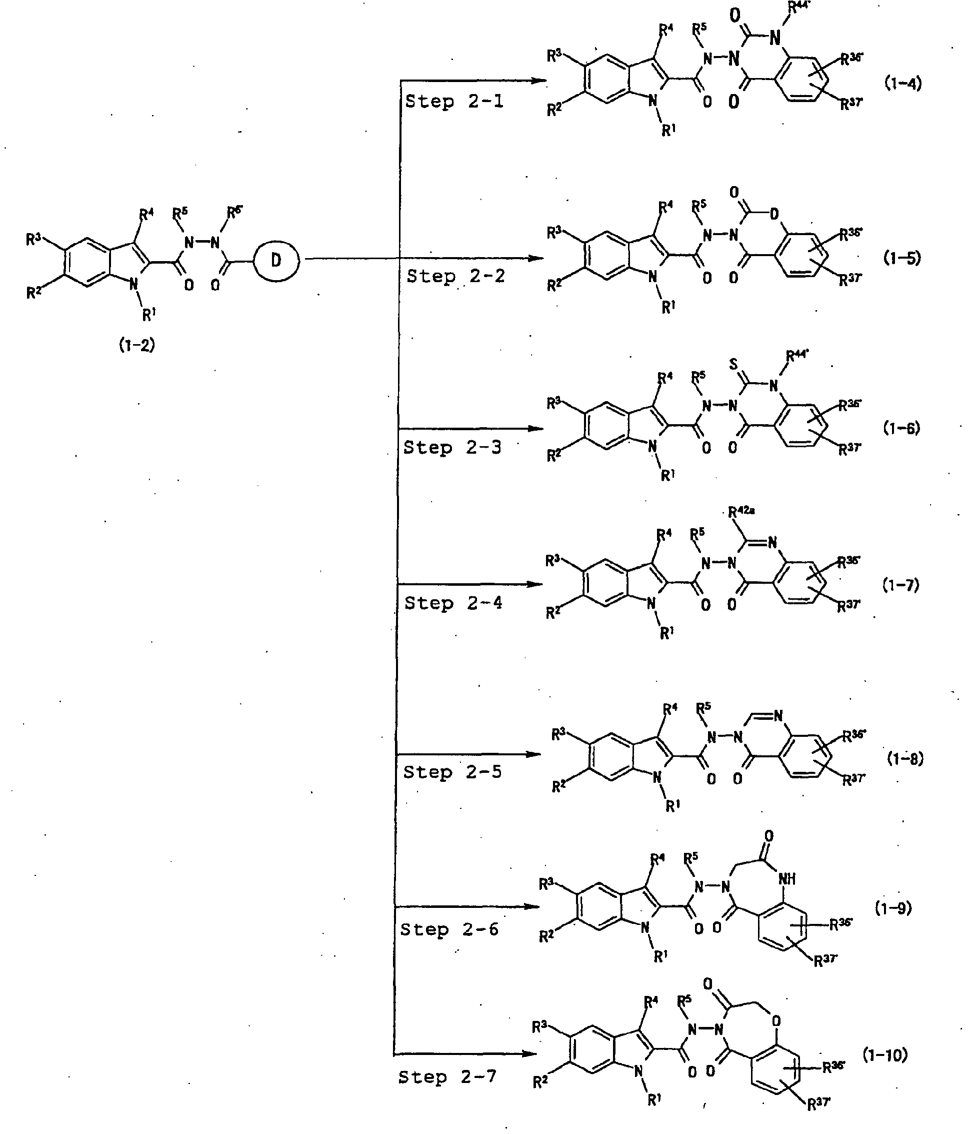

wherein R¹, R², R³, R⁴, R⁵, R^{44'} and ring D are as defined above, R^{6'} is a hydrogen atom, a C₁₋₆ alkyl group or an aralkyl group (aralkyl group is optionally substituted by a halogen atom), R^{36'} and R^{37'} are each a hydrogen atom, a halogen atom, a C₁₋₆ alkyl group, a C₁₋₆ alkoxy group or a hydroxyl group, and R^{42a} is a C₁₋₆ alkyl group).

### Step 2-1

A compound represented by the formula (1-4), which is one of the object compounds, can be obtained by reacting a compound wherein ring D is 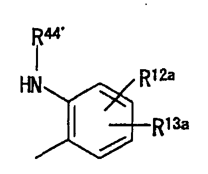 wherein R^{12a} and R^{13a} are the same or different and each is a hydrogen atom, a halogen atom, a C₁₋₆ alkyl group, a C₁₋₆ alkoxy group or a hydroxyl group (R^{12a} and R^{13a} are as defined for R^{36'} and R^{37'}) and R⁴⁴ is as defined above, from among the compounds represented by the formula (1-2) obtained in Steps 1-3 and 1-5, in a solvent, using a phosgene equivalent (e.g., carbodiimidazole, triphosgene etc., preferably triphosgene). As the reagent, diphosgene, chloroethyl carbonate and the like can be used, other than the above-mentioned two kinds of reagents.

As the solvent to be used in the reaction, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethylsulfoxide, water etc.; and the like can be mentioned, These may be used alone or in combination. Preferable solvents for this reaction are N,N-dimethylformamide, and a mixture of THF and water.

For this reaction, a base is preferably used. As the usable base, for example, inorganic bases such as sodium hydroxide, sodium hydrogen carbonate (sodium bicarbonate), potassium carbonate etc.; organic bases such as triethylamine, diazabicycloundecene, 1,8-diazabicyclo[5.4.0]undec-7-ene etc. can be mentioned, with preference given to sodium hydrogen carbonate.

The reaction temperature is generally -10°C to 60°C, preferably 0°C to room temperature.

The reaction time is generally not less than 1hr, preferably 1-24 hr, more preferably 3-24 hr, preferably 6-12 hr.

### Step 2-2

A compound represented by the formula (1-5), which is one of the object compounds, can be obtained by subjecting a compound wherein ring D is 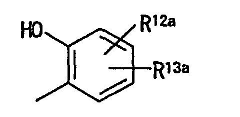 wherein R^{12a} and R^{13a} are as defined above, from among the compounds represented by the formula (1-2) obtained in Steps 1-3 and 1-5, to the same reaction as in Step 2-1.

### Step 2-3

A compound represented by the formula (1-6), which is one of the object compounds, can be obtained by reacting a compound wherein ring D is 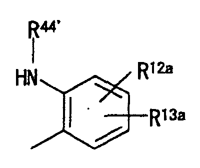 wherein R^{12a}, R^{13a} and R^{44'} are as defined above, from among the compounds represented by the formula (1-2) obtained in Steps 1-3 and 1-5, with a thiocarbonyl compound in a solvent.

As the solvent to be used in the reaction, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide, water etc.; and the like can be mentioned, These may be used alone or in combination. Preferable solvent for this reaction is tetrahydrofuran.

As the thiocarbonyl compound, for example, thiocarbonyldiimidazole, carbon disulfide, thiophosgene and the like can be mentioned, with preference given to thiocarbonyldiimidazole.

In this reaction, the use of base may be desired. As the usable base, for example, inorganic bases such as sodium hydroxide, sodium hydrogen carbonate, potassium carbonate etc.; and organic bases such as triethylamine, 1,8-diazabicyclo[5.4.0]undec-7-ene etc. can be mentioned.

The reaction temperature is generally -10°C to 60°C, preferably 0°C to room temperature.

The reaction time is generally 1-12 hr, preferably 2-6 hr.

### Step 2-4

A compound represented by the formula (1-7), which is one of the object compounds, can be obtained by reacting a compound wherein ring D is 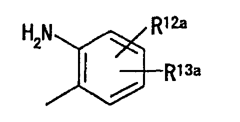 wherein R^{12a} and R^{13a} are as defined above, from among the compounds represented by the formula (1-2) obtained in Steps 1-3 and 1-5, with orthoacetic acid ester, or formic acid or a derivative thereof in a solvent, in the presence of an acid.

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide, formic acid etc.; and the like can be mentioned. These may be used alone or in combination. Preferable solvent for this reaction is N,N-dimethylformamide.

As the acid to be used in the reaction, for example, inorganic acids such as hydrochloric acid, sulfuric acid, nitric acid etc.; organic acids such as trifluoroacetic acid, trichloroacetic acid, acetic acid, methanesulfonic acid, p-toluenesulfonic acid etc. can be mentioned, with preference given to methanesulfonic acid.

As the orthoacetic acid ester, methyl orthoacetate is preferable. As the formic acid and a derivative thereof, for example, formic acid, ethyl orthoformate and the like can be mentioned, with preference given to formic acid.

The reaction temperature is generally -10°C to 100°C, preferably 0°C to room temperature.

The reaction time is generally 30 min.-12 hr, preferably 30 min.-6 hr, more preferably 1-6 hr.

### Step 2-5

A compound represented by the formula (1-8), which is one of the object compounds, can be obtained by reacting, from among the compounds represented by the formula (1-2) obtained in Step 1-3 or 1-5, a compound wherein ring D is represented by 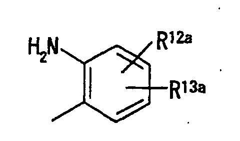 wherein R^{12a} and R^{13a} are as defined above, with formic acid under heating.

The reaction temperature is generally 80°C to 300°C, preferably 100°C to 200°C.

The reaction time is generally 4-24 hr, preferably 5-12 hr.

### Step 2-6

A compound represented by the formula (1-9), can be obtained by reacting, from among the compounds represented by the formula (1-2) obtained in Step 1-3 or 1-5, a compound
wherein ring D is represented by 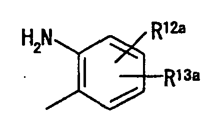 wherein R^{12a} and R^{13a} are as defined above (i) with an acetyl halide compound (e.g., acetyl chloride, acetyl bromide etc.) in a solvent in the presence of an organic base (e.g., pyridine, triethylamine, sodium hydrogen carbonate etc.), and (ii) by cyclizing the obtained compound without isolation using a base and sodium iodide and the like.

As the solvent in (i) and (ii), for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ester solvents such as ethyl acetate, methyl acetate, butyl acetate etc.; polar solvents such as acetone, N,N-dimethylformamide, dimethyl sulfoxide etc.; and the like can be mentioned. These may be used alone or in combination. Preferable solvents for this reaction are tetrahydrofuran, ethyl acetate, N,N-dimethylformamide, and a mixed solvent thereof.

As the base to be used for the reaction, for example, alkali metal hydrides such as sodium hydride, potassium hydride etc.; alkali metal alkoxides such as sodium ethoxide, sodium methoxide, potassium tert-butoxide and the like; alkyllithiums such as n-butyllithium, sec-butyllithium etc.; alkali metal amides such as lithium diisopropylamide, sodium amide, lithium bistrimethylsilylazide etc.; alkali metal carbonates such as sodium carbonate, potassium carbonate etc.; alkali metal hydrogen carbonates such as sodium hydrogen carbonate, potassium hydrogen carbonate etc.; alkali metal hydroxides such as lithium hydroxide, sodium hydroxide, potassium hydroxide etc.; alkali metal carboxylates such as sodium acetate, potassium acetate etc.; alkali metal phosphates such as sodium phosphate, potassium phosphate etc.; and organic bases such as triethylamine, pyridine, N-methylmorpholine, 1,8-diazabicyclo[5.4.0]undec-7-ene etc. can be mentioned, with preference given to potassium carbonate.

The reaction temperature is generally -30°C to 100°C, preferably 0°C to 80°C, more preferably 0°C to room temperature.

The reaction time is generally 1-24 hr, preferably 1-15 hr, more preferably 2-12 hr.

### Step 2-7

A compound represented by the formula (1-10), which is one of the object compounds, can be obtained by cyclizing, from among the compound represented by the formula (1-2) obtained in Step 1-3 or 1-5, a compound wherein ring D is 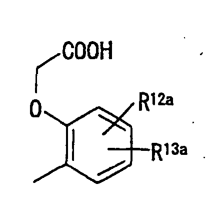 wherein R^{12a} and R^{13a} are as defined above, in a solvent in the presence of a condensing agent.

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ether solvents such as ethyl acetate, methyl acetate, butyl acetate etc.; polar solvents such as acetone, N,N-dimethylformamide, dimethyl sulfoxide etc.; and the like can be mentioned. These may be used alone or in combination. Preferable solvent for this reaction is N,N-dimethylformamide.

As the condensing agent, any condensing agent used for general peptide condensation method (e.g., acid chloride method, mixed acid anhydride method etc.). Of these, a combination of 1-hydroxybenzotriazole monohydrate and 1-(3-(dimethylamino)propyl)-3-ethylcarbodiimide hydrochloride is preferable.

The reaction temperature is generally -30°C to 60°C, preferably 0°C to room temperature.

The reaction time is generally 5-24 hr, preferably 10-20 hr.

### Production Method 3

A production method for a compound represented by the formula (1), wherein R⁶ and R⁷ form, together with the adjacent nitrogen atom, 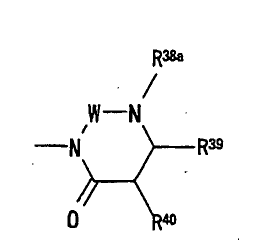 wherein W, R³⁸, R³⁹ and R⁴⁰ are as defined above, and R^{38a} is an aryl group, is exemplarily shown in the following.

### Production Method 3

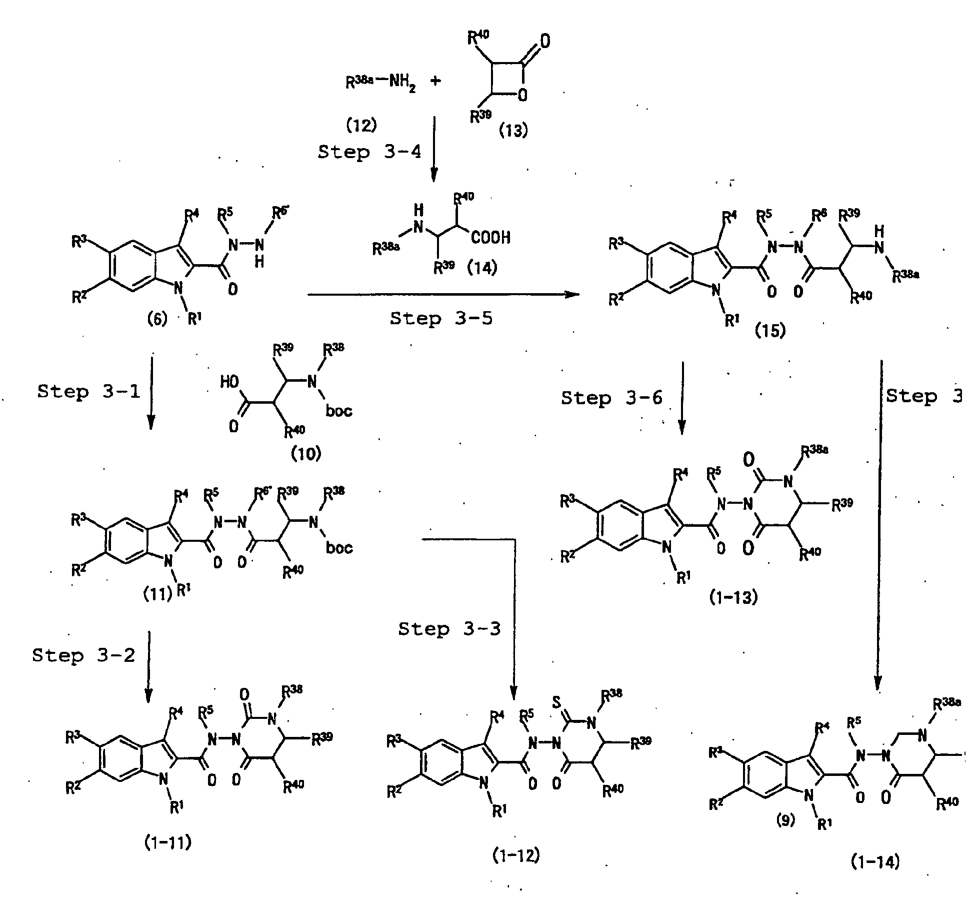

wherein R¹, R², R³, R⁴, R⁵, R^{6'}, R³⁸, R^{38a}, R³⁹ and R⁴⁰ are as defined above and boc is a tert-butoxycarbonyl group.

### Step 3-1

A compound represented by the formula (11) can be obtained by subjecting a compound represented by the formula (6) obtained in Step 1-2 and a compound represented by the formula (10) to a condensation reaction as in Step 1-3.

### Step 3-2

A tert-butoxycarbonyl group of a compound represented by the formula (11) is deprotected with an acid (e.g., trifluoroacetic acid, hydrochloric acid, hydrobromic acid, p-toluenesulfonic acid etc.) and then the obtained amino compound is cyclized with a phosgene equivalent (e.g., carbonyldiimidazole, triphosgene etc.) in the presence of a base to give a compound represented by the formula (1-11), which is one of the object compounds.

As the solvent to be used in the reaction, for example, ether solvents such as diethyl ether, tetrahydrofuran, dioxane, 1,2-dimethoxyethane, diglyme, diphenyl ether etc.; hydrocarbon solvents such as benzene, toluene, hexane, xylene etc.; halogen solvents such as dichloromethane, chloroform, carbon tetrachloride, chlorobenzene, 1,2-dichloroethane etc.; alcohol solvents such as methanol, ethanol, isopropyl alcohol, tert-butanol etc.; polar solvents such as N,N-dimethylformamide, dimethyl sulfoxide, water etc.; and the like can be mentioned. These may be used alone or in combination or the reaction can be carried out without solvent. Preferable solvent for this reaction is tetrahydrofuran.

As the base to be used for the reaction, for example, alkali metal hydrides such as sodium hydride, potassium hydride etc.; alkali metal alkoxides such as sodium ethoxide, sodium methoxide, potassium tert-butoxide and the like; alkyllithiums such as n-butyllithium, sec-butyllithium etc.; alkali metal amides such as lithium diisopropylamide, sodium amide, lithium bistrimethylsilylamide etc.; alkali metal carbonates such as sodium carbonate, potassium carbonate etc.; alkali metal hydrogen carbonates such as sodium hydrogen carbonate, potassium hydrogen carbonate etc.; alkali metal hydroxides such as lithium hydroxide, sodium hydroxide, potassium hydroxide etc.; alkali metal carboxylates such as sodium acetate, potassium acetate etc.; alkali metal phosphates such as sodium phosphate, potassium phosphate etc.; and organic bases such as triethylamine, pyridine, N-methylmorpholine, 1,8-diazabicyclo[5.4.0]undec-7-ene etc. can be mentioned, with preference given to triethylamine.

The reaction temperature is generally 0°C to 100°C, preferably room temperature to 60°C.

The reaction time is generally 1-48 hr, preferably 1-25 hr, more preferably 2-24 hr.

### Step 3-3

In the same manner as in Step 3-2, a tert-butoxycarbonyl group of a compound represented by the formula (11) is deprotected with an acid (e.g., trifluoroacetic acid, hydrochloric acid etc.) and then the obtained compound is cyclized in the presence of a base using a thiocarbonylating reagent (e.g., thiocarbonyldiimidazole, carbon disulfide, thiophosgene etc.) to give a compound represented by the formula (1-12), which is one of the object compounds.

### Step 3-4

A compound represented by the formula (14) can be obtained by reacting a compound represented by the formula (12) with a compound represented by the formula (13) in acetonitrile.

The reaction temperature is generally 50°C to 200°C, preferably 80°C to 100°C.

The reaction time is generally 1-10 hr, preferably 2-5 hr.

### Step 3-5

In the same manner as in Step 1-3, a compound represented by the formula (15) can be obtained by reacting a compound represented by the formula (6) with a compound represented by the formula (14).

### Step 3-6

A compound represented by the formula (1-13), which is one of the object compounds can be obtained by cyclizing a compound represented by the formula (15) with a phosgene equivalent (e.g., triphosgene, carbonyldiimidazole, diphosgene etc.) in a solvent in the presence of a base.

As the solvent to be used in the reaction, for example, ether solvents such as diethyl ether, tetrahydrofuran, dioxane, 1,2-dimethoxyethane, diglyme, diphenyl ether etc.; hydrocarbon solvents such as benzene, toluene, hexane, xylene etc.; halogen solvents such as dichloromethane, chloroform, chlorobenzene, carbon tetrachloride, 1,2-dichloroethane etc.; alcohol solvents such as methanol, ethanol, isopropyl alcohol, tert-butanol etc.; polar solvents such as N,N-dimethylformamide, dimethyl sulfoxide, water etc.; dimethylaniline; and the like can be mentioned. These may be used alone or in combination or the reaction can be carried out without solvent. Preferable solvent for this reaction is tetrahydrofuran.

As the base to be used for the reaction, for example, alkali metal hydrides such as sodium hydride, potassium hydride etc.; alkali metal alkoxides such as sodium ethoxide, sodium methoxide, potassium tert-butoxide and the like; alkyllithiums such as n-butyllithium, sec-butyllithium etc.; alkali metal azides such as lithium diisopropylazide, sodium amide, lithium bistrimethylsilylazide etc.; alkali metal carbonates such as sodium carbonate, potassium carbonate etc.; alkali metal hydrogen carbonates such as sodium hydrogen carbonate, potassium hydrogen carbonate etc.; alkali metal hydroxides such as lithium hydroxide, sodium hydroxide, potassium hydroxide etc.; alkali metal carboxylates such as sodium acetate, potassium acetate etc.; alkali metal phosphates such as sodium phosphate, potassium phosphate etc.; and organic bases such as triethylamine, pyridine, N-methylmorpholine, 1,8-diazabicyclo-[5.4.0]undec-7-ene etc. can be mentioned, with preference given to triethylamine.

The reaction temperature is generally 0°C to 100°C, preferably 0°C to room temperature.

The reaction time is generally 1-24 hr, preferably 4-10 hr.

### Step 3-7

A compound represented by the formula (1-14), which is one of the object compounds, can be obtained by cyclizing a compound represented by the formula (15) with a formalin equivalent (e.g., paraformaldehyde, dimethoxymethane, dibromomethane etc.) in a solvent.

As the solvent to be used in the reaction, for example, ether solvents such as diethyl ether, tetrahydrofuran, dioxane, 1,2-dimethoxyethane, diglyme, diphenyl ether etc.; hydrocarbon solvents such as benzene, toluene, hexane, xylene etc.; halogen solvents such as dichloromethane, chloroform, chlorobenzene, carbon tetrachloride, 1,2-dichloroethane etc.; alcohol solvents such as methanol, ethanol, isopropyl alcohol, tert-butanol etc.; polar solvents such as N,N-dimethylformamide, dimethyl sulfoxide, water etc.; dimethylaniline and the like can be mentioned. These may be used alone or in combination or the reaction can be carried out without solvent. Preferable solvents for this reaction are methanol and ethanol.

This reaction is preferably carried out in the presence of an acid or a base.

The reaction temperature is generally 0°C to 100°C, preferably 0°C to room temperature.

The reaction time is generally 1 hr-10 days, preferably 1 day-10 days, more preferably 1 day -3 days.

A compound represented by the formula (1-14) (wherein R^{38a} is a hydrogen atom) can be produced by the following method in addition to the method of the above-mentioned Step 3-7:
a compound represented by 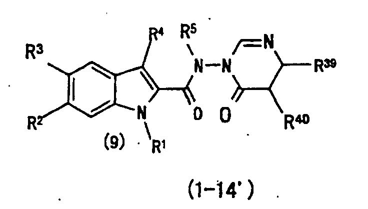 wherein each symbol is as defined above is reduced. The reduction can be conducted by a conventional method. For example, a compound represented by the formula (1-14') is reacted with a reducing agent such as sodium cyanoborohydride and the like to give a desired compound.

### Production Method 4

A compound represented by the formula (1), wherein R⁶ and R⁷ form, together with the adjacent nitrogen atom, 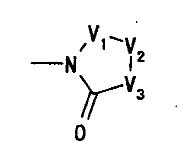 wherein V₁, V₂ and V₃ are as defined above is exemplarily shown in the following.

### Production Method 4

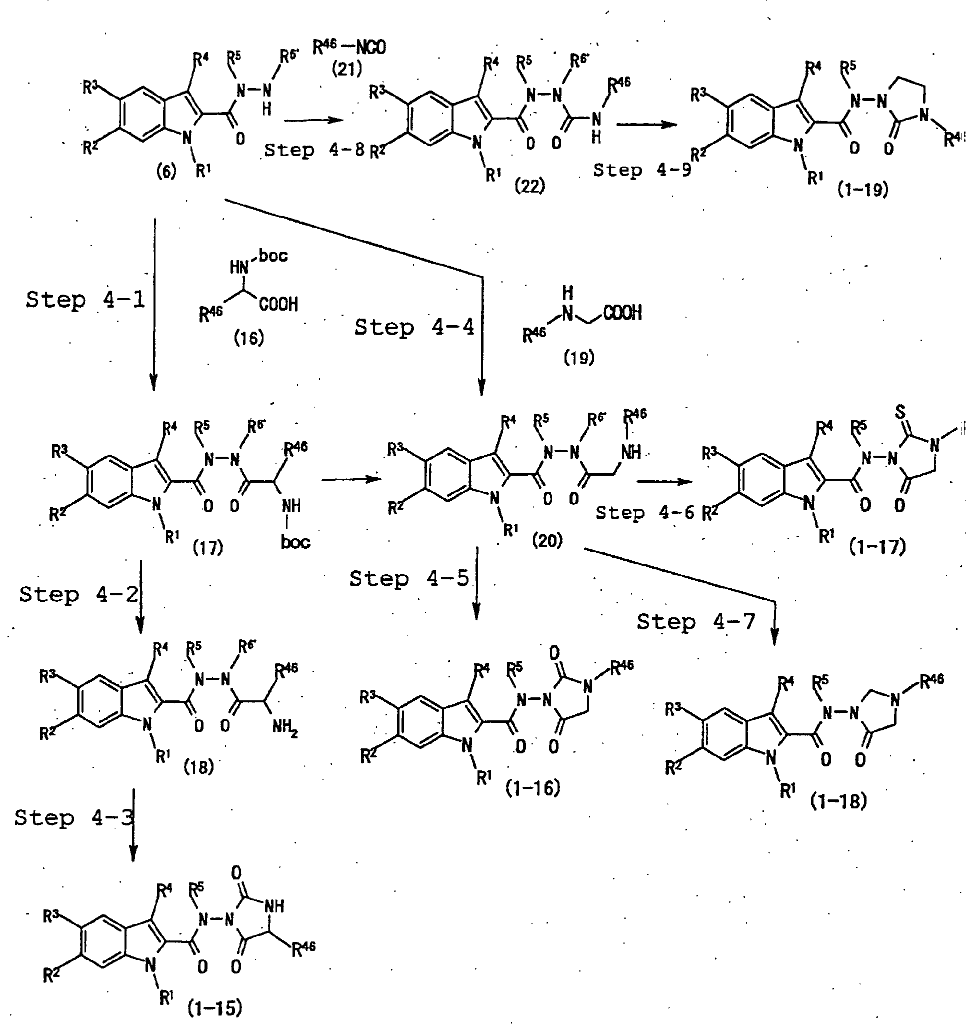

wherein R¹, R², R³, R⁴, R⁵, R^{5'}, R^{6'} and R⁴⁶ are as defined above and boc is a tert-butoxycarbonyl group.

### Step 4-1

In the same manner as in Step 1-3, a compound represented by the formula (17) can be obtained by reacting a compound represented by the formula (6) with a compound represented by the formula (16).

### Step 4-2

This step is deprotection of an amino-protecting group generally performed, wherein a compound represented by the formula (17) is deprotected in a solvent using an acid to give a compound represented by the formula (18).

As the solvent, for example, ether solvents such as diethyl ether, tetrahydrofuran, dioxane, 1,2-dimethoxyethane, diglyme etc.; hydrocarbon solvents such as benzene, toluene, hexane, xylene etc.; halogen solvents such as dichloromethane, chloroform, carbon tetrachloride, 1,2-dichloroethane etc.; alcohol solvents such as methanol, ethanol, isopropyl alcohol, tert-butanol etc.; ether solvents such as ethyl acetate, methyl acetate, butyl acetate etc.; polar solvents such as N,N-dimethylformamide, dimethyl sulfoxide etc.; and the like can be mentioned. These may be used alone or in combination or the reaction can be carried out without solvent. Preferable solvents for this reaction are dioxane, dichloroethane and trifluoroacetic acid.

As an acid to be used for the reaction, for example, inorganic acids such as hydrochloric acid, hydrobromic acid, sulfuric acid, nitric acid etc.; organic acids such as trifluoroacetic acid, trichloroacetic acid, acetic acid, methanesulfonic acid, p-toluenesulfonic acid etc. can be mentioned, with preference given to trifluoroacetic acid.

The reaction temperature is generally -10°C to 60°C, preferably 0°C to 60°C, more preferably 0°C to room temperature.

The reaction time is generally not less than 1 hr, preferably 1-20 hr, more preferably 1-12 hr, still more preferably 4-6 hr.

### Step 4-3

A compound represented by the formula (1-15), which is one of the object compounds, can be obtained by cyclizing a compound represented by the formula (18) with a phosgene equivalent (e.g., carbonyldiimidazole, triphosgene, diphosgene, ethyl chlorocarbonate etc.) in a solvent.

As the solvent, for example, ether solvents such as diethyl ether, tetrahydrofuran, dioxane, 1,2-dimethoxyethane, diglyme, diphenyl ether etc.; hydrocarbon solvents such as benzene, toluene, hexane, xylene etc.; halogen solvents such as dichloromethane, chloroform, carbon tetrachloride, 1,2-dichloroethane, chlorobenzene etc.; alcohol solvents such as methanol, ethanol, isopropyl alcohol, tert-butanol etc.; polar solvents such as N,N-dimethylformamide, dimethyl sulfoxide, water etc.; and the like can be mentioned. These may be used alone or in combination or the reaction can be carried out without solvent. Preferable solvent for this reaction is tetrahydrofuran.

In this reaction, the reaction may proceed smoothly when a base is used.

The reaction temperature is generally -10°C to 100°C, preferably 0°C to room temperature.

The reaction time is generally 0.5-24 hr, preferably 1-3 hr.

### Step 4-4

A compound represented by the formula (20) can be obtained by cyclizing a compound represented by the formula (6) with a compound represented by the formula (19) in the same manner as in Step 1-3.

### Step 4-5

In the same manner as in Step 4-3, a compound represented by the formula (20) is cyclized with a phosgene equivalent (e.g., carbonyldiimidazole, triphosgene, diphosgene, ethyl chlorocarbonate etc.) to give a compound represented by the formula (1-16), which is one of the object compounds.

### Step 4-6

A compound represented by the formula (1-17), which is one of the object compounds, can be obtained by cyclizing a compound represented by the formula (20) with a thiocarbonylating reagent (e.g., thiocarbonyldiimidazole, carbon disulfide, thiophosgene etc.) in the same manner as in Step 3-3.

### Step 4-7

A compound represented by the formula (1-18), which is one of the object compounds, can be obtained by cyclizing a compound represented by the formula (20) with a formalin equivalent (e.g., paraformaldehyde, dimethoxymethane, dibromomethane etc.) in the same manner as in Step 3-7.

### Step 4-8

In the same manner as in Step 1-3, a compound represented by the formula (22) can be obtained by reacting a compound represented by the formula (6) with a compound represented by the formula (21).

### Step 4-9

A compound represented by the formula (1-19), which is one of the object compounds, can be obtained by reacting a compound represented by the formula (22) with 1,2-dibromoethane or 1,2-dichloroethane in a solvent in the presence of a base.

One of 1,2-dibromoethane and 1,2-dichloroethane is used for the reaction.

As the solvent, for example, ether solvents such as diethyl ether, tetrahydrofuran, dioxane, 1,2-dimethoxyethane, diglyme, diphenyl ether etc.; hydrocarbon solvents such as 'benzene, toluene, hexane, xylene etc.; halogen solvents such as chlorobenzene etc.; alcohol solvents such as methanol, ethanol, isopropyl alcohol, tert-butanol etc.; polar solvents such as N,N-dimethylformamide, dimethyl sulfoxide etc.; and the like can be mentioned. These may be used alone or in combination or the reaction can be carried out without solvent. Preferable solvent for this reaction is N,N-dimethylformamide.

As the base to be used for the reaction, for example, alkali metal hydrides such as sodium hydride, potassium hydride etc.; alkali metal alkoxides such as sodium ethoxide, sodium methoxide, potassium tert-butoxide and the like; alkyllithiums such as n-butyllithium, sec-butyllithium etc.; alkali metal amides such as lithium diisopropylamide, sodium amide, lithium bistrimethylsilylazide etc.; alkali metal carbonates such as sodium carbonate, potassium carbonate etc.; alkali metal hydrogen carbonates such as sodium hydrogen carbonate, potassium hydrogen carbonate etc.; alkali metal hydroxides such as lithium hydroxide, sodium hydroxide, potassium hydroxide etc.; alkali metal carboxylates such as sodium acetate, potassium acetate etc.; alkali metal phosphates such as sodium phosphate, potassium phosphate etc.; and organic bases such as triethylamine, pyridine, N-methylmorpholine, 1,8-diazabicyclo[5.4.0]undec-7-ene etc. can be mentioned, with preference given to potassium carbonate.

The reaction temperature is generally 0°C to 100°C, preferably room temperature to 70°C.

The reaction time is generally 1-48 hr, preferably 2-24 hr.

### Production Method 5

A production method of a compound represented by the formula (1) wherein R⁷ is -C(=O)-A'- 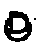 wherein A' is -N(R⁸)-, -C(R⁹)(R¹⁰)- or -CO- and ring D is as defined above is exemplarily shown in the following.

### Production Method 5

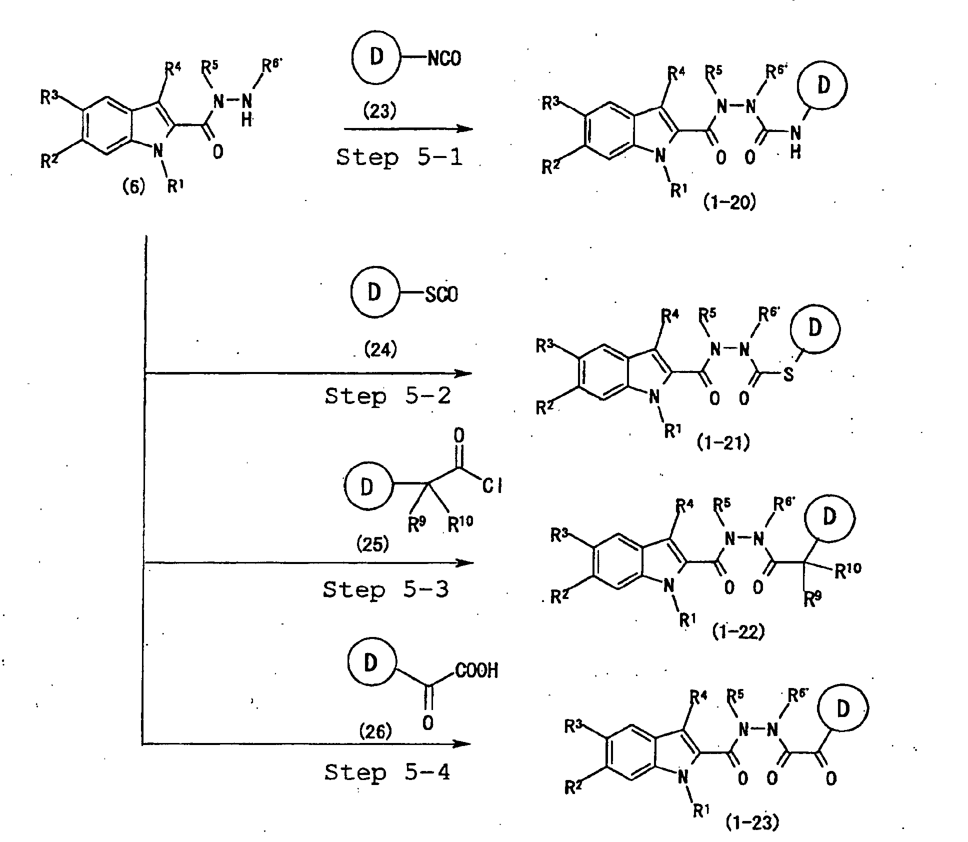

wherein R¹, R², R³, R⁴, R⁵, R^{6'}, R⁹, R¹⁰ and ring D are as defined above.

### Step 5-1

A compound represented by the formula (1-20) , which is one of the object compounds, can be obtained by reacting a compound represented by the formula (6) obtained in Step 1-2 with a compound represented by the formula (23) in a solvent.

As the solvent, for example, ether solvents such as diethyl ether, tetrahydrofuran, dioxane, 1,2-dimethoxyethane, diglyme, diphenyl ether etc.; hydrocarbon solvents such as benzene, toluene, hexane, xylene etc.; halogen solvents such as dichloromethane, chloroform, carbon tetrachloride, chlorobenzene, 1,2-dichloroethane, etc.; alcohol solvents such as methanol, ethanol, isopropyl alcohol, tert-butanol etc.; polar solvents such as N,N-dimethylformamide, dimethyl sulfoxide, etc.; dimethylaniline; and the like can be mentioned. These may be used alone or in combination or the reaction can be carried out without solvent. Preferable solvent for this reaction is dioxane.

The reaction temperature is generally 0°C to 100°C, preferably room temperature to 80°C.

The reaction time is generally 1-12 hr, preferably 1-6 hr, more preferably 2-6 hr.

### Step 5-2

A compound represented by the formula (1-21), which is one of the object compounds, can be obtained by reacting a compound represented by the formula (6) with a compound represented by the formula (24) in the same manner as in Step 5-1.

### Step 5-3

A compound represented by the formula (1-22), which is one of the object compounds, can be obtained by reacting a compound represented by the formula (6) with a compound represented by the formula (25) in a solvent in the presence of a base.

As the solvent, for example, ether solvents such as diethyl ether, tetrahydrofuran, dioxane, 1,2-dimethoxyethane, diglyme, diphenyl ether etc.; hydrocarbon solvents such as benzene, toluene, hexane, xylene etc.; halogen solvents such as dichloromethane, chloroform, carbon tetrachloride, chlorobenzene, 1,2-dichloroethane, etc.; alcohol solvents such as methanol, ethanol, isopropyl alcohol, tert-butanol etc.; polar solvents such as N,N-dimethylformamide, dimethyl sulfoxide, etc.; and the like can be mentioned. These may be used alone or in combination or the reaction can be carried out without solvent. Preferable solvent for this reaction is tetrahydrofuran.

As the base to be used for the reaction, for example, alkali metal hydrides such as sodium hydride, potassium hydride etc.; alkali metal alkoxides such as sodium ethoxide, sodium methoxide, potassium tert-butoxide and the like; alkyllithiums such as n-butyllithium, sec-butyllithium etc.; alkali metal carbonates such as sodium carbonate, potassium carbonate etc.; alkali metal hydrogen carbonates such as sodium hydrogen carbonate, potassium hydrogen carbonate etc.; alkali metal hydroxides such as lithium hydroxide, sodium hydroxide, potassium hydroxide etc.; alkali metal carboxylates such as sodium acetate, potassium acetate etc.; alkali metal phosphates such as sodium phosphate, potassium phosphate etc.; and organic bases such as triethylamine, pyridine, N-methylmorpholine etc. can be mentioned, with preference given to triethylamine.

The reaction temperature is generally 0°C to 100°C, preferably room temperature to 60°C.

The reaction time is generally 1-12 hr, preferably 1-6 hr, more preferably 2-6 hr.

### Step 5-4

In the same manner as in Step 1-3, a compound represented by the formula (1-23), which is one of the object compounds, can be obtained by reacting a compound represented by the formula (6) with a compound represented by the formula (26).

When a salt of the compound (1) of the present invention is desired, a free compound (1) of the present invention obtained in the above-mentioned Production Method 1-5 can be converted to a salt according to conventional methods.

### Examples

The present invention is explained in detail by referring to Examples, which are not to be construed as limitative. In the following formulas, "boc" means tert-butoxycarbonyl.

### Example 1

### benzoic acid 2-(5-chloro-1H-indole-2-carbonyl)hydrazide

### a) 5-chloro-1H-indole-2-carboxylic acid hydrazide

5-Chloro-1H-indole-2-carboxylic acid (1.96 g) and 1-hydroxybenzotriazole monohydrate (HOBt·H₂O) (1.58 g) were dissolved in N,N-dimethylformamide (DMF) (10 ml) and 1-(3-(dimethylamino)propyl)-3-ethylcarhodiimide hydrochloride (EDC) (2.58 g) was added. This solution was stirred at room temperature for 3 hr. This solution was cooled in an ice-bath and hydrazine hydrate (2.4 ml) was added. This solution was stirred at room temperature for 11 hr. Water (20ml) was added slowly to this reaction mixture. The precipitated solid was collected by filtration and dried in vacuo to give the title compound (2.24 g) (containing DMF (15%) as a residual solvent). This sample was boiled in tetrahydrofuran (THF), allowed to cool and filtered to give a purer sample.
¹H-NMR(δ, 300MHz,DMSO-d₆).
4.52(2H,brs),7.05(1H,d,J=1.5Hz),7.16(1H,dd,J=8.7Hz,2.1Hz),7.42 (1H,d,J=8.7Hz),7.66(1H,d,J=1.9Hz),9.86(1H,brs),11.84(1H,s).

### b) benzoic acid 2-(5-chloro-1H-indole-2-carbonyl)hydrazide

5-Chloro-1H-indole-2-carboxylic acid hydrazide (98 mg) obtained in Example 1 a) was suspended in THF (2 ml) and triethylamine (73 µl) was added. This mixture was cooled in an ice-bath and benzoyl chloride (61 µl) was added slowly. The reaction mixture was stirred at room temperature for 10 min. Thereto were added ethyl acetate (5 ml), THF (3 ml) and ice water (10 ml) and the mixture was stirred. The separated organic layer was washed successively with aqueous sodium hydroxide solution (0.1N) and water and dried over anhydrous sodium sulfate. This solution was filtered and concentrated to allow precipitation of white crystals, which were collected by filtration and dried in vacuo to give the title compound (29 mg) (see Table 1).

### Example 1-2

### 2-amino-benzoic acid 2-(5-chloro-1H-indole-2-carbonyl)hydrazide

5-Chloro-1H-indole-2-carboxylic acid hydrazide obtained in Example 1 a) (6.00 g), anthranilic acid (3.42 g) and HOBt· H₂O (3.882 g) were suspended in DMF (100 ml) and EDC (4.79 g) was added. This mixture was stirred at room temperature for one day. To the reaction mixture were added THF (200 ml) and half-saturated brine (200 ml). The separated organic layer was washed successively with aqueous sodium hydroxide solution (0.1N), aqueous hydrochloric acid (0.5N) and pure water. This solution was dried over anhydrous sodium sulfate and filtrated. This solution was concentrated to a small amount and left standing to allow precipitation of a white powder, which was collected by filtration and dried in vacuo to give the title compound (4.35 g, yield 55%) (see Table 1).

### Example 1-3

### 2-hydroxy-benzoic acid 2-(5-chloro-1H-indole-2-carbonyl)hydrazide

The title compound was obtained as crystals according to the same method as Example 1-2 but using salicylic acid instead of anthranilic acid (see Table 1).

### Example 1-4

### 3-[{2-(2-(5-chloro-1H-indole-2-carbonyl)hydrazinocarbonyl)pheny}carbamoyloxy]-2,2-dimethylpropionic acid

### a) 3-hydroxy-2,2-dimethylpropionic acid benzyl ester

Hydroxypivalic acid (5.03 g) was dissolved in DMF (50 ml) and benzyl bromide (5.50 ml) and potassium carbonate (11.38 g) were successively added. The mixture was stirred at room temperature for 12 hr. Water (100 ml) was added to the reaction mixture and the mixture was extracted with ether (2×100 ml). The organic layer was washed successively with water (2×50 ml) and saturated aqueous sodium chloride solution (50 ml), dried and concentrated under reduced pressure. The obtained oily substance was purified by column chromatography to give the title compound (6.54 g) as a colorless oil.
¹H-NMR(δ, 300MHz, CDCl₃)
1.22(6H,s),2.37(1H,t,J=6.8Hz),3.58(2H,d,J=6.8Hz),5.15(2H,s),7. 30-7.40(5H,m).

### b)2-(2-benzyloxycarbonyl-2-methylpropoxycarbonylamino)benzoic acid

tert-Butyl phthalate (5.38 g) was dissolved in toluene (50 ml). To this solution were successively added triethylamine (3.7 ml) and diphenylphosphoryl azide (5.7 ml) and the mixture was heated to 130°C and stirred for 1 hr. The reaction mixture was allowed to cool to room temperature and 3-hydroxy-2,2-dimethylpropionic acid benzyl ester obtained in Example 1-4 a) (4.95 g) was added. The mixture was stirred at room temperature for 17 hr. The reaction mixture was diluted with ethyl acetate (50 ml), washed successively with water (50 ml) and saturated aqueous sodium chloride solution (5 ml), dried and concentrated under reduced pressure. The obtained oily substance was purified by silica gel column chromatography (n-hexane/ethyl acetate=1:1). The oily substance (3.28 g) after purification was dissolved in dichloromethane (30 ml). This solution was cooled to 0°C and trifluoroacetic acid (30 ml) was added. The mixture was heated to room temperature and stirred for 3 hr. The reaction mixture was concentrated under reduced pressure, after which water (50 ml) was added, and the mixture was extracted with ethyl acetate (150 ml). The organic layer was washed successively with water (50 ml×3) and saturated aqueous sodium chloride solution (50 ml), dried and concentrated under reduced pressure. The obtained oily substance was purified by column chromatography to give the title compound (2.54 g, yield 89%) as a colorless oil.
¹H-NMR(δ, 300MHz, DMSO-d₆)
1.22(6H,s),4.20(2H,s), 5.13(2H,s),7.12(1H,m),7.25-7.34 (5H,m),7.60(1H,m),
7.99(1H,dd,J=1.6,7.9Hz),8.23(1H,d,J=7.9Hz),10.72(1H,s),13.63(1 H,brs).

### c) 3-[{2-(2-(5-chloro-1H-indole-2-carbonyl)hydrazinocarbonyl)phenyl}carbamoyloxy]-2,2-dimethylpropionic acid benzyl ester

5-Chloro-1H-indole-2-carboxylic acid hydrazide (containing 14% DMF) obtained in Example 1 a) (1.59 g), 2-(2-benzyloxycarbonyl-2-methylpropoxycarbonylamino)benzoic acid obtained in Example 1-4 b) (2.54 g) and HOBt·H₂O (1.23 g) were dissolved in DMF (25 ml). To this solution was added EDC (1.59 g), and the mixture was stirred at room temperature for 3 hr. The reaction mixture was diluted with ethyl acetate (150 ml) and washed with water (50 ml) and saturated aqueous sodium chloride solution (50 ml). This solution was dried over anhydrous sodium sulfate and concentrated under reduced pressure. The obtained solid was washed with ether in a slurry form to give the title compound (2.58 g, yield 78%) as a colorless amorphous form.
¹H-NMR(σ, 300MHz, DMSO-d₆)
1.20(6H,s),4.18(2H,s),5.11.(2H,s),7.14-7.29(8H,m),7.48(1H,d,J=8.8Hz),
7.60(1H,d,J=7.3Hz),7.77(1H,d,J=1.8Hz),7.91(1H,d,J=7.3Hz),8.24( 1H,d,J=8.4Hz),10.62(1H,s),10.72(1H,s),10.82(1H,s),11.97(1H,s).

### d) 3-[{2-(2-(5-chloro-2H-indole-2-carbonyl)hydrazinocarbonyl)phenyl}carbamoyloxy]-2,2-dimethylpropionic acid

3-[{2-(2-(5-Chloro-1H-indole-2-carbonyl)hydrazinocarbonyl)phenyl}carbamoyloxy]-2,2-dimethylpropionic acid benzyl ester obtained in Example 1-4 c) (1.64 g) was dissolved in THF (50 ml). To this solution was added 10% palladium-carbon (224 mg) and the mixture was stirred under a hydrogen atmosphere at room temperature for 6 hr and 30 min. The reaction mixture was filtered through a glass filter packed with celite and the residue was washed with THF (25 ml). The filtrate and washings were combined and concentrated under reduced pressure. The obtained solid was crystallized from ethanol to give the title compound (831 mg, yield 60%) as colorless needle crystals (see Table 1).

### Example 1-5

### benzoic acid 2-(5-chloro-1H-indole-2-carbonyl)-1-methylhydrazide

### a)benzoic acid 1-methylhydrazide

To a solution (10 ml) of benzoic acid (500 mg) in DMF were added 1-hydroxybenzotriazole (752 mg) and EDC (942 mg), and the mixture was stirred at room temperature for 3 hr. Then, methylhydrazine (2.18 ml) was added to the reaction solution under ice-cooling. The mixture was stirred at room temperature for 16 hr, and water was added. This mixture was extracted with ethyl acetate and the organic layer was washed successively with saturated aqueous sodium hydrogen carbonate, water and saturated brine and dried over sodium sulfate. This solution was filtered, concentrated under reduced pressure and the residue was purified by silica gel column chromatography (n-hexane :ethyl acetate=1:2) to give the title compound (213 mg, yield 35%).
¹H-NMR (δ, 300MHz,DMSO-d₆).
3.16(3H,s),4.77(1H,brs),5.10(1H,brs),7.32-7.52(5H,m).

### b) benzoic acid 2-(5-chloro-1H-indole-2-carbonyl)-1-methylhydrazide

To a solution (10 ml) of 5-chloro-1H-indole-2-carboxylic acid (100 mg) and benzoic acid 1-methylhydrazide obtained in Example 1-5 a) (130 mg) in DMF were added 1-hydroxybenzotriazole (122 mg) and EDC (153 mg). The mixture was stirred at room temperature for 16 hr and water was added to the reaction solution. The precipitated solid was collected by filtration and washed with water and diethyl ether. The resulting solid was dried in vacuo to give the title compound (144 mg, yield 66%) (see Table 1).

### Example 1-6

### benzoic acid 2-(1-acetyl-5-chloro-1H-indole-2-carbonyl)hydrazide

### a)1-acetyl-5-chloro-1H-indole-2-carboxylic acid

5-Chloro-1H-indole-2-carboxylic acid (5.50 g) was dissolved in dry DMF (50 ml) and ice-cooled. Sodium hydride (60% in oil) (2.35 g) was added to this solution. The mixture was stirred at room temperature for 20 min. This solution was cooled again in an ice bath and acetyl chloride (2.3 ml) was added dropwise. This solution was stirred at room temperature for 1 hr, and cooled in an ice bath. Acetic acid (4 ml) was added to this solution, after which ethyl acetate and ice water (each 200 ml) were added. The separated organic layer was washed with water and dried over anhydrous sodium sulfate. The extract solution was filtered and concentrated. The thick solution was stood to give pale-yellow crystals. The crystals were collected by filtration and dried in vacuo to give the title compound (3.12 g, yield 49%).
¹H-NMR (δ, 300MHz,DMSO-d₆).
2.77 (3H,s) ,7.37 (3H,s) ,7.49 (1H,m),7.82 (1H,m),7.97 (1H,d)

### b) benzoic acid 2-(1-acetyl-5-chloro-1H-indole-2-carbonyl)hydrazide

In the same manner as in Example 1-5, the title compound (yield 17%) was obtained by reacting 1-acetyl-5-chloro-1H-indole-2-carboxylic acid with benzoic acid hydrazide (see Table 1).

### Example 1-7

### 5-chloro-1H-indole-2-carboxylic acid 2-(imino-phenyl-methyl)hydrazide

### a) thiobenzimide acid methyl ester hydroiodic acid salt

To a solution (60 ml) of thiobenzamide (10.0 g) in acetone was added methyl iodide (10.3 g) at room temperature. This solution was stirred at room temperature for 13 hr. The precipitated crystals were collected by filtration, washed with diethyl ether and dried in vacuo to give the title compound (18.5 g, yield 91%).

### b) 5-chloro-1H-indole-2-carboxylic acid 2-(imino-phenyl-methyl)hydrazide

To a suspension (700 ml) of 5-chloro-1H-indole-2-carboxylic acid hydrazide obtained in Example 1 a) (70.0 g) in methanol (MeOH) was added thiobenzimide acid methyl ester hydroiodic acid salt obtained in Example 1-7 a) (84.2 g) at room temperature. This suspension was stirred at room temperature for 19 hr. To the reaction mixture was added aqueous sodium hydrogen carbonate to alkalize the mixture. The precipitated solid was collected by filtration, washed with water and washed with diethyl ether. The product collected by filtration was boiled in THF-MeOH and allowed to cool. Diisopropyl ether was added and the precipitated solid was collected by filtration and dried to give the title compound (76.8 g, yield 85%) (see Table 1).

### Example 1-8

### 5-aminothiazole-4-carboxylic acid 2-(5-chloro-1H-indole-2-carbonyl)hydrazide

### a) 5-aminothiazole-4-carboxylic acid hydrazine

5-Amino-thiazole-4-carboxylic acid ethyl ester (460 mg) obtained in the same manner as in Chem. Pharm. Bull, 19(1) 119-123 (1971) was dissolved in ethanol (7 ml). Hydrazine monohydrate (1.3 ml) was added to this solution and the mixture was refluxed at 100°C for 22 hr. The reaction mixture was cooled to room temperature and the obtained solid was collected by filtration and washed with ethanol. This solid was dried in vacuo to give the title compound (280 mg).
¹H-NMR(σ, 300MHz, DMSO-d6)
4.26(2H,s),7.07(2H,s),8.00(1H,s),8.83(1H,s).

### b)5-aminothiazole-4-carboxylic acid 2-(5-chloro-1H-indole-2-carbonyl)hydrazide

In the same manner as in Example 1-2, 5-chloro-1H-indole-2-carboxylic acid was reacted with 5-aminothiazole-4-carboxylic acid hydrazine obtained in Example 1-7 a) to give the title compound (yield 79%) (see Table 1).

### Examples 1-9 to 1-143

In the same manner as in Examples 1 to 1-8, the compounds of Examples 1-9 to 1-143 were obtained. The obtained compounds are shown in Tables 1-18.

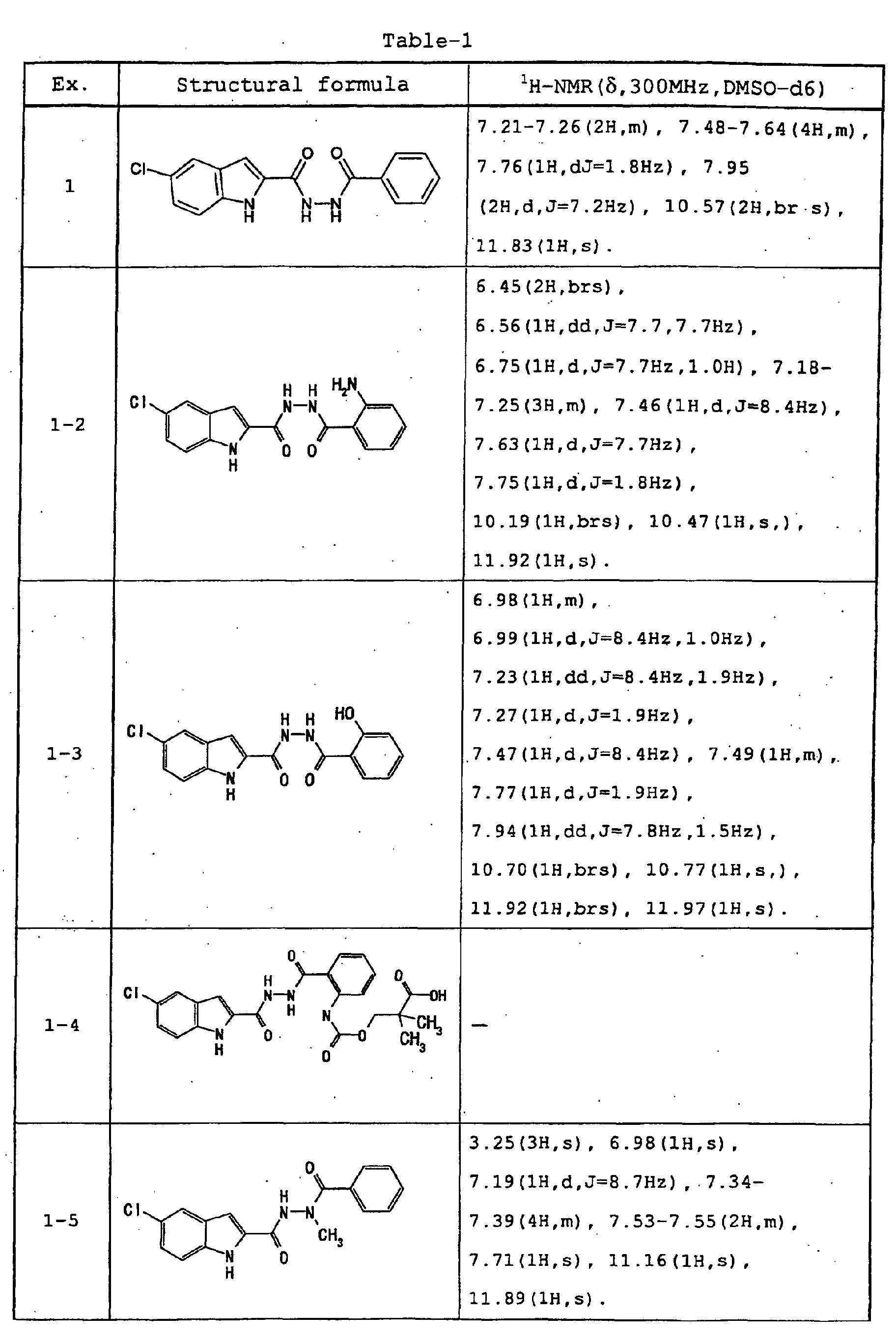

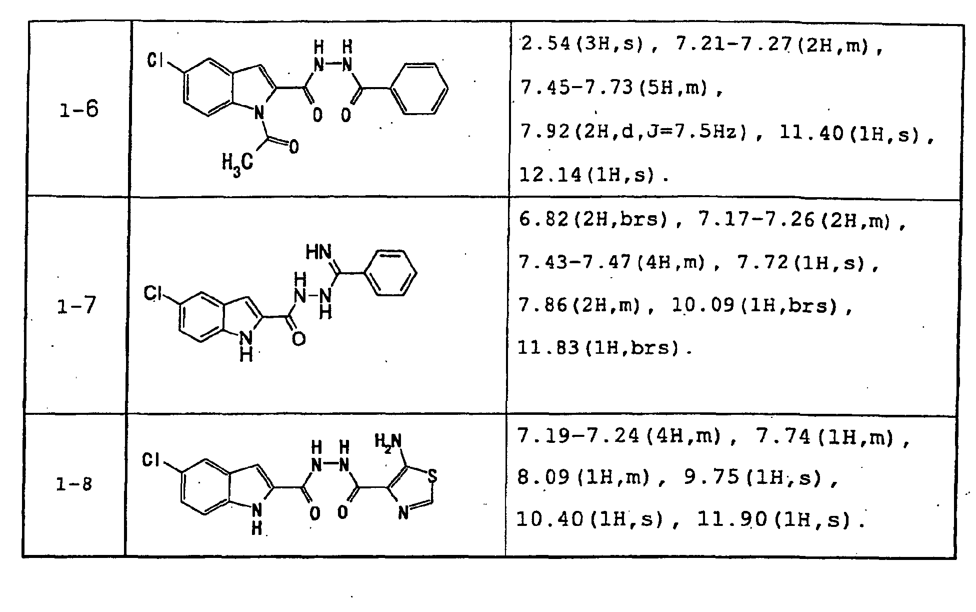

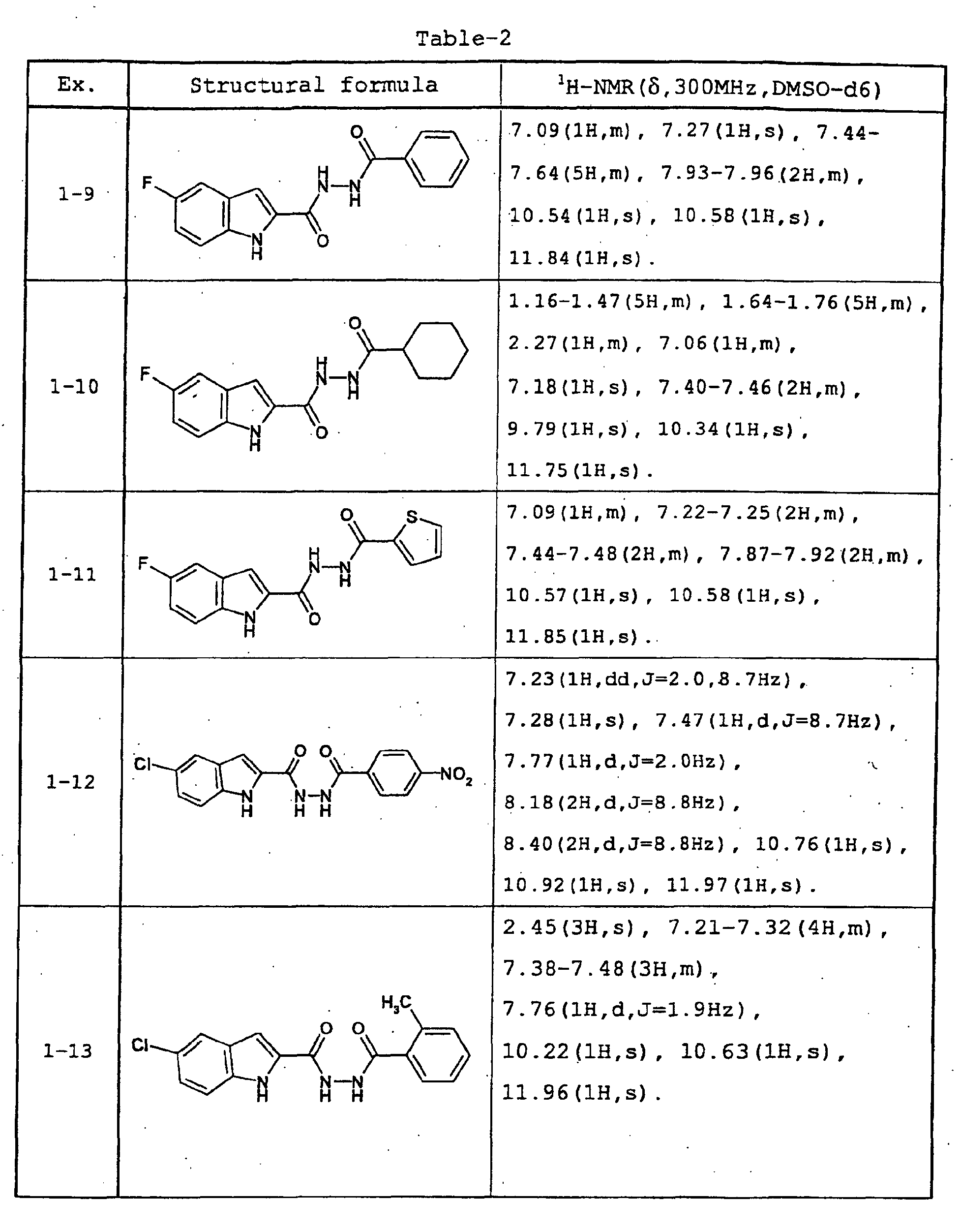

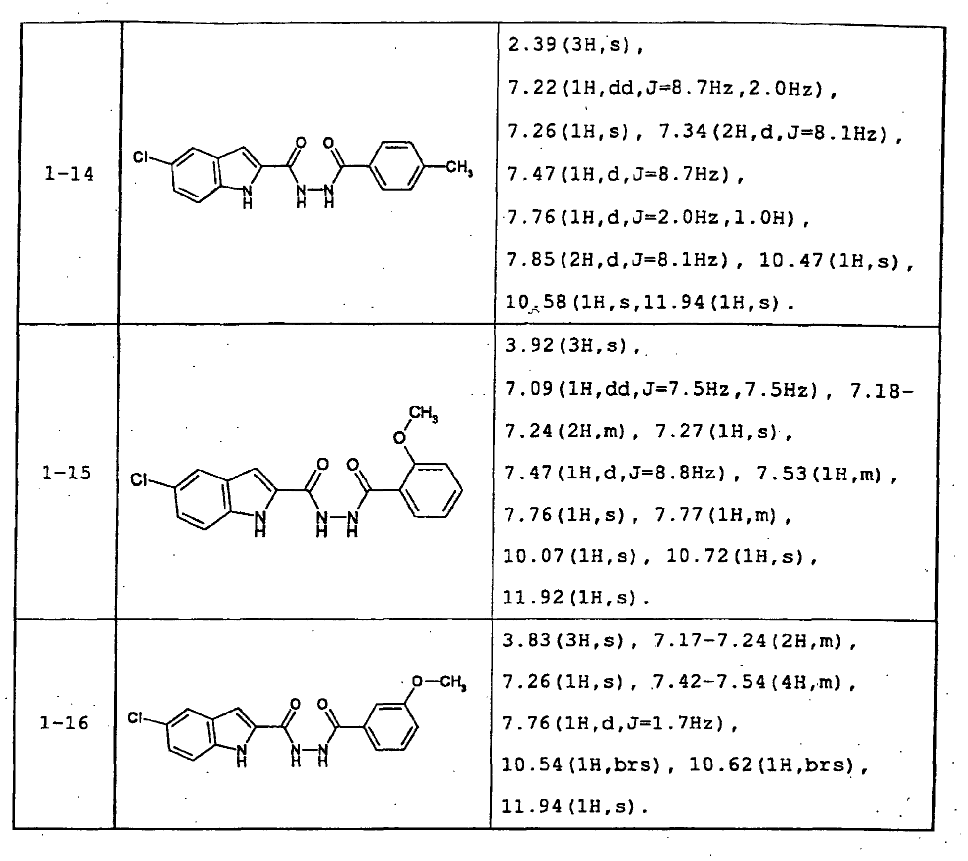

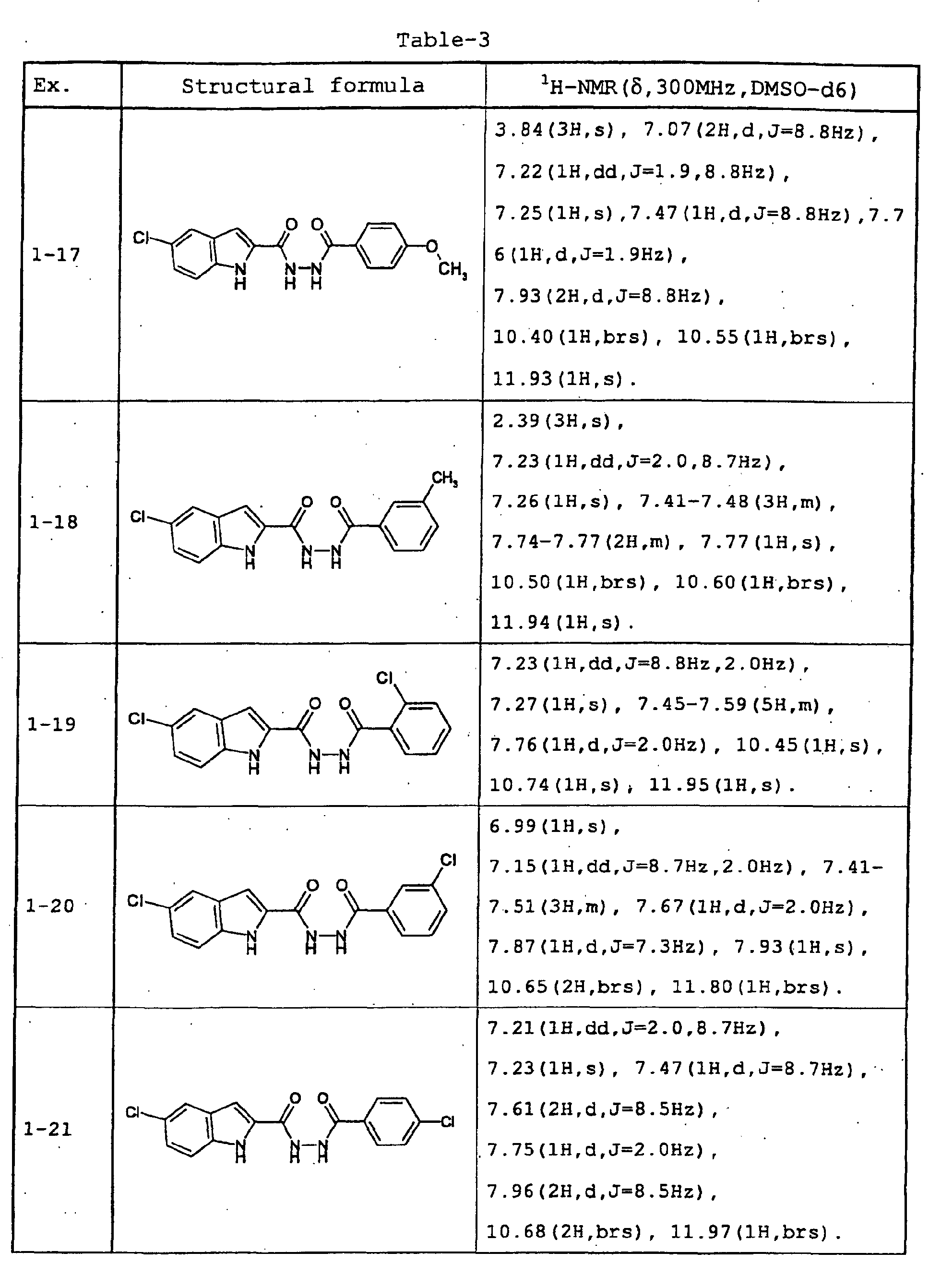

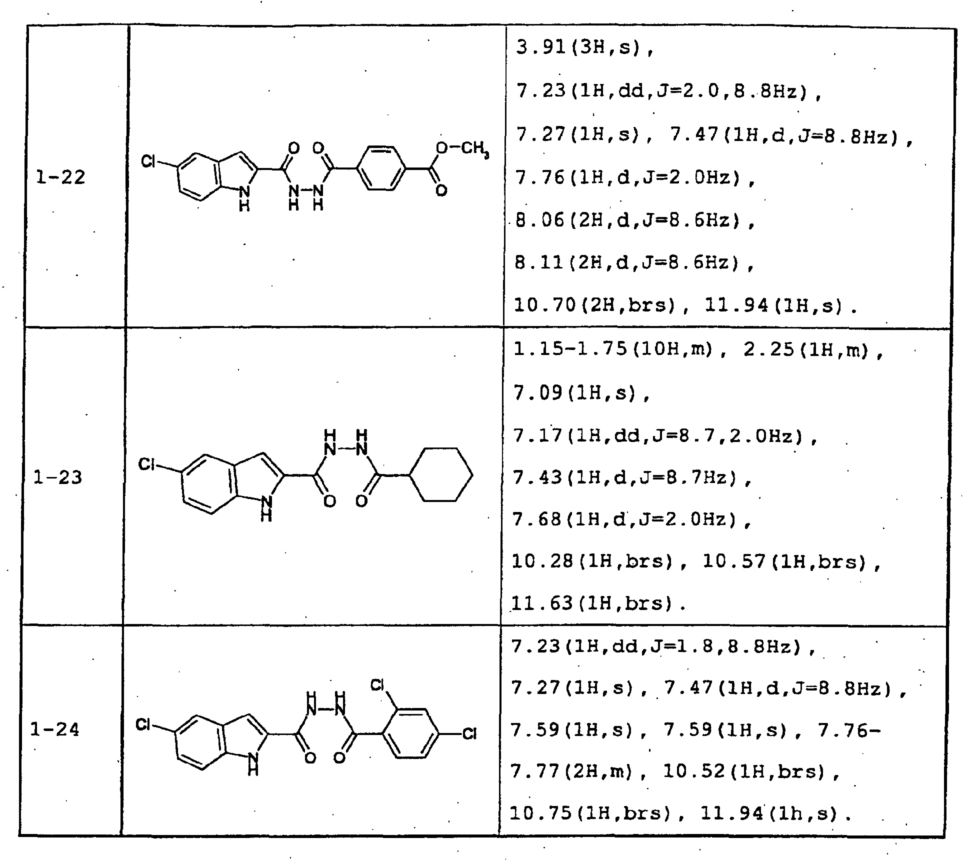

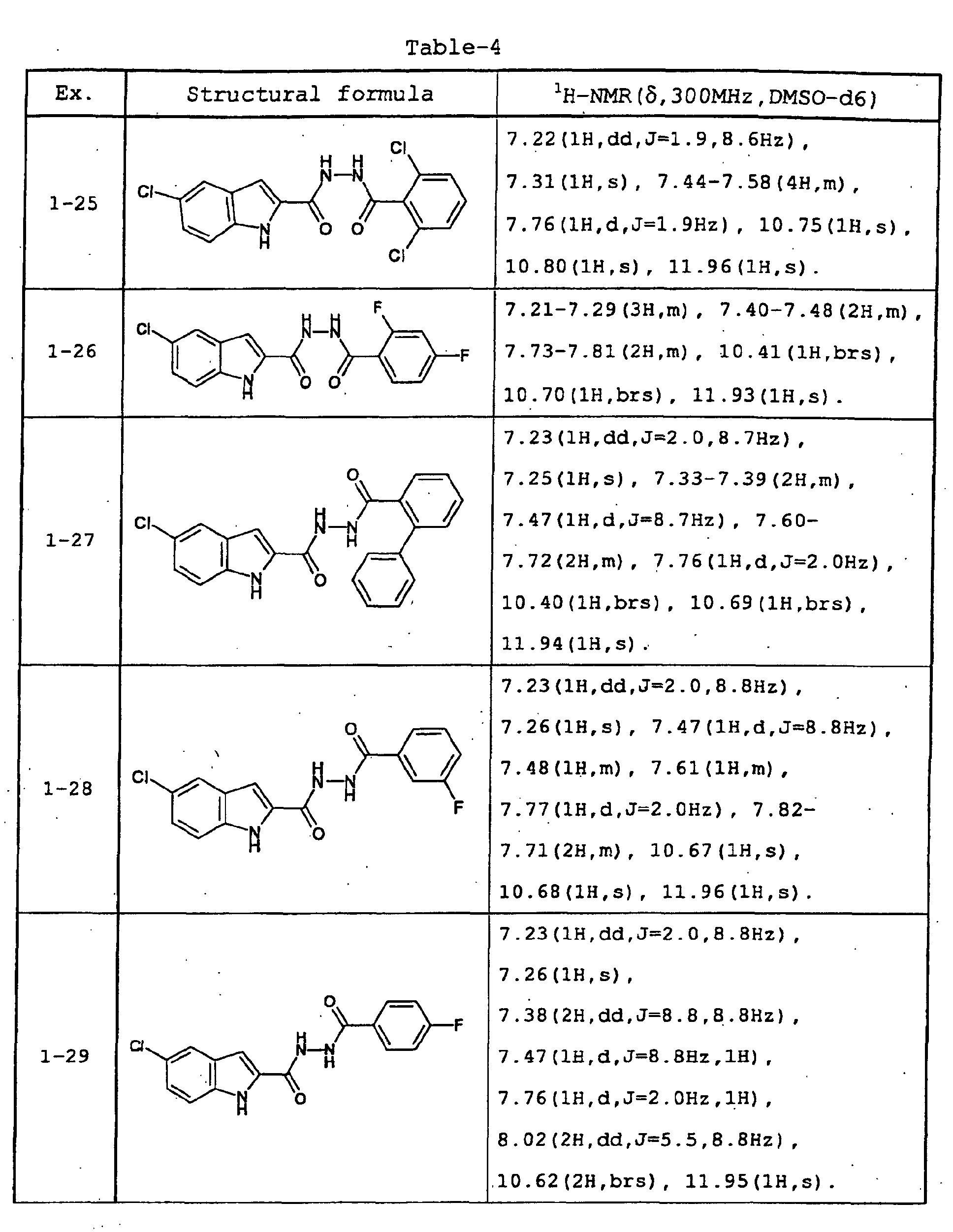

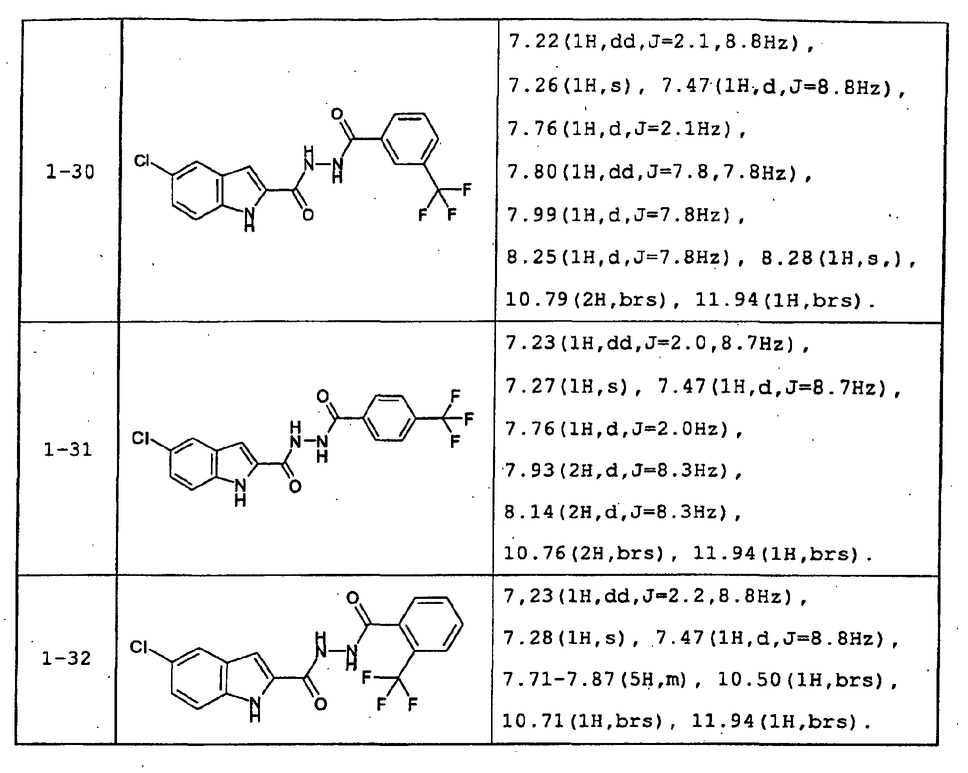

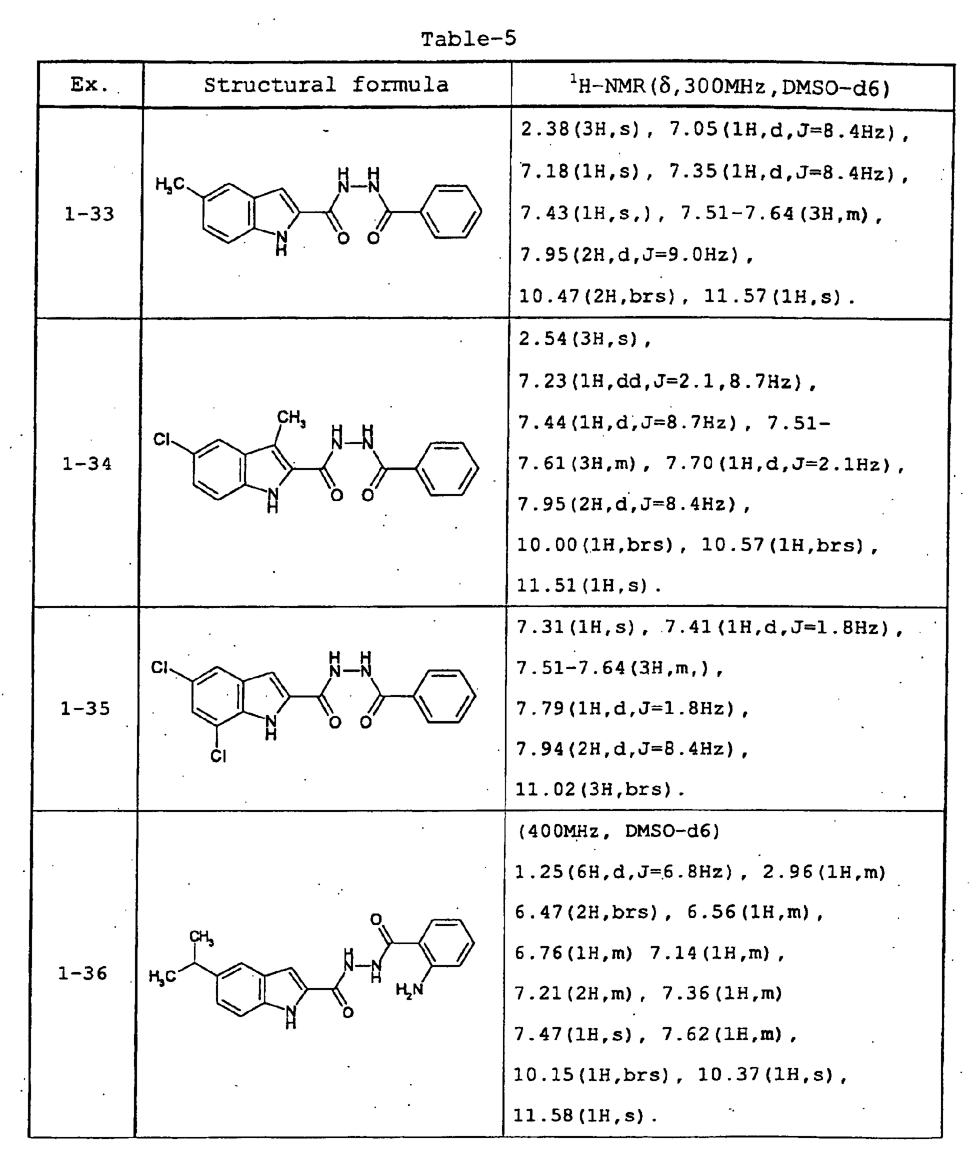

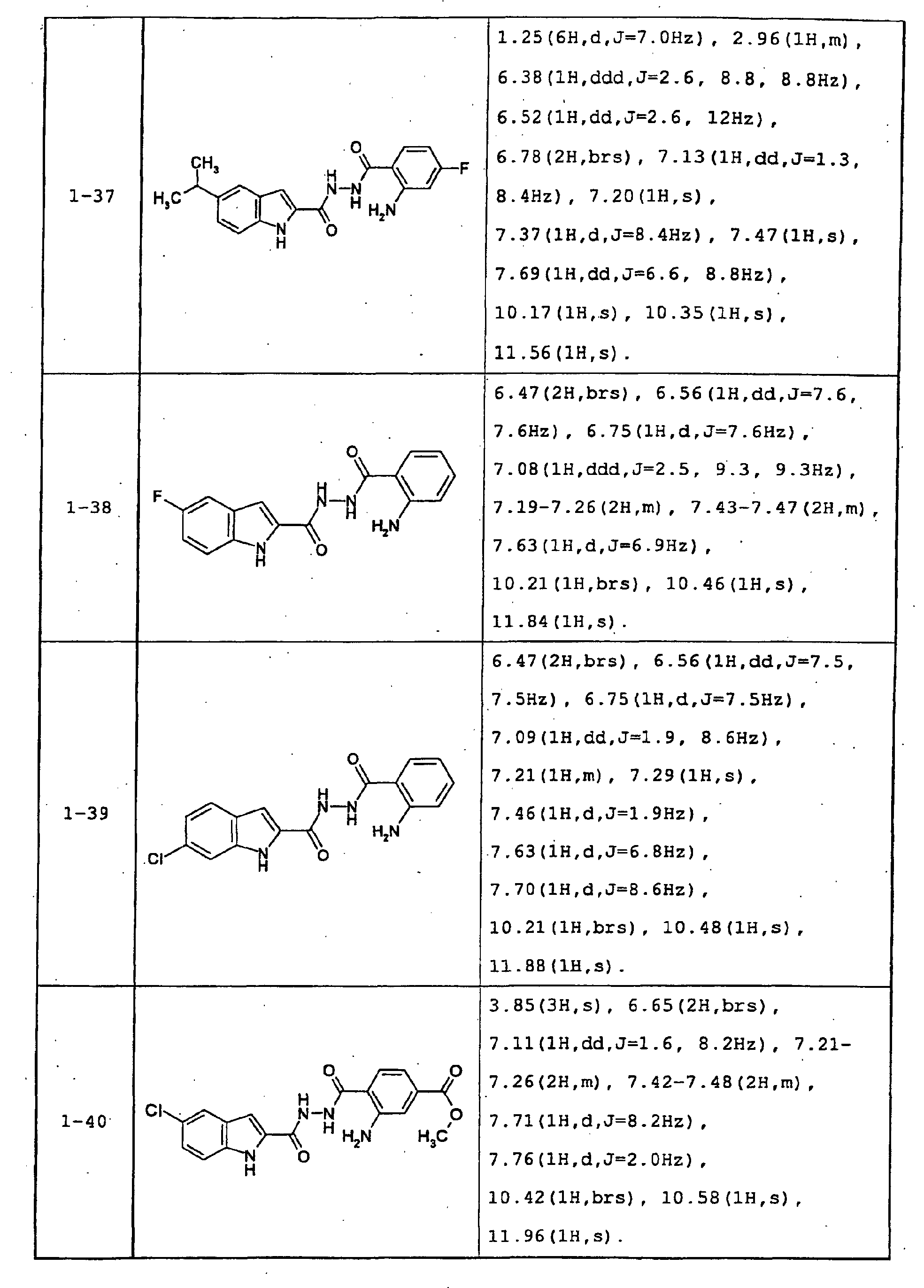

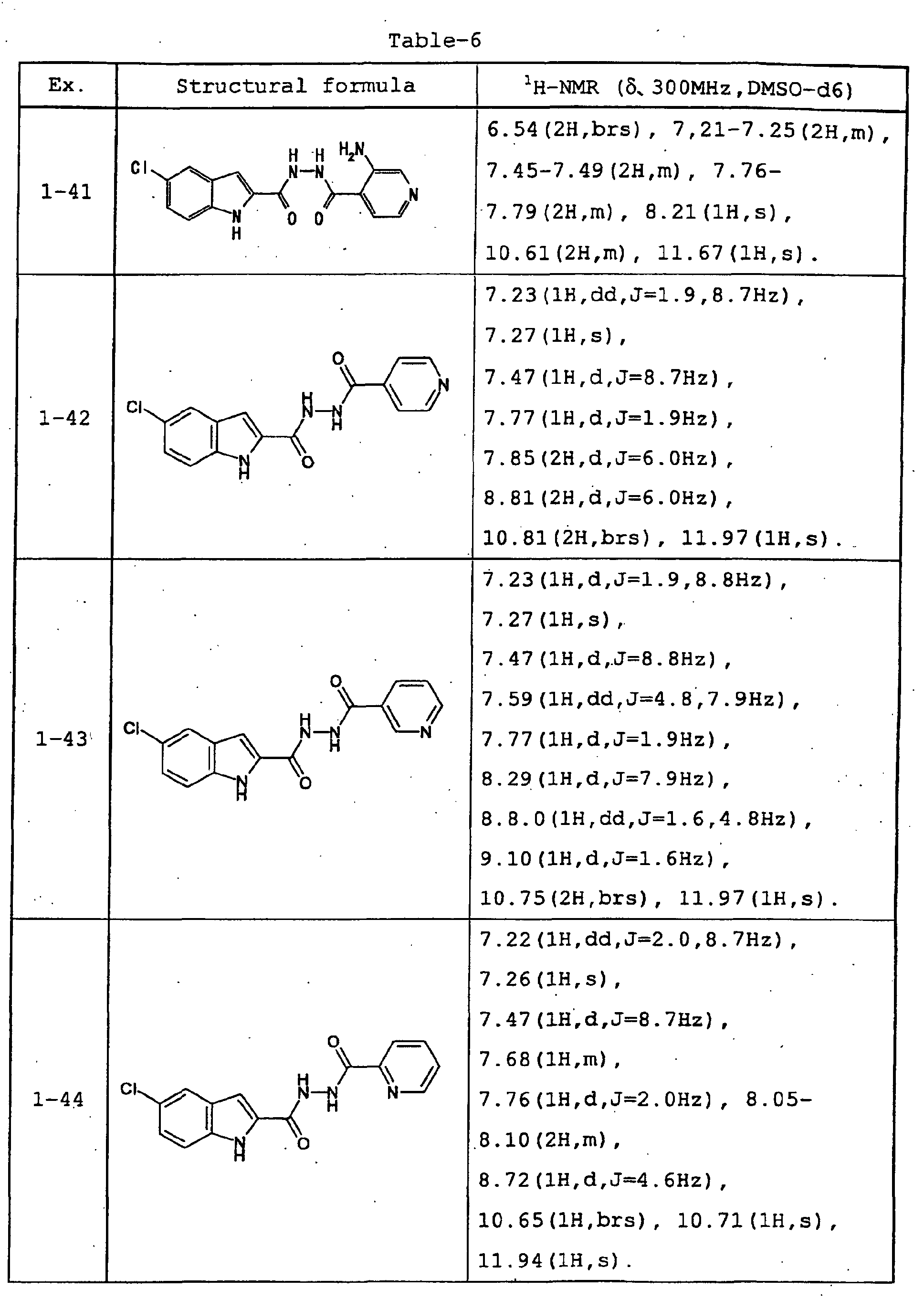

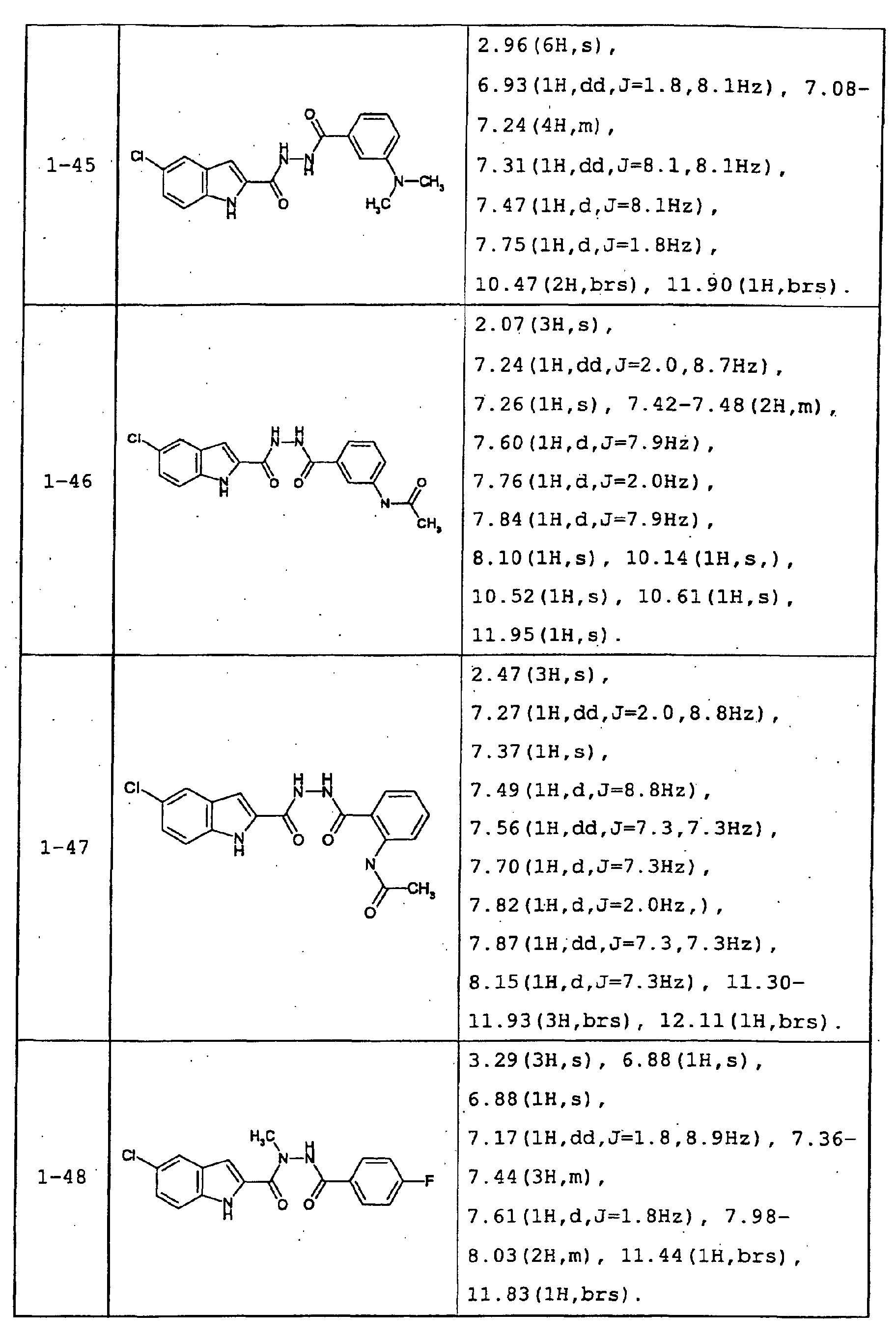

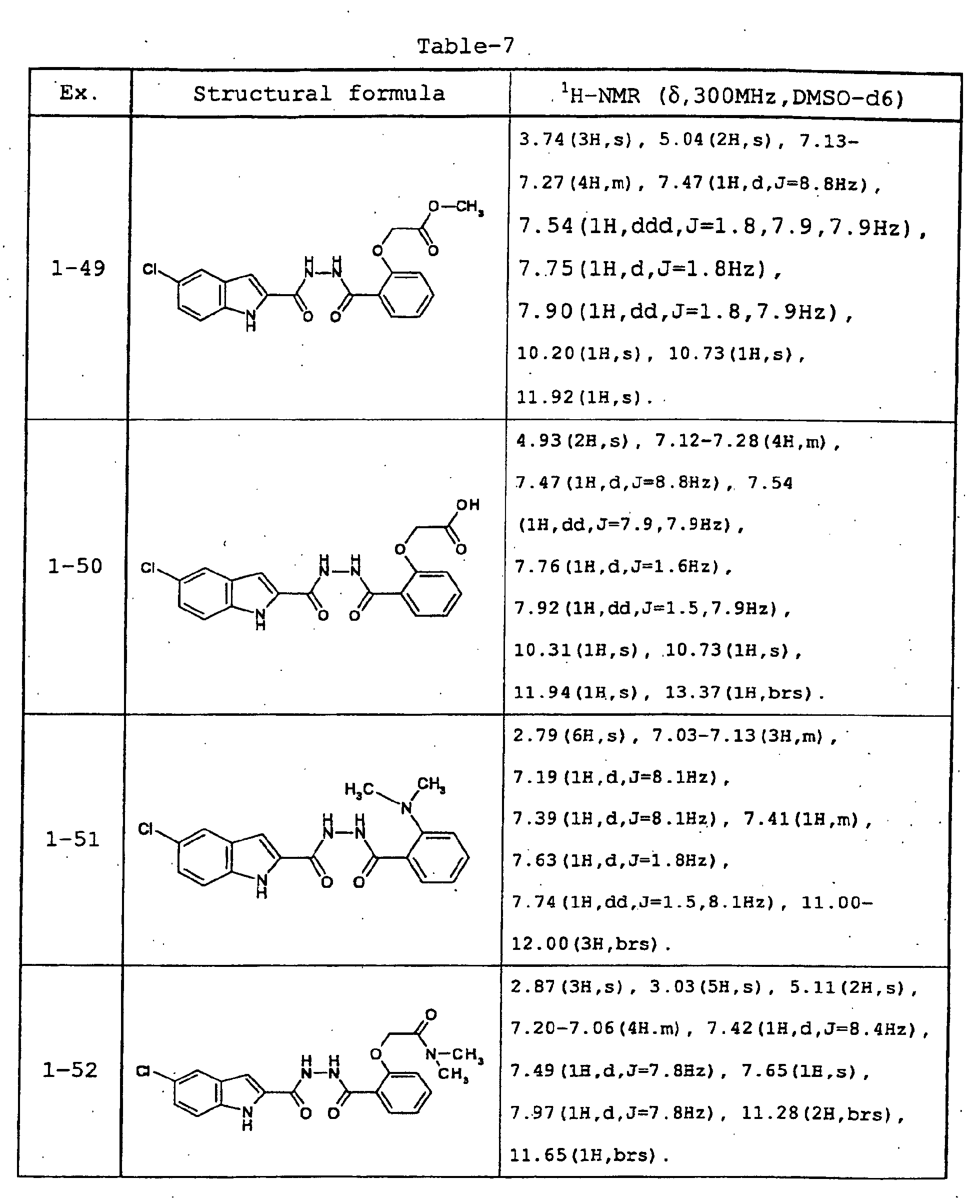

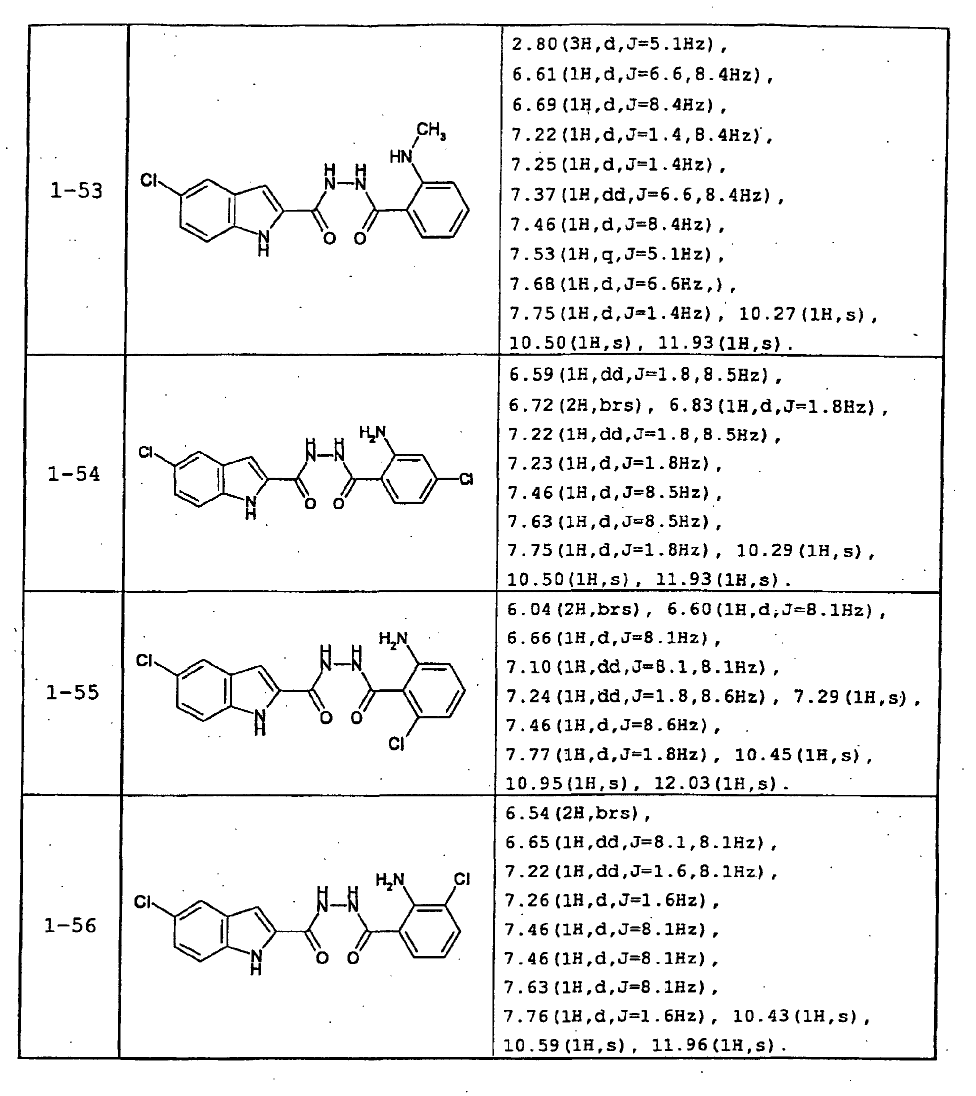

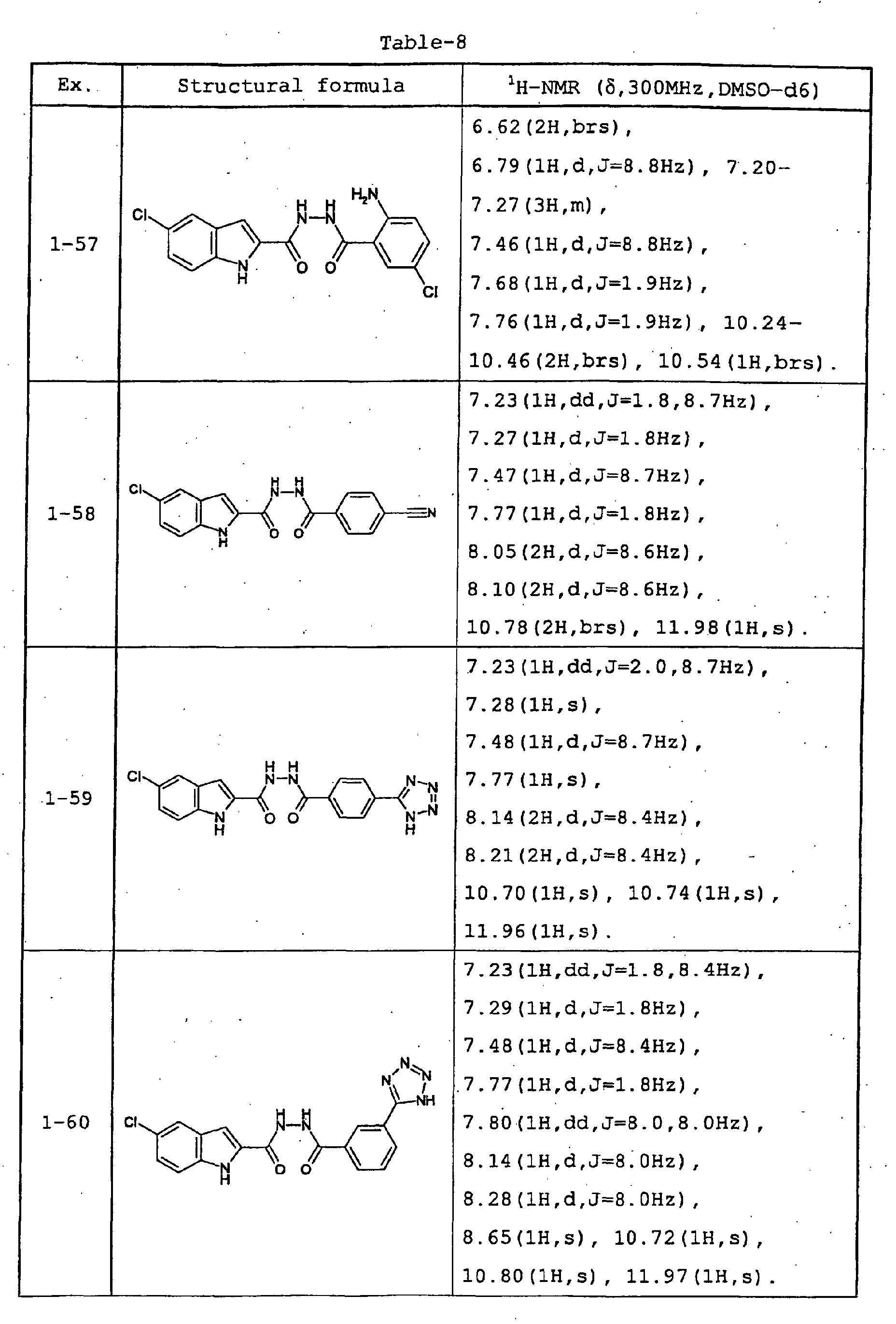

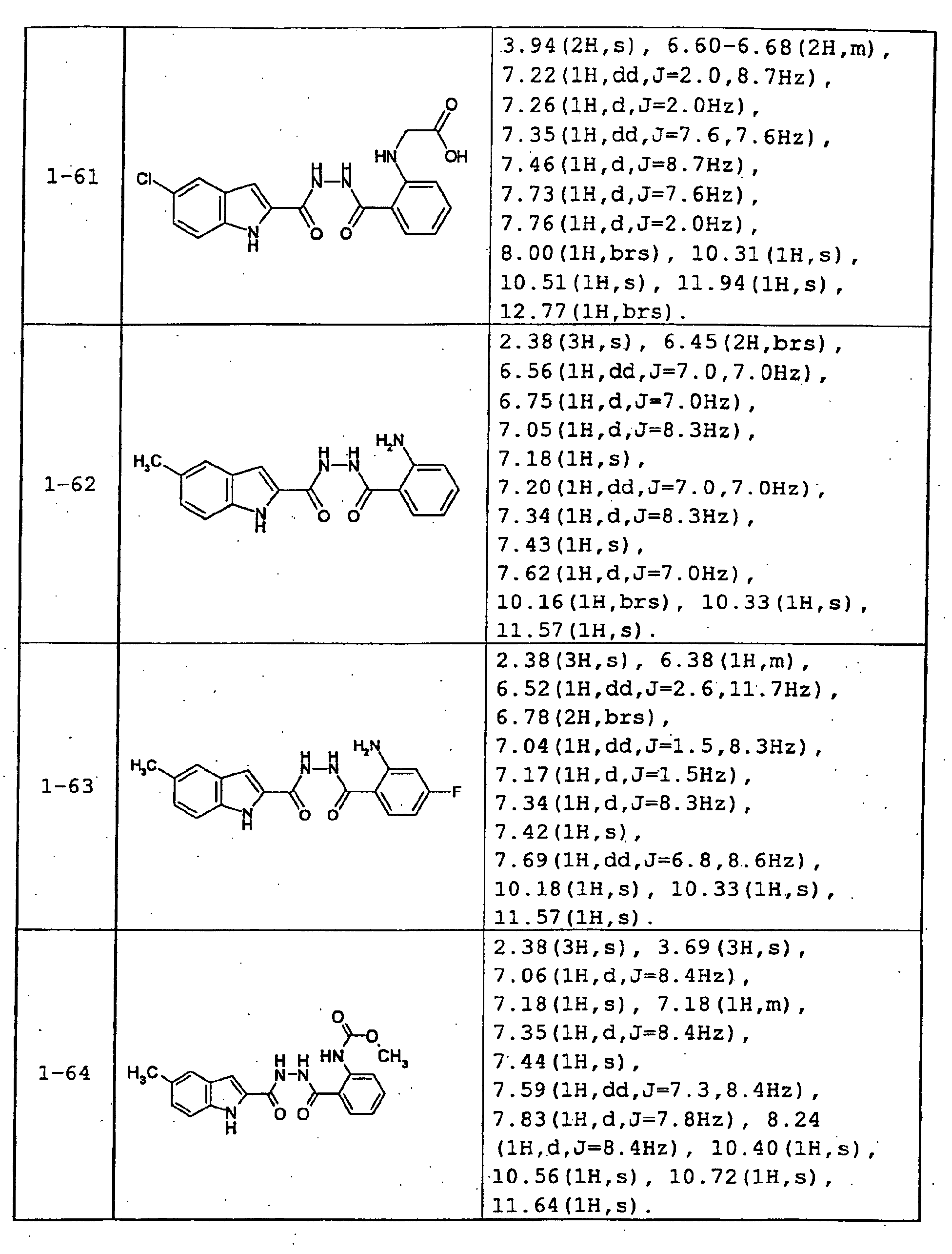

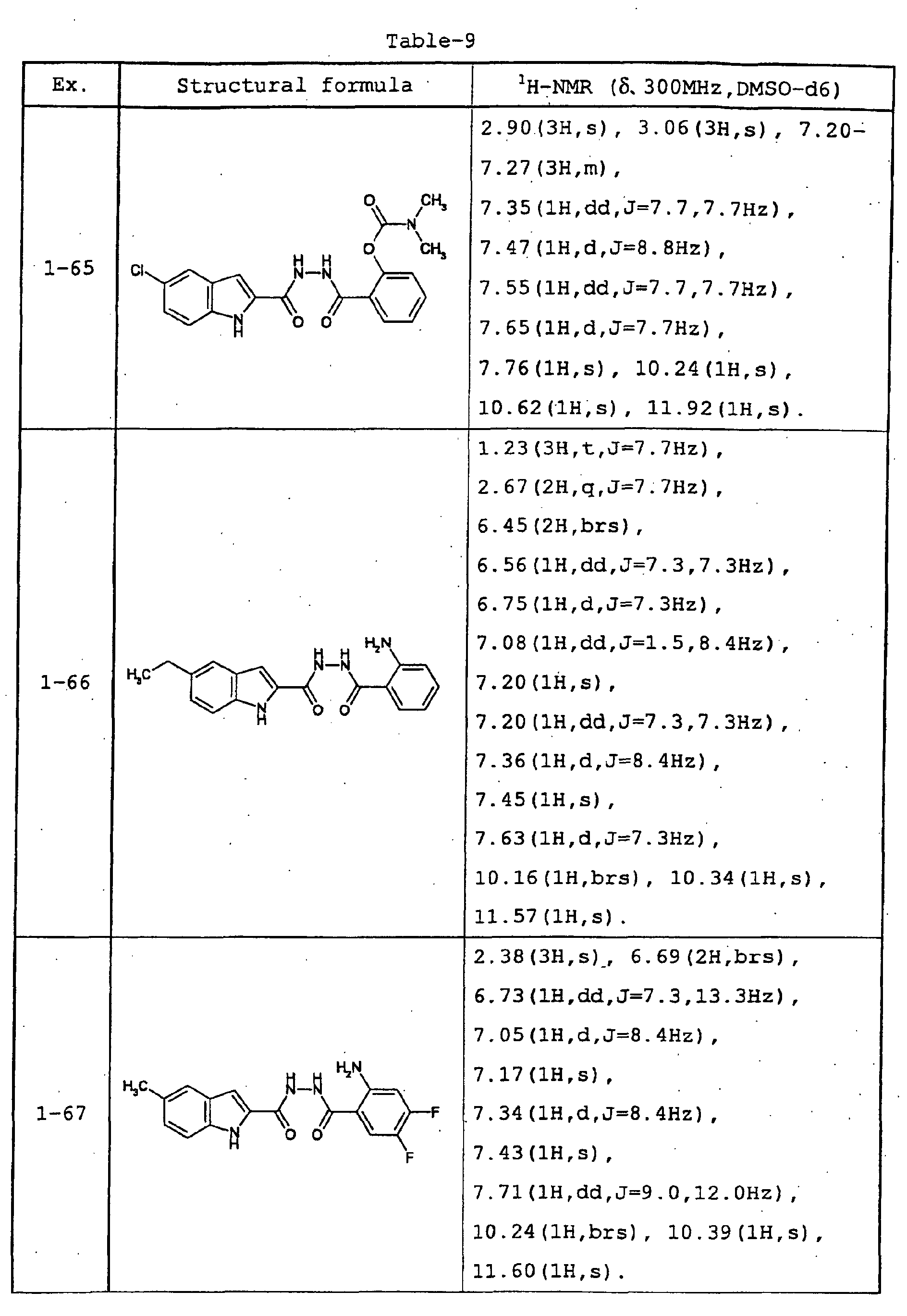

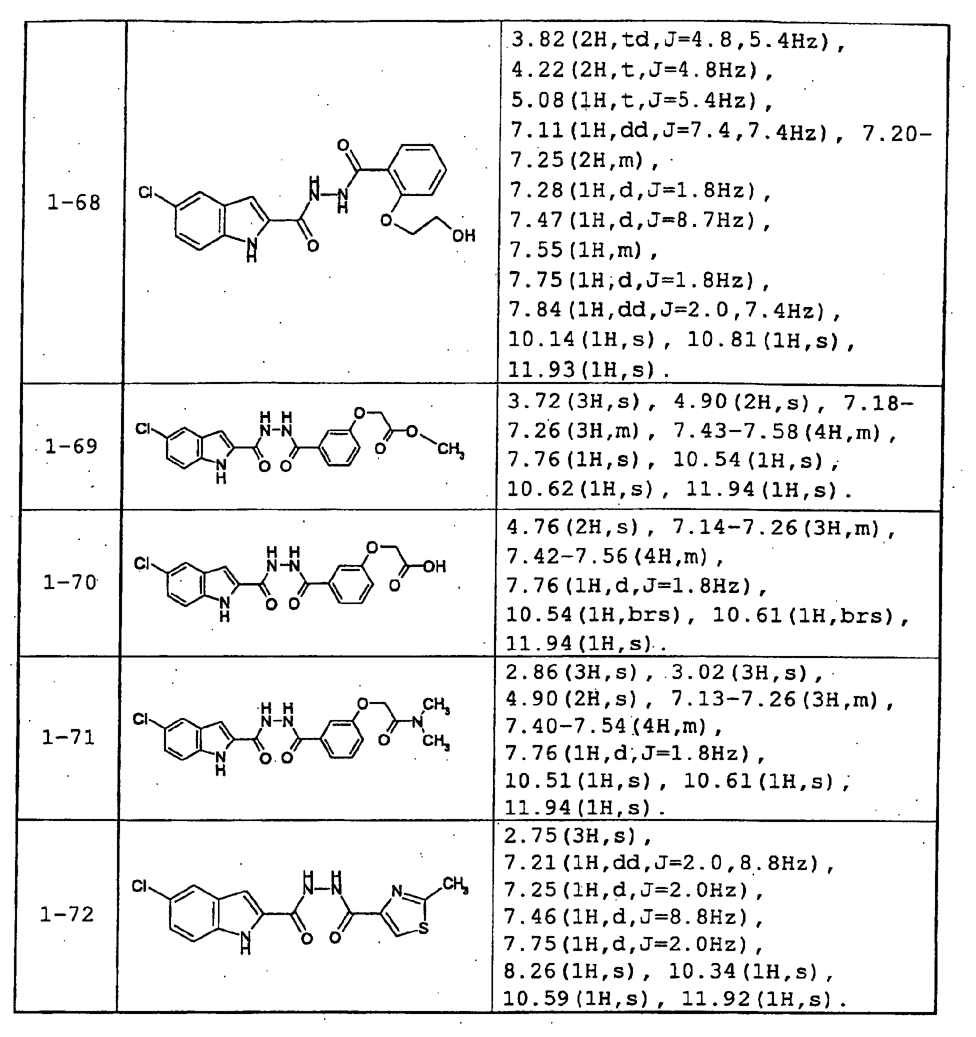

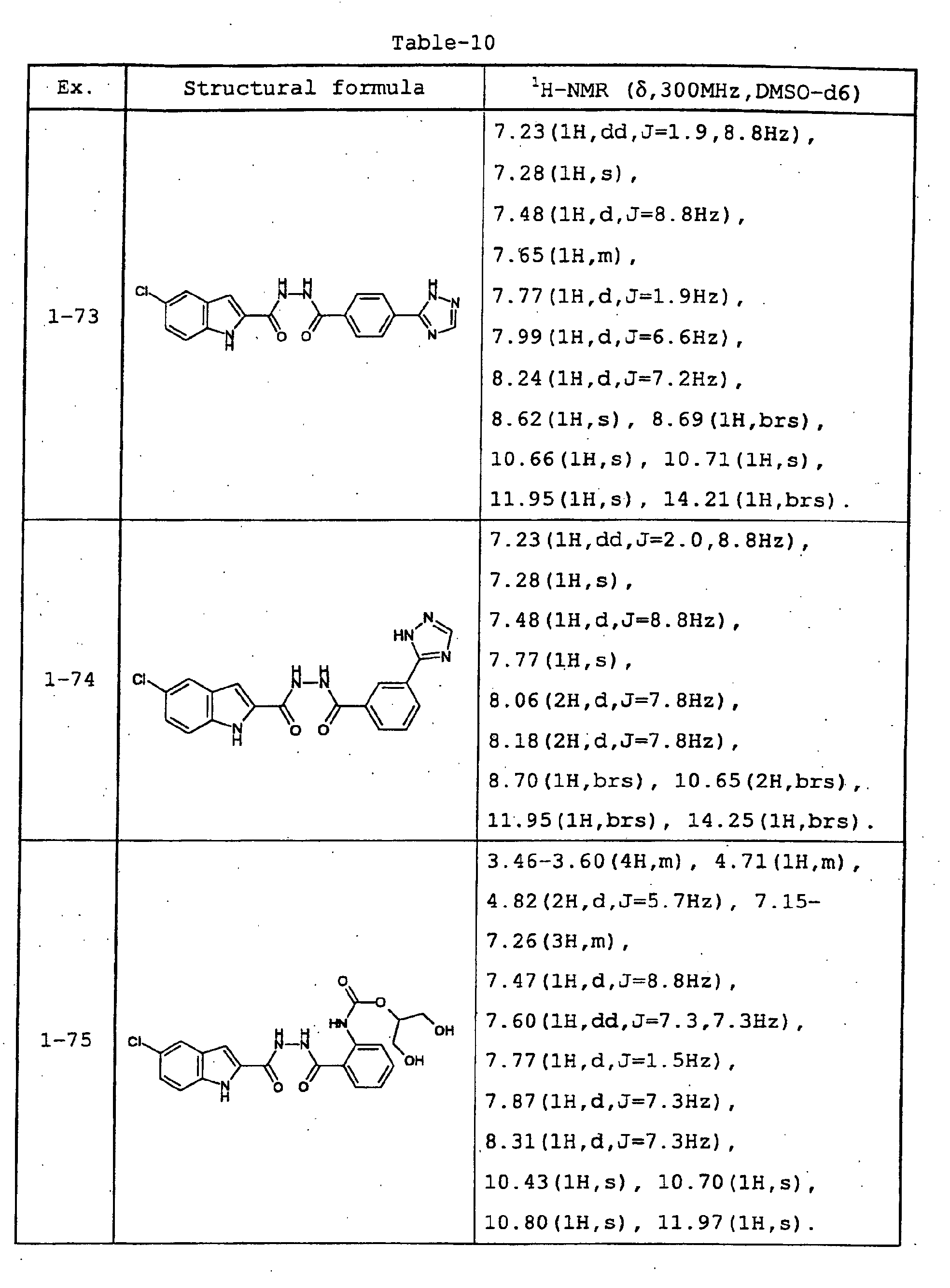

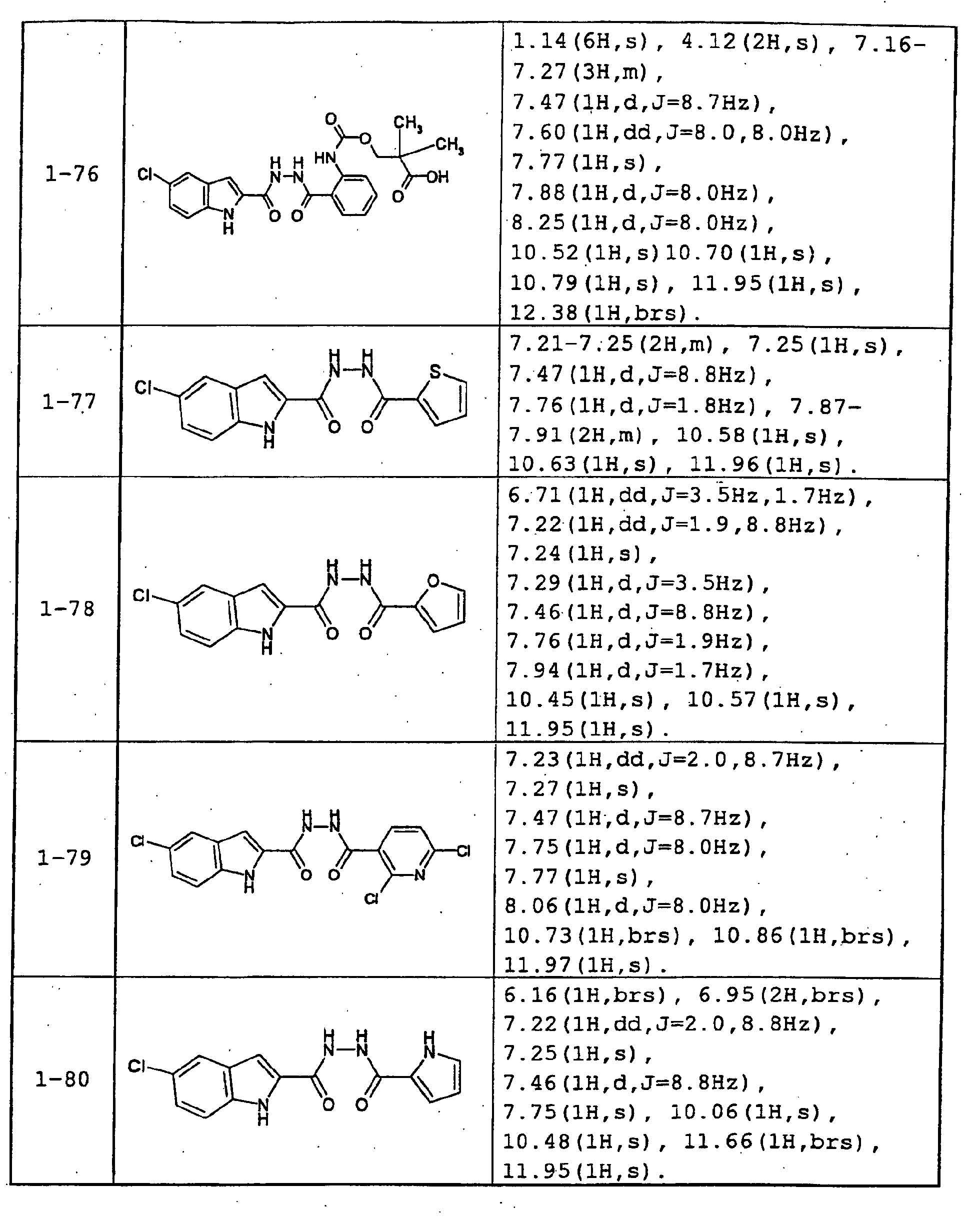

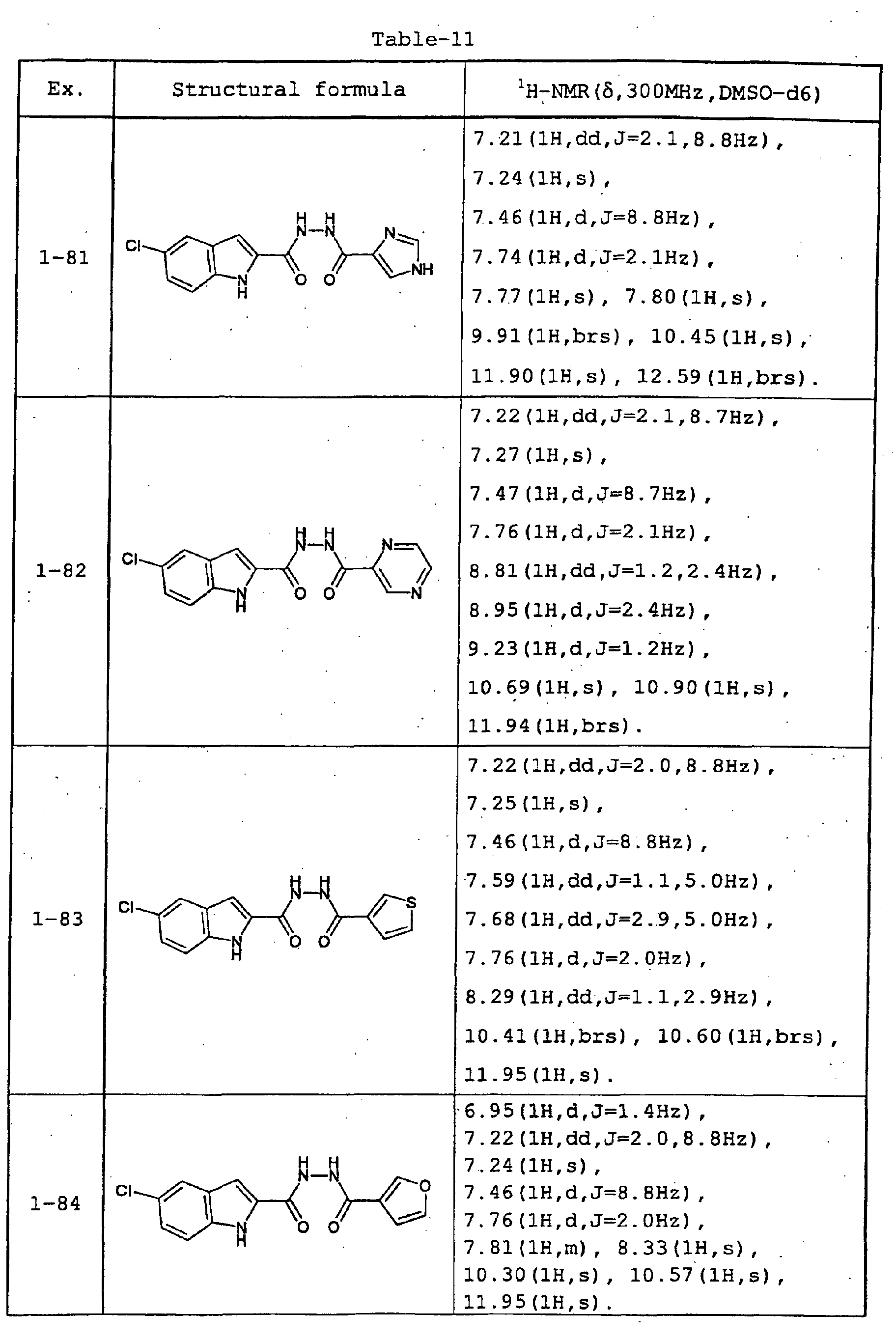

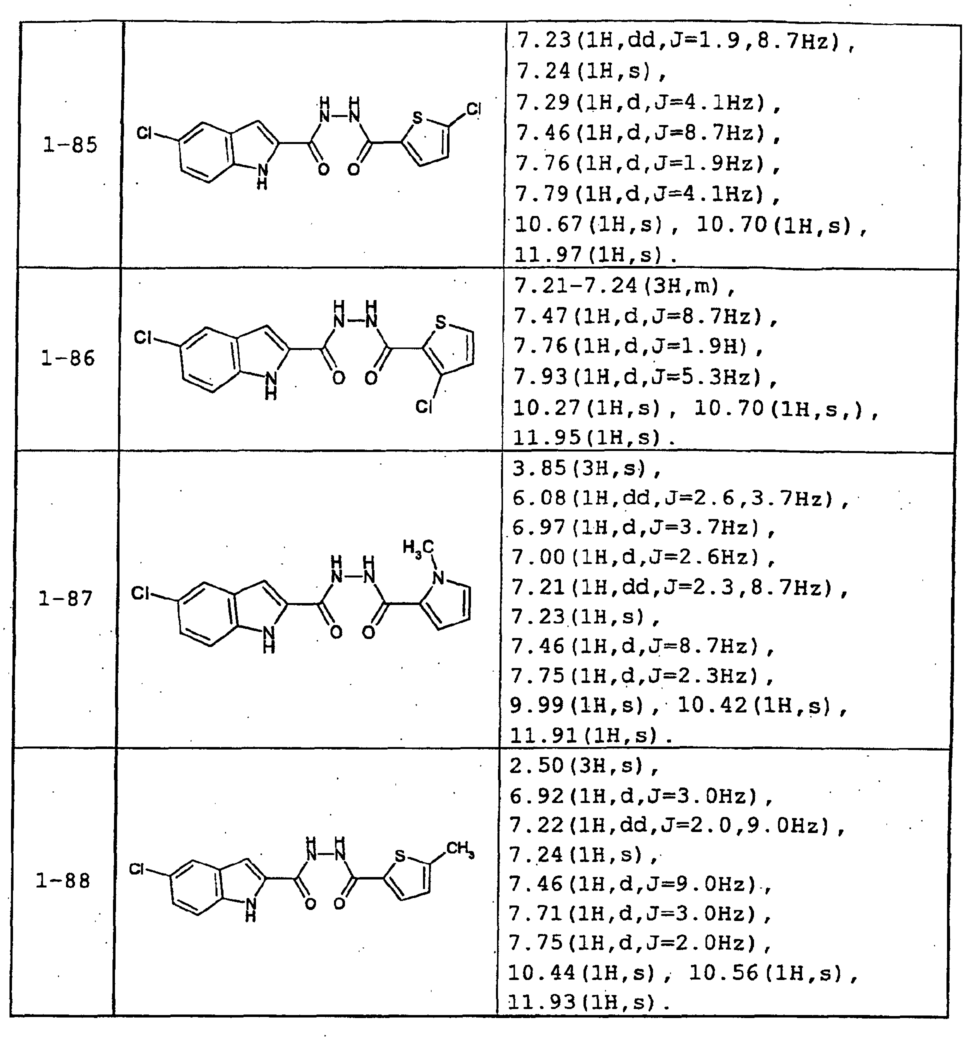

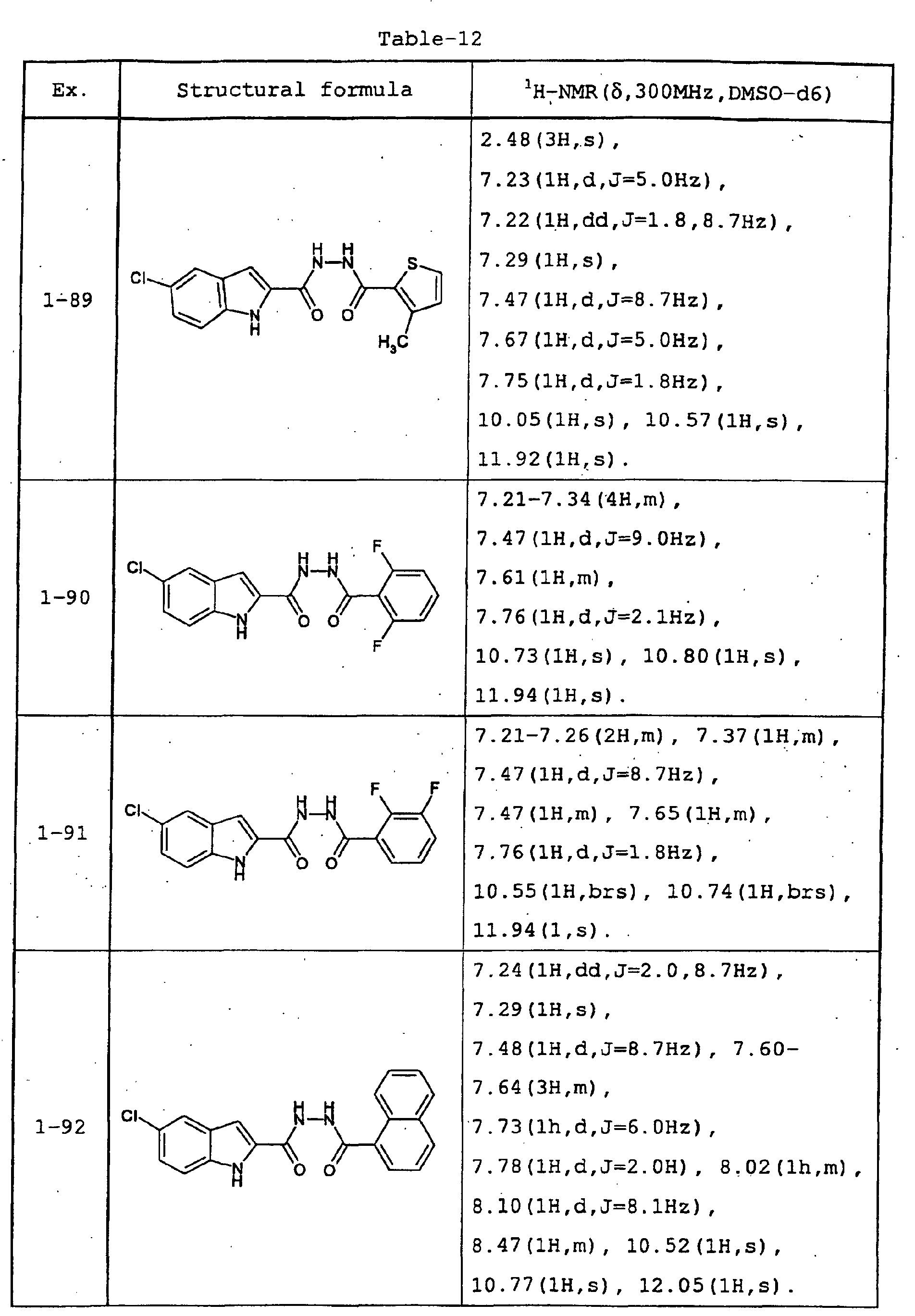

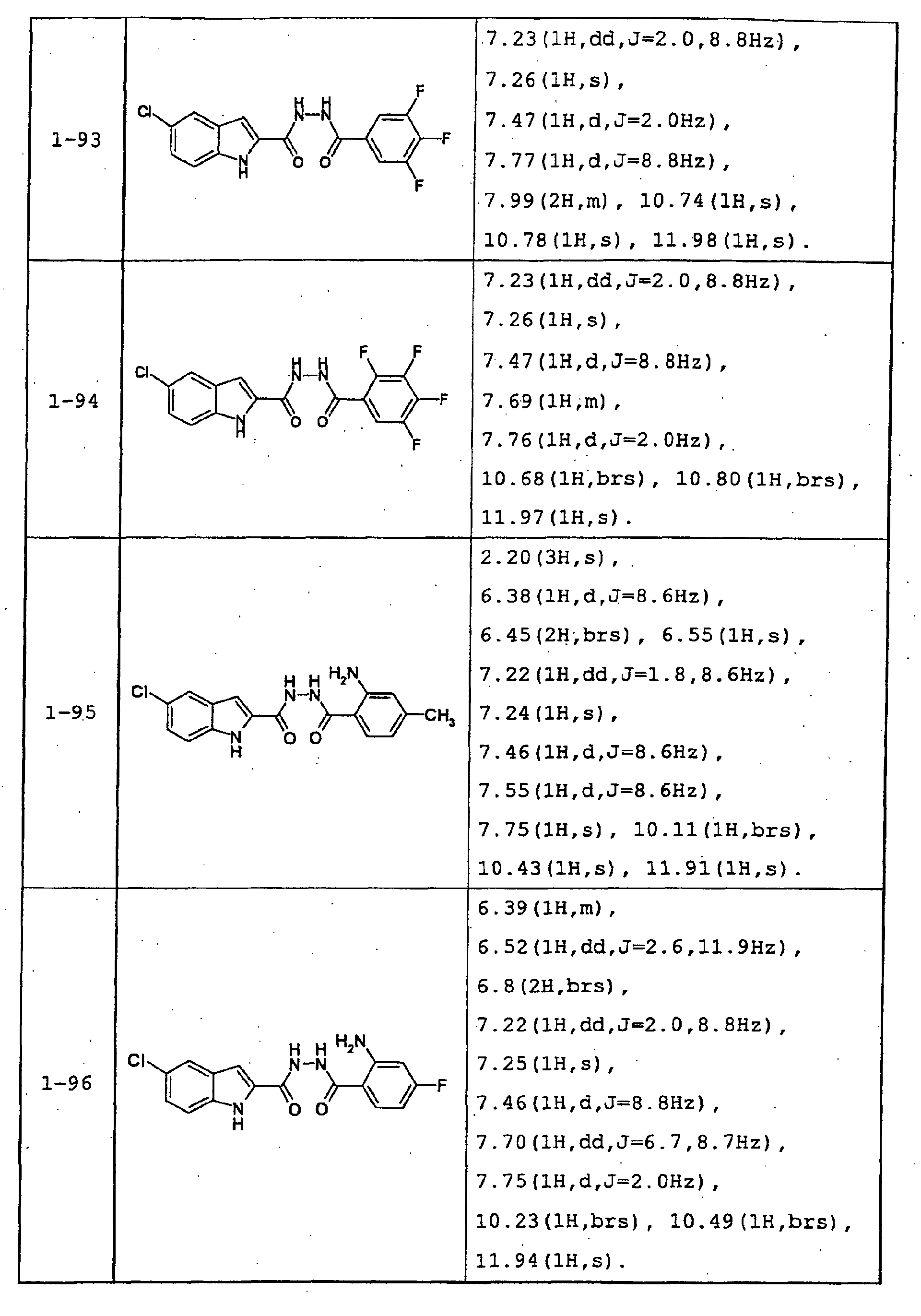

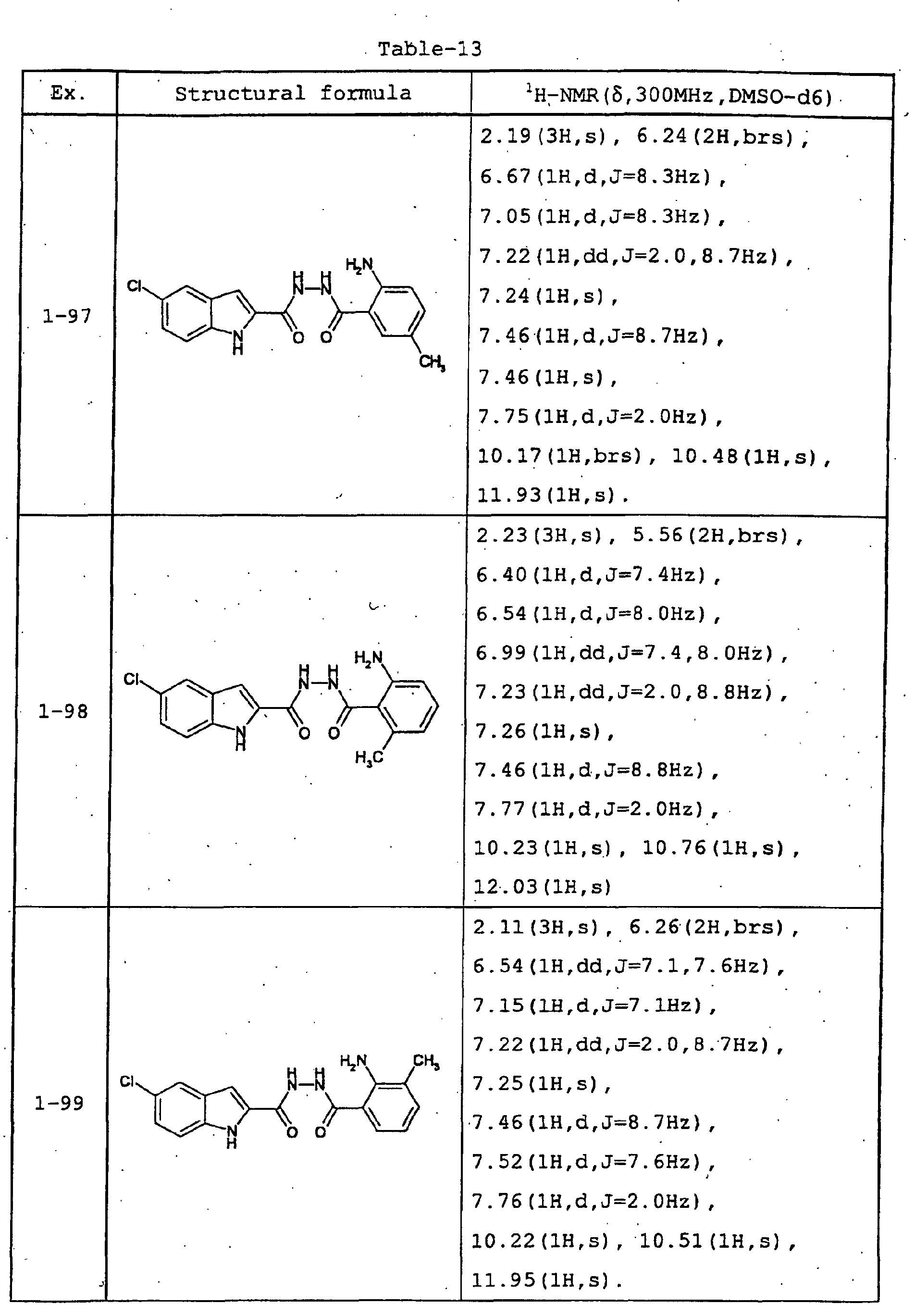

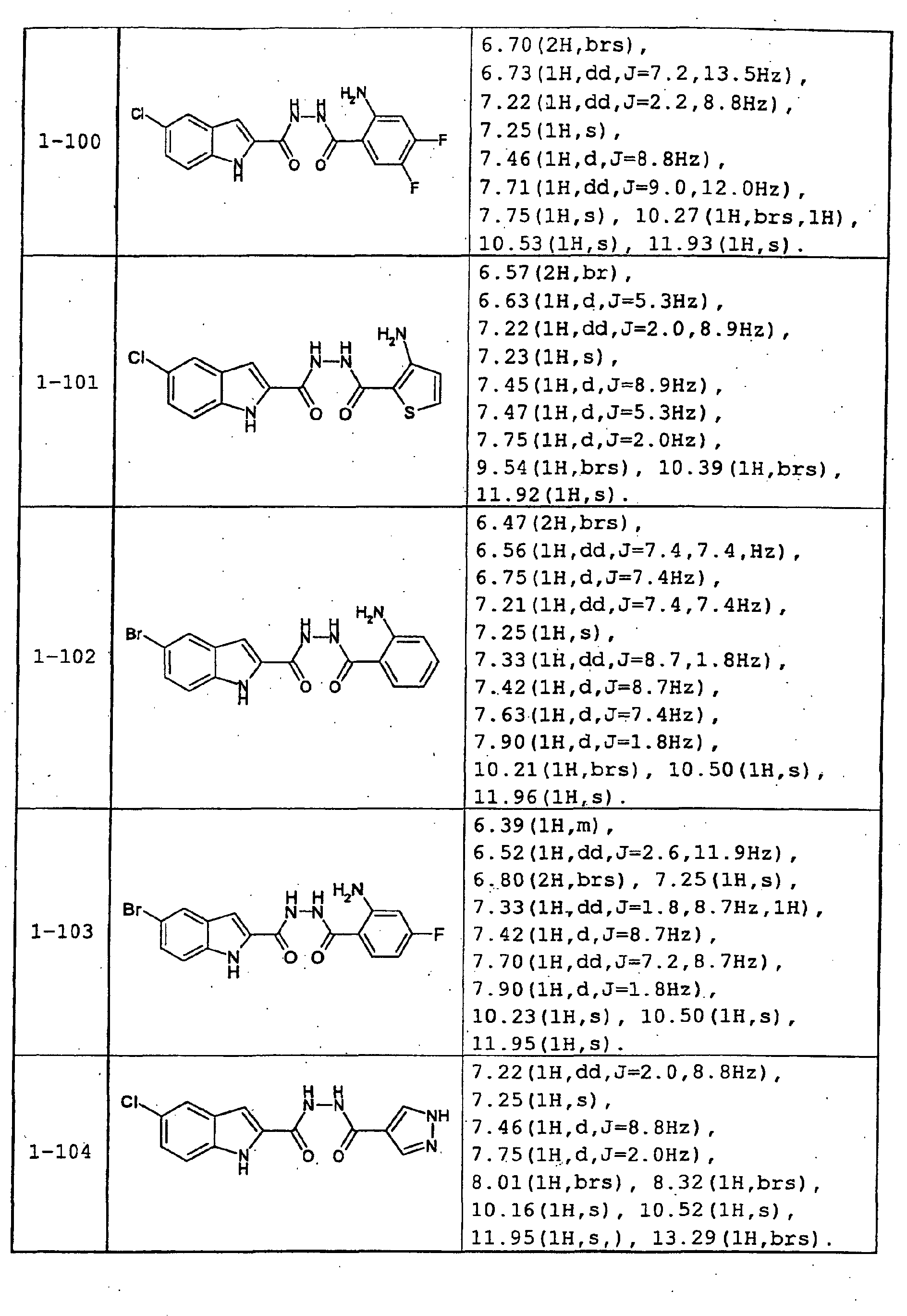

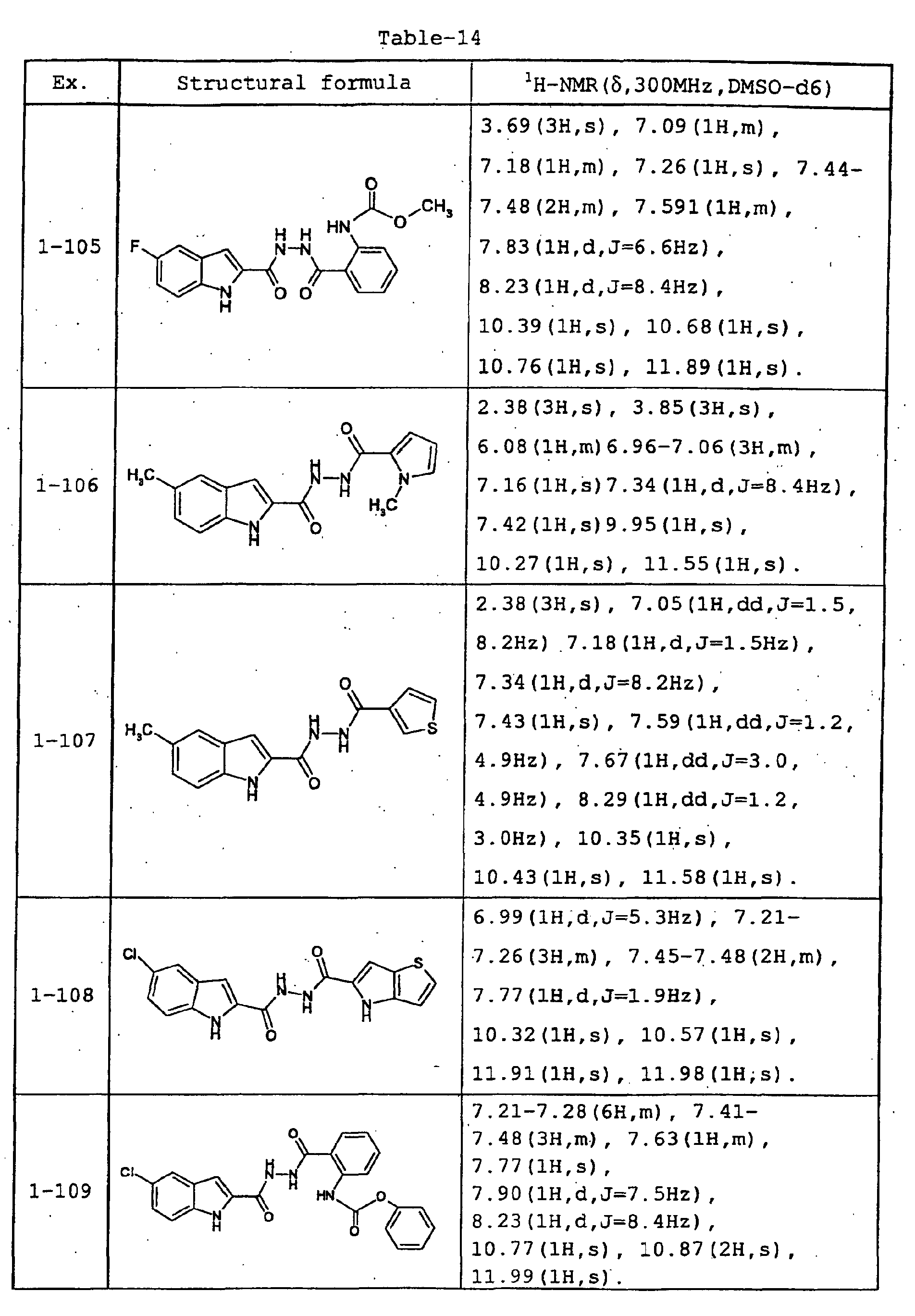

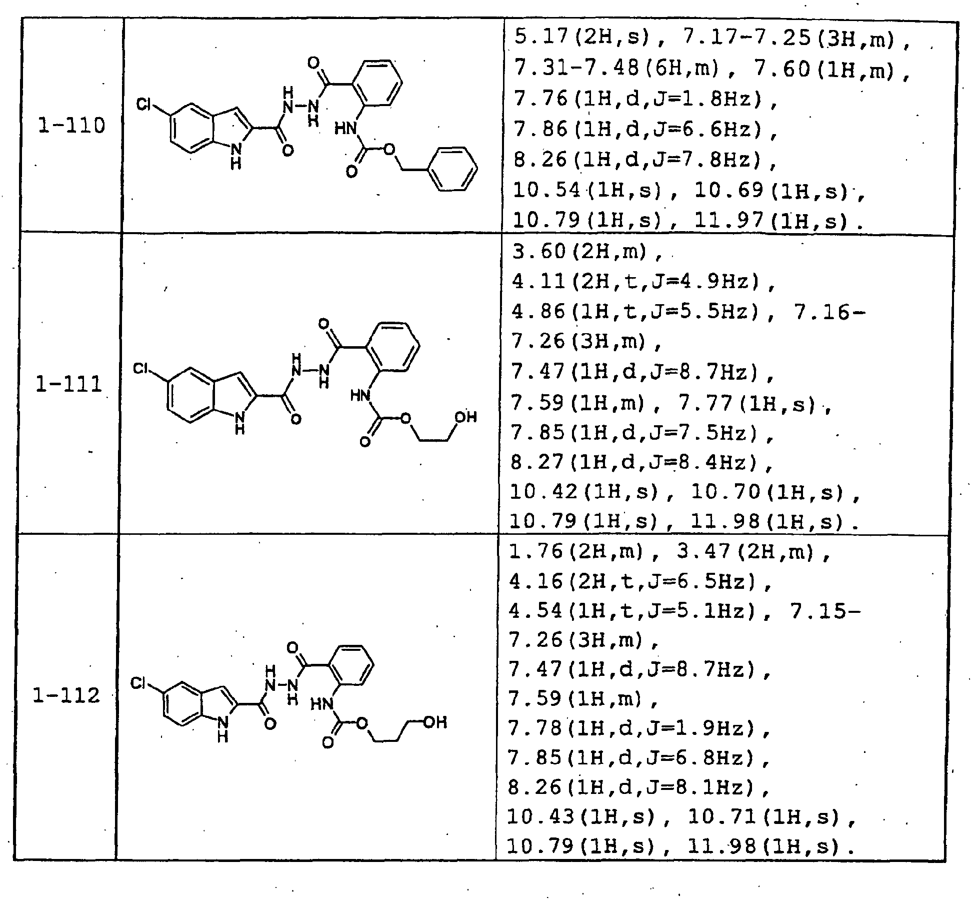

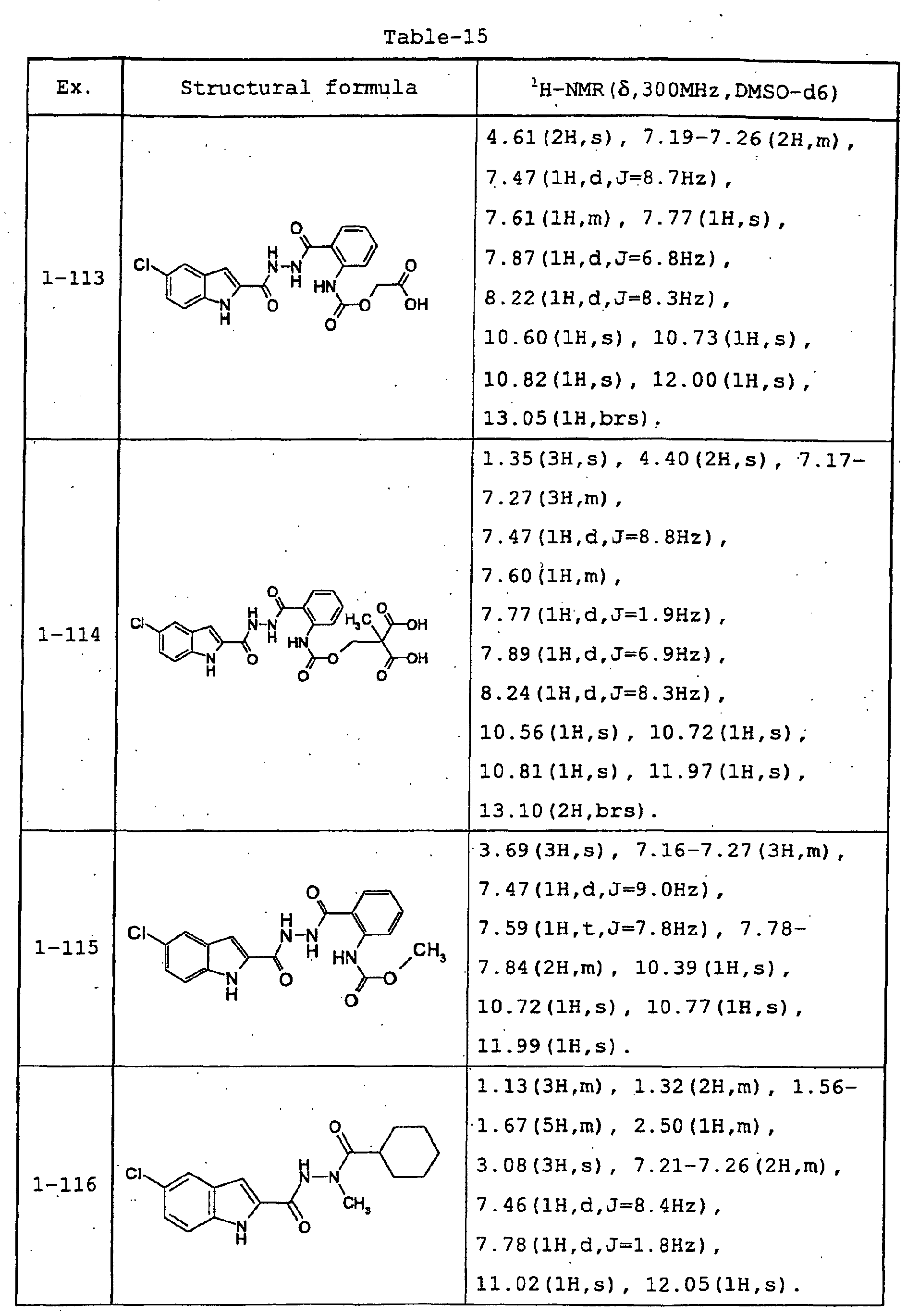

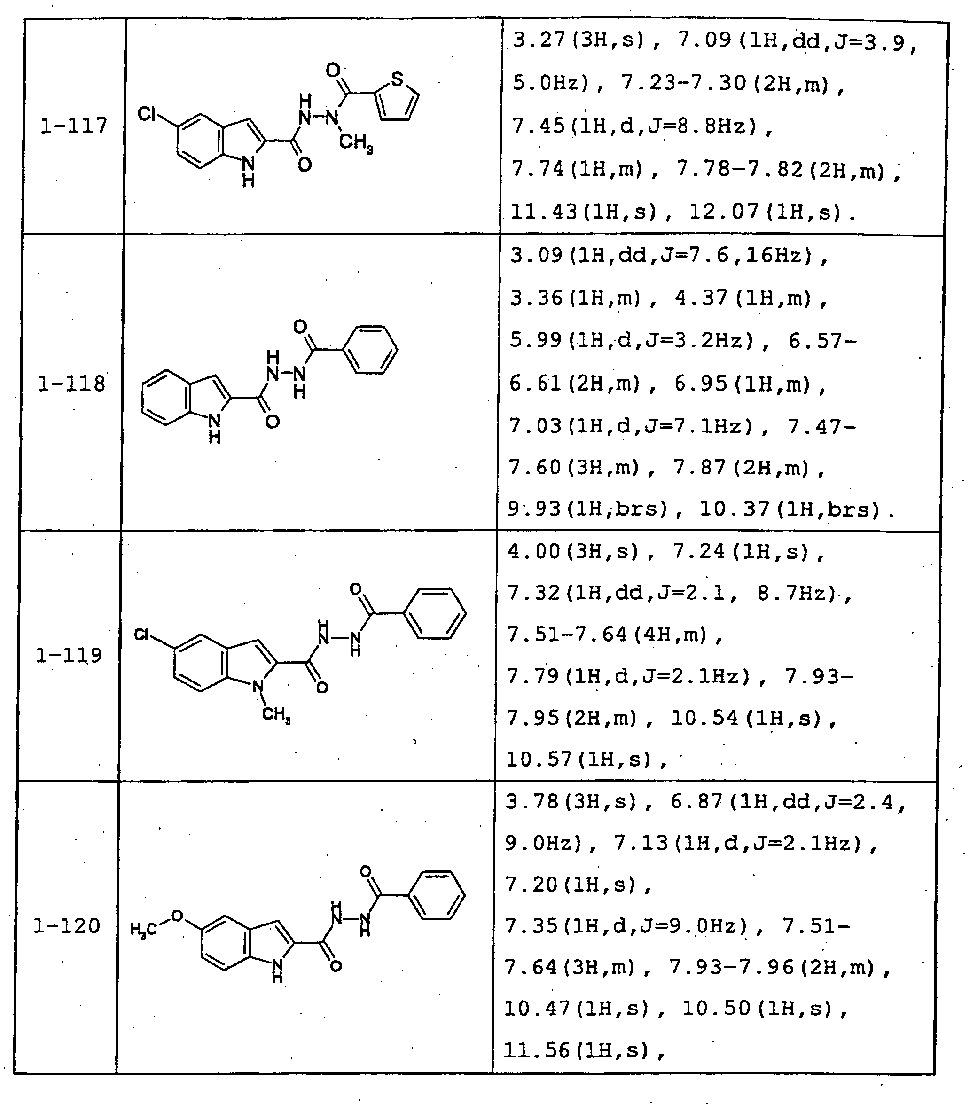

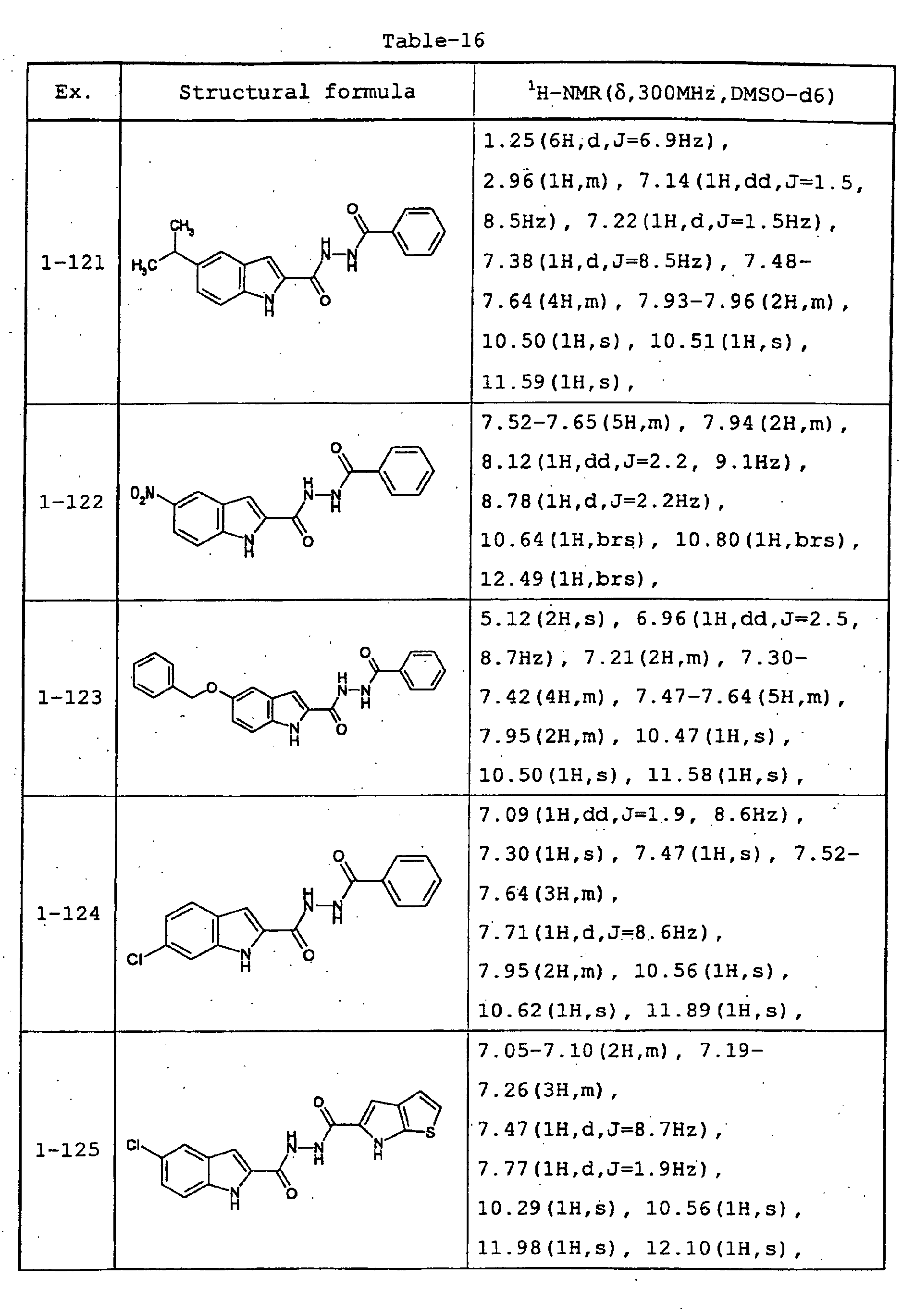

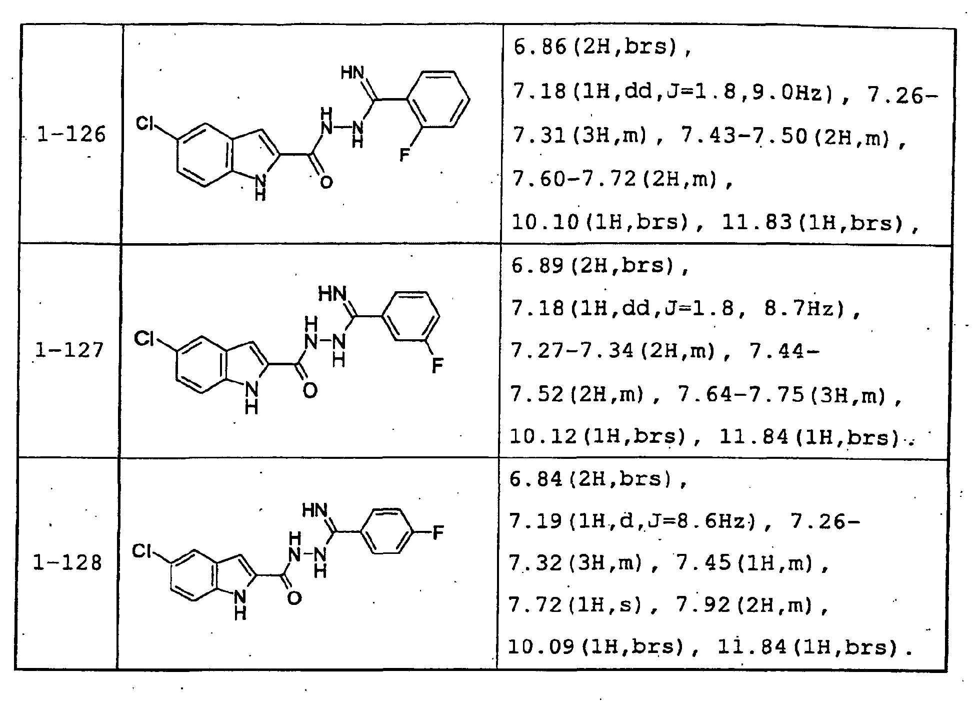

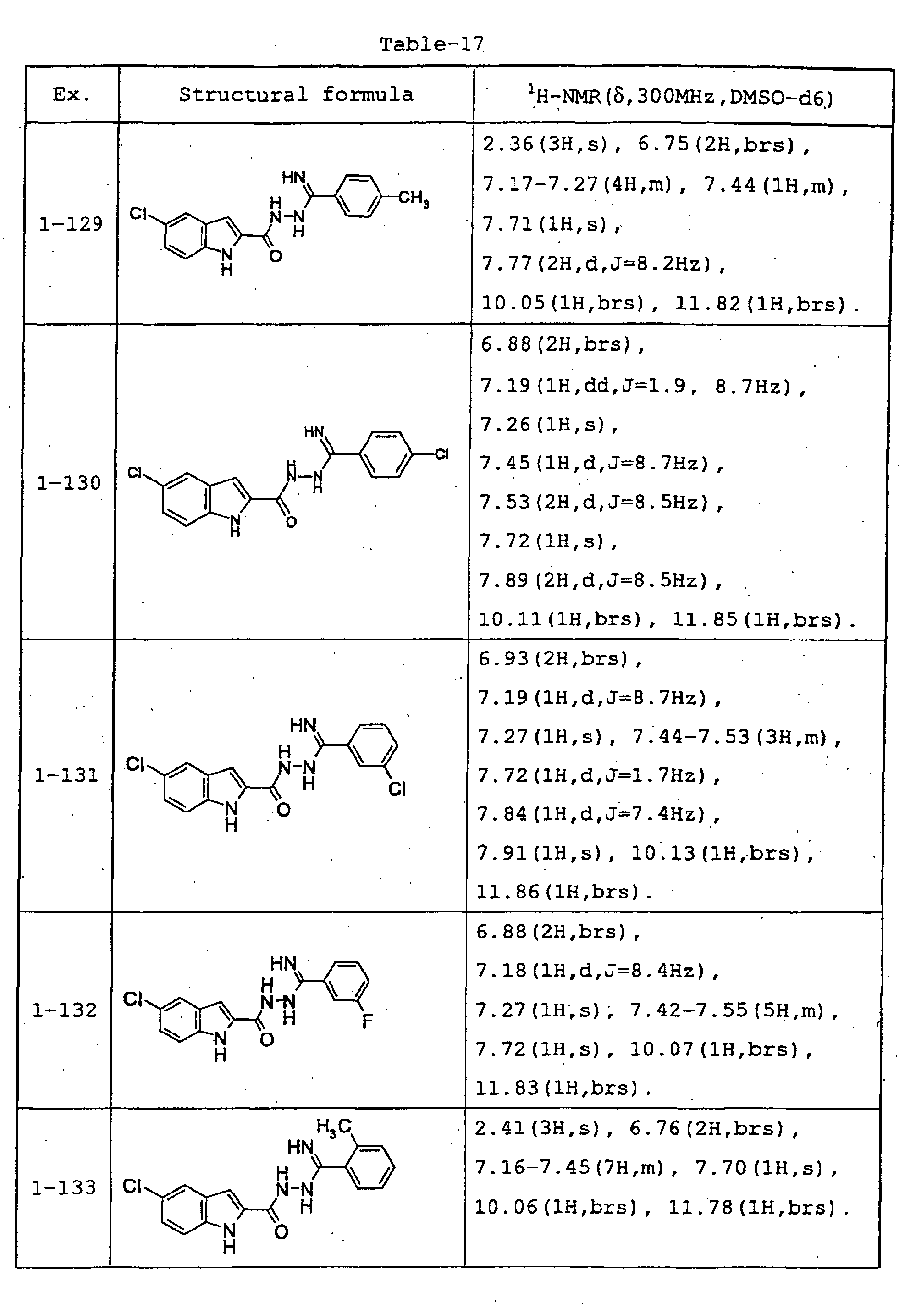

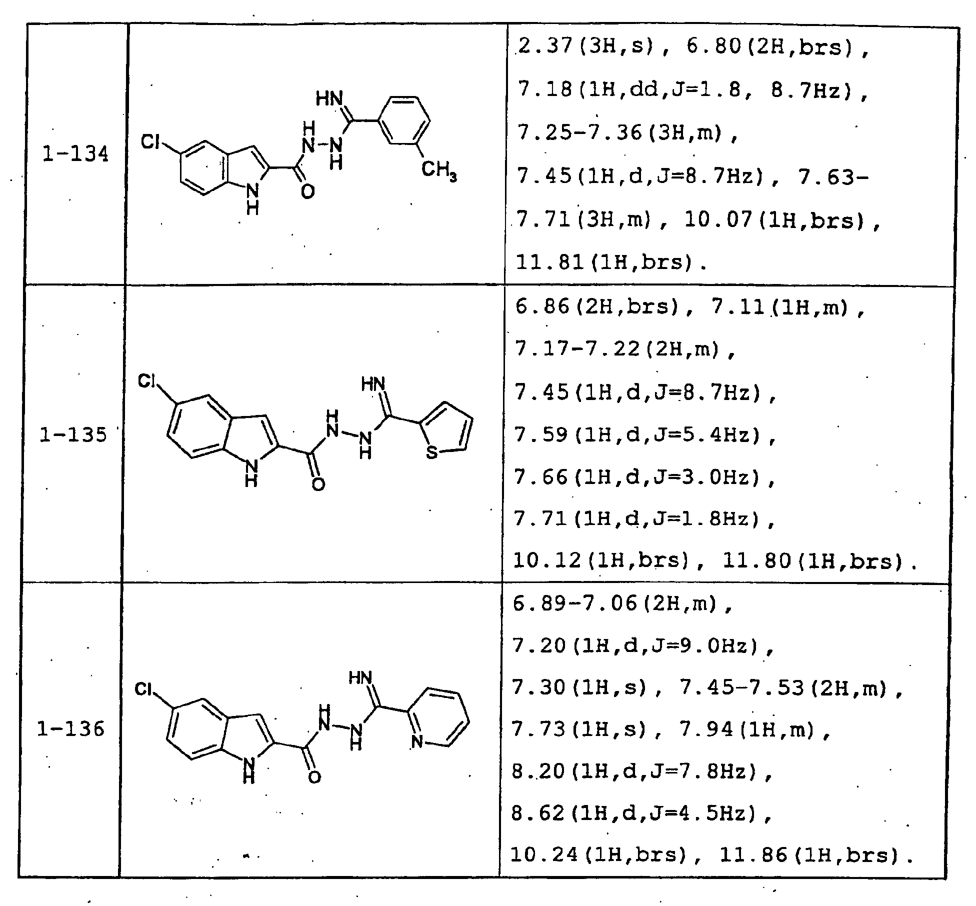

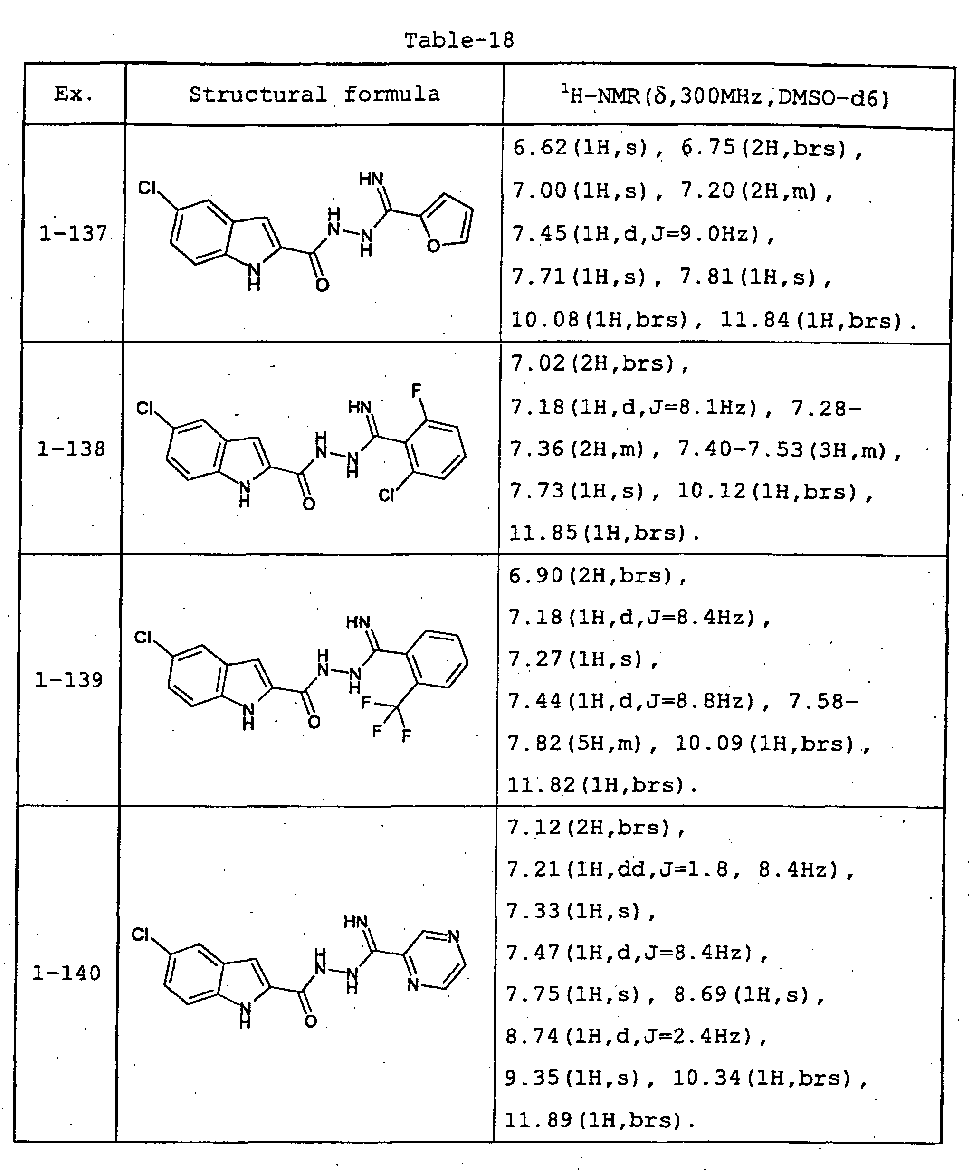

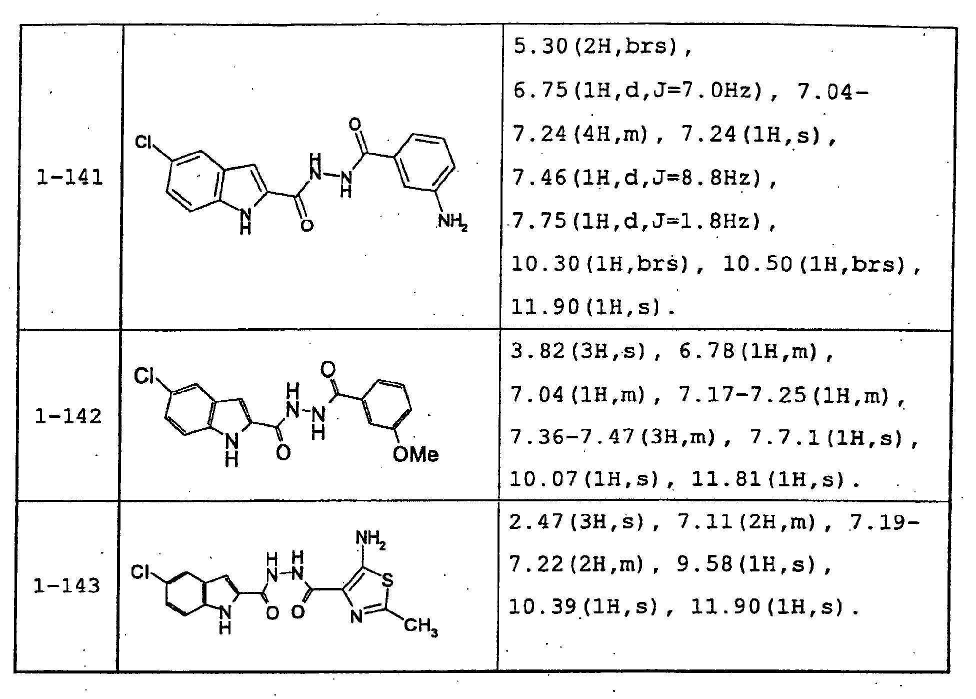

### Example 2

### N-(1,2,3,4-tetrahydro-2,4-dioxoquinazolin-3-yl)-5-chloro-1H-indole-2-carboxamide

2-Aminobenzoic acid 2-(5-chloro-1H-indole-2-carbonyl)hydrazide obtained in Example 1-2 (329 mg) and sodium hydrogencarbonate (168 mg) were suspended in a mixed solvent of THF (10 ml) and water (3 ml) and cooled in an ice bath. Triphosgene (168 mg) was added in divided portions over 20 min. This reaction solution was stirred at room temperature for 1 hr. The separated organic layer was washed 3 times with half-saturated brine and dried over anhydrous sodium sulfate. This solution was filtered and concentrated to give an oily substance. Thereto was added chloroform to give the title compound (317 mg, yield 89%) as crystals (see Table 19).

### Example 2-2

### N-(1,2,3,4-tetrahydro-2,4-dioxobenzo[e][1,3]oxazin-3-yl)-5-chloro-1H-indole-2-carboxamide

In the same manner as in Example 1-2, the title compound was obtained from 2-hydroxybenzoic acid 2-(5-chloro-1H-indole-2-carbonyl)hydrazide obtained in Example 1-3 (see Table 19).

### Example 2-3

### N-(1,2,3,4-tetrahydro-4-oxo-2-thioxoquinazolin-3-yl)-5-chloro-1H-indole-2-carboxamide

2-Aminobenzoic acid 2-(5-chloro-1H-indole-2-carbonyl)-hydrazide obtained in Example 1-2 (1.31 g) was dissolved in THF (26 ml). Thiocarbonyldiimidazole (867 mg) was.added and the mixture was stirred at room temperature for 2 hr. The reaction solvent was evaporated under reduced pressure and the obtained residue was dissolved in ethyl acetate. This solution was washed successively with water, aqueous hydrochloric acid (0.5N), saturated aqueous sodium hydrogen carbonate and water. This solution was dried over anhydrous sodium sulfate and filtrated. The filtrate was evaporated under reduced pressure and the residue was crystallized from ethyl acetate and diethyl ether to give the title compound (910 mg, yield 61.3%) as pale-yellow crystals (see Table 19).

### Example 2-4

### 5-chloro-1H-indole-2-carboxylic acid (2-methyl-4-oxo-4H-quinazolin-3-yl)-amide

2-Amino-benzoic acid 2-(5-chloro-1H-indole-2-carbonyl)-hydrazide obtained in Example 1-2 (90 mg) was dissolved in DMF (1 ml). To this solution were successively added methyl orthoacetate (3 ml) and methanesulfonic acid (0.05 ml) and the mixture was stirred at room temperature for 30 min. Water (2 ml) was added to the reaction mixture and extracted with ethyl acetate-THF (1:1) (50 ml). The organic layer was washed successively with water (2×20 ml) and saturated aqueous sodium chloride solution (20 ml), dried and concentrated under reduced pressure. The obtained solid was washed with ether in a slurry form to give the title compound (77 mg, yield 80%) as a colorless solid (see Table 19).

### Example 2-5

### 5-chloro-1H-indole-2-carboxylic acid (4-oxo-4H-quinazolin-3-yl)-amide

2-Amino-benzoic acid 2-(5-chloro-1H-indole-2-carbonyl)-hydrazide obtained in Example 1-2 (87.1 mg) was suspended in formic acid (3 ml). The mixture was heated to 120°C and stirred for 6 hr. The reaction mixture was allowed to cool to room temperature and water (30 ml) was added. The mixture was extracted with ethyl acetate-THF (1:1) (50 ml). The organic layer was washed successively with saturated aqueous sodium hydrogen carbonate solution (30 ml) and saturated aqueous sodium chloride solution (30 ml), dried and concentrated under reduced pressure. The obtained solid was washed with MeOH in a slurry form to give the title compound (65 mg, yield 72%) as a colorless solid (see Table 19).

### Example 2-6

### 5-chloro-1H-indole-2-carboxylic acid (4-oxo-1,4-dihydro-2H-quinazolin-3-yl)-amide

5-Chloro-1H-indole-2-carboxylic acid (4-oxo-4H-quinazolin-3-yl)amide obtained in Example 2-5 (44 mg) was dissolved in THF-MeOH (1:1) (16 ml). To this solution were successively added acetic acid (0.4 ml) and sodium cyanoborohydride (145 mg) and the mixture was heated to 85°C and stirred for 42 hr. Water (30 ml) was added to the reaction mixture and the mixture was extracted with ethyl acetate-THF (1:1) (80 ml). The organic layer was washed with saturated aqueous sodium chloride solution (30 ml), dried and concentrated under reduced pressure. The obtained solid was washed with ether in a slurry form to give the title compound (42 mg, yield 94%) as a colorless solid (see Table 19).

### Example 2-7

### 5-chloro-1H-indole-2-carboxylic acid (2,5-dioxo-1,2,3,5-tetrahydro-benzo[e][1,4]diazepin-4-yl)-amide

2-Amino-benzoic acid 2-(5-chloro-1H-indole-2-carbonyl)-hydrazide obtained in Example 1-2 (203 mg) was dissolved in THF (25 ml). Pyridine (0.28 ml) was added to this solution and the mixture was cooled to 0°C. Acetyl chloride (0.054 ml) was added and the reaction mixture was heated to room temperature and stirred for 12 hr and 30 min. Water (50 ml) was added to the reaction mixture and the mixture was extracted with THF-ethyl acetate (1:1) (100 ml). The organic layer was washed successively with 10% aqueous citric acid solution (30 ml), water (30 ml) and saturated aqueous sodium chloride solution (30 ml), dried and concentrated under reduced pressure. The obtained solid was washed with ether in a slurry form.

This solid was dissolved in DMF (7 ml). To this solution were successively added potassium carbonate (225 mg) and sodium iodide (catalytic amount) and the mixture was heated to 80°C and stirred for 2 hr. The reaction mixture was allowed to cool to room temperature and water (30 ml) was added. The mixture was extracted with THF-ethyl acetate (1:1) (100 ml). The organic layer was washed with saturated aqueous sodium chloride solution (30 ml), dried and concentrated under reduced pressure. The obtained solid was washed with THF in a slurry form to give the title compound (117 mg, yield 67%) as a colorless solid (see Table 19).

### Example 2-8

### 5-chloro-1H-indole-2-carboxylic acid (2,5-dioxo-2,3-dihydro-5H-benzo[e][1,4]oxazepin-4-yl)-amide

2-(Carboxymethyl)hydroxy-benzoic acid 2-(5-chloro-1H-indole-2-carbonyl)hydrazide (131 mg) and HOBt·H₂O (85 mg) were dissolved in DMF (2.5 ml) and EDC (85 mg) was added. The mixture was stirred at room temperature for 16 hr. The reaction mixture was diluted with THF-ethyl acetate (1:1) (80 ml) and washed successively with water (30 ml), 10% aqueous citric acid solution (30 ml), water (30 ml) and saturated aqueous sodium chloride solution (30 ml). This solution was dried over anhydrous sodium sulfate, filtered and concentrated under reduced pressure. The obtained solid was washed with ether in a slurry form to give the title compound (89 mg, yield 58%) as a colorless solid (see Table 19).

### Examples 2-9 to 2-31

In the same manner as in Examples 2 to 2-8, the compounds of Examples 2-9 to 2-31 were obtained. The obtained compounds are shown in Tables 19-22.

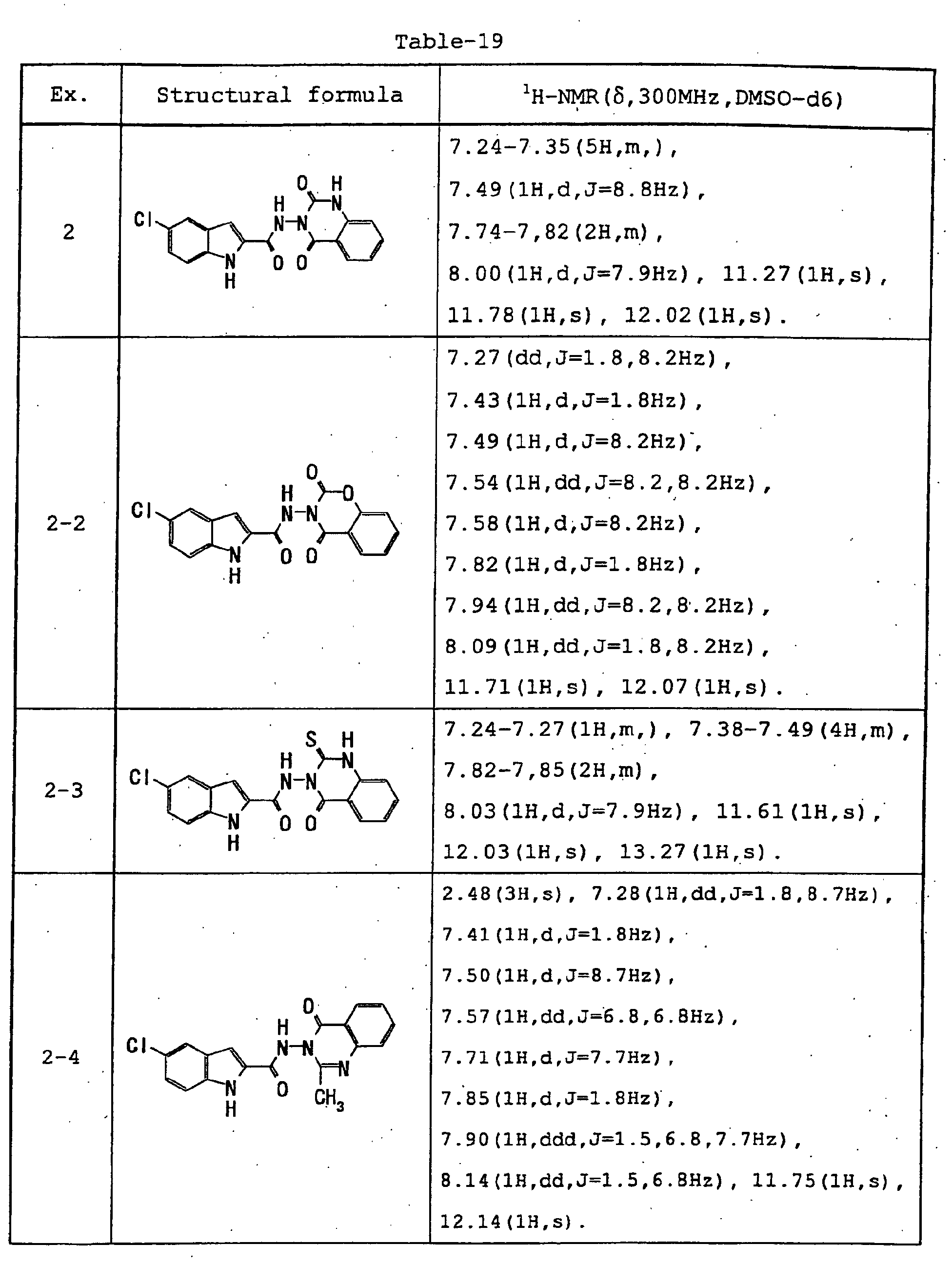

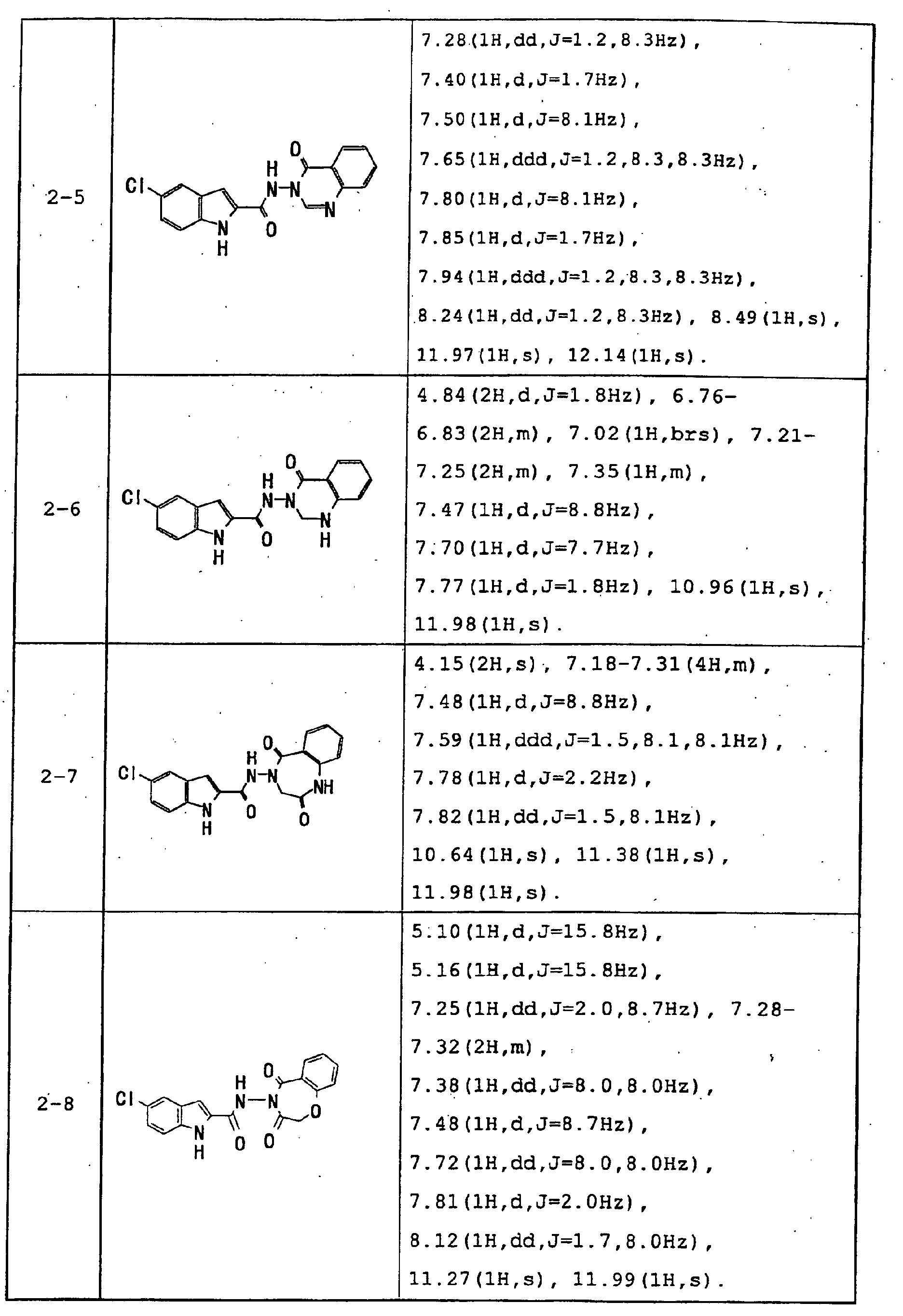

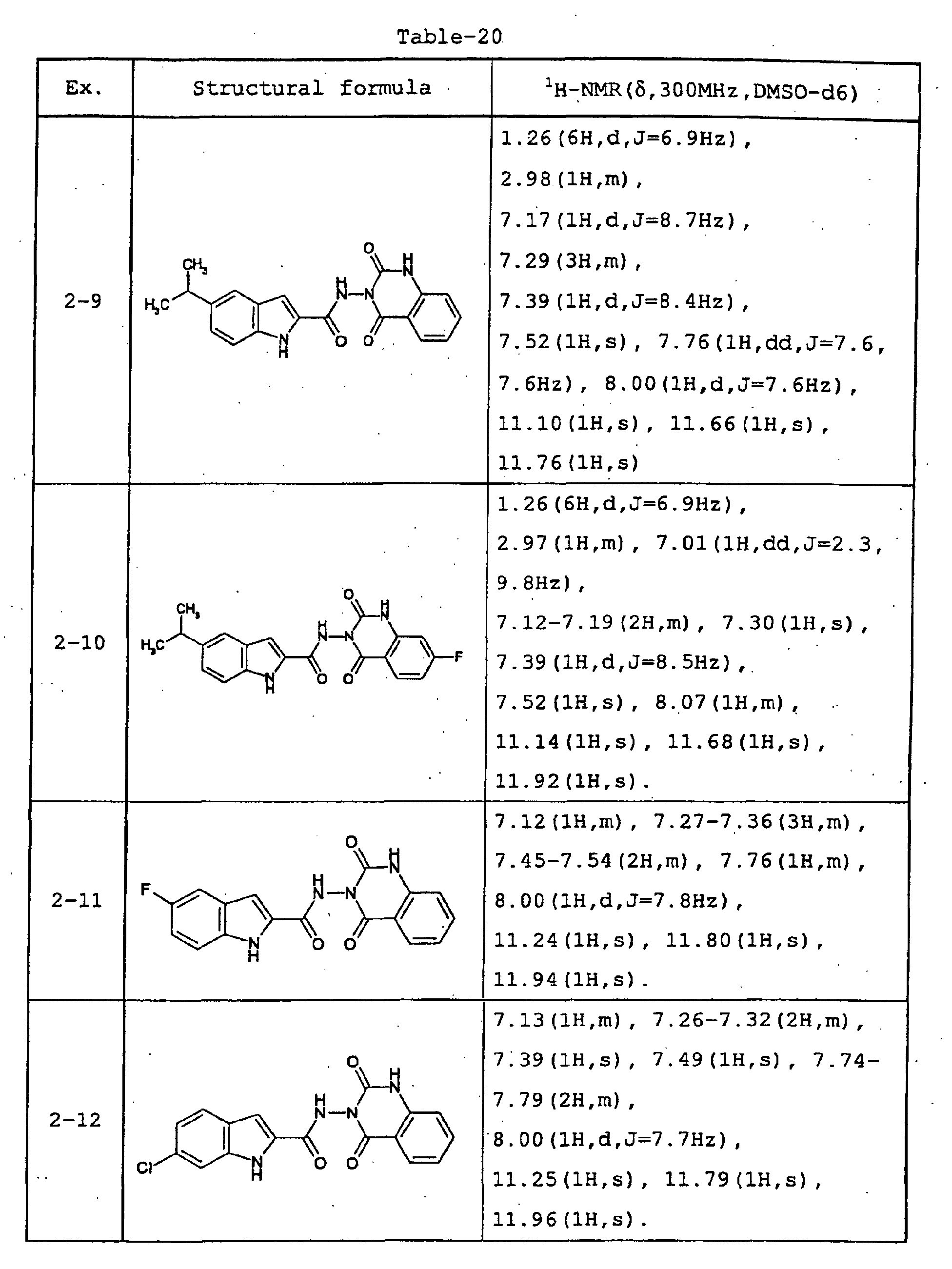

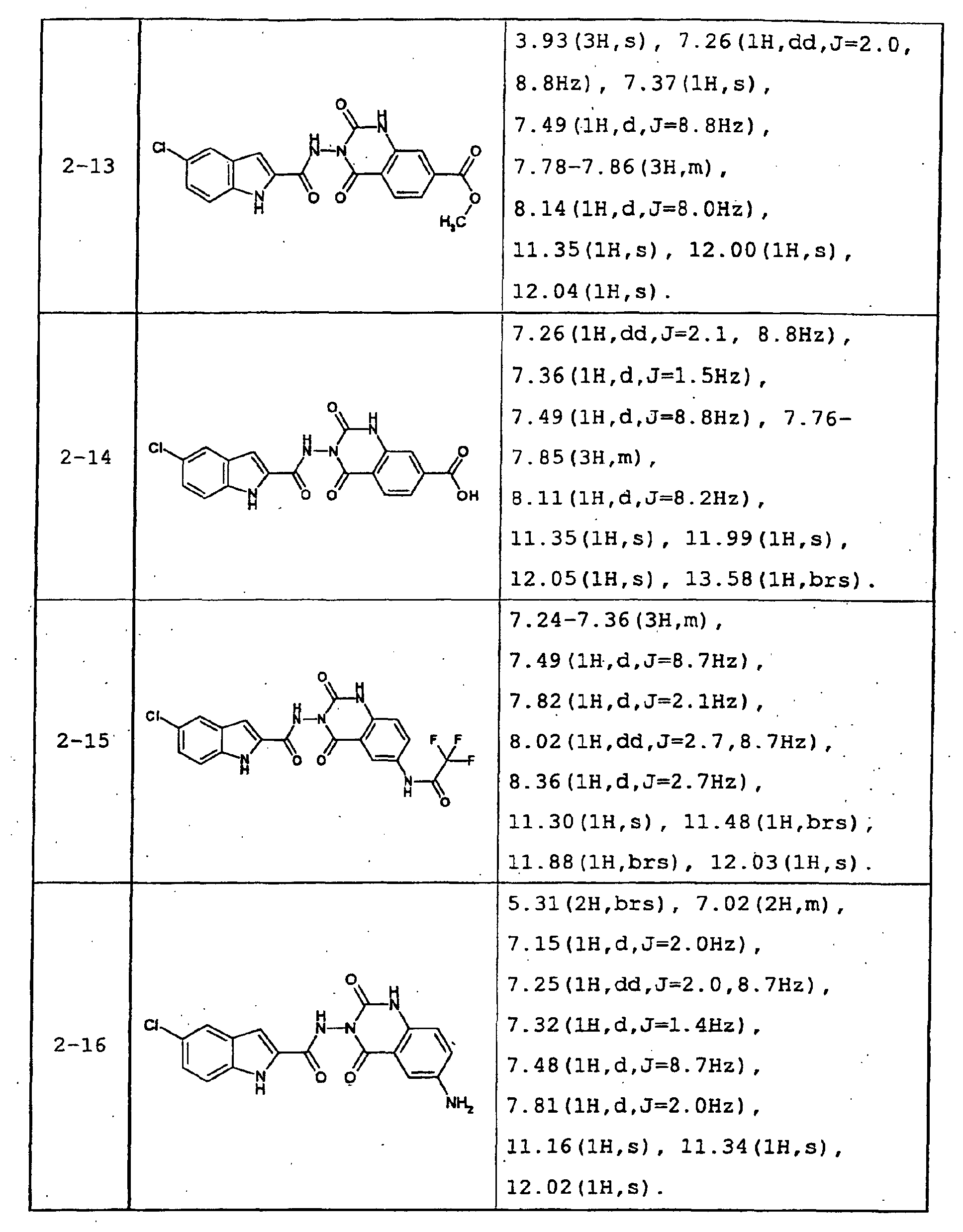

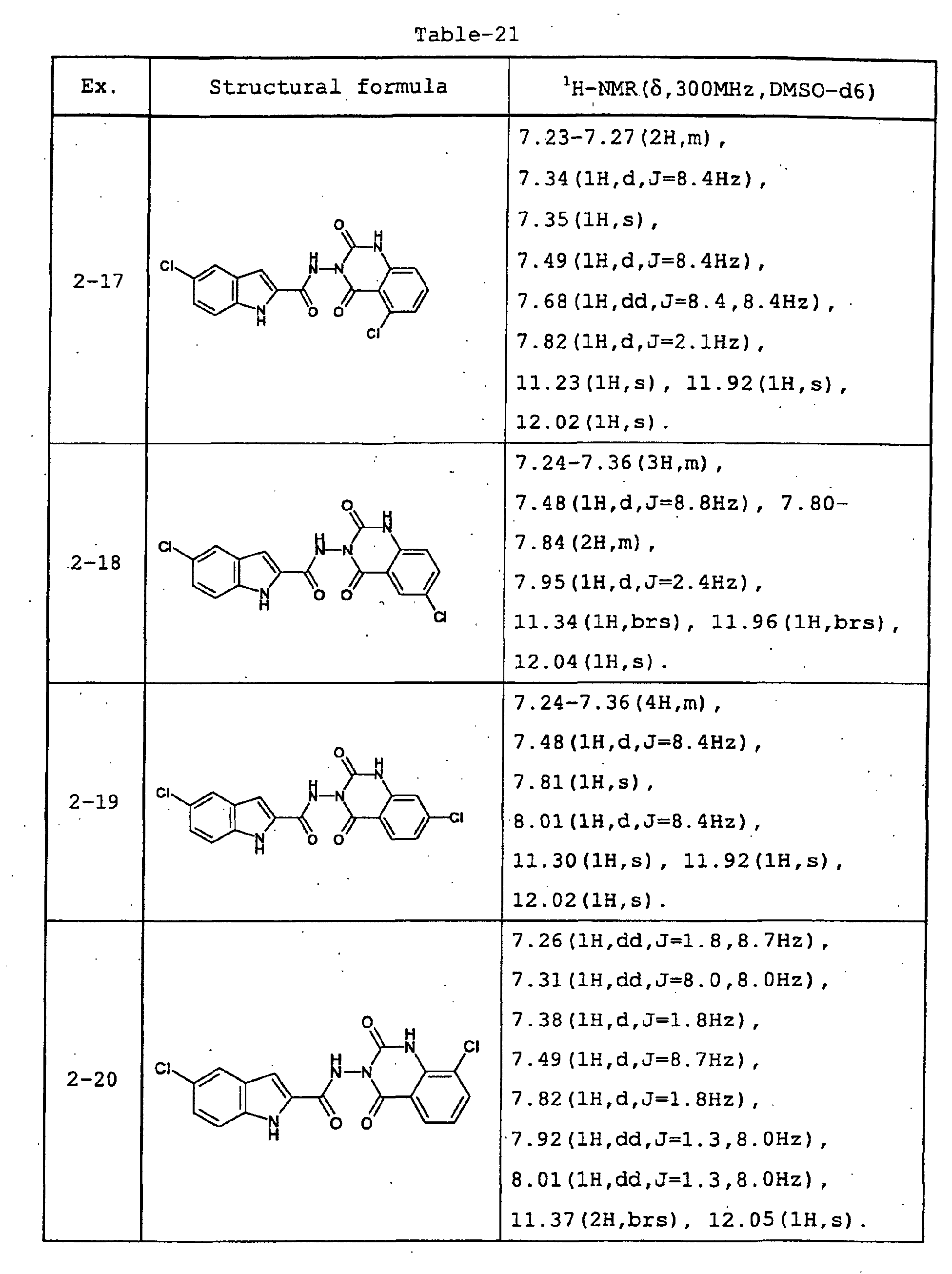

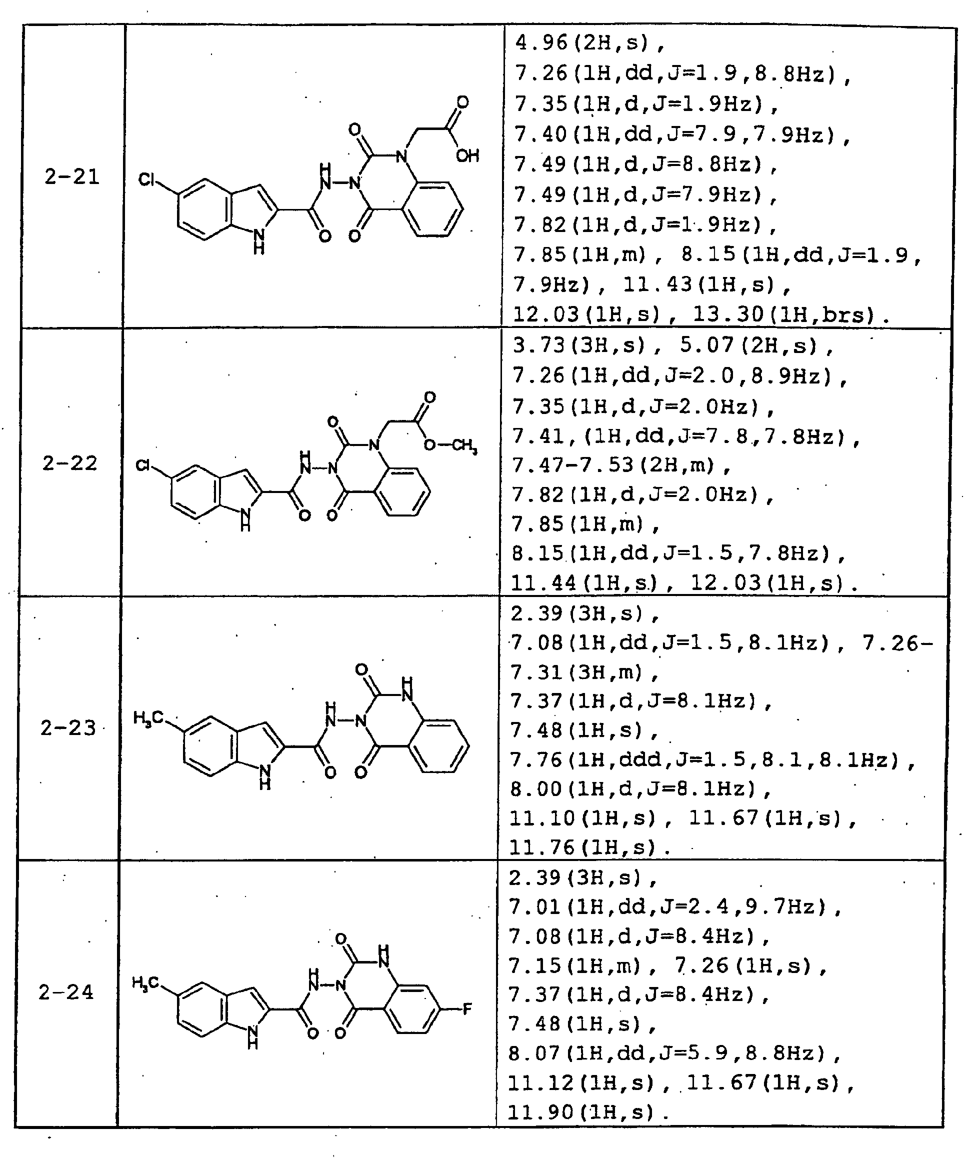

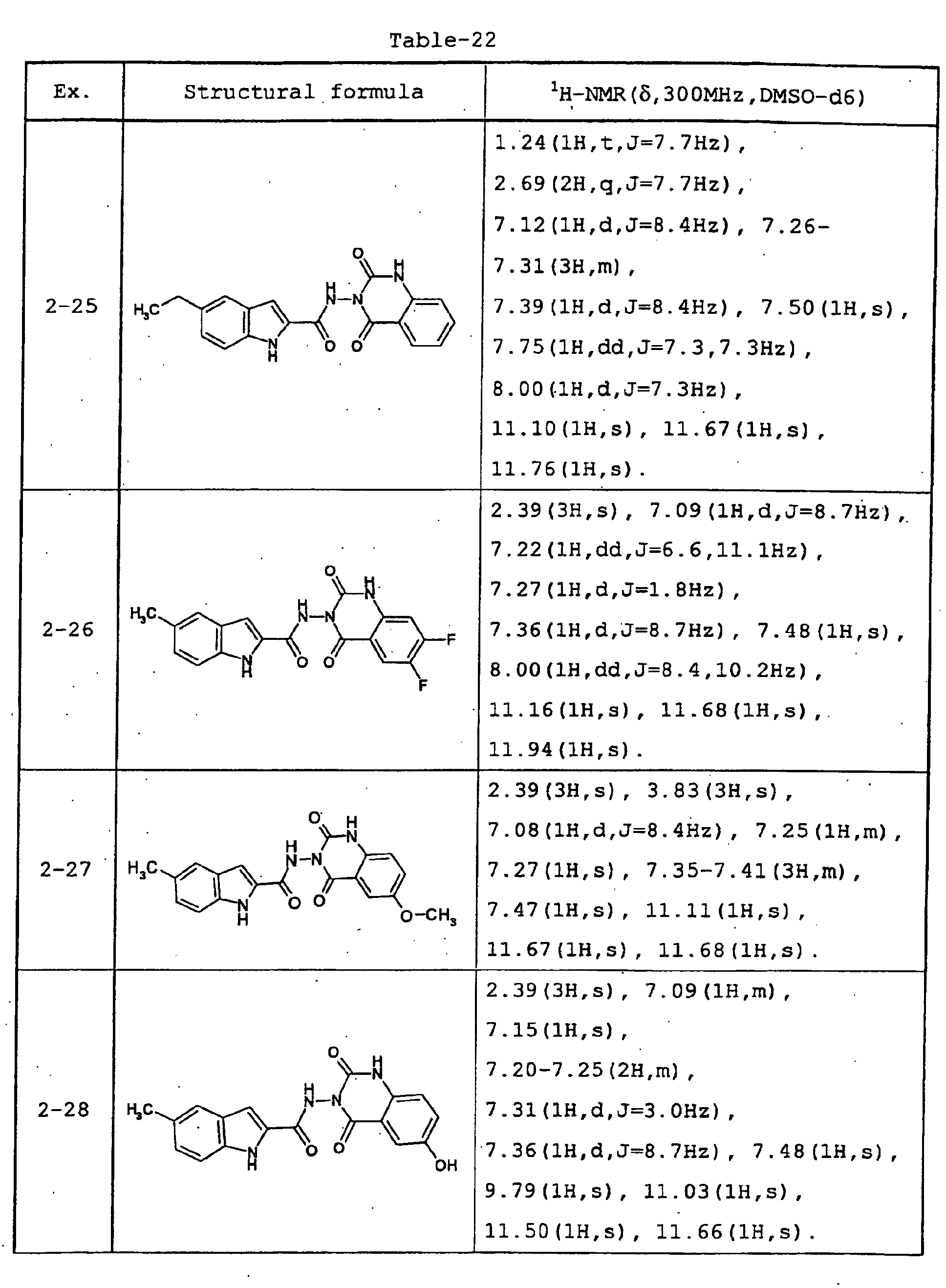

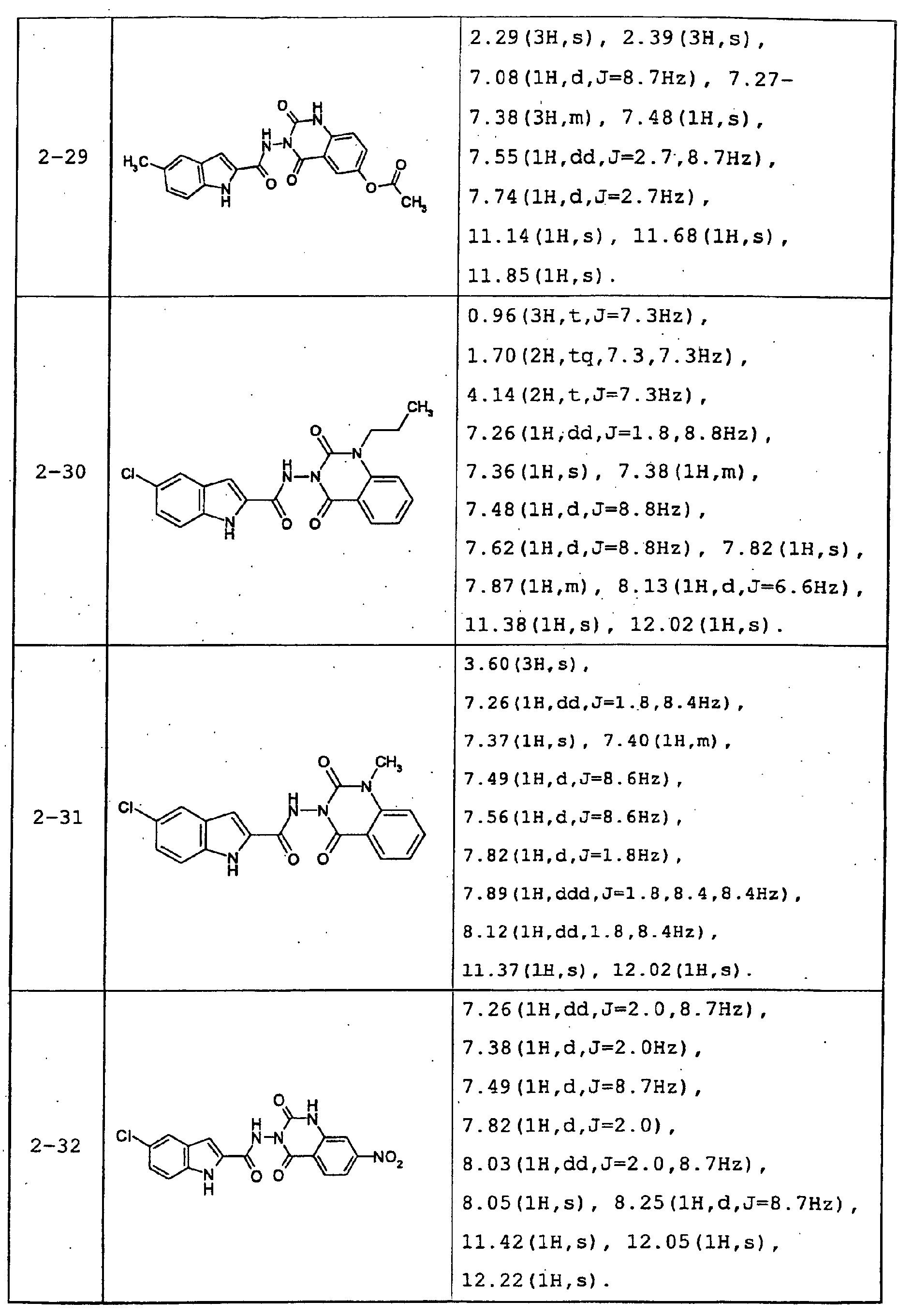

### Example 3

### N-(2,4-dioxo-perhydropyrimidin-3-yl)-5-chloro-1H-indole-2-carboxamide

### a) tert-butyl {3-[2-(5-chloro-1H-indole-2-carbonyl)hydrazino]-3-oxopropyl}carbamate

In the same manner as in Example 1-2 and using N-(tert-butoxycarbonyl)-β-alanine instead of anthranilic acid, the title compound (yield 48%) was obtained.
¹H-NMR (δ, 300MHz,DMSO-d₆).
1.39(9H,s),2.37(2H,t,J=7.5Hz),3.19(2H,m),6.81(1H,m),7.19-7.23(2H,m),7.45(1H,d,J=8.7Hz),7.73(1H,m),9.97(1H,brs),11.83(1H ,s).

### b)N-(2,4-dioxo-perhydropyrimidin-3-yl)-5-chloro-1H-indole-2-carboxamide

tert-Butyl {3-[2-(5-chloro-1H-indole-2-carbonyl)hydrazino]-3-oxopropyl}carbamate obtained in Example 3 a) (577 mg) was dissolved in trifluoroacetic acid (6 ml) under ice-cooling. Trifluoroacetic acid was evaporated under reduced pressure to give a solid residue. This residue was dissolved in THF (3 ml) and triethylamine (0.31 ml) was added. To this solution was added carbonyldiimidazole (357 mg) and the mixture was stirred at room temperature for one day and at 50°C for 1 hr. The reaction solvent was evaporated under reduced pressure and the obtained residue was dissolved in ethyl acetate. This solution was washed successively with water, aqueous hydrochloric acid (0.5N), saturated aqueous sodium hydrogen carbonate and water. This solution was dried over anhydrous sodium sulfate and filtrated. The filtrate was evaporated under reduced pressure and the residue was crystallized from ethyl acetate, diethyl ether to give the title compound (80 mg, yield 12%) as white crystals (see Table 23).

### Example 3-2

### N-(4-oxo-2-thioxo-perhydropyrimidin-3-yl)-5-chloro-1H-indole-2-carboxamide

In the same manner as in Example 3 b) but using thiocarbonyldiimidazole instead of carbonyldiimidazol, the title compound (yield 74%) was obtained (see Table 23).

### Example 3-3

### N-(2,4-dioxo-1-phenyl-perhydropyrimidin-3-yl)-5-chloro-1H-indole-2-carboxamide

### a) 3-anilinopropionic acid

A solution (30 ml) of aniline (5.00 g) in acetonitrile was heated under reflux and β-propiolactone (3.36 ml) was added dropwise. The mixture was heated under reflux for 3 hr and the solvent was evaporated under reduced pressure. The residue was dissolved in aqueous sodium hydroxide solution. This mixture was washed with diethyl ether, adjusted to pH 4-5 with hydrochloric acid and extracted with diethyl ether. The organic layer was washed with water and saturated brine, dried over sodium sulfate, filtered and concentrated under reduced pressure. The residue was purified by silica gel column chromatography (chloroform:MeOH=9:1) to give 3-anilinopropionic acid (2.08 g, yield 23%).

### b) 5-chloro-1H-indole-2-carboxylic acid 2-(3-anilinopropionyl)hydrazide

To a suspension (10 ml) of 5-chloro-1H-indole-2-carboxylic acid hydrazide obtained in Example 1 a) (419 mg) and compound 3 obtained in Example 3-3 a) (330 mg) in DMF were added 1-hydroxybenzotriazole (368 mg) and EDC (460 mg), and the mixture was stirred at room temperature for 12 hr. Water was added to the reaction solution and the mixture was extracted with ethyl acetate. The organic layer was washed successively with 10% aqueous citric acid solution, saturated aqueous sodium hydrogen carbonate, water and saturated brine and dried over sodium sulfate. This solution was filtered and concentrated under reduced pressure. The residue was recrystallized from ethyl acetate·n-hexane to give the title compound (468 mg, yield 66%).

### c) N-(2,4-dioxo-1-phenyl-perhydropyrimidin-3-yl)-5-chloro-1H-indole-2-carboxamide

To a solution (3 ml) of 5-chloro-1H-indole-2-carboxylic acid 2-(3-anilinopropionyl)hydrazide obtained in Example 3-3 b) (51.0 mg) in THF were added triethylamine (44 mg) and triphosgene (15.6 mg). The mixture was stirred at room temperature for 4 hr and water was added. This mixture was extracted with ethyl acetate. The organic layer was washed with 10% aqueous citric acid solution, saturated aqueous sodium hydrogen carbonate and saturated brine and dried over sodium sulfate. This was filtered and concentrated under reduced pressure. The residue was purified by silica gel column chromatography (n-hexane:ethyl acetate=1:2) to give the title compound (34 mg, yield 62%) (see Table 23).

### Example 3-4

### N-(4-oxo-1-phenyl-perhydropyrimidin-3-yl)-5-chloro-1H-indole-2-carboxamide

To a suspension of 5-chloro-1H-indole-2-carboxylic acid 2-(3-anilinopropionyl)hydrazide obtained in Example 3-3 b) (100 mg) in ethanol (5 ml) was added paraformaldehyde (68 mg). The mixture was stirred at room temperature for 7 days and water was added. The mixture was extracted with ethyl acetate and the organic layer was washed with saturated brine and dried over sodium sulfate. This solution was filtered and concentrated under reduced pressure. The residue was purified by silica gel column chromatography (chloroform:MeOH=20:1) to give the title compound (48 mg) (see Table 23).

### Examples 3-5 and 2-15

In the same manner as in Examples 3 to 3-4, the compounds of Examples 3-5 to 3-15 were obtained. The obtained compounds are shown in Tables 23-24.

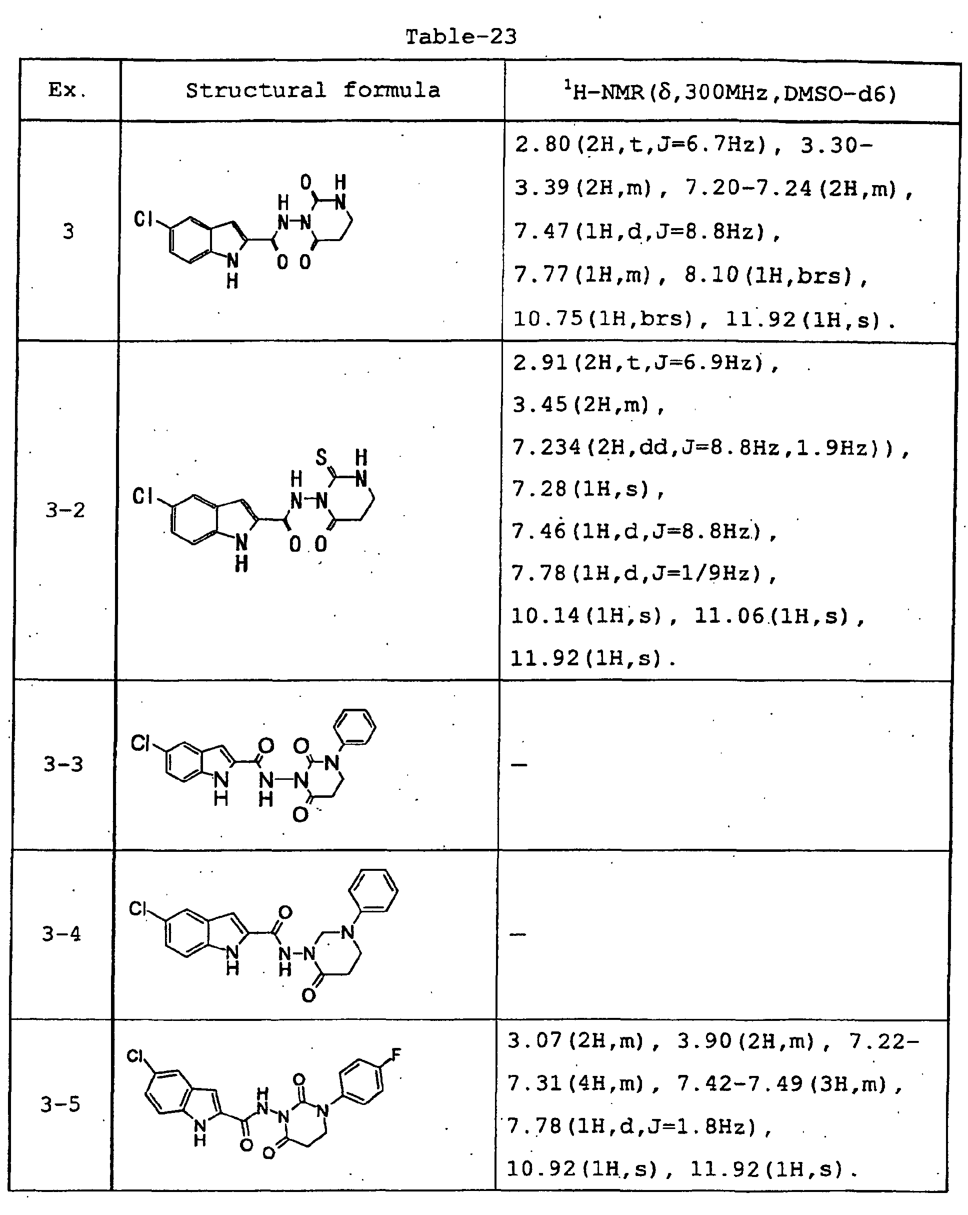

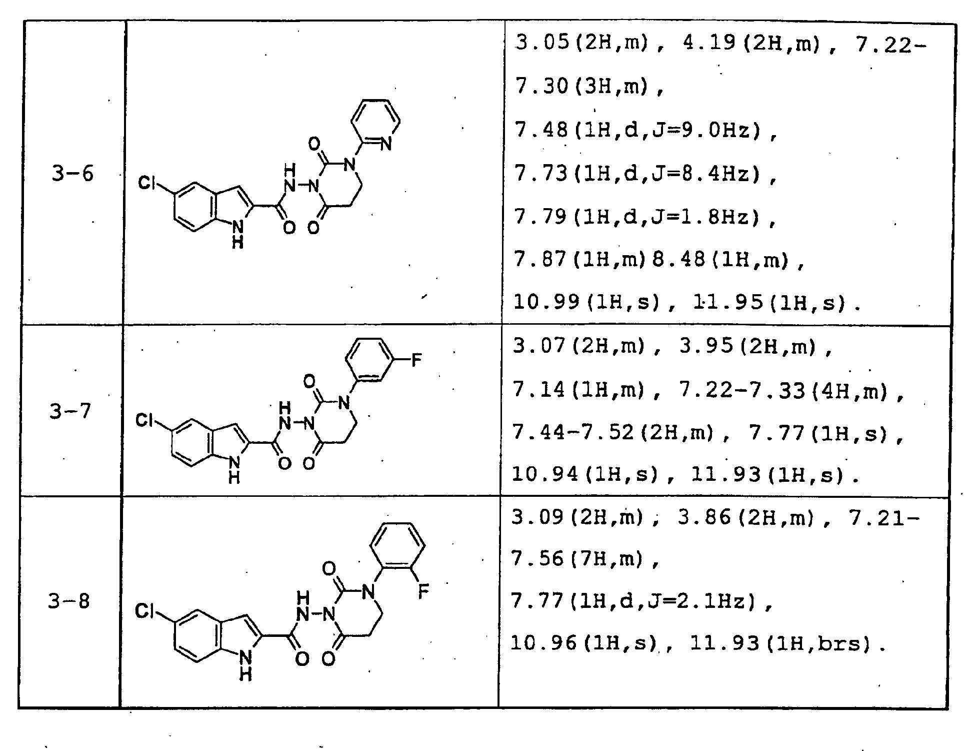

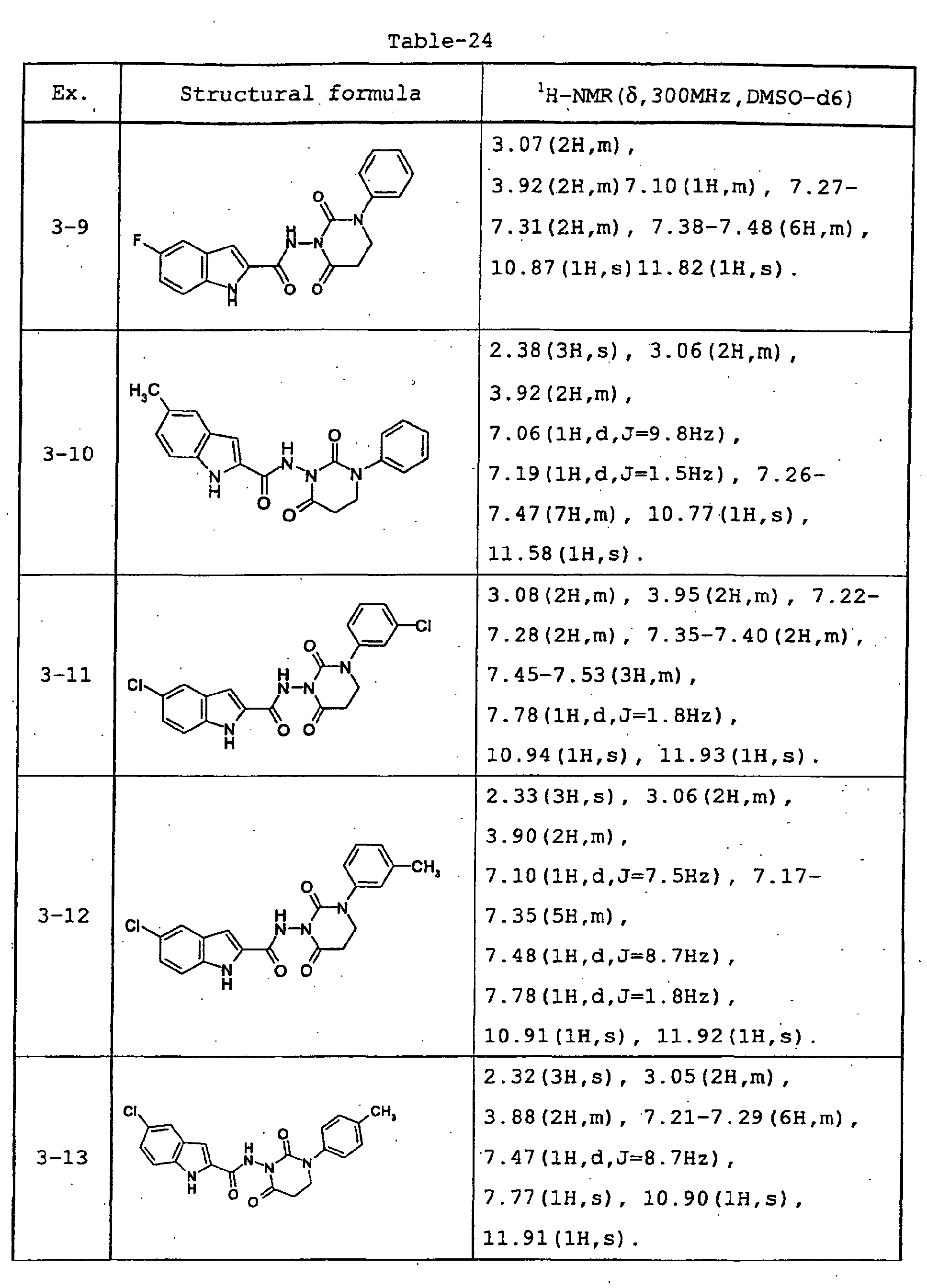

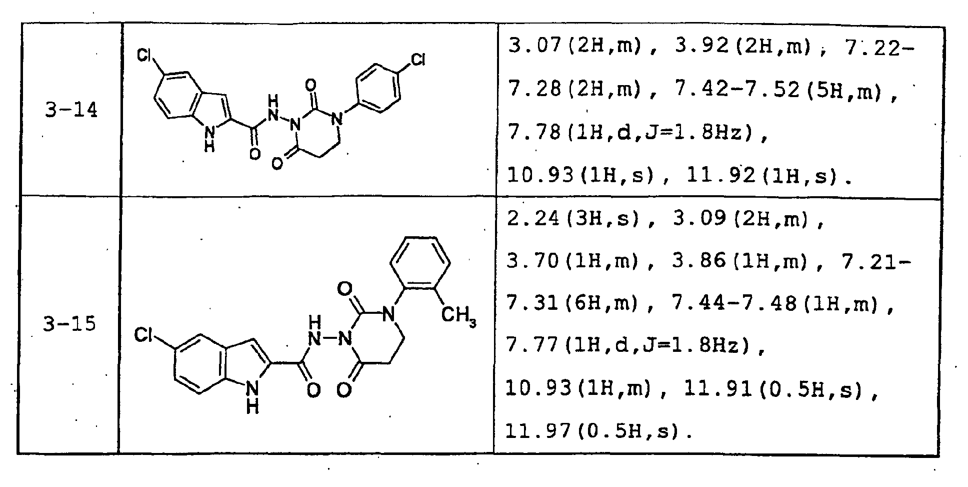

### Example 4

### N-(2,4-dioxo-5-phenylimidazolidin-3-yl)-5-chloro-1H-indole-2-carboxamide

### a) tert-butyl 2-[2-(5-chloro-1H-indole-2-carbonyl)hydrazino]-2-oxo-1-phenyl-ethyl)carbamate

To a suspension (10 ml) of 5-chloro-1H-indole-2-carboxylic acid hydrazide obtained in Example 1 a) (420 mg) and 2-(tert-butoxycarbonylamino)-2-phenylacetic acid (503 mg) in DMF were added 1-hydroxybenzotriazole monohydrate (368 mg) and EDC (460 mg). This reaction mixture was stirred at room temperature for 14 hr and water was added to the reaction solution. The reaction mixture was extracted with ethyl acetate. The organic layer was washed successively with 10% aqueous citric acid solution, saturated aqueous sodium hydrogen carbonate, water and saturated brine, and dried over sodium sulfate. This solution was filtered and concentrated under reduced pressure and the residue was recrystallized from ethyl acetate·n-hexane to give the title compound (790 mg, yield 89%).

### b) 5-chloro-1H-indole-2-carboxylic acid 2-((a-aminobenzyl)carbonyl)hydrazide

To a suspension (5 ml) of tert-butyl 2-[2-(5-chloro-1H-indole-2-carbonyl)hydrazino]-2-oxo-1-phenyl-ethyl}carbamate obtained in Example 4 a) (500 mg) in dichloromethane was added trifluoroacetic acid (5 ml) at room temperature. This reaction mixture was stirred at room temperature for 5 hr and alkalized with aqueous sodium hydrogen carbonate. This mixture was extracted with ethyl acetate and the organic layer was washed with water and saturated brine, and dried over sodium sulfate. This solution was filtered and concentrated under reduced pressure. The residue was recrystallized from ethyl acetate·n-hexane to give the title compound (366 mg, yield 94%).
¹H-NMR (s) ppm (300MHz,DMSO-d₆)
4.54(1H,s),7.19-7.37(5H,m),7.44(1H,d,J=8.7Hz),7.54(2H,d,J=7.5Hz),7.71(1H,d,J=1 .8Hz),11.91(1H,s).

### c) N-(2,4-dioxo-5-phenylimidazolidin-3-yl)-5-chloro-1H-indole-2-carboxamide

To a solution (3 ml) of 5-chloro-1H-indole-2-carboxylic acid 2-((α-aminobenzyl)carbonyl)hydrazide obtained in Example 4 b) (80.0 mg) in THF was added carbonyldiimidazole (45.8 mg) at room temperature. The mixture was stirred at room temperature for 16 hr and 10% aqueous citric acid solution was added. This mixture was extracted with ethyl acetate. The organic layer was washed with water and saturated brine and dried over sodium sulfate. This was filtered and concentrated under reduced pressure. The residue was purified by silica gel column chromatography (hexane:ethyl acetate=1:2) to give the title compound (75 mg, yield 87%) (see Table 25).

### Example 4-2

### N-(2,4-dioxo-1-phenylimidazolidin-3-yl)-5-chloro-1H-indole-2-carboxamide

### a) 5-chloro-1H-indole-2-carboxylic acid 2-(anilinoacetyl)hydrazide

To a suspension (5 ml) of 5-chloro-1H-indole-2-carboxylic acid hydrazide obtained in Example 1 a) (210 mg) and anilinoacetic acid (151 mg) in DMF were added 1-hydroxybenzotriazole (184 g) and EDC (230 mg). The mixture was stirred at room temperature for 13 hr and water was added to the reaction solution. This mixture was extracted with ethyl acetate. The organic layer was washed successively with 10% aqueous citric acid solution, saturated aqueous sodium hydrogen carbonate, water and saturated brine and dried over sodium sulfate. This solution was filtered and concentrated under reduced pressure. The residue was recrystallized from ethyl acetate-n-hexane to give the title compound (321 mg, yield 94%).

### b) N-(2,4-dioxo-1-phenylimidazolidin-3-yl)-5-chloro-1H-indole-2-carboxamide

To a solution (10 ml) of 5-chloro-1H-indole-2-carboxylic acid 2-(anilinoacetyl)hydrazide obtained in Example 4-2 a) (203 mg) in THF was added carbonyldiimidazole (140 mg). The mixture was stirred at 60°C for 13 hr, allowed to cool and 10% aqueous citric acid solution was added. The mixture was extracted with ethyl acetate ester. The organic layer was washed with saturated aqueous sodium hydrogen carbonate and saturated brine and dried over sodium sulfate. This was filtered and concentrated under reduced pressure. The residue was purified by silica gel column chromatography (hexane:ethyl acetate=2:1) to give the title compound (21 mg, yield 10%) (see Table 25).

### Example 4-3

### N-(4-oxo-1-phenyl-2-thioxoimidazolidin-3-yl)-5-chloro-1H-indole-2-carboxamide

In the same manner as in Example 4-2 b) but using thiocarbonyldiimidazole instead of carbonyldiimidazole, the title compound was obtained (see Table 25).

### Example 4-4

### N-(1-oxo-4-phenylimidazolidin-2-yl)-5-chloro-1H-indole-2-carboxamide

To a suspension of 5-chloro-1H-indole-2-carboxylic acid 2-(anilinoacetyl)hydrazide obtained in Example 4-2 a) (103 mg) in ethanol (3 ml) was added paraformaldehyde (36 mg). The mixture was stirred at room temperature for 5 days, and water was added. This mixture was extracted with ethyl acetate. The organic layer was washed with saturated brine and dried over sodium sulfate. This solution was filtered and concentrated under reduced pressure. The residue was purified by silica gel column chromatography (hexane:ethyl acetate=2:1) to give the title compound (73 mg, yield 69%) (see Table 25).

### Example 4-5

### N-(2-oxo-1-phenylimidazolidin-3-yl)-5-chloro-1H-indole-2-carboxamide

### a) 1-(5-chloro-1H-indole-2-carbonyl)-4-phenylsemicarbazide

To a suspension of 5-chloro-1H-indole-2-carboxylic acid hydrazide obtained in Example 1 a) (800 mg) in THF (10 ml) was added phenylisocyanate (477 mg). The mixture was stirred at room temperature for 2 hr and diethyl ether was added. The precipitated solid was collected by filtration and dried in vacuo to give the title compound (1.24 g, yield 99%).

### b) N-(2-oxo-1-phenylimidazolidin-3-yl)-5-chloro-1H-indole-2-carboxamide

To a solution of 1-(5-chloro-1H-indole-2-carbonyl)-4-phenylsemicarbazide obtained in Example 4-5 a) (100 mg) in DMF (5 ml) were added 1,2-dibromoethane (63 mg) and potassium carbonate (92 mg) and the mixture was stirred at 60°C for 18 hr. The mixture was allowed to cool and 10% aqueous citric acid solution was added. The mixture was extracted with ethyl acetate and the organic layer was washed with saturated aqueous sodium hydrogen carbonate, water and saturated brine, and dried over sodium sulfate. This solution was filtered and concentrated under reduced pressure. The residue was purified by silica gel column chromatography (hexane:ethyl acetate=2:1) to give the title compound (45 mg, yield 42%) (see Table 25).

### Examples 4-6 to 4-20

In the same manner as in Examples 4 to 4-5, the compounds of Examples 4-6 to 4-20 were obtained. The obtained compounds are shown in Tables 25-27.

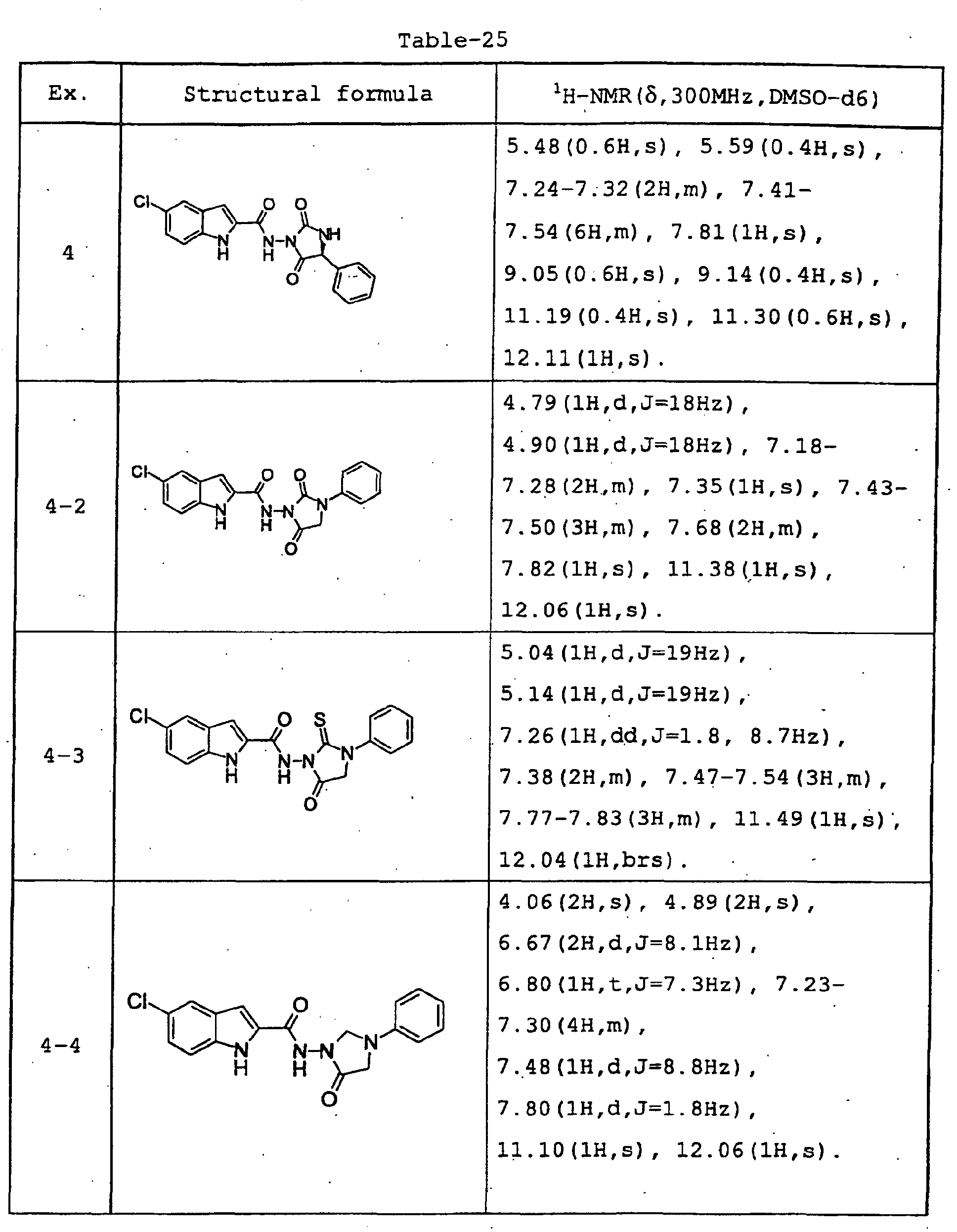

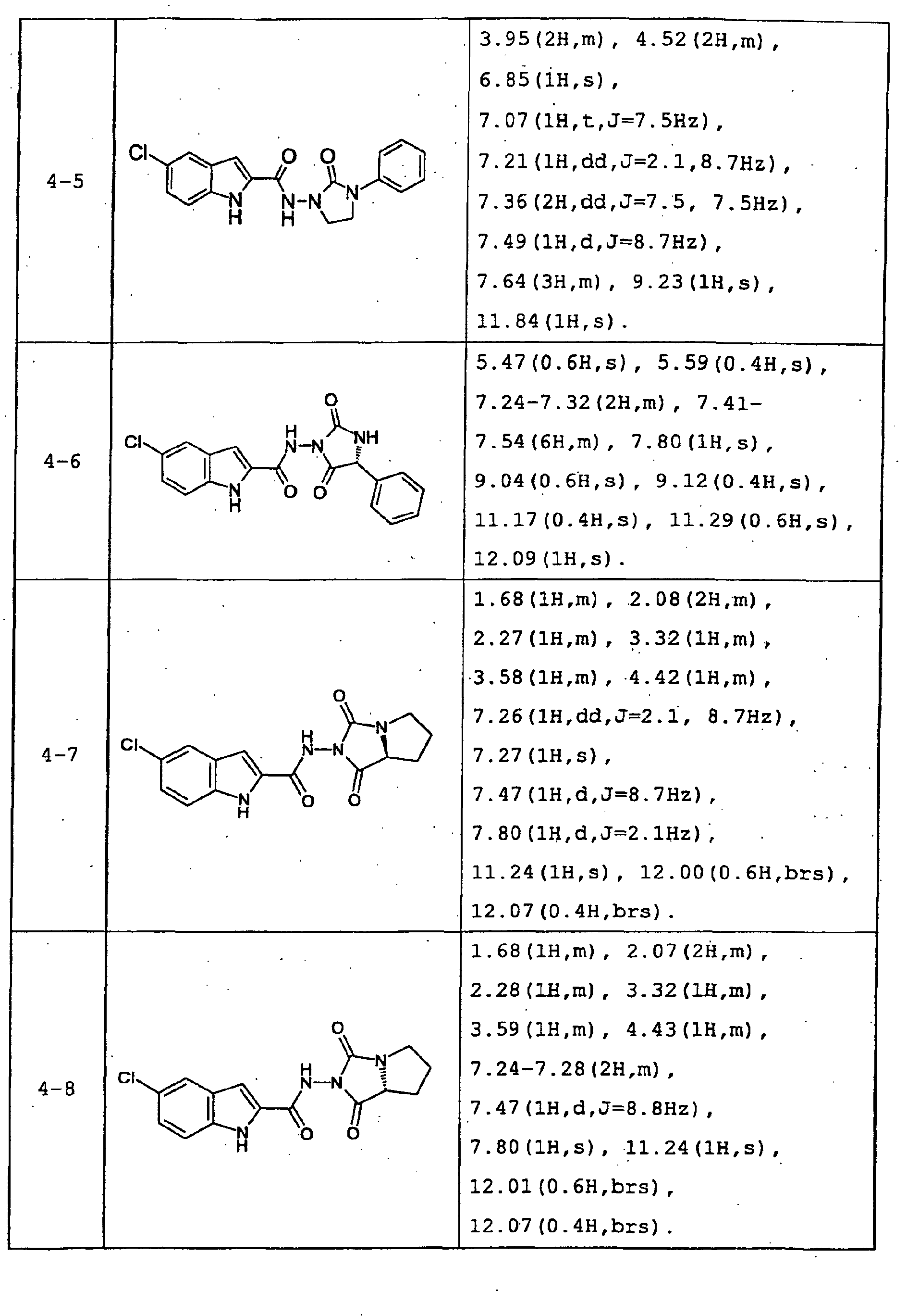

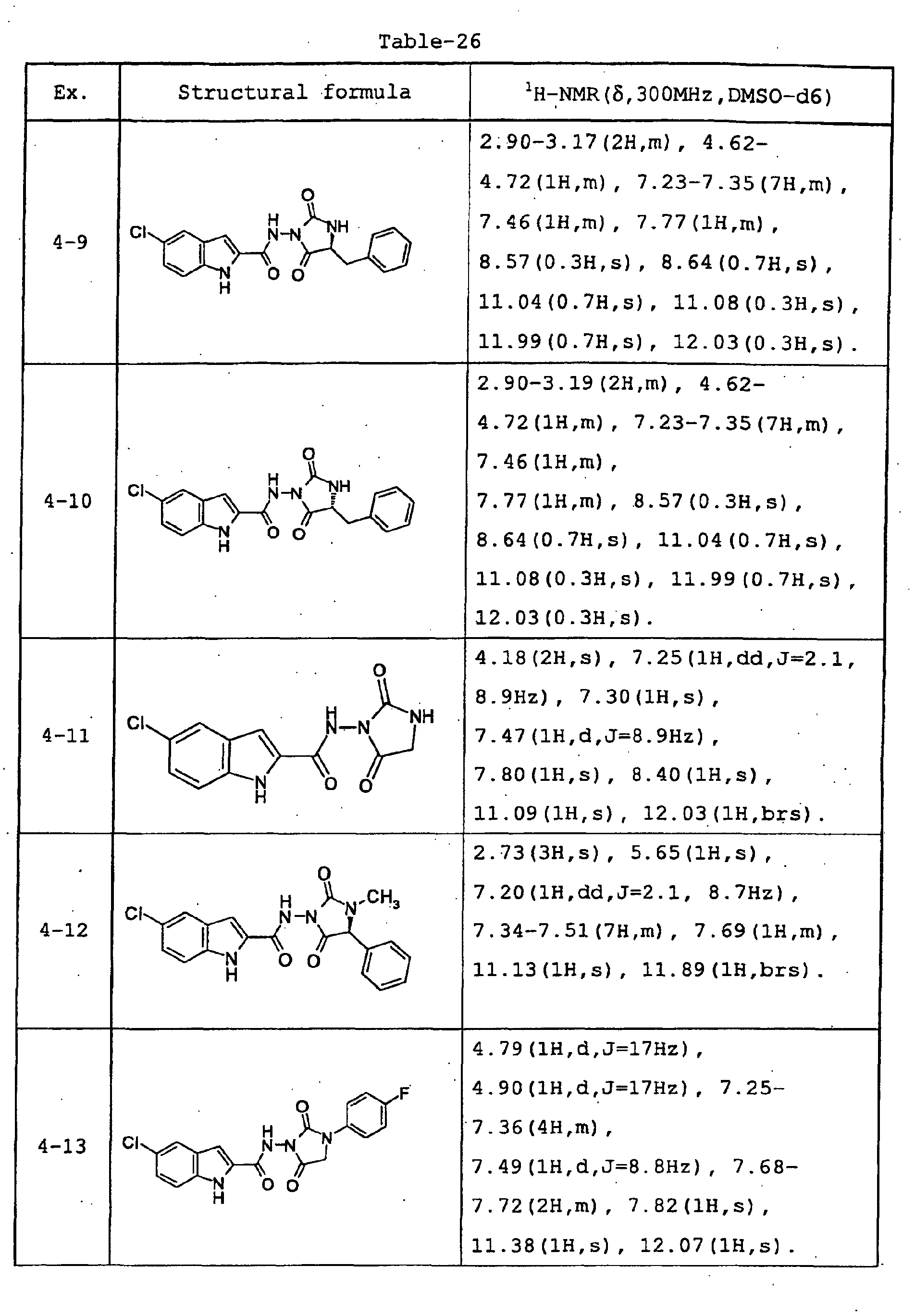

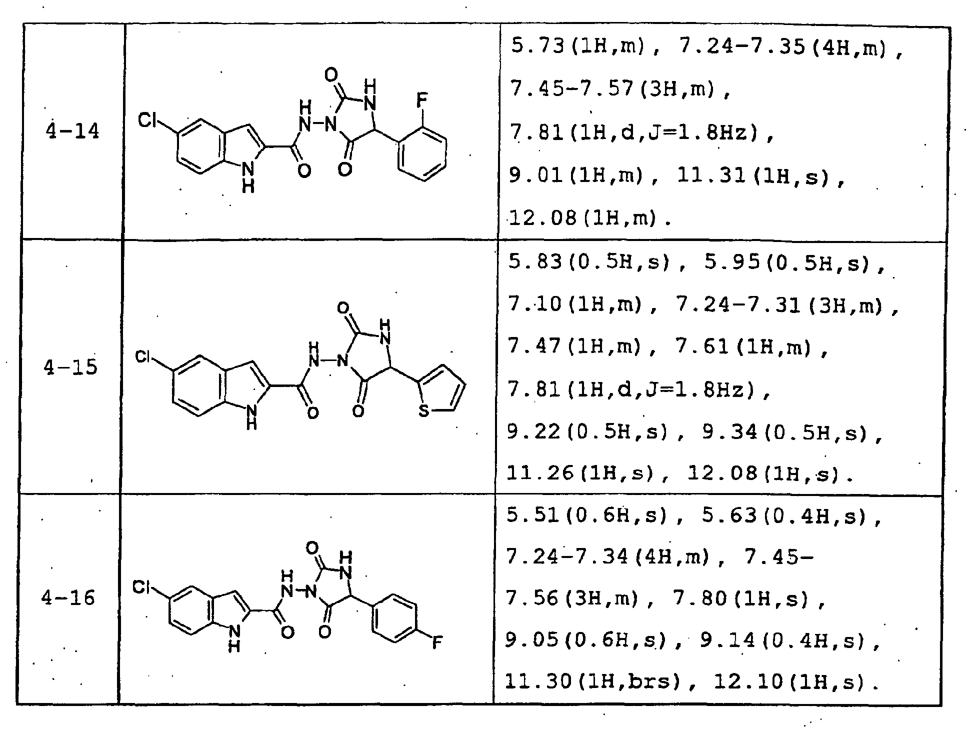

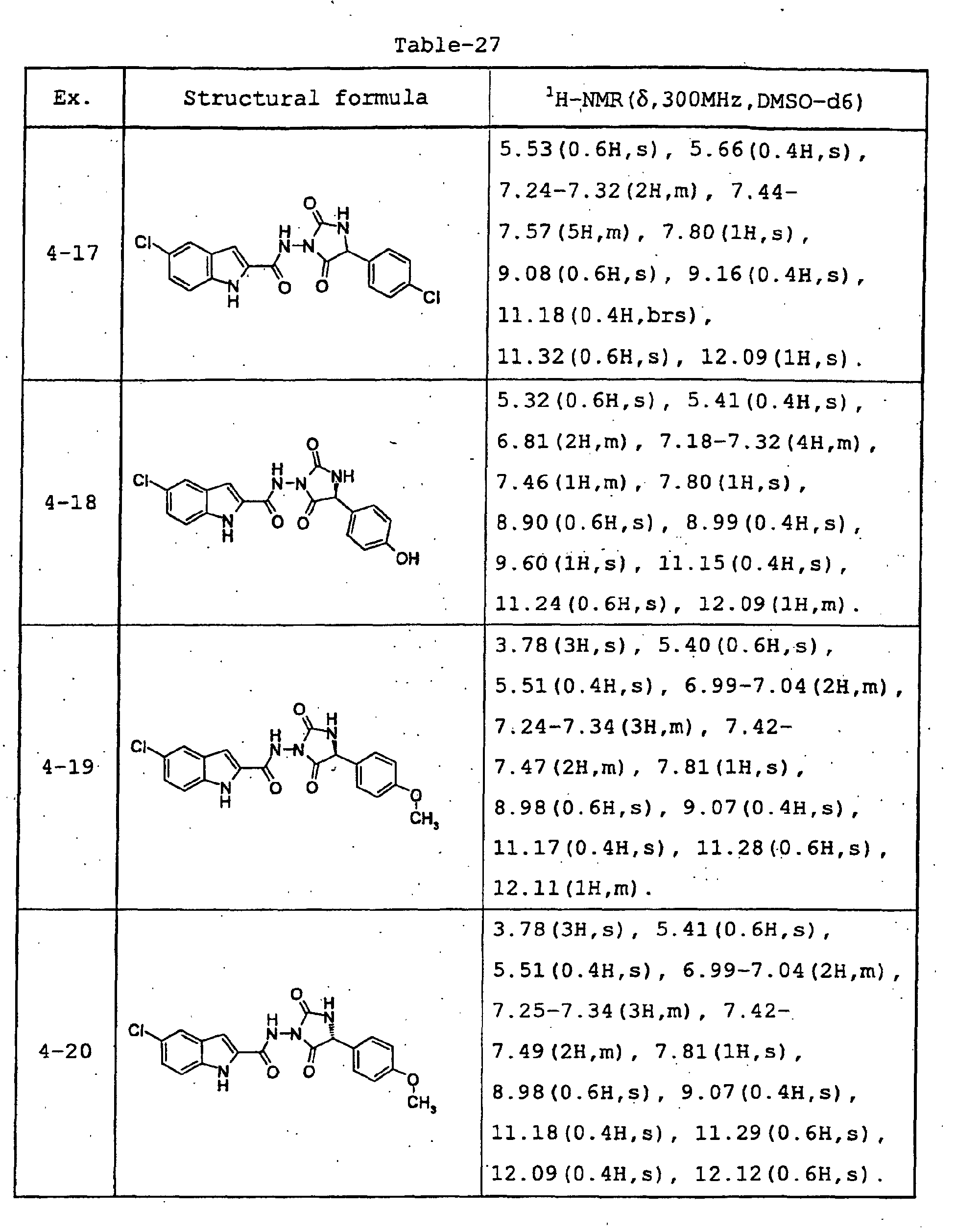

### Example 5

### 1-(5-chloro-1H-indole-2-carbonyl)-4-phenylsemicarbazide

5-Chloro-1H-indole-2-carboxylic acid hydrazide obtained in Example 1 a) (105 mg) and phenylisocyanate (54 µl) were reacted in dioxane (1 ml) for 1 hr. THF (5 ml) was added to the reaction mixture and the mixture was heated at 60°C for 1 hr. This reaction solution was concentrated and stood to allow precipitation of crystals. The crystals were collected by filtration and dried in vacuo to give the title compound (144 mg, yield 88%) (see Table 28).

### Example 5-2

### 5-chloro-1H-indole-2-carboxylic acid 2-(phenylthio)carbonylhydrazide

In the same manner as in Example 5-1 but using phenyl isothiocyanate instead of phenylisocyanate, the title compound was obtained (yield 23%) (see Table 28).

### Example 5-3

### 5-chloro-1H-indole-2-carboxylic acid 2-phenylacetylhydrazide

5-chloro-1H-indole-2-carboxylic acid hydrazide obtained in Example 1 a) (91.1 mg) was suspended in THF, and triethylamine (0.05 ml) and phenylacetylchloride (0.065 ml) were successively added. The mixture was stirred at room temperature for 1 hr and 30 min. Water (2 ml) and sodium hydrogen carbonate (34 mg) were successively added to the reaction mixture and the mixture was stirred for 1 hr. The precipitated solid was collected by filtration to give the title compound (112 mg, yield 79%) as a colorless solid(see Table 28).

### Example 5-4

### 5-chloro-1H-indole-2-carboxylic acid 2-(benzoylformyl)hydrazide

To a suspension (5 ml) of 5-chloro-1H-indole-2-carboxylic acid hydrazide obtained in Example 1 a) (240 mg) and benzoylformic acid (150 mg) in DMF were added 1-hydroxybenzotriazole (184 mg) and EDC (230 mg). The mixture was stirred at room temperature for 14 hr and water was added to the reaction solution. This mixture was extracted with ethyl acetate. The organic layer was washed successively with 10% aqueous citric acid solution, saturated aqueous sodium hydrogen carbonate, water and saturated brine, and dried over sodium sulfate. This solution was filtered and concentrated under reduced pressure. The residue was washed with diethyl ether to give the title compound (106 mg) (see Table 28).

### Examples 5-5 to 5-19

In the same manner as in Examples 5 to 5-4, the compounds of Examples 5-5 to 5-19 were obtained. The obtained compounds are shown in Tables 28-30.

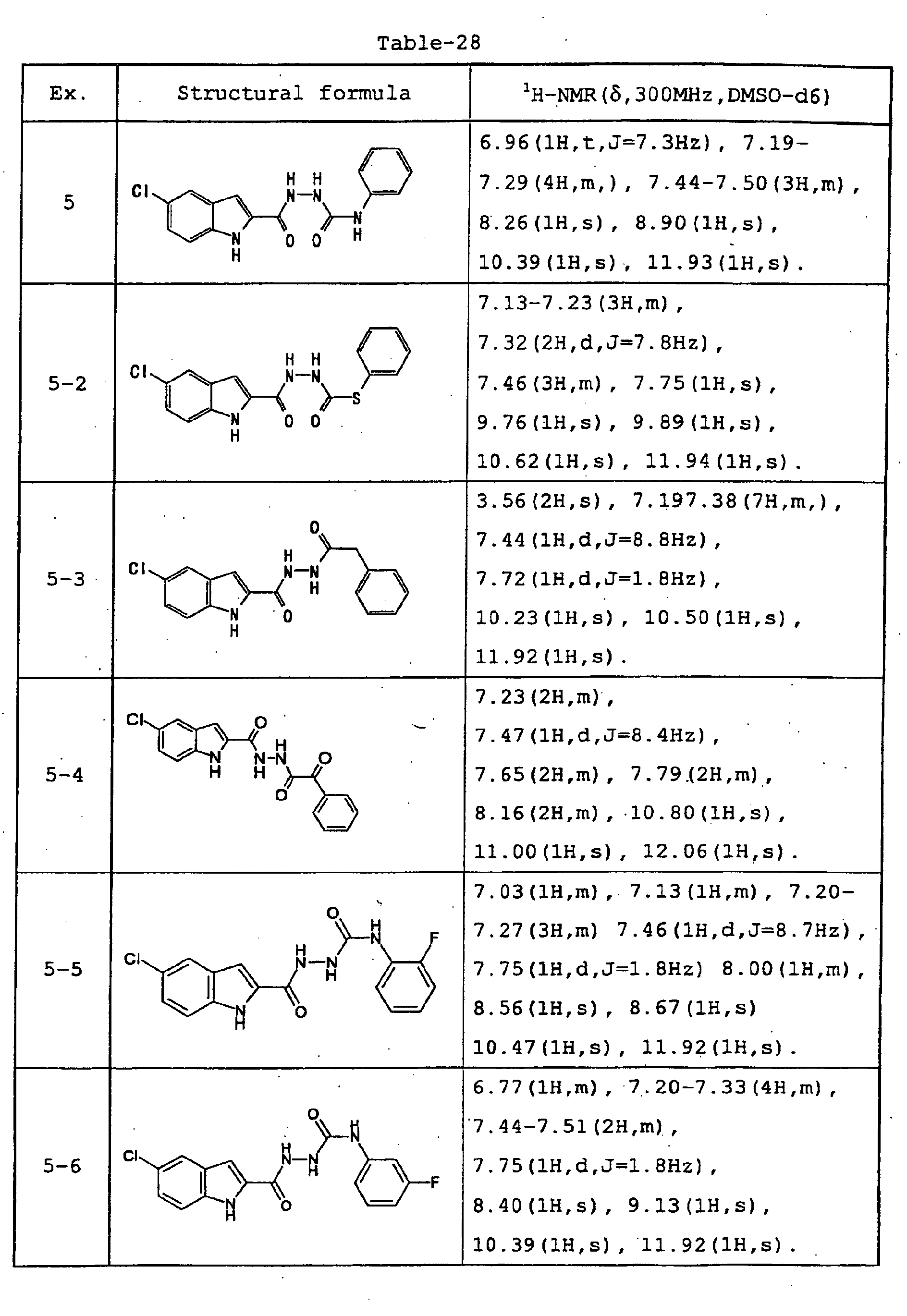

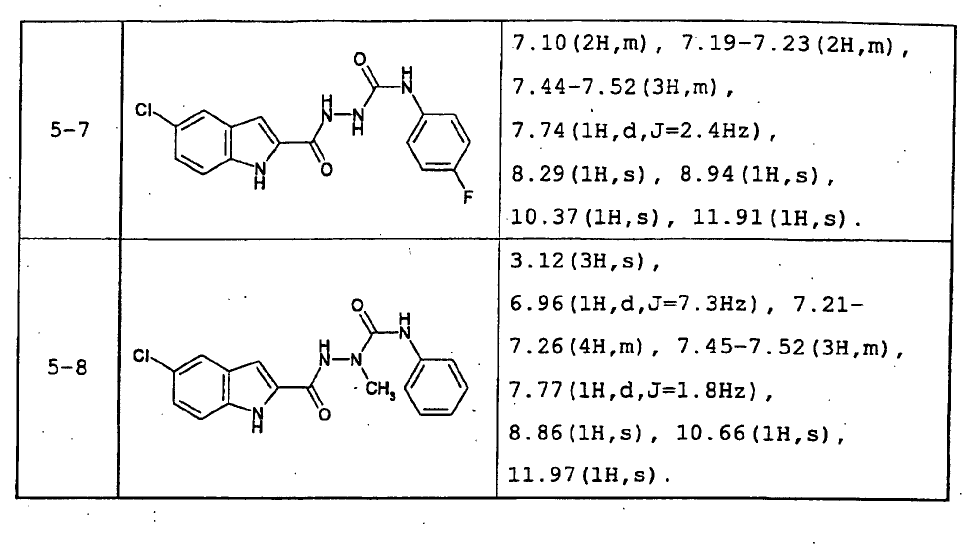

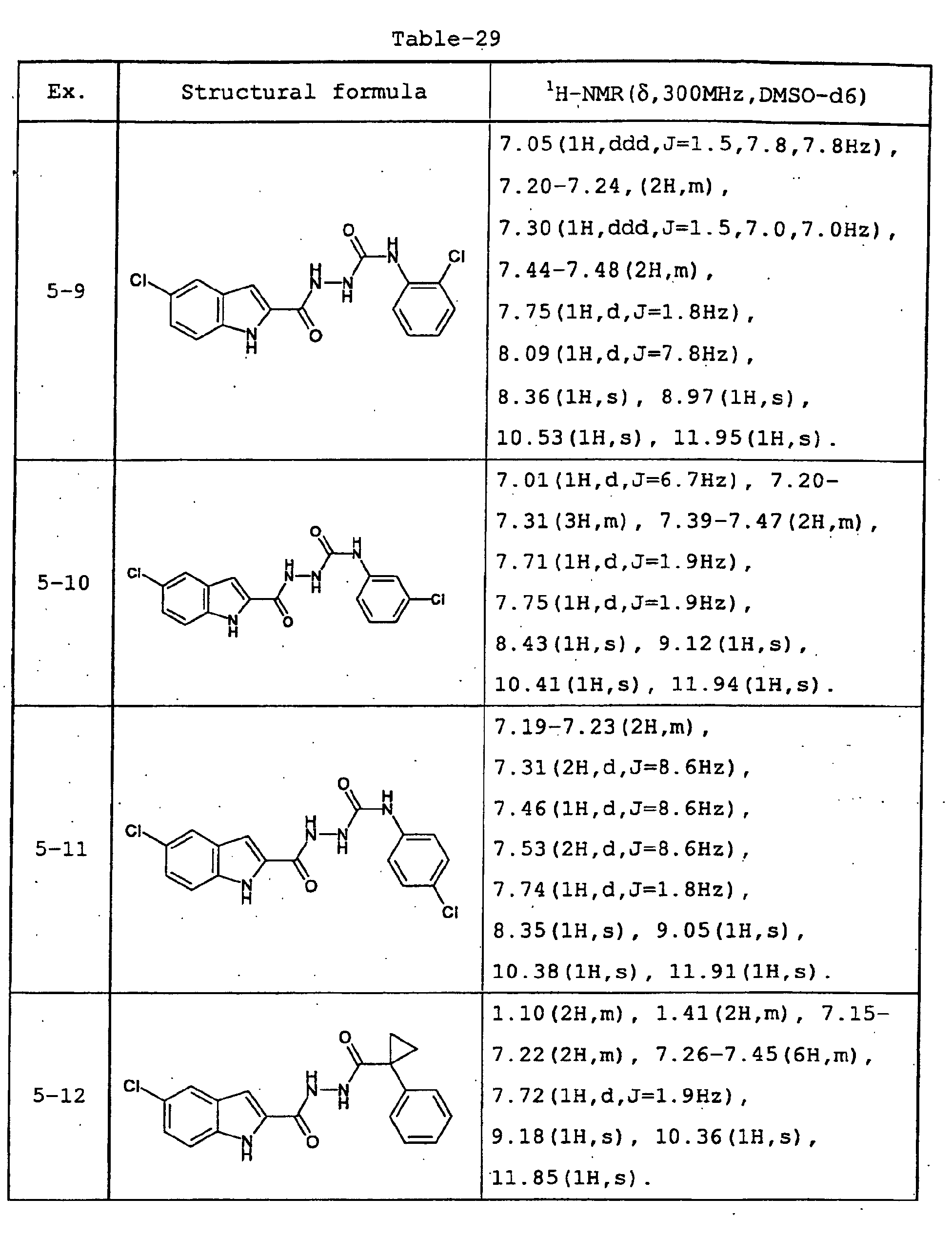

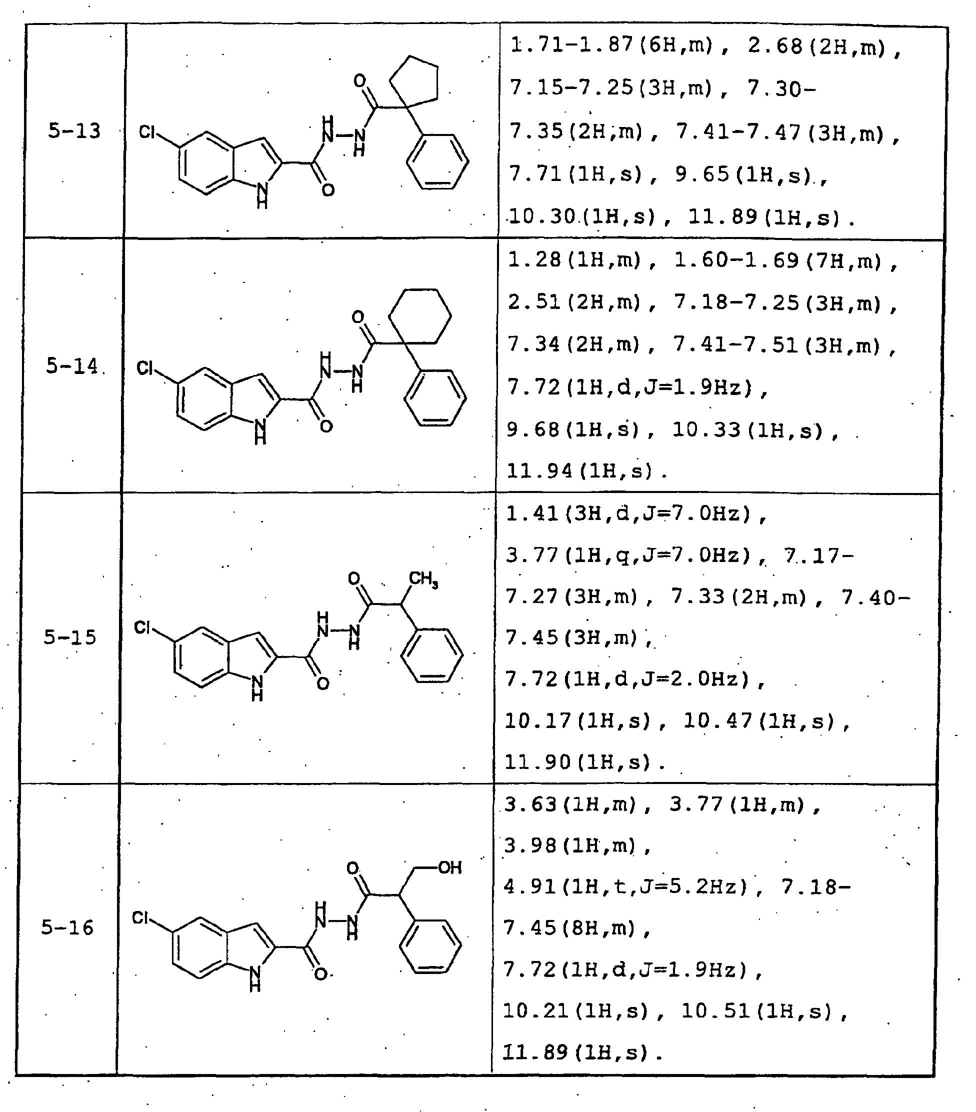

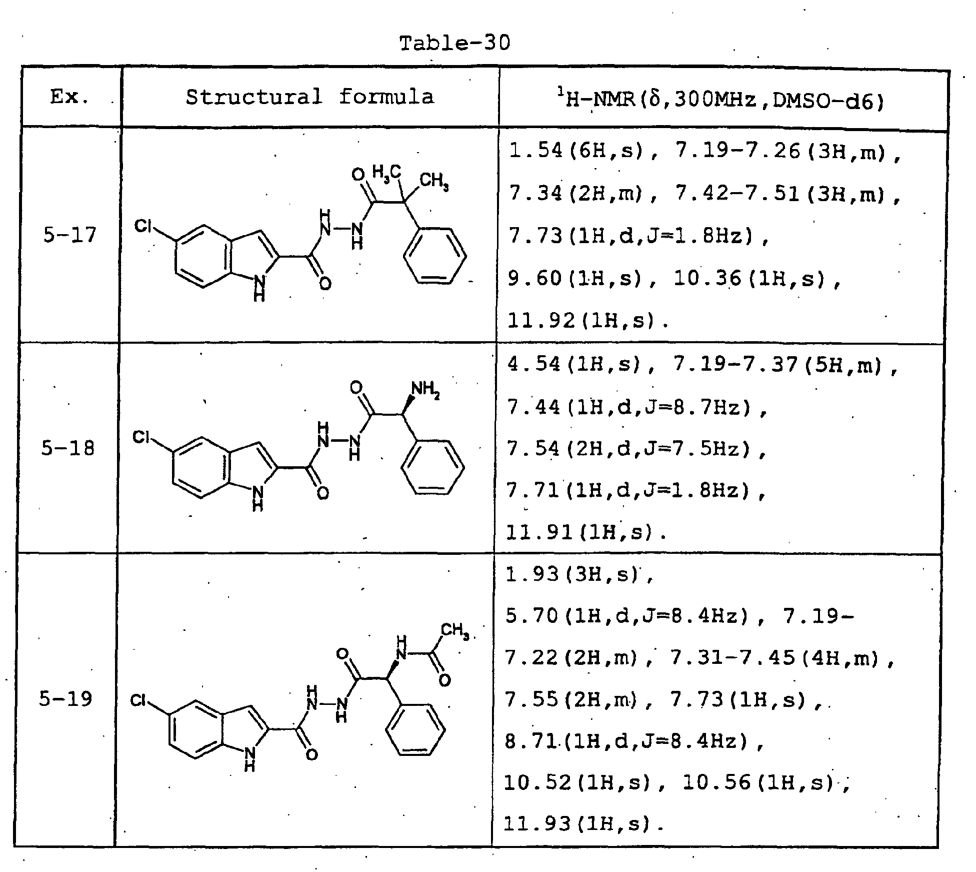

### Example 6

### 5-chloro-1H-indole-2-carboxylic acid 2-(imino-phenyl-methyl)hydrazide hydrochloride

To a suspension (530 ml) of 5-chloro-1H-indole-2-carboxylic acid 2-(imino-phenyl-methyl)hydrazide obtained in Example 1-7 (35.6 g) in MeOH was added dropwise 4N hydrochloric acid/ethyl acetate solution (34.2 ml) under ice-cooling and the mixture was stirred at room temperature for 1 hr. To this reaction solution was added isopropyl ether and the precipitated crystals were collected by filtration and washed with isopropyl ether. This was dried in vacuo to give the title compound (30.3 g, yield 76%) (see Table 31).

### Example 6-2

### 2-amino-4,5-difluorobenzoic acid 2-(5-methyl-1H-indole-2-carbonyl)hydrazide benzenesulfonate

2-Amino-4,5-difluorobenzoic acid 2-(5-methyl-1H-indole-2-carbonyl)hydrazide obtained in Example 1-67 (67.6 mg) and benzenesulfonic acid monohydrate (51.2 mg) were heated at 60°C in THF (1.0 ml) to give a solution. This solution was stood at room temperature for 2 hr to allow precipitation of crystals. The crystals were separated, washed with THF and dried in vacuo to give the title compound (83.3 mg, yield 85%) as pale-yellow needle crystals (see Table 31).

### Example 6-3

### 2-amino-4-fluorobenzoic acid 2-(5-chloro-1H-indole-2-carbonyl)hydrazide benzenesulfonate

2-Amino-4-fluorobenzoic acid 2-(5-chloro-1H-indole-2-carbonyl)hydrazide (694.0 mg) obtained in the same manner as in Example 1-97 and benzenesulfonic acid monohydrate (475.0 mg) were heated at 65°C in methanol (70 ml) to give a solution. This solution was concentrated to about 10 ml to allow precipitation of crystals. The crystals were separated, washed with ethanol and dried in vacuo to give the title compound (900.0 mg, 89%) as colorless needle crystals (see Table 31).

### Example 6-4

### 3-(dimethylamino)benzoic acid 2-(5-chloro-1H-indole-2-carbonyl)hydrazide methanesulfonate

3-(Dimethylamino)benzoic acid 2-(5-chloro-1H-indole-2-carbonyl)hydrazide obtained in Example 1-45 (68.3 mg) and methanesulfonic acid (19 µl) were dissolved in methanol (0.7 ml) at room temperature. Diethyl ether (1.0 ml) was added dropwise to this solution to allow precipitation of needle crystals. The crystals were separated, washed with diethyl ether and dried in vacuo to give the title compound (72.8 mg, 84%) as colorless needle crystals (see Table 31).

### Example 6-5

### 2-amino-4-fluorobenzoic acid 2-(5-chloro-1H-indole-2-carbonyl)hydrazide hydrochloride

2-Amino-4-fluorobenzoic acid 2-(5-chloro-1H-indole-2-carbonyl)hydrazide obtained in Example 1-97 (93 mg) was suspended in methanol (7.0 ml). A 4N hydrochloric acid-dioxane solution (0.55 ml) was added to this mixture and the mixture was heated to 60°C to give a solution. Diethyl ether (5 ml) was added dropwise to the obtained solution. The obtained mixture was stood for 1 hr to allow precipitation of white crystals. The precipitated crystals were collected by filtration, washed with methanol and dried in vacuo to give the title compound (670 mg, 87%) as colorless needle crystals (see Table 31).

### Example 6-6

### 2-amino-4-fluorobenzoic acid 2-(5-chloro-1H-indole-2-carbonyl)hydrazide p-toluenesulfonate

2-Amino-4-fluorobenzoic acid 2-(5-chloro-1H-indole-2-carbonyl)hydrazide obtained in Example 1-97 (693 mg) and p-toluenesulfonic acid monohydrate (571 mg) were dissolved in DMSO (2.5 ml). The obtained solution was added dropwise to methanol (6 ml) over 3 min with stirring. The precipitated crystals were collected by filtration, washed with methanol and dried in vacuo to give the title compound (897 mg, 86%) as colorless needle crystals (see Table 31).

### Example 6-7

### 2-amino-4-fluorobenzoic acid 2-(5-chloro-1H-indole-2-carbonyl)hydrazide p-toluenesulfonate

2-Amino-4-fluorobenzoic acid 2-(5-chloro-1H-indole-2-carbonyl)hydrazide obtained in Example 1-97 (1.73 g) was dissolved in a THF:H₂O (10:1) mixed solvent (10 ml). p-Toluenesulfonic acid monohydrate (1.05 g) was dissolved in THF (1 ml) and the mixture was heated to 60°C. A solution of 2-amino-4-fluorobenzoic acid 2-(5-chloro-1H-indole-2-carbonyl)hydrazide in water-containing THF was added dropwise to a solution of p-toluenesulfonic acid in THF at 60°C. After the completion of the dropwise addition, this mixture was stirred at room temperature for 3 hr. The precipitated crystals were collected by filtration, washed with THF and dried in vacuo to give the title compound (1.99 g, 77%) as colorless needle crystals (see Table 31).

### Example 6-8

### 2-amino-4-fluorobenzoic acid 2-(5-chloro-1H-indole-2-carbonyl)hydrazide p-toluenesulfonate

2-Amino-4-fluorobenzoic acid 2-(5-chloro-1H-indole-2-carbonyl)hydrazide obtained in Example 1-97 (5.00 g) was dissolved in a mixture (47.5 ml) of THF:methanol:H₂O (70:30:10) at 60°C. To this solution was added dropwise a mixture (4.1 ml) of p-toluenesulfonic acid (4.11 g) in THF:methanol:H₂O (70:30:10) over 5 min with stirring while maintaining at 60°C. After the completion of the dropwise addition, this mixture was stirred at room temperature for 3 hr. The precipitated crystals were treated in the same manner as in Example 1 to give the title compound (6.42 g, 86%) as colorless needle crystals (see Table 31).

### Example 6-9

### 2-amino-4-fluorobenzoic acid 2-(5-chloro-1H-indole-2-carbonyl)hydrazide p-toluenesulfonate

2-Amino-4-fluorobenzoic acid 2-(5-chloro-1H-indole-2-carbonyl)hydrazide obtained in Example 1-97 (347 mg) and p-toluenesulfonic acid monohydrate (190 mg) were suspended in n-hexane (15 ml). This mixture was stirred at room temperature for one week. The obtained crystals were collected by filtration, washed with n-hexane and dried in vacuo to give the title compound (430mg,83%) as a white powder (see Table 32).

### Example 6-10

### 2-aminobenzoic acid 2-(5-methyl-1H-indole-2-carbonyl) hydrazide p-toluenesulfonate

The compound was produced using a compound obtained in Example 1-62 as a starting material and according to the method of Example 6-8 (see Table 32).

### Example 6-11

### 2-aminobenzoic acid 2-(5-methyl-1H-indole-2-carbonyl)hydrazide benzenesulfonate

The compound was produced using a compound obtained in Example 1-62 as a starting material and according to the method of Example 6-2 (see Table 32).

### Example 6-12

### 2-amino-4-fluorobenzoic acid 2-(5-methyl-1H-indole-2-carbonyl)hydrazide benzenesulfonate

The compound was produced using a compound obtained in Example 1-63 as a starting material and according to the method of Example 6-2 (see Table 32).

### Example 6-13

### 3-aminobenzoic acid 2-(5-methyl-1H-indole-2-carbonyl)hydrazide p-toluenesulfonate

The compound was produced using a compound obtained in Example 1-142 as a starting material and according to the method of Example 6-8 (see Table 32).

### Examples 6-14 to 6-35

In the same manner as in Examples 6 to 6-13, the compounds of Examples 6-14 to 6-35 were obtained. The obtained compounds are shown in Tables 31-35.

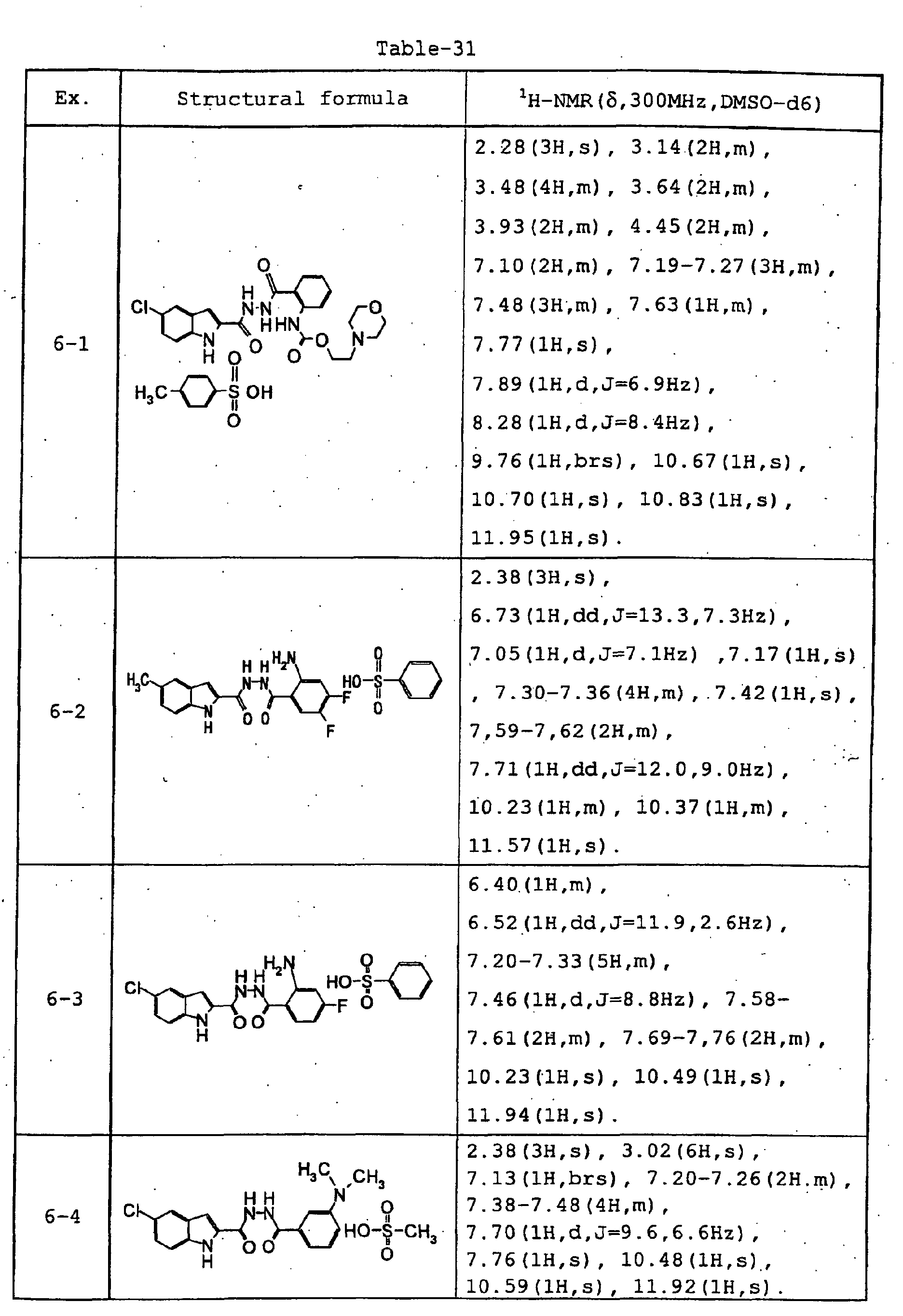

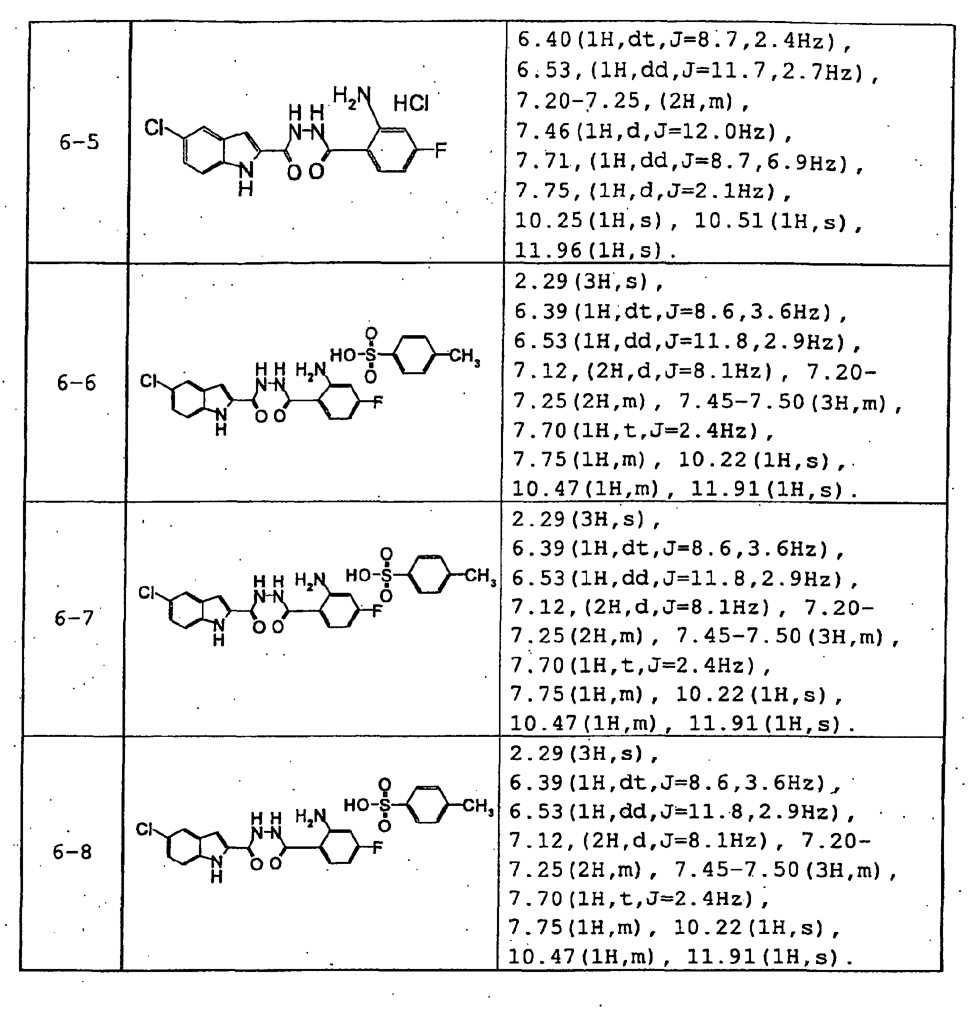

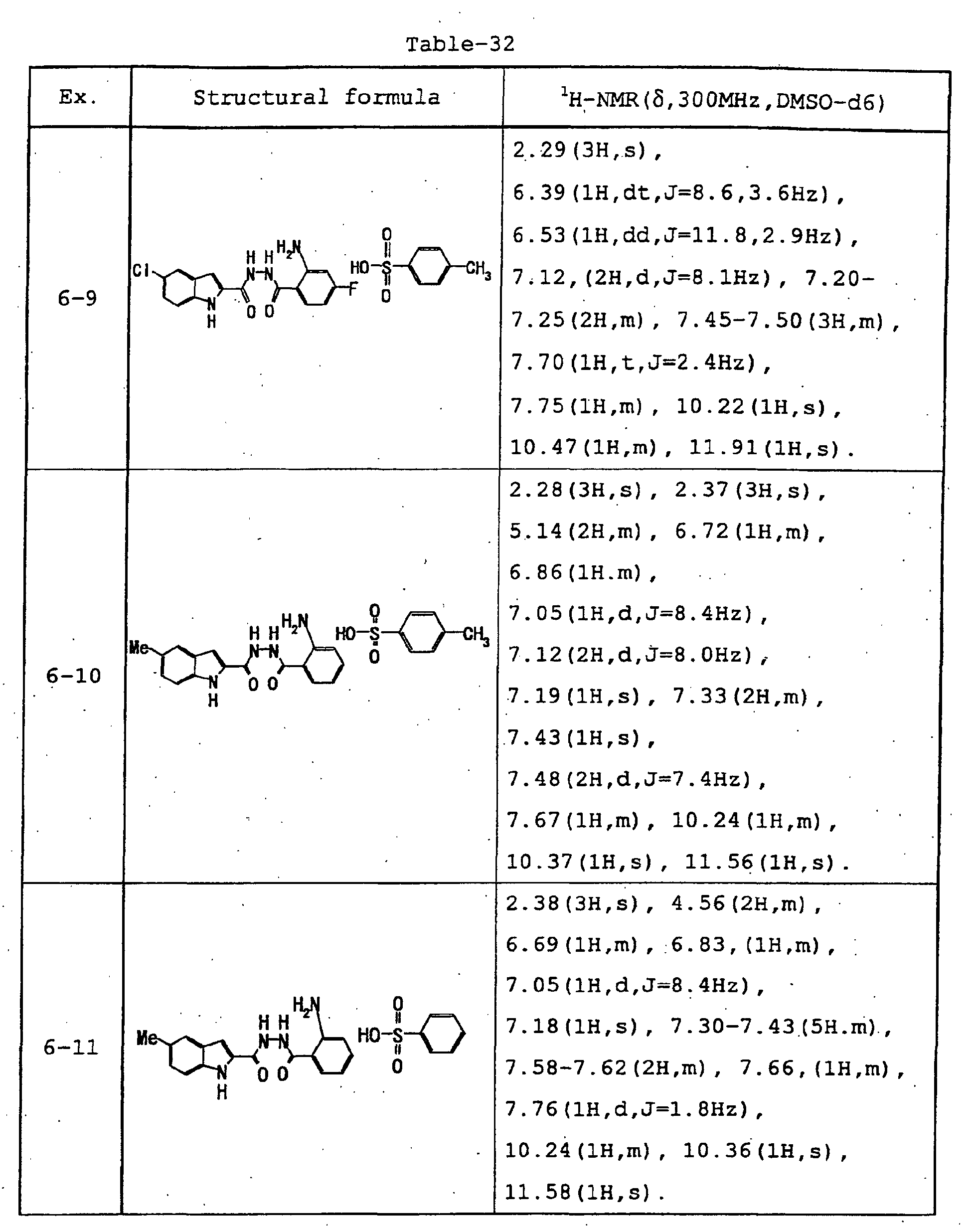

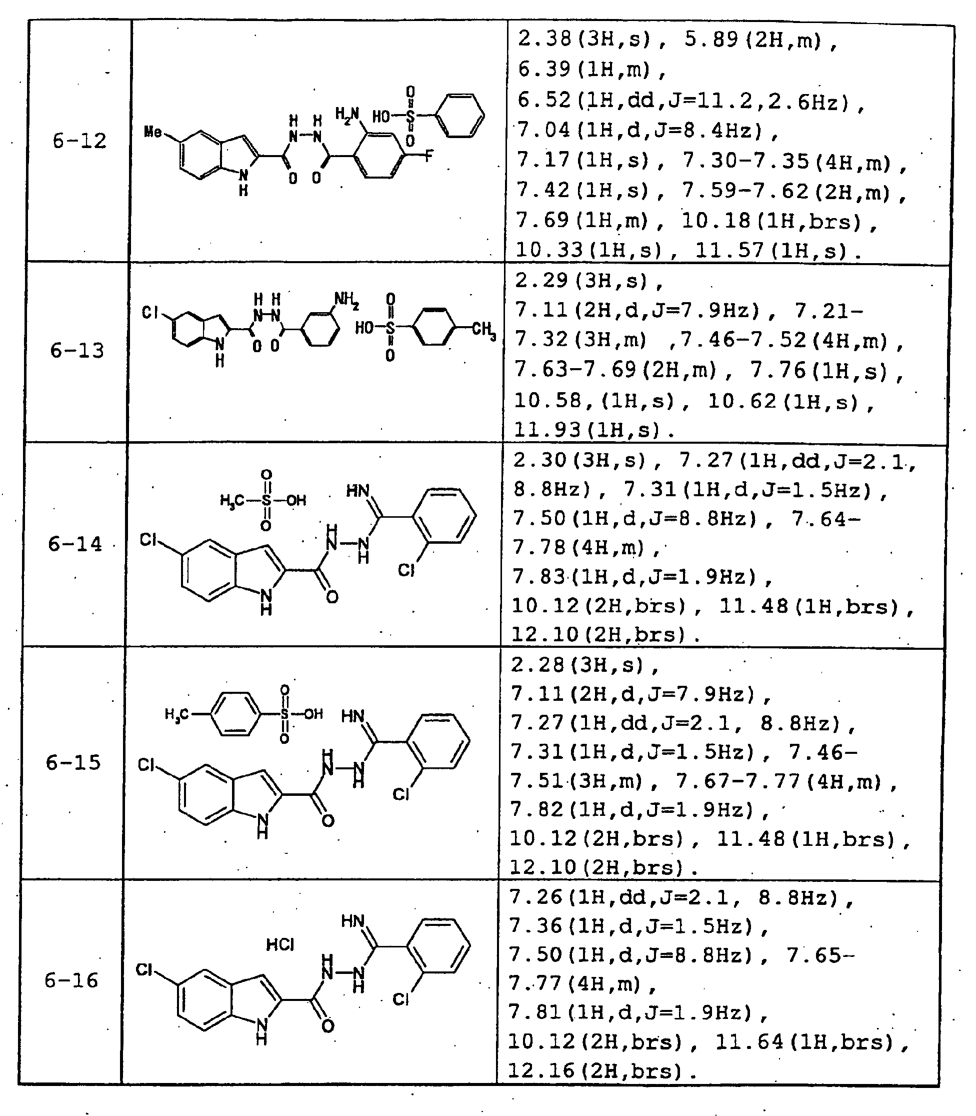

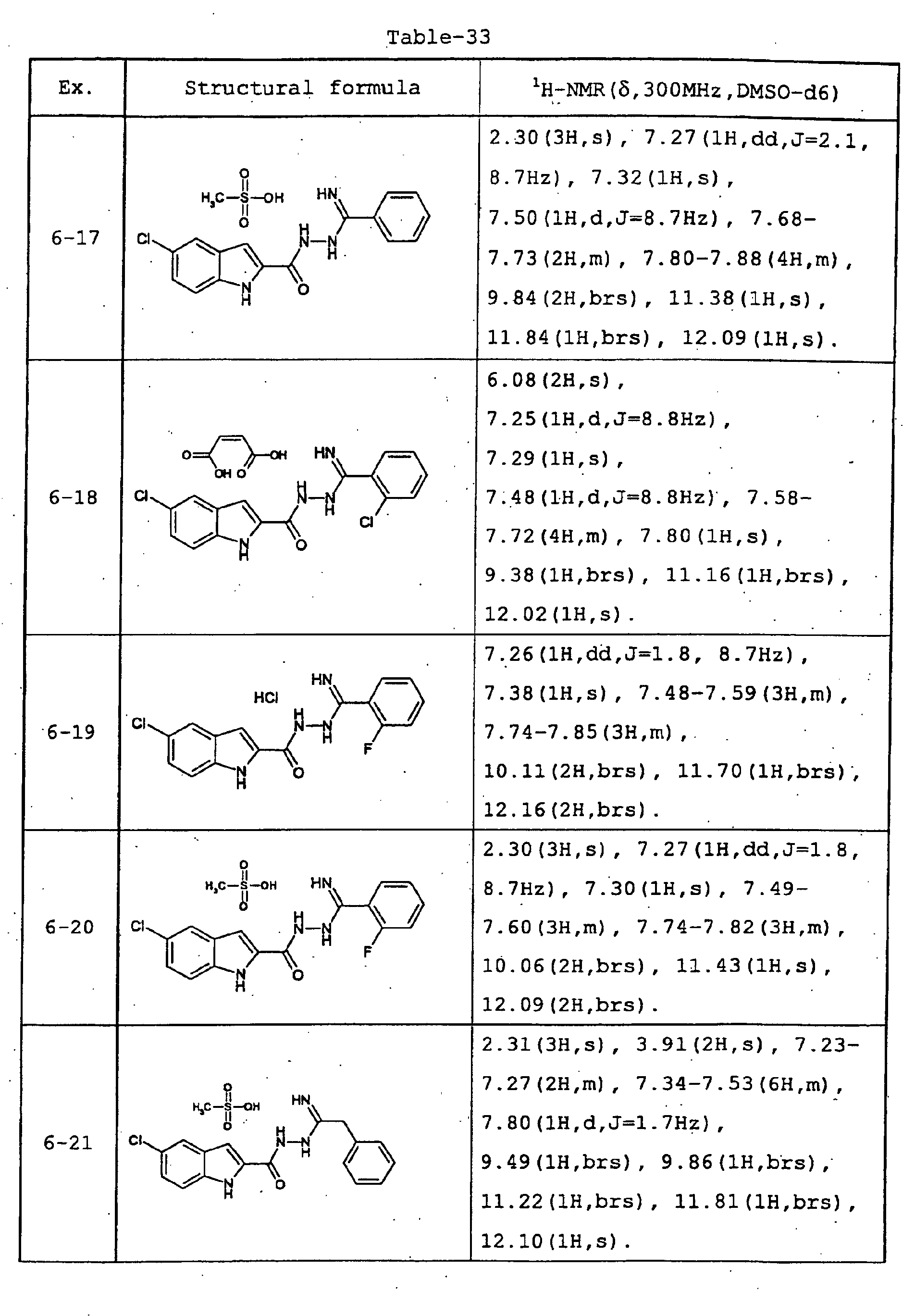

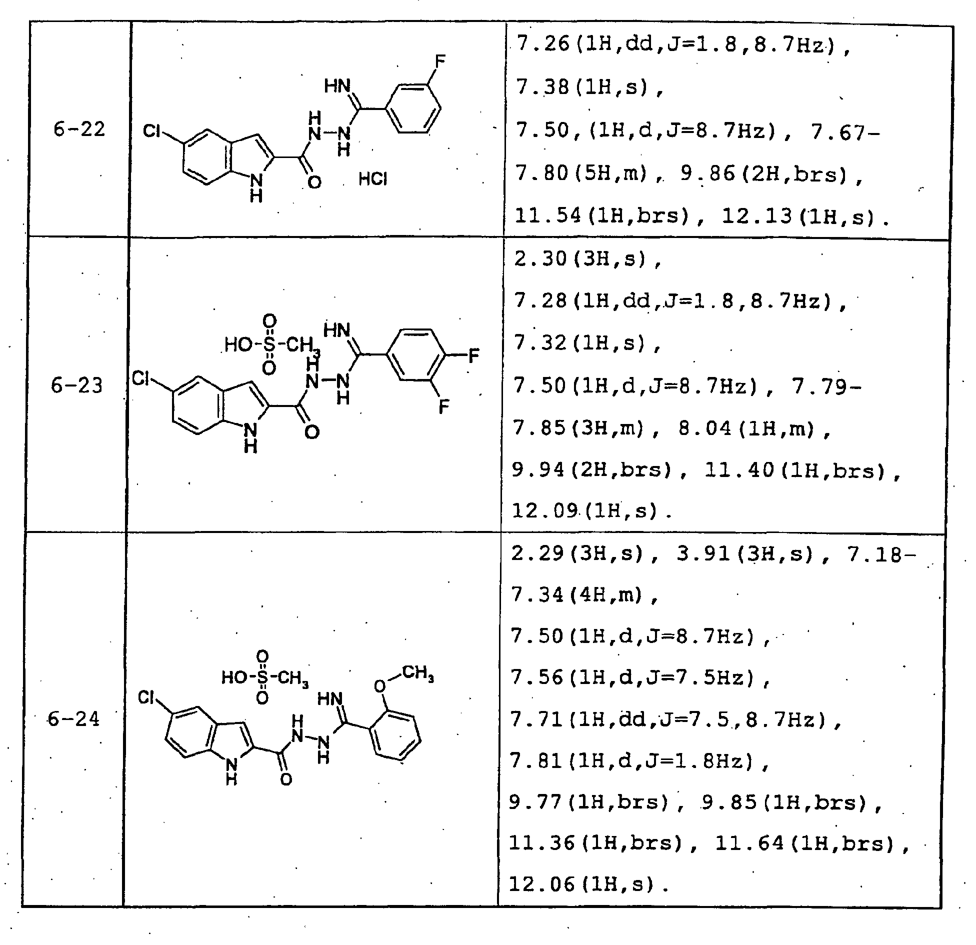

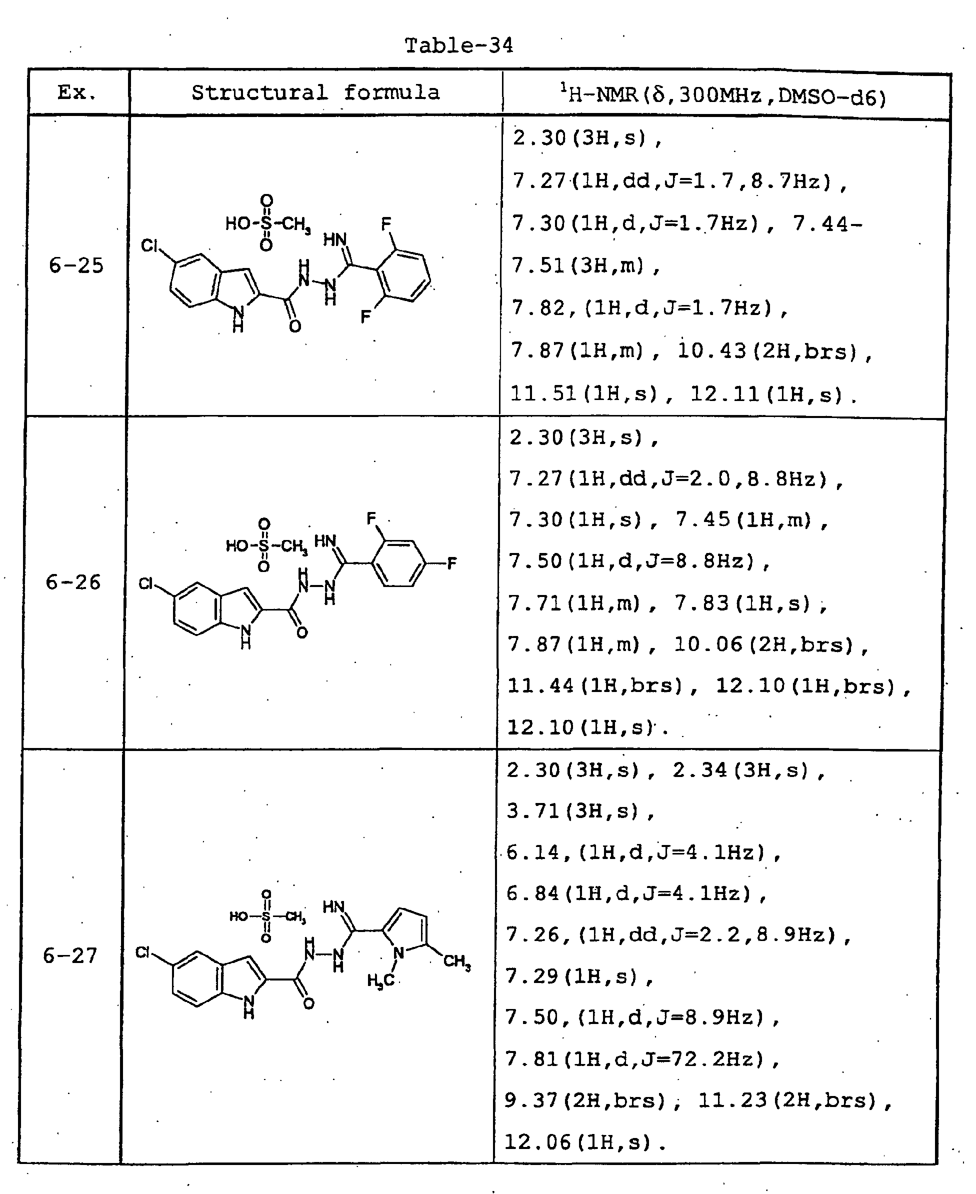

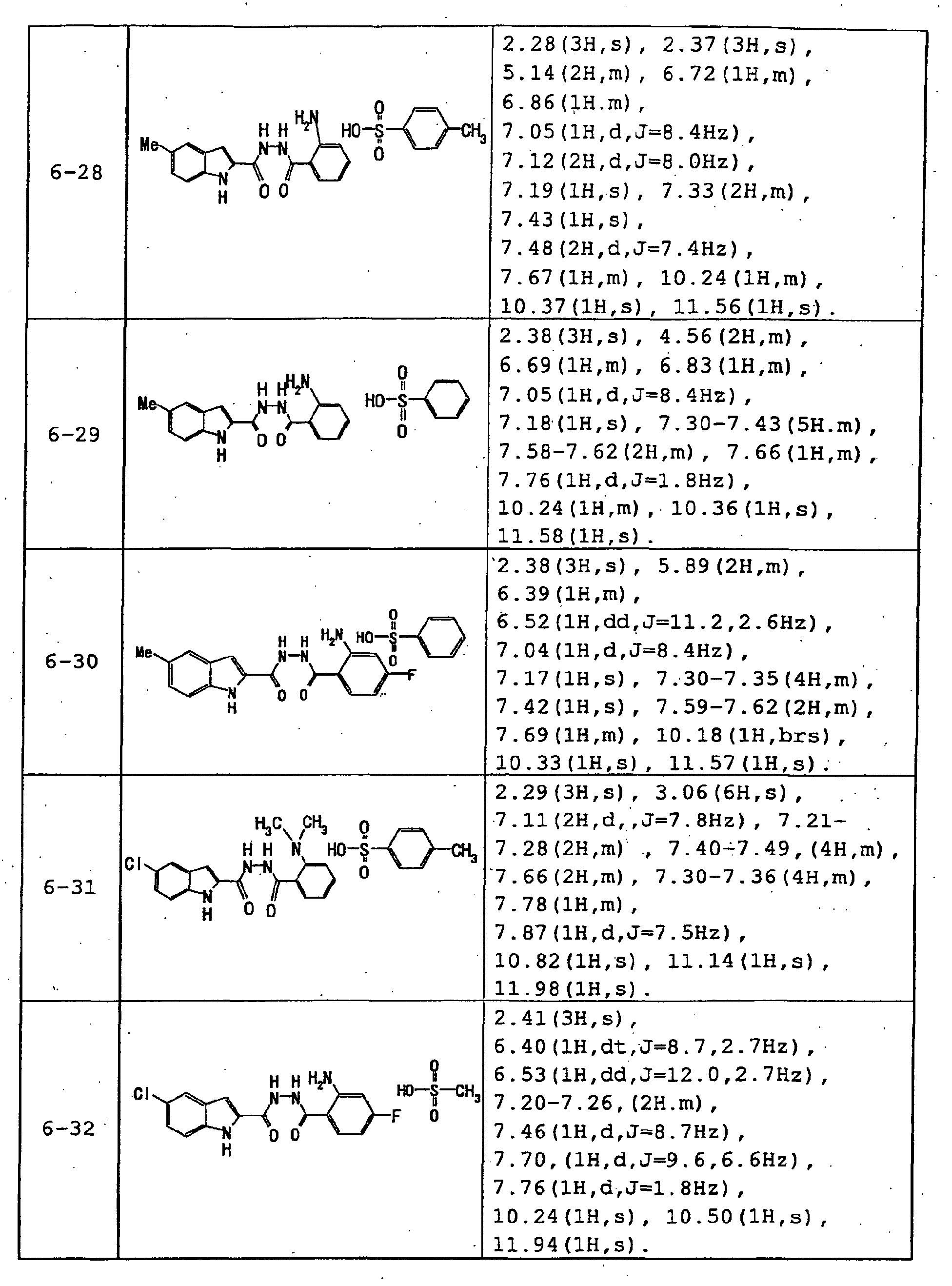

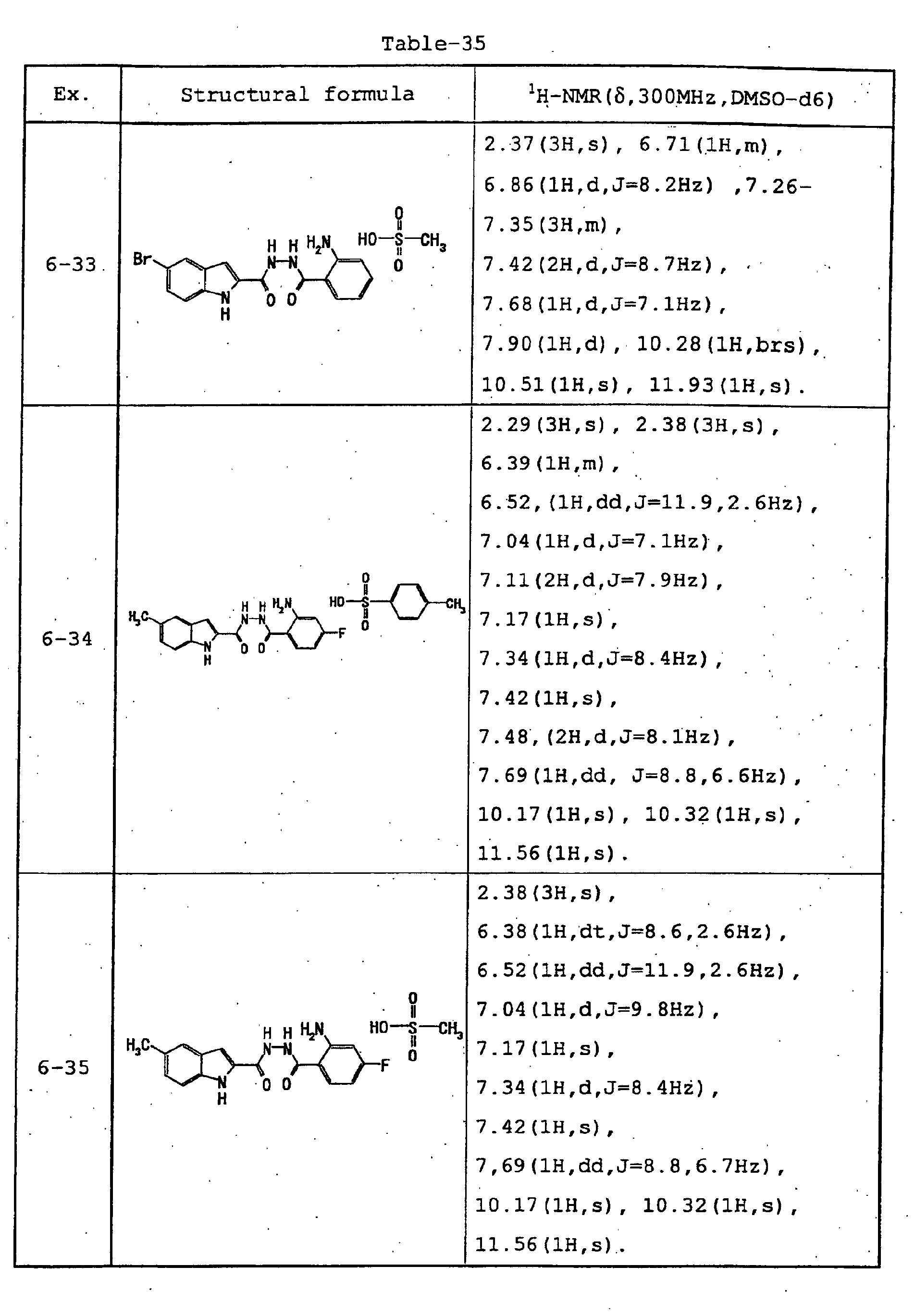

### Pharmacological Tests

### Experimental Example (1) Measurement method of liver glycogen phosphorylase activity

The measurement of the glycogen phosphorylase activity was performed by measuring the concentration of phosphoric acic produced by a reverse reaction, that is, a reaction wherein the glycogen phosphorylase synthesizes glycogen from G1-P. A cell lysate of Sf9 cells that forcibly expressed recombinant human liver glycogen phosphorylase was diluted to a protein amount of 80 µg/mL with 1 mM imidazole-hydrochloric acid buffer (pH 7.0, containing 0.2 mM PMSF, 250 mM NaCl, 0.025% BSA) and the dilute cell lysate was used as an enzyme solution of human liver glycogen phosphorylase. As a substrate solution, 25 mM Tris-HCl buffer (pH 7.2, containing 250 mM KCl, 6.25 mM MgCl₂, 6.25 mM EGTA, 1.25 mM glucose-1-phosphate, 2.5 mg/ml glycogen, 7.5 mM glucose) was used. The test drug was dissolved in 0.5% dimethyl sulfoxide (DMSO). To a mixture of the test drug (10 µl) and the substrate solution (20 µl) was added an enzyme solution (20 µl) to start the enzyme reaction. As a control, 0.5% DMSO was added instead of the test drug. One free of addition of enzyme was used as a blank. The mixture was reacted at room temperature for 60 min, and a malachite green solution (50 µl) was added. The mixture was further reacted at room temperature for 20 min and absorbance at 650 nm was measured. An enzyme solution was added to the blank simultaneously with the malachite green solution and the measurement was done in the same manner. The percent inhibition (%) of the test drug was calculated from ((value of control - value of the test drug)/(value of control - value of blank) ) ×100 (%) .

The test results of the above-mentioned Experimental Example are shown in Tables 36-38.

**Table 36**

| Ex. No. | Enzyme inhibitory activity against HLGPa IC₅₀ (µM) | Ex. No. | Enzyme inhibitory activity against HLGPa IC₅₀ (µM) | Ex. No. | Enzyme inhibitory activity against HLGPa IC₅₀ (µM) |
|---|---|---|---|---|---|
| 1 | 0.038 | 1-36 | >0.1 | 1-65 | >0.1 |
| 1-2 | 0.013 | 1-37 | >0.1 | 1-66 | >0.1 |
| 1-3 | 0.037 | 1-38 | >0.1 | 1-67 | 0.054 |
| 1-9 | 0.073 | 1-39 | 0.81 | 1-68 | 0.16 |
| 1-10 | 0.20 | 1-41 | 0.31 | 1-69 | 0.15 |
| 1-11 | 0.072 | 1-42 | 0.26 | 1-70 | 0.15 |
| 1-12 | 0.79 | 1-43 | 0.095 | 1-71 | 0.062 |
| 1-13 | 0.072 | 1-44 | 0.10 | 1-72 | >0.1 |
| 1-14 | 0.13 | 1-46 | 0.29 | 1-73 | 0.076 |
| 1-15 | 0.12 | 1-47 | 0.66 | 1-75 | 0.25 |
| 1-16 | 0.073 | 1-49 | 0.15 | 1-76 | 0.037 |
| 1-18 | 0.099 | 1-50 | 0.027 | 1-78 | 0.018 |
| 1-19 | 0.091 | 1-51 | 0.089 | 1-79 | 0.083 |
| 1-20 | 0.035 | 1-52 | 0.091 | 1-80 | 0.40 |
| 1-21 | 0.049 | 1-53 | 0.23 | 1-81 | 0.028 |
| 1-23 | 0.075 | 1-54 | 0.030 | 1-82 | 0.36 |
| 1-24 | 0.17 | 1-55 | 0.087 | 1-83 | 0.88 |
| 1-25 | 0.14 | 1-57 | 0.030 | 1-84 | 0.02 |
| 1-26 | 0.018 | 1-58 | >0.1 | 1-85 | 0.43 |
| 1-27 | 0.047 | 1-59 | >0.1 | 1-86 | 0.028 |
| 1-28 | 0.049 | 1-60 | >0.1 | 1-87 | 0.056 |
| 1-29 | 0.015 | 1-61 | 0.049 | 1-88 | 0.020 |
| 1-30 | 0.24 | 1-62 | 0.088 | 1-89 | 0.45 |
| 1-32 | 0.35 | 1-63 | 0.028 | 1-91 | 0.056 |
| 1-33 | 0.086 | 1-64 | >0.1 | 1-92 | 0.071 |

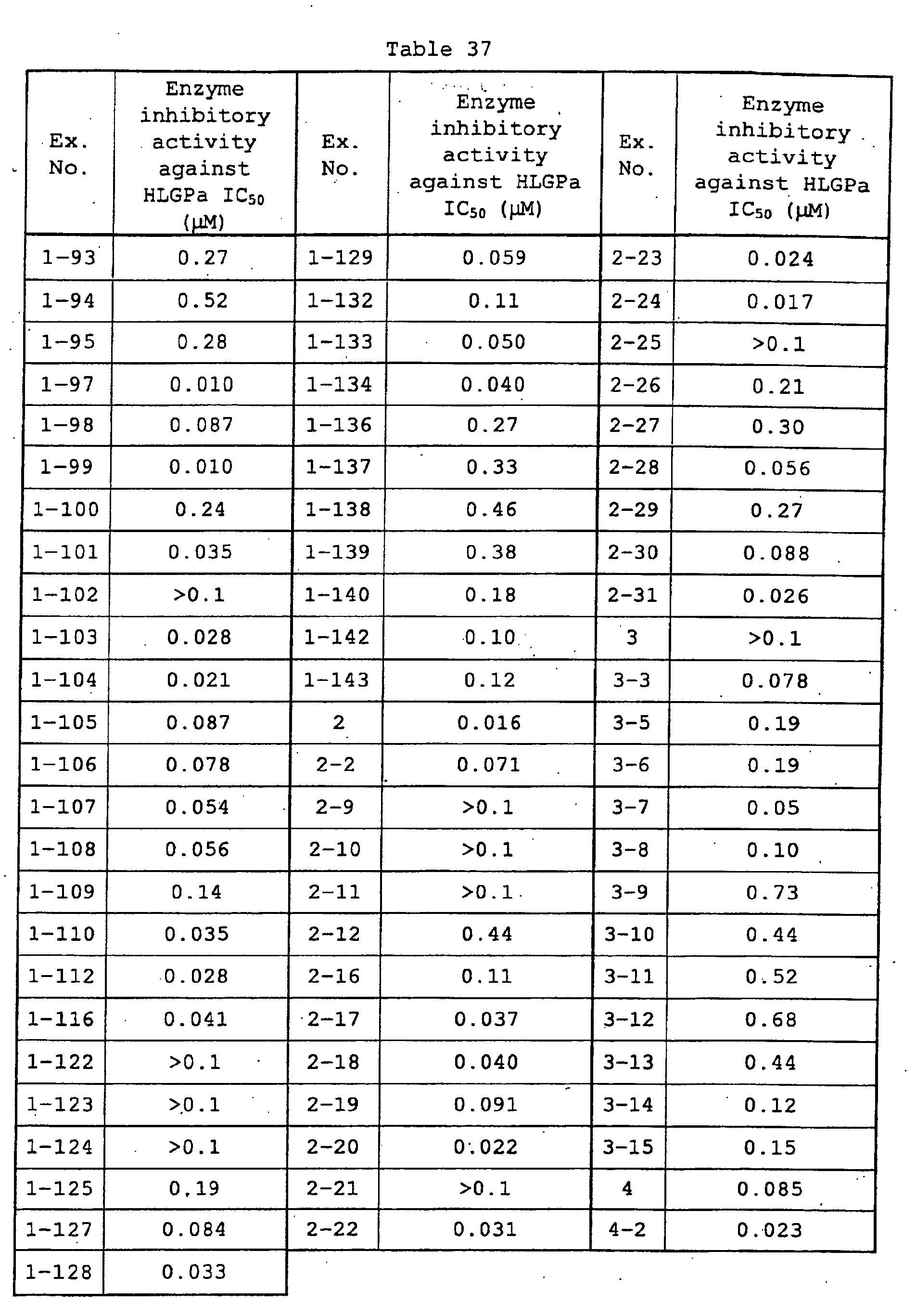

**Table 38**

| Ex. No. | Enzyme inhibitory activity against HLGPa IC₅₀ (µM) | Ex. No. | Enzyme inhibitory activity against HLGPa IC₅₀ (µM) | Ex. No. | Enzyme inhibitory activity against HLGPa IC₅₀ (µM) |
|---|---|---|---|---|---|
| 4-3 | >0.1 | 4-20 | 0.17 | 6 | 0.062 |
| 4-4 | >0.1 | 5-5 | 0.079 | 6-14 | 0.015 |
| 4-5 | >0.1 | 5-6 | 0.049 | 6-15 | 0.059 |
| 4-6 | 0.065 | 5-7 | 0.088 | 6-16 | 0.059 |
| 4-7 | >0.1 | 5-8 | >0.1 | 6-17 | 0.080 |
| 4-8 | >0.1 | 5-9 | >0.1 | 6-18 | 0.049 |
| 4-9 | >0.1 | 5-10 | >0.1 | 6-19 | 0.053 |
| 4-10 | >0.1 | 5-11 | >0.1 | 6-20 | 0.11 |
| 4-11 | >0.1 | 5-12 | 0.29 | 6-21 | 0.084 |
| 4-12 | >0.1 | 5-13 | 0.41 | 6-23 | 0.067 |
| 4-13 | >0.1 | 5-14 | 0.30 | 6-24 | 0.16 |
| 4-14 | 0.11 | 5-15 | 0.063 | 6-25 | 0.33 |
| 4-18 | 0.075 | 5-18 | >0.1 | 6-27 | 0.17 |
| 4-19 | 0.096 | 5-19 | 0.36 | 6-28 | 0.25 |

### Experimental Example (2) Measurement method of plasma glucose concentration

Using obesity type diabetes model db/db mice, the effect of the compound (1) of the present invention on glucose concentration in plasma was examined. The glucose concentration in the plasma of db/db mice (10-15 weeks of age) was measured, and the mice were grouped into 5 mice per group such that the average glucose concentration of plasma is leveled. After fasting for 4 hr, the test drug or a solvent (0.5% methyl cellulose) was orally administered to db/db mice, and plasma glucose concentration at 1 and 3 hr after the administration was measured. The hypoglycemic effect of the test drug was evaluated by detecting a significant difference every hour between the solvent administration group and the test drug administration group (Dunnett's test).

The test results of the above-mentioned Experimental Example are shown in Table 39.

**Table 39**

| Ex. No. | Minimum effective dose db/db mouse (mg/kg, p.o.) | Ex. No. | Minimum effective dose db/db mouse (mg/kg, p.o.) |
|---|---|---|---|
| 1-2 | 10 | 4-16 | 10 |
| 1-81 | 10 | 4-19 | 10 |
| 1-88 | 10 | 6 | 3 |
| 1-134 | 10 | 6-15 | 3 |
| 2-17 | 10 | 6-16 | 3 |
| 2-23 | 3 | 6-17 | 3 |
| 2-24 | 3 | 6-18 | 3 |
| 3 | 10 | 6-19 | 3 |
| 4 | 10 | 6-20 | 3 |
| 4-6 | 10 | 6-21 | 10 |

### Industrial Applicability

As is clear from the above-mentioned tests, the novel compound and a pharmaceutically acceptable salt thereof of the present invention strongly suppressed human liver glycogen phosphorylase. Because of the presence of such action mechanism, the compound (1) of the present invention is useful as a therapeutic agent for diabetes.

This application is based on a patent application No. 331501/2001 filed in Japan, the contents of which are hereby incorporated by reference.

## Claims

1. An indole compound represented by the formula (1) 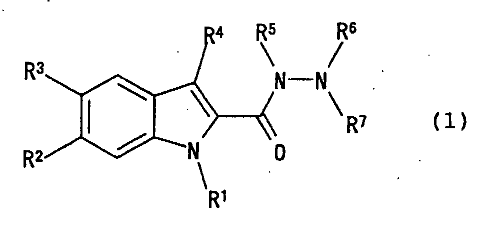 wherein
R¹ is a hydrogen atom, a C₁₋₆ alkyl group or an acyl group;
R² is a hydrogen atom or a halogen atom;
R³ is a halogen atom, a C₁₋₆ alkyl group, a C₁₋₆ alkoxy group, a nitro group, an amino group, a hydroxyl group, a cyano group, an acyl group, an aralkyloxy group or a thiazolyl group
wherein the thiazolyl group is optionally substituted by a C₁₋₆ alkyl group or an amino group;
R⁴ is a hydrogen atom or a C₁₋₆ alkyl group;
R⁵ is a hydrogen atom, a C₁₋₆ alkyl group or a C₂₋₇₋ alkoxycarbonyl group;
R⁶ is a hydrogen atom, a C₁₋₆ alkyl group or an aralkyl group
wherein the aralkyl group is optionally substituted by a halogen atom;
R⁷ is 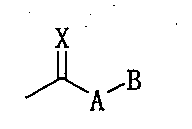
wherein X is =O, =S or =NH;
A is -N(R⁸)- wherein R⁸ is a hydrogen atom, a C₁₋₆ alkyl group or an aryl group optionally having substituents, -C(R⁹)(R¹⁰)- wherein R⁹ and R¹⁰ are the same or different and each is independently a hydrogen atom, a hydroxyl group, an amino group, a C₁₋₆ alkyl group, a C₁₋₆ hydroxyalkyl group, a C₂₋₇ alkoxycarbonylamino group or an acylamino group, or R⁹ and R¹⁰ may form a C₃₋₇ cycloalkyl group together with the adjacent carbon atom, -(CH₂)ₘ-NH- wherein m is an integer of 1 to 4, -CO-, -S- or a single bond; and
B is 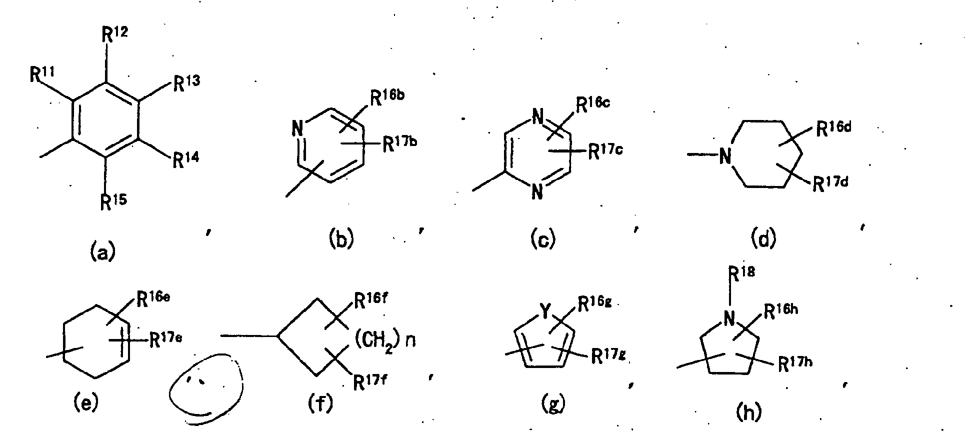 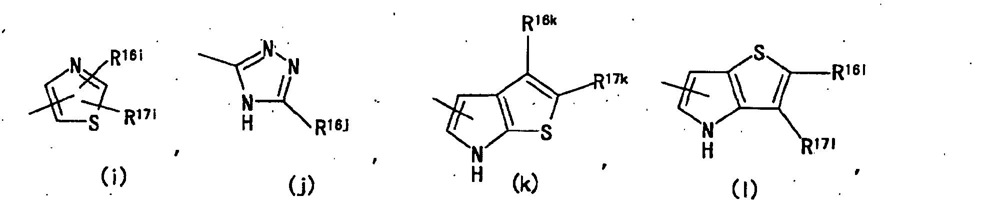 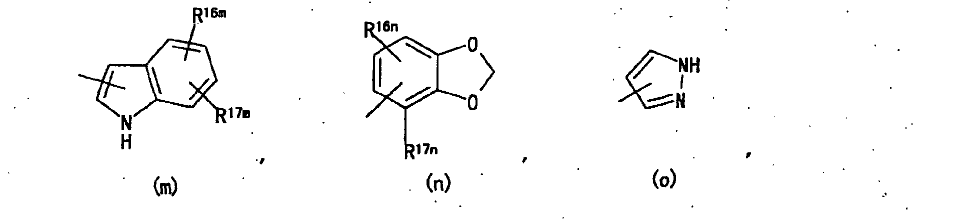 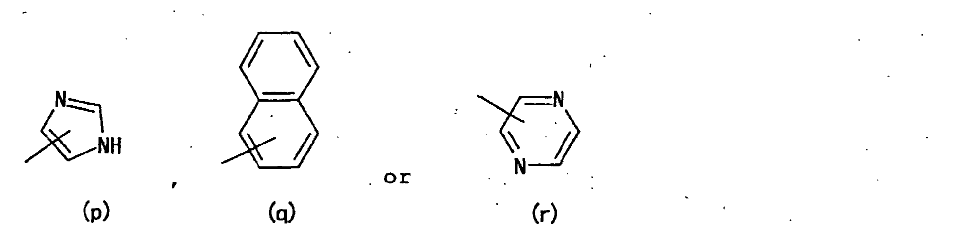 wherein R¹¹, R¹², R¹³, R¹⁴ and R¹⁵ are the same or different and each is independently a hydrogen atom, a halogen atom, a C₁₋₆ alkyl group, a C₁₋₆ alkoxy group, a nitro group, a hydroxyl group, a cyano group, a haloalkyl group, an aralkyl group, an aryl group optionally having substituents, an aryloxy group, a tetrazolyl group, a triazolyl group, -(CH₂)ₚ-CO-R¹⁹ wherein p is 0 or an integer of 1 to 4 and R¹⁹ is an aryl group optionally having substituents, a hydroxyl group, a C₁₋₆ alkoxy group or -N(R²⁰)(R²¹) wherein R²⁰ and R²¹ are the same or different and each is independently a hydrogen atom, a C₁₋₆ alkyl group, an aralkyl group or a C₃₋₁₃ alkoxycarbonylalkyl group, or R²⁰ and R²¹ may form, together with the adjacent nitrogen atom, 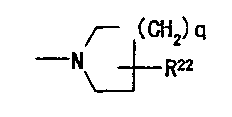 wherein q is an integer of 1 to 3 and R²² is a hydrogen atom, a hydroxyl group, a C₁₋₆ alkoxy group, an amino group, a C₂₋₁₂ dialkylamino group or a C₂₋₇ alkoxycarbonylamino group, -O-(CH₂)ᵣ-R²³ wherein r is an integer of 1 to 4 and R²³ is a hydroxyl group, an amino group, a C₂₋₇ alkylcarbonyloxy group or -CO-R²⁴ wherein R²⁴ is a hydroxyl group, a C₁₋₆ alkoxy group or -N(R²⁵)(R²⁶) wherein R²⁵ and R²⁶ are the same or different and each is a hydrogen atom, a C₁₋₆ alkyl group or an aralkyl group, or R²⁵ and R²⁶ may form, together with the adjacent nitrogen atom, 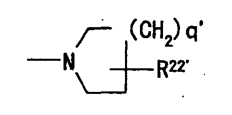 or 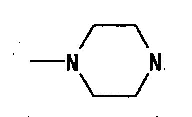 wherein q' and R^{22'} are as defined for q and R²², respectively, -O-CO-R²⁷ wherein R²⁷ is a C₁₋₆ alkylamino group or a C₂₋₁₂ dialkylamino group, or -N(R²⁸)(R²⁹) wherein R²⁸ and R²⁹ are the same or different and each is a hydrogen atom, a C₁₋₆ alkyl group, an aryl group optionally having substituents, an acyl group, -(CH₂)_{p'}-COO-R³⁰ wherein p' is as defined for p and R³⁰ is a hydrogen atom, an aryl group optionally having substituents or a C₁₋₆ alkyl group wherein the C₁₋₆ alkyl group is optionally substituted by a hydroxyl group, a trifluoromethyl group, an aryl group optionally having substituents, a morpholino group or a carboxyl group, -CON(R³¹)(R³²) wherein R³¹ and R³² are the same or different and each is a hydrogen atom, a C₁₋₆ alkyl group or an aryl group optionally having substituents, -CO-R³³ wherein R³³ is a C₁₋₆ alkyl group or an aryl group optionally having substituents or -CO-(CH₂)_{r'}-R³⁴ wherein r' is as defined for r and R³⁴ is a C₁₋₆ alkylamino group, a C₂₋₁₂ dialkylamino group, a C₁₋₆ alkoxy group or a C₂₋₇ alkylcarbonyloxy group,
R^{16b}-R¹⁶ⁿ and R^{17b}-R¹⁷ⁿ are the same or different and each is a hydrogen atom, a halogen atom, a C₁₋₆ alkyl group, an amino group, a hydroxyl group, a C₁₋₆ alkoxy group or -CON(R^{31'})(R^{32'}) wherein R^{31'} and R^{32'} are as defined for R³¹ and R³²,
R¹⁸ is a hydrogen atom or a C₂₋₇ alkoxycarbonyl group,
Y is -S-, -O- or -N (R³⁵)- wherein R³⁵ is a hydrogen atom or a C₁₋₆ alkyl group, and
n is 0 or an integer of 1 to 4, or
R⁶ and R⁷ may form, together with the adjacent nitrogen atom, 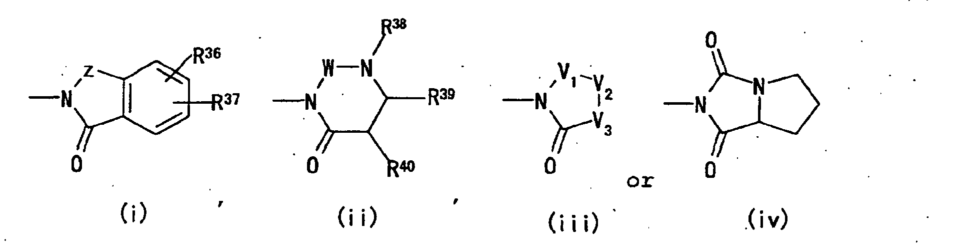 wherein R³⁶ and R³⁷ are the same or different and each is a hydrogen atom, a halogen atom, a C₁₋₆ alkyl group, a C₁₋₆ alkoxy group, an amino group, a nitro group, a hydroxyl group, a C₂₋₇ alkoxycarbonyl group, a carboxyl group, a C₂₋₇ haloalkylcarbonylamino group or -O-CO-R⁴¹ wherein R⁴¹ is a C₁₋₆ alkyl group, a C₁₋₆ alkylamino group or a C₂₋₁₂ dialkylamino group;
Z is -CH₂-CH₂-, -C(R⁴²)=CH-, -C(R^{42'})=N-, -N=N-, -CO-, -CO-O-, -CO-CH₂-O-, -CH₂-CO-NH-, -C(R^{42''})(R⁴³)-N(R⁴⁴)- wherein R⁴², R^{42'}, R^{42''} and R⁴³ are the same or different and each is a hydrogen atom, a C₁₋₆ alkyl group or an aryl group optionally having substituents and R⁴⁴ is a hydrogen atom, a C₂₋₇ alkoxycarbonyl group or a C₁₋₆ alkyl group wherein the C₁₋₆ alkyl group is optionally substituted by a carboxyl group or a C₂₋₇ alkoxycarbonyl group, or -C(U)-N(R^{44'})- wherein U is =O or =S and R^{44'} is as defined for R⁴⁴ wherein an atom adjacent to the nitrogen atom on the fused ring in the formula (i) is described on the left end of each group;
R³⁸ is a hydrogen atom, an aryl group optionally having substituents or a heteroaryl group;
R³⁹ and R⁴⁰ are the same or different and each is a hydrogen atom, a C₁₋₆ alkyl group, a C₁₋₆ alkoxy group or a C₂₋₇ alkoxycarbonyl group, or R³⁹ and R⁴⁰ may form, together with the adjacent carbon atom, 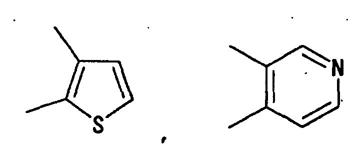 or 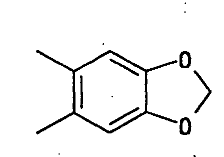
W is -CO-, -CS- or -CH₂-;
V₁ is -CO-, -CS- or -CH₂-;
V₂ is -O-, -CH₂- or -N(R⁴⁵)- wherein R⁴⁵ is a hydrogen atom, a C₁₋₆ alkyl group or an aryl group optionally having substituents; and
V₃ is -CH(R⁴⁶)- or -N(R^{46'})- wherein R⁴⁶ and R^{46'} are each a hydrogen atom, an aralkyl group, a heteroaryl group or an aryl group optionally having substituents,
a pharmaceutically acceptable salt thereof or a prodrug thereof.

2. The indole compound of claim 1,
wherein
R⁶ is a hydrogen atom, a C₁₋₆ alkyl group or an aralkyl group wherein the aralkyl group is optionally substituted by a halogen atom;
R⁷ is 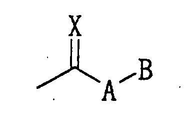 wherein X is =O, =S or =NH;
A is -N(R^{8'})- wherein R^{8'} is a hydrogen atom, a C₁₋₆ alkyl group or a phenyl group optionally having substituents, -C(R^{9'})(R^{10'})- wherein R^{9'} and R^{10'} are the same or different and each is a hydrogen atom, a hydroxyl group, an amino group, a C₁₋₆ alkyl group, a C₁₋₆ hydroxyalkyl group, a C₂₋₇ alkoxycarbonylamino group or an acylamino group, or R^{9'} and R^{10'} may form, together with the adjacent carbon atom, a C₃₋₇ cycloalkyl group, -(CH₂)ₘ-NH- wherein m is an integer of 1 to 4, -CO-, -S- or a single bond; and
B is 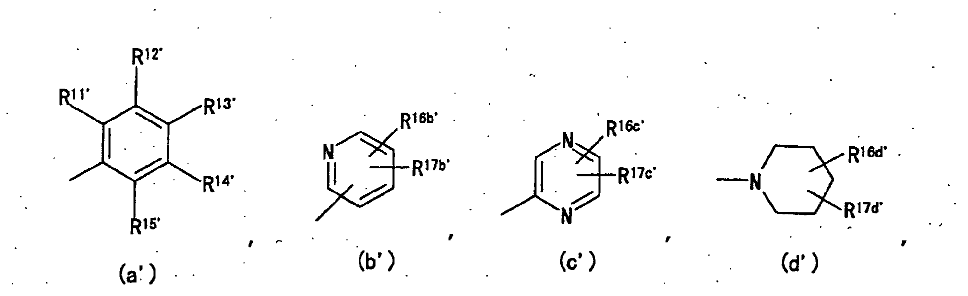 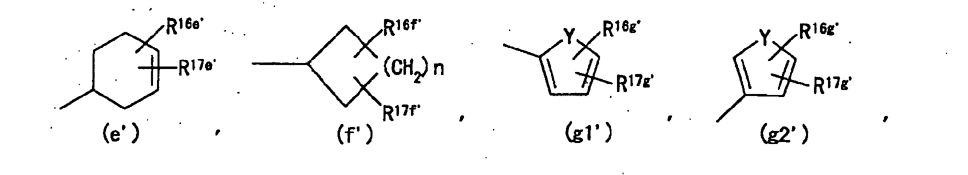 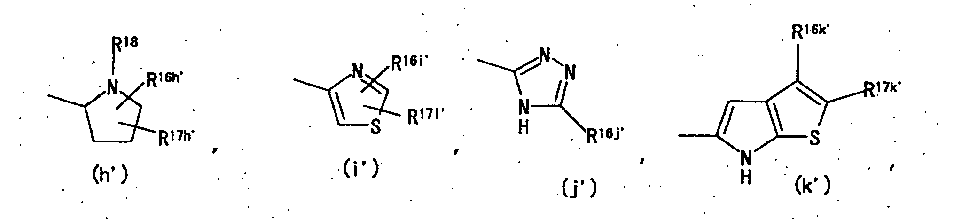 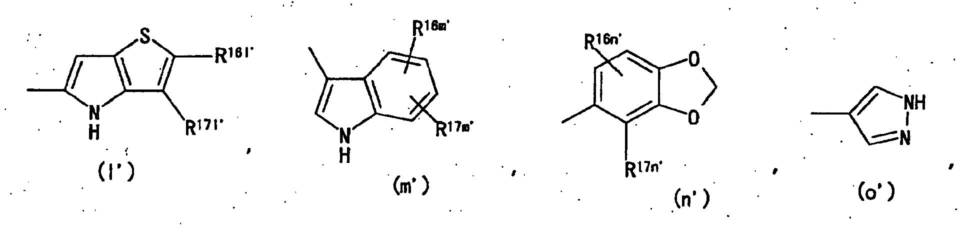 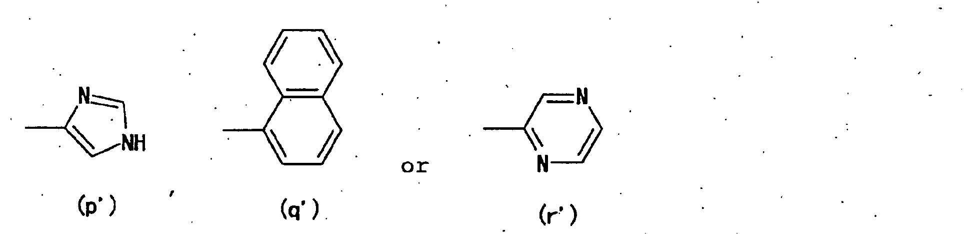 wherein R^{11'}, R^{12'}, R^{13'}, R^{14'} and R^{15'} are the same or different and each is a hydrogen atom, a halogen atom, a C₁₋₆ alkyl group, a C₁₋₆ alkoxy group, a nitro group, a hydroxyl group, a cyano group, a haloalkyl group, an aralkyl group, a phenyl group optionally having substituents, an aryloxy group, a tetrazolyl group, a triazolyl group, -(CH₂)ₚ-CO-R^{19'} wherein p is 0 or an integer of 1 to 4 and R^{19'} is a phenyl group optionally having substituents, a hydroxyl group, a C₁₋₆ alkoxy group or -N(R²⁰)(R²¹)- wherein R²⁰ and R²¹ are as defined in claim 1, -O-(CH₂)ᵣ-R²³ wherein r and R²³ are as defined in claim 1, -O-CO-R²⁷ wherein R²⁷ is as defined in claim 1, or -N(R^{28'})(R^{29'}) wherein R^{28'} and R^{29'} are the same or different and each is a hydrogen atom, a C₁₋₆ alkyl group, a phenyl group optionally having substituents, an acyl group, -(CH₂)_{p'}-COO-R^{30'} wherein p' is as defined in claim 1 and R^{30'} is a hydrogen atom, a phenyl group optionally having substituents or a C₁₋₆ alkyl group wherein the C₁₋₆ alkyl group is optionally substituted by a hydroxyl group, a trifluoromethyl group, a phenyl group optionally having substituents, a morpholino group or a carboxyl group, -CON(R^{31''})(R^{32''}) wherein R^{31''} and R^{32''} are the same or different and each is a hydrogen atom, a C₁₋₆ alkyl group or a phenyl group optionally having substituents, -CO-R^{33'} wherein R^{33'} is a C₁₋₆ alkyl group or a phenyl group optionally having substituents or -CO-(CH₂)_{r'}-R³⁴ wherein r' and R³⁴ are as defined in claim 1,
R^{16b'}-R^{16n'} and R^{17b'}-R^{17n'} are the same or different and each is a hydrogen atom, a halogen atom, a C₁₋₆ alkyl group, an amino group, a hydroxyl group, a C₁₋₆ alkoxy group or -CON(R^{31'''})(R^{32'''}) wherein R^{31'''} and R^{32'''} are as defined for R^{31''} and R^{32''}, and
R¹⁸, Y and n are as defined in claim 1, or
R⁶ and R⁷ may form, together with the adjacent nitrogen atom, 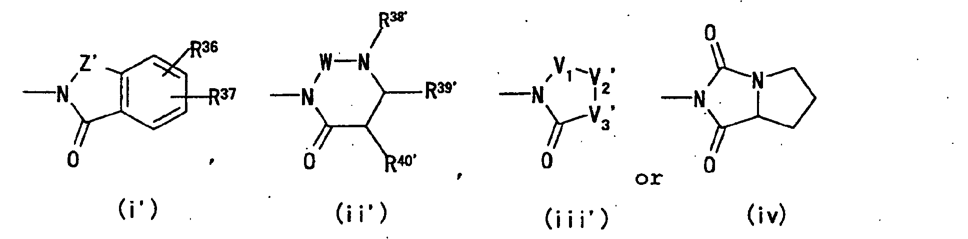 wherein R³⁶ and R³⁷ are as defined in claim 1;
Z' is -CH₂-CH₂-, -C(R⁴²)=CH-, -C(R^{42'})=N-, -N=N-, -CO-, -CO-O-, -CO-CH₂-O-, -CH₂-CO-NH-, -C(R^{42''})(R⁴³)-N(R⁴⁴)- wherein R⁴², R^{42'}, R^{42''} and R⁴³ are the same or different and each is a hydrogen atom, a C₁₋₆ alkyl group or a phenyl group optionally having substituents and R⁴⁴ are as defined in claim 1 or -C(U)-N(R^{44'})- wherein U and R^{44'} are as defined in claim 1;
R³⁸' is a hydrogen atom, a phenyl group optionally substituted by a halogen atom or a C₁₋₆ alkyl group, or a pyridyl group;
R^{39'} and R^{40'} are both hydrogen atoms, or R^{39'} and R^{40'} may form, together with the adjacent carbon atom, 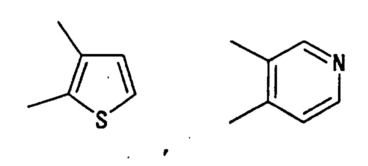 or 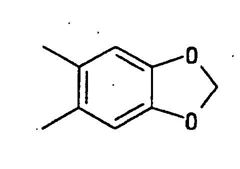
W and V₁ are as defined in claim 1;
V₂' is -O-, -CH₂- or -N(R⁴⁵)- wherein R⁴⁵ is a hydrogen atom, a C₁₋₆ alkyl group, a phenyl group optionally substituted by a halogen atom; and
V₃' is -CH(R⁴⁶)- or -N(R^{46'})- wherein R⁴⁶ and R^{46'} are each a hydrogen atom, a benzyl group, a thienyl group, or a phenyl group optionally substituted by a halogen atom, a hydroxy group or a C₁₋₆ alkoxy group,
a pharmaceutically acceptable salt thereof or a prodrug thereof.

3. The indole compound of claim 1,
wherein
R⁶ is a hydrogen atom, a C₁₋₆ alkyl group or an aralkyl group wherein the aralkyl group is optionally substituted by a halogen atom;
R⁷ is 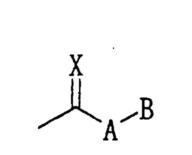 wherein X is as defined in claim 1;
A is -N(R^{8''})- wherein R^{8''} is a hydrogen atom, a C₁₋₆ alkyl group or an aryl group optionally having substituents, -C(R^{9''})(R^{10''})- wherein R^{9''} and R^{10''} are the same or different and each is a hydrogen atom, a hydroxyl group, an amino group, a C₁₋₆ alkyl group, a C₁₋₆ hydroxyalkyl group or a C₂₋₇ alkoxycarbonylamino group, or R^{9''} and R^{10''} may form, together with the adjacent carbon atom, a C₃₋₇ cycloalkyl group, -(CH₂)ₘ-NH- wherein m is as defined in claim 1, -CO- or a single bond; and
B is 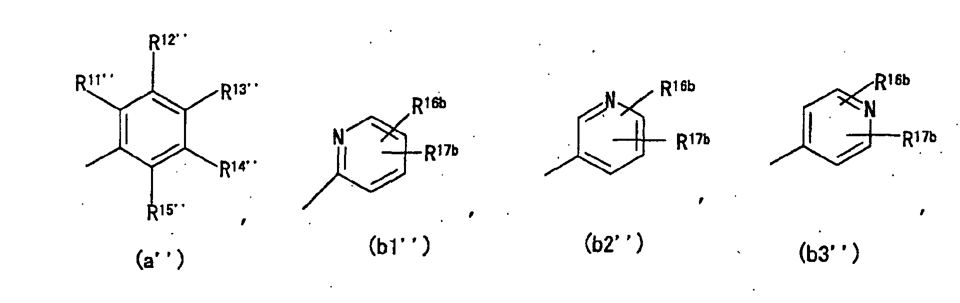 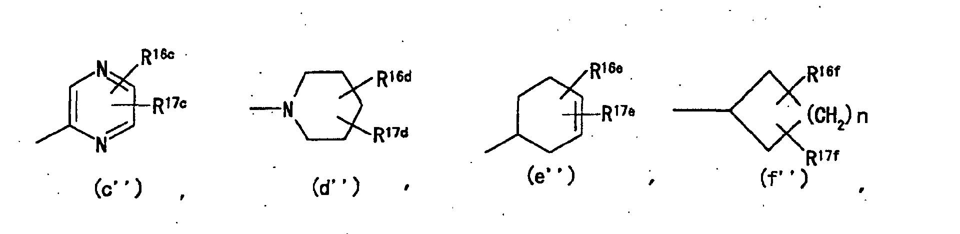 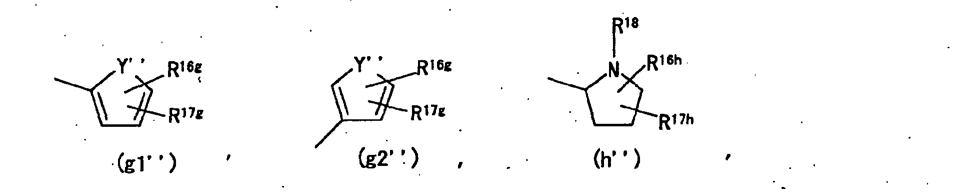 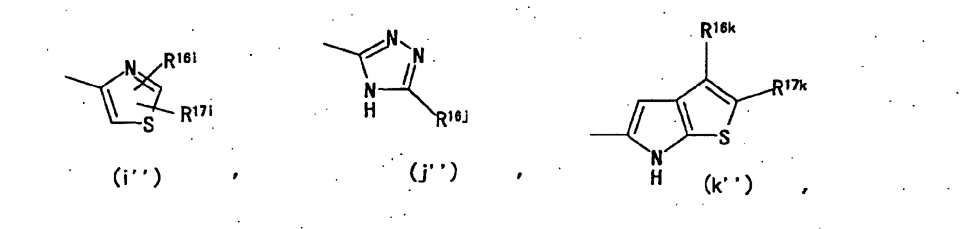 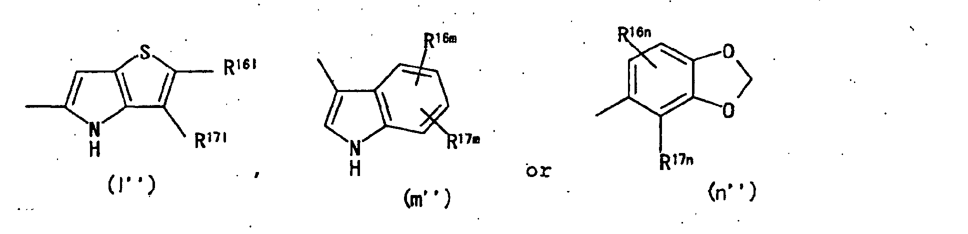 wherein R^{11''}, R^{12''}, R^{13''}, R^{14''} and R^{15''} are the same or different and each is a hydrogen atom, a halogen atom, a C₁₋₆ alkyl group, a C₁₋₆ alkoxy group, a nitro group, a hydroxyl group, a cyano group, a haloalkyl group, an aralkyl group, an aryl group optionally having substituents, an aryloxy group, a tetrazolyl group, a triazolyl group, -(CH₂)ₚ-CO-R¹⁹ wherein p and R¹⁹ are as defined in claim 1, -O-(CH₂)ᵣ-R²³ wherein r and R²³ are as defined in claim 1, -O-CO-R²⁷ wherein R²⁷ is as defined in claim 1 or -N(R^{28''})(R^{29''}) wherein R^{28''} and R^{29''} are the same or different and each is a hydrogen atom, a C₁₋₆ alkyl group, an aryl group optionally having substituents, -(CH₂)_{p'}-COO-R^{30''} wherein p' is as defined for p and R^{30''} is a hydrogen atom or a C₁₋₆ alkyl group wherein the C₁₋₆ alkyl group is optionally substituted by a hydroxyl group, a trifluoromethyl group or a carboxyl group, -CON(R³¹)(R³²) wherein R³¹ and R³² are as defined in claim 1, -CO-R³³ wherein R³³ is as defined in claim 1 or -CO-(CH₂)_{r'}-R³⁴ wherein r' and R³⁴ are as defined in claim 1,
R^{16b}-R¹⁶ⁿ and R^{17b}-R¹⁷ⁿ are as defined in claim 1,
R¹⁸ is as defined in claim 1,
Y'' is -S- or -N(R³⁵)- wherein R³⁵ is as defined in claim 1, and
n is as defined in claim 1, or
R⁶ and R⁷ may form, together with the adjacent nitrogen atom, 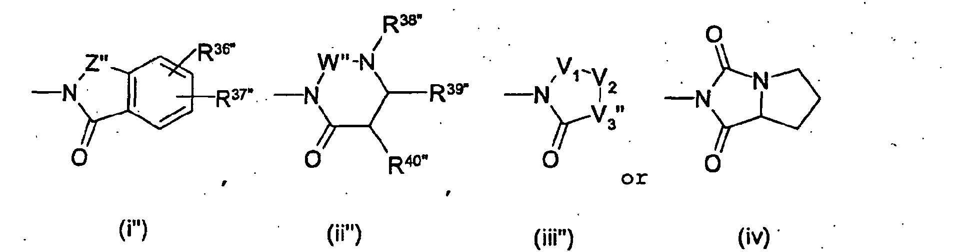 wherein R^{36''} and R^{37''} are the same or different and each is a hydrogen atom, a halogen atom, a C₁₋₆ alkyl group, a C₁₋₆ alkoxy group, an amino group, a hydroxyl group or -O-CO-R⁴¹ wherein R⁴¹ are as defined in claim 1;
Z'' is, -CH₂-CH₂-, -C(R⁴²)=CH-, -N=N- , -CO- , -CO-O- , -CO-CH₂-O-, -CH₂-CO-NH-, -C(R^{42''})(R⁴³)-N(R^{44''})- wherein R⁴², R^{42''} and R⁴³ are as defined in claim 1 and R^{44''} is a hydrogen atom, a C₁₋₆ alkyl group or a C₂₋₇ alkoxycarbonyl group or -C(U)-N(R^{44'''})- wherein U is =O or =S and R^{44'''} is as defined for R^{44''};
R^{38''} is a hydrogen atom or an aryl group optionally having substituents;
R^{39''} and R^{40''} are the same or different and each is a hydrogen atom, a C₁₋₆ alkyl group or a C₂₋₇ alkoxycarbonyl group, or R^{39''} and R^{40"} may form, together with the adjacent carbon atom, 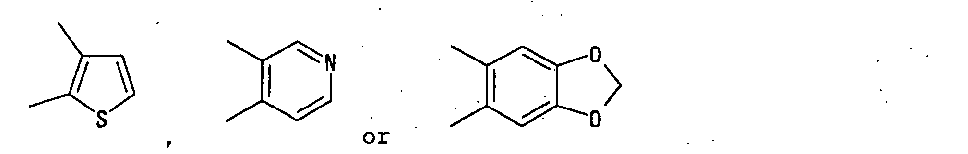
W'' is -CO- or -CH₂-;
V₁ and V₂ are as defined in claim 1; and
V₃'' is -CH(R^{46''})- or -N(R^{46'''})- wherein R^{46''} and R^{46'''} are the same or different and each is a hydrogen atom or an aryl group optionally having substituents,
a pharmaceutically acceptable salt thereof or a prodrug thereof.

4. The indole compound of claim 1, wherein R¹, R², R⁴, R⁵ and R⁶ are each a hydrogen atom, a pharmaceutically acceptable salt thereof or a prodrug thereof.

5. The indole compound of claim 4, wherein R³ is a halogen atom or a C₁₋₆ alkyl group, a pharmaceutically acceptable salt thereof or a prodrug thereof.

6. The indole compound of claim 4, wherein X=O, A is a single bond and B is 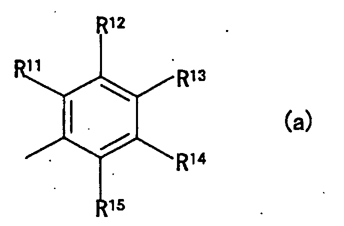 a pharmaceutically acceptable salt thereof or a prodrug thereof.

7. The indole_compound of claim 4, wherein X=NH, A is a single bond and B is 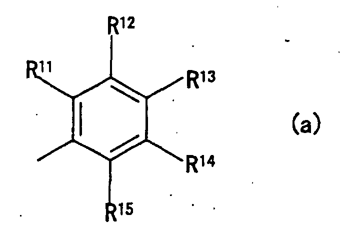 a pharmaceutically acceptable salt thereof or a prodrug thereof.

8. The indole compound of claim 1, wherein R⁶ and R⁷ may form, together with the adjacent nitrogen atom, 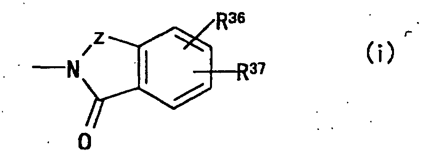 a pharmaceutically acceptable salt thereof or a prodrug thereof.

9. The indole compound of claim 1, which is selected from the group consisting of
benzoic acid 2-(5-chloro-1H-indole-2-carbonyl)hydrazide,
2-aminobenzoic acid 2-(5-chloro-1H-indole-2-carbonyl)hydrazide,
2-hydroxybenzoic acid 2-(5-chloro-1H-indole-2-carbonyl)hydrazide,
3-(2-(2-(5-chloro-1H-indole-2-carbonyl)hydrazinocarbonyl)phenylcarbamoyloxy)-2,2-dimethylpropionic acid,
benzoic acid 2-(5-chloro-1H-indole-2-carbonyl)-1-methylhydrazide,
benzoic acid 2-(1-acetyl-5-chloro-1H-indole-2-carbonyl)hydrazide,
5-chloro-1H-indole-2-carboxylic acid 2-(imino-phenyl-methyl)hydrazide,
5-aminothiazole-4-carboxylic acid 2-(5-chloro-1H-indole-2-carbonyl)hydrazide,
benzoic acid 2-(5-fluoro-1H-indole-2-carbonyl)hydrazide,
cyclohexanecarboxylic acid 2-(5-fluoro-1H-indole-2-carbonyl)hydrazide,
thiophene-2-carboxylic acid 2-(5-fluoro-1H-indole-2-carbonyl)hydrazide,
4-nitrobenzoic acid 2-(5-chloro-1H-indole-2-carbonyl)hydrazide,
2-methylbenzoic acid 2-(5-chloro-1H-indole-2-carbonyl)hydrazide,
4-methylbenzoic acid 2-(5-chloro-1H-indole-2-carbonyl)hydrazide,
2-methoxybenzoic acid 2-(5-chloro-1H-indole-2-carbonyl)hydrazide,
3-methoxybenzoic acid 2-(5-chloro-1H-indole-2-carbonyl)hydrazide,
4-methoxybenzoic acid 2-(5-chloro-1H-indole-2-carbonyl)hydrazide,
3-methylbenzoic acid 2-(5-chloro-1H-indole-2-carbonyl)hydrazide,
2-chlorobenzoic acid 2-(5-chloro-1H-indole-2-carbonyl)hydrazide,
3-chlorobenzoic acid 2-(5-chloro-1H-indole-2-carbonyl)hydrazide,
4-chlorobenzoic acid 2-(5-chloro-1H-indole-2-carbonyl)hydrazide,
4-(2-(5-chloro-1H-indole-2-carbonyl)hydrazinocarbonyl)benzoic acid methyl ester,
cyclohexanecarboxylic acid 2-(5-chloro-1H-indole-2-carbonyl)hydrazide,
2,4-dichlorobenzoic acid 2-(5-chloro-1H-indole-2-carbonyl)hydrazide,
2,6-dichlorobenzoic acid 2-(5-chloro-1H-indole-2-carbonyl)hydrazide,
2,4-difluorobenzoic acid 2-(5-chloro-1H-indole-2-carbonyl)hydrazide,
biphenyl-2-carboxylic acid 2-(5-chloro-1H-indole-2-carbonyl)hydrazide,
3-fluorobenzoic acid 2-(5-chloro-1H-indole-2-carbonyl)hydrazide,
4-fluorobenzoic acid 2-(5-chloro-1H-indole-2-carbonyl)hydrazide,
3-trifluoromethylbenzoic acid 2-(5-chloro-1H-indole-2-carbonyl)hydrazide,
4-trifluoromethylbenzoic acid 2-(5-chloro-1H-indole-2-carbonyl)hydrazide,
2-trifluoromethylbenzoic acid 2-(5-chloro-1H-indole-2-carbonyl)hydrazide,
benzoic acid 2-(5-methyl-1H-indole-2-carbonyl)hydrazide,
benzoic acid 2-(5-chloro-3-methyl-1H-indole-2-carbonyl)hydrazide,
benzoic acid 2-(5,7-dichloro-1H-indole-2-carbonyl)hydrazide,
2-aminobenzoic acid 2-(5-isopropyl-1H-indole-2-carbonyl)hydrazide,
2-amino-4-fluorobenzoic acid 2-(5-isopropyl-1H-indole-2-carbonyl)hydrazide,
2-aminobenzoic acid 2-(5-fluoro-1H-indole-2-carbonyl)hydrazide,
2-aminobenzoic acid 2-(6-chloro-1H-indole-2-carbonyl)hydrazide,
3-amino-4-(2-(5-chloro-1H-indole-2-carbonyl)hydrazinocarbonyl)benzoic acid methyl ester,
3-aminoisonicotinic acid 2-(5-chloro-1H-indole-2-carbonyl)hydrazide,
isonicotinic acid 2-(5-chloro-1H-indole-2-carbonyl)hydrazide,
nicotinic acid 2-(5-chloro-1H-indole-2-carbonyl)hydrazide,
pyridine-2-carboxylic acid 2-(5-chloro-1H-indole-2-carbonyl)hydrazide,
3-(dimethylamino)benzoic acid 2-(5-chloro-1H-indole-2-carbonyl)hydrazide,
N-(3-(2-(5-chloro-1H-indole-2-carbonyl)hydrazinocarbonyl)phenyl)acetamide,
N-(2-(2-(5-chloro-1H-indole-2-carbonyl)hydrazinocarbonyl)phenyl)acetamide,
4-fluorobenzoic acid 2-(5-chloro-1H-indole-2-carbonyl)-2-methylhydrazide,
2-(2-(2-(5-chloro-1H-indole-2-carbonyl)hydrazinocarbonyl)phenoxy)acetic acid methyl ester,
2-(2-(2-(5-chloro-1H-indole-2-carbonyl)hydrazinocarbonyl)phenoxy)acetic acid,
2-(dimethylamino)benzoic acid 2-(5-chloro-1H-indole-2-carbonyl)hydrazide,
2-(2-(5-chloro-1H-indole-2-carbonyl)hydrazinocarbonyl)phenoxy-N,N-dimethylacetamide,
2-methylaminobenzoic acid 2-(5-chloro-1H-indole-2-carbonyl)hydrazide,
2-amino-4-chlorobenzoic acid 2-(5-chloro-1H-indole-2-carbonyl)hydrazide,
2-amino-6-chlorobenzoic acid 2-(5-chloro-1H-indole-2-carbonyl)hydrazide,
2-amino-3-chlorobenzoic acid 2-(5-chloro-1H-indole-2-carbonyl)hydrazide,
2-amino-5-chlorobenzoic acid 2-(5-chloro-1H-indole-2-carbonyl)hydrazide,
4-cyanobenzoic acid 2-(5-chloro-1H-indole-2-carbonyl)hydrazide,
4-(1H-tetrazol-5-yl)benzoic acid 2-(5-chloro-1H-indole-2-carbonyl)hydrazide,
3-(1H-tetrazol-5-yl)benzoic acid 2-(5-chloro-1H-indole-2-carbonyl)hydrazide,
2-(2-(2-(5-chloro-1H-indole-2-carbonyl)hydrazinocarbonyl)anilino)acetic acid,
2-aminobenzoic acid 2-(5-methyl-1H-indole-2-carbonyl)hydrazide,
2-amino-4-fluorobenzoic acid 2-(5-methyl-1H-indole-2-carbonyl)hydrazide,
methyl (2-(2-(5-methyl-1H-indole-2-carbonyl)hydrazinocarbonyl)phenyl)carbamate,
2-(2-(5-chloro-1H-indole-2-carbonyl)hydrazinocarbonyl)phenyl dimethylcarbamate,
2-aminobenzoic acid 2-(5-ethyl-1H-indole-2-carbonyl)hydrazide,
2-amino-4,5-difluorobenzoic acid 2-(5-methyl-1H-indole-2-carbonyl)hydrazide,
2-(2-hydroxyethoxy)benzoic acid 2-(5-chloro-1H-indole-2-carbonyl)hydrazide,
2-(3-(2-(5-chloro-1H-indole-2-carbonyl)hydrazinocarbonyl)phenoxy)acetic acid methyl ester,
2-(3-(2-(5-chloro-1H-indole-2-carbonyl)hydrazinocarbonyl)phenoxy)acetic acid,
2-(3-(2-(5-chloro-1H-indole-2-carbonyl)hydrazinocarbonyl)phenoxy)-N,N-dimethylacetamide,
2-methylthiazole-4-carboxylic acid 2-(5-chloro-1H-indole-2-carbonyl)hydrazide,
4-(2H-[1,2,4]triazol-3-yl)benzoic acid 2-(5-chloro-1H-indole-2-carbonyl)hydrazide,
3-(2H-[1,2,4]triazol-3-yl)benzoic acid 2-(5-chloro-1H-indole-2-carbonyl)hydrazide,
1,3-dihydroxy-2-propyl (2-(2-(5-chloro-1H-indole-2-carbonyl)hydrazinocarbonyl)phenyl)carbamate,
3-((2-(2-(5-chloro-1H-indole-2-carbonyl)hydrazinocarbonyl)phenyl)carbamoyloxy)-2,2-dimethylpropionic acid,
thiophene-2-carboxylic acid 2-(5-chloro-1H-indole-2-carbonyl)hydrazide,
furan-2-carboxylic acid 2-(5-chloro-1H-indole-2-carbonyl)hydrazide,
2,6-dichloronicotinic acid 2-(5-chloro-1H-indole-2-carbonyl)hydrazide,
1H-pyrrole-2-carboxylic acid 2-(5-chloro-1H-indole-2-carbonyl)hydrazide,
1H-imidazole-4-carboxylic acid 2-(5-chloro-1H-indole-2-carbonyl)hydrazide,
pyrazine-2-carboxylic acid 2-(5-chloro-1H-indole-2-carbonyl)hydrazide,
thiophene-3-carboxylic acid 2-(5-chloro-1H-indole-2-carbonyl)hydrazide,
furan-3-carboxylic acid 2-(5-chloro-1H-indole-2-carbonyl)hydrazide,
5-chlorothiophene-2-carboxylic acid 2-(5-chloro-1H-indole-2-carbonyl)hydrazide,
3-chlorothiophene-2-carboxylic acid 2-(5-chloro-1H-indole-2-carbonyl)hydrazide,
1-methyl-1H-pyrrole-2-carboxylic acid 2-(5-chloro-1H-indole-2-carbonyl)hydrazide,
5-methylthiophene-2-carboxylic acid 2-(5-chloro-1H-indole-2-carbonyl)hydrazide,
3-methylthiophene-2-carboxylic acid 2-(5-chloro-1H-indole-2-carbonyl)hydrazide,
2,6-difluorobenzoic acid 2-(5-chloro-1H-indole-2-carbonyl)hydrazide,
2,3-difluorobenzoic acid 2-(5-chloro-1H-indole-2-carbonyl)hydrazide,
5-chloro-1H-indole-2-carboxylic acid 2-(naphthalene-1-carbonyl)hydrazide,
3,4,5-trifluorobenzoic acid 2-(5-chloro-1H-indole-2-carbonyl)hydrazide,
2,3,4,5-tetrafluorobenzoic acid 2-(5-chloro-1H-indole-2-carbonyl)hydrazide,
2-amino-4-methylbenzoic acid 2-(5-chloro-1H-indole-2-carbonyl)hydrazide,
2-amino-4-fluorobenzoic acid 2-(5-chloro-1H-indole-2-carbonyl)hydrazide,
2-amino-5-methylbenzoic acid 2-(5-chloro-1H-indole-2-carbonyl)hydrazide,
2-amino-6-methylbenzoic acid 2-(5-chloro-1H-indole-2-carbonyl)hydrazide,
2-amino-3-methylbenzoic acid 2-(5-chloro-1H-indole-2-carbonyl)hydrazide,
2-amino-4,5-difluorobenzoic acid 2-(5-chloro-1H-indole-2-carbonyl)hydrazide,
3-aminothiophene-2-carboxylic acid 2-(5-chloro-1H-indole-2-carbonyl)hydrazide,
2-aminobenzoic acid 2-(5-bromo-1H-indole-2-carbonyl)hydrazide,
2-amino-4-fluorobenzoic acid 2-(5-bromo-1H-indole-2-carbonyl)hydrazide,
1H-pyrazole-4-carboxylic acid 2-(5-chloro-1H-indole-2-carbonyl)hydrazide,
methyl (2-(2-(5-fluoro-1H-indole-2-carbonyl)hydrazinocarbonyl)phenyl)carbamate,
1-methyl-1H-pyrrole-2-carboxylic acid 2-(5-methyl-1H-indole-2-carbonyl)hydrazide,
thiophene-3-carboxylic acid 2-(5-methyl-1H-indole-2-carbonyl)hydrazide,
4H-thieno[3,2-b]pyrrole-5-carboxylic acid 2-(5-chloro-1H-indole-2-carbonyl)hydrazide,
phenyl (2-(2-(5-chloro-1H-indole-2-carbonyl)hydrazinocarbonyl)phenyl)carbamate,
benzyl (2-(2-(5-chloro-1H-indole-2-carbonyl)hydrazinocarbonyl)phenyl)carbamate,
2-hydroxyethyl (2-(2-(5-chloro-1H-indole-2-carbonyl)hydrazinocarbonyl)phenyl)carbamate,
3-hydroxypropyl (2-(2-(5-chloro-1H-indole-2-carbonyl)hydrazinocarbonyl)phenyl)carbamate,
2-((2-(2-(5-chloro-1H-indole-2-carbonyl)hydrazinocarbonyl)phenyl)carbamoyloxy)acetic acid,
2-((2-(2-(5-chloro-1H-indole-2-carbonyl)hydrazinocarbonyl)phenyl)carbamoyloxymethyl)-2-methylmalonic acid,
methyl 2-(2-(5-chloro-1H-indole-2-carbonyl)hydrazinocarbonyl)phenylcarbamate,
cyclohexanecarboxylic acid 2-(5-chloro-1H-indole-2-carbonyl)-1-methylhydrazide,
thiophene-2-carboxylic acid 2-(5-chloro-1H-indole-2-carbonyl)-1-methylhydrazide,
benzoic acid 2-(1H-indole-2-carbonyl)hydrazide,
benzoic acid 2-(5-chloro-1-methyl-1H-indole-2-carbonyl)hydrazide,
benzoic acid 2-(5-methoxy-1H-indole-2-carbonyl)hydrazide,
benzoic acid 2-(5-isopropyl-1H-indole-2-carbonyl)hydrazide,
benzoic acid 2-(5-nitro-1H-indole-2-carbonyl)hydrazide,
benzoic acid 2-(5-benzyloxy-1H-indole-2-carbonyl)hydrazide,
benzoic acid 2-(6-chloro-1H-indole-2-carbonyl)hydrazide,
6H-thieno[2,3-b]pyrrole-5-carboxylic acid 2-(5-chloro-1H-indole-2-carbonyl)hydrazide,
5-chloro-1H-indole-2-carboxylic acid 2-((2-fluorophenyl)-imino-methyl)hydrazide,
5-chloro-1H-indole-2-carboxylic acid 2-((3-fluorophenyl)-imino-methyl)hydrazide,
5-chloro-1H-indole-2-carboxylic acid 2-((4-fluorophenyl)-imino-methyl)hydrazide,
5-chloro-1H-indole-2-carboxylic acid 2-(imino-(p-tolyl)-methyl)hydrazide,
5-chloro-1H-indole-2-carboxylic acid 2-((4-chlorophenyl)-imino-methyl)hydrazide,
5-chloro-1H-indole-2-carboxylic acid 2-((3-chlorophenyl)-imino-methyl) hydrazide,
5-chloro-1H-indole-2-carboxylic acid 2-((3-fluorophenyl)-imino-methyl)hydrazide,
5-chloro-1H-indole-2-carboxylic acid 2-(imino-(o-tolyl)-methyl)hydrazide,
5-chloro-1H-indole-2-carboxylic acid 2-(imino-(m-tolyl)-methyl)hydrazide,
5-chloro-1H-indole-2-carboxylic acid 2-(imino-(thiophen-2-yl)-methyl)hydrazide,
5-chloro-1H-indole-2-carboxylic acid 2-(imino-(pyridin-2-yl)-methyl)hydrazide,
5-chloro-1H-indole-2-carboxylic acid 2-((furan-2-yl)-imino-methyl)hydrazide,
5-chloro-1H-indole-2-carboxylic acid 2-((2-chloro-6-fluorophenyl)-imino-methyl)hydrazide,
5-chloro-1H-indole-2-carboxylic acid 2-(imino-(2-trifluoromethylphenyl)-methyl)hydrazide,
5-chloro-1H-indole-2-carboxylic acid 2-(imino-(pyrazin-2-yl)-methyl)hydrazide,
3-aminobenzoic acid 2-(5-chloro-1H-indole-2-carbonyl)hydrazide,
3-methoxybenzoic acid 2-(5-chloro-1H-indole-2-carbonyl)hydrazide,
5-amino-2-methylthiazole-4-carboxylic acid 2-(5-chloro-1H-indole-2-carbonyl)hydrazide,
5-chloro-1H-indole-2-carboxylic acid (1,2,3,4-tetrahydro-2,4-dioxoquinazolin-3-yl)amide,
5-chloro-1H-indole-2-carboxylic acid (2,3-dihydro-2,4-dioxo-4H-benzo[e][1,3]oxazin-3-yl)amide,
5-chloro-1H-indole-2-carboxylic acid (1,2,3,4-tetrahydro-4-oxo-2-thioxoquinazolin-3-yl)amide,
5-chloro-1H-indole-2-carboxylic acid (3,4-dihydro-2-methyl-4-oxoquinazolin-3-yl)amide,
5-chloro-1H-indole-2-carboxylic acid (3,4-dihydro-4-oxoquinazolin-3-yl)amide,
5-chloro-1H-indole-2-carboxylic acid (1,2,3,4-tetrahydro-4-oxoquinazolin-3-yl)amide,
5-chloro-1H-indole-2-carboxylic acid (1,2,3,4-tetrahydro-2,5-dioxo-5H-benzo[e][1,4]diazepin-4-yl)amide,
5-chloro-1H-indole-2-carboxylic acid (2,3,4,5-tetrahydro-3,5-dioxo-benzo[f][1,4]oxazepin-4-yl)amide,
5-isopropyl-1H-indole-2-carboxylic acid (1,2,3,4-tetrahydro-2,4-dioxoquinazolin-3-yl)amide,
5-isopropyl-1H-indole-2-carboxylic acid (7-fluoro-1,2,3,4-tetrahydro-2,4-dioxoquinazolin-3-yl)amide,
5-fluoro-1H-indole-2-carboxylic acid (1,2,3,4-tetrahydro-2,4-dioxoquinazolin-3-yl)amide,
6-chloro-1H-indole-2-carboxylic acid (1,2,3,4-tetrahydro-2,4-dioxoquinazolin-3-yl)amide,
3-((5-chloro-1H-indole-2-carbonyl)amino)-1,2,3,4-tetrahydro-2,4-dioxoquinazoline-7-carboxylic acid methyl ester,
3-((5-chloro-1H-indole-2-carbonyl)amino)-1,2,3,4-tetrahydro-2,4-dioxoquinazoline-7-carboxylic acid,
5-chloro-1H-indole-2-carboxylic acid (1,2,3,4-tetrahydro-2,4-dioxo-6-(trifluoroacetylamino)quinazolin-3-yl)amide,
5-chloro-1H-indole-2-carboxylic acid (6-amino-1,2,3,4-tetrahydro-2,4-dioxoquinazolin-3-yl)amide,
5-chloro-1H-indole-2-carboxylic acid (5-chloro-1,2,3,4-tetrahydro-2,4-dioxoquinazolin-3-yl)amide,
5-chloro-1H-indole-2-carboxylic acid (6-chloro-1,2,3,4-tetrahydro-2,4-dioxoquinazolin-3-yl)amide,
5-chloro-1H-indole-2-carboxylic acid (7-chloro-1,2,3,4-tetrahydro-2,4-dioxoquinazolin-3-yl)amide,
5-chloro-1H-indole-2-carboxylic acid (8-chloro-1,2,3,4-tetrahydro-2,4-dioxoquinazolin-3-yl)amide,
2-(3-((5-chloro-1H-indole-2-carbonyl)amino)-1,2,3,4-tetrahydro-2,4-dioxoquinazolin-1-yl)acetic acid,
2-(3-((5-chloro-1H-indole-2-carbonyl)amino)-1,2,3,4-tetrahydro-2,4-dioxoquinazolin-1-yl)acetic acid methyl ester,
5-methyl-1H-indole-2-carboxylic acid (1,2,3,4-tetrahydro-2,4-dioxoquinazolin-3-yl)amide,
5-methyl-1H-indole-2-carboxylic acid (7-fluoro-1,2,3,4-tetrahydro-2,4-dioxoquinazolin-3-yl)amide,
5-ethyl-1H-indole-2-carboxylic acid (1,2,3,4-tetrahydro-2,4-dioxoquinazolin-3-yl)amide,
5-methyl-1H-indole-2-carboxylic acid (6,7-difluoro-1,2,3,4-tetrahydro-2,4-dioxoquinazolin-3-yl)amide,
5-methyl-1H-indole-2-carboxylic acid (1,2,3,4-tetrahydro-6-methoxy-2,4-dioxoquinazolin-3-yl)amide,
5-methyl-1H-indole-2-carboxylic acid (1,2,3,4-tetrahydro-6-hydroxy-2,4-dioxoquinazolin-3-yl)amide,
acetic acid 3-((5-methyl-1H-indole-2-carbonyl)amino)-1,2,3,4-tetrahydro-2,4-dioxoquinazolin-6-yl ester,
5-chloro-1H-indole-2-carboxylic acid (1,2,3,4-tetrahydro-2,4-dioxo-1-propylquinazolin-3-yl)amide,
5-chloro-1H-indole-2-carboxylic acid (1,2,3,4-tetrahydro-1-methyl-2,4-dioxoquinazolin-3-yl)amide,
N-(1,2,3,4-tetrahydro-7-nitro-2,4-dioxoquinazolin-3-yl)-5-chloro-1H-indole-2-carboxylic acid amide,
5-chloro-1H-indole-2-carboxylic acid (2,4-dioxoperhydropyrimidin-3-yl)amide,
5-chloro-1H-indole-2-carboxylic acid (4-oxo-2-thioxoperhydropyrimidin-3-yl)amide,
5-chloro-1H-indole-2-carboxylic acid (2,4-dioxo-1-phenylperhydropyrimidin-3-yl)amide,
5-chloro-1H-indole-2-carboxylic acid (4-oxo-1-phenylperhydropyrimidin-3-yl) amide,
5-chloro-1H-indole-2-carboxylic acid (1-(4-fluorophenyl)-2,4-dioxoperhydropyrimidin-3-yl)amide,
5-chloro-1H-indole-2-carboxylic acid (2,4-dioxo-1-(pyridin-2-yl)perhydropyrimidin-3-yl)amide,
5-chloro-1H-indole-2-carboxylic acid (1-(3-fluorophenyl)-2,4-dioxoperhydropyrimidin-3-yl)amide,
5-chloro-1H-indole-2-carboxylic acid (1-(2-fluorophenyl)-2,4-dioxoperhydropyrimidin-3-yl)amide,
5-fluoro-1H-indole-2-carboxylic acid (2,4-dioxo-1-phenyl-perhydropyrimidin-3-yl)amide,
5-methyl-1H-indole-2-carboxylic acid (2,4-dioxo-1-phenyl-perhydropyrimidin-3-yl) amide,
5-chloro-1H-indole-2-carboxylic acid (1-(3-chlorophenyl)-2,4-dioxoperhydropyrimidin-3-yl)amide,
5-chloro-1H-indole-2-carboxylic acid (2,4-dioxo-1-(m-tolyl)perhydropyrimidin-3-yl)amide,
5-chloro-1H-indole-2-carboxylic acid (2,4-dioxo-1-(p-tolyl)perhydropyrimidin-3-yl)amide,
5-chloro-1H-indole-2-carboxylic acid (1-(4-chlorophenyl)-2,4-dioxoperhydropyrimidin-3-yl)amide,
5-chloro-1H-indole-2-carboxylic acid (2,4-dioxo-1-(o-tolyl)perhydropyrimidin-3-yl)amide,
5-chloro-1H-indole-2-carboxylic acid ((4S)-2,5-dioxo-4-phenylimidazolidin-1-yl)amide,
5-chloro-1H-indole-2-carboxylic acid (2,4-dioxo-1-phenylimidazolidin-3-yl)amide,
5-chloro-1H-indole-2-carboxylic acid (4-oxo-1-phenyl-2-thioxoimidazolidin-3-yl)amide,
5-chloro-1H-indole-2-carboxylic acid (4-oxo-1-phenylimidazolidin-3-yl)amide,
5-chloro-1H-indole-2-carboxylic acid (2-oxo-1-phenylimidazolidin-3-yl)amide,
5-chloro-1H-indole-2-carboxylic acid ((4R)-2,5-dioxo-4-phenylimidazolidin-1-yl)amide,
5-chloro-1H-indole-2-carboxylic acid ((4S)-1,3-dioxo-perhydropyrrolo[1,2-c]imidazol-2-yl)amide,
5-chloro-1H-indole-2-carboxylic acid ((4R)-1,3-dioxo-perhydropyrrolo[1,2-c]imidazol-2-yl)amide,
5-chloro-1H-indole-2-carboxylic acid ((4S)-4-benzyl-2,5-dioxoimidazolidin-1-yl)amide,
5-chloro-1H-indole-2-carboxylic acid ((4R)-4-benzyl-2,5-dioxoimidazolidin-1-yl)amide,
5-chloro-1H-indole-2-carboxylic acid (2,4-dioxoimidazolidin-3-yl)amide,
5-chloro-1H-indole-2-carboxylic acid (1-methyl-2,5-dioxo-4-phenylimidazolidin-1-yl)amide,
5-chloro-1H-indole-2-carboxylic acid (2,4-dioxo-1-(4-fluorophenyl)imidazolidin-3-yl)amide,
5-chloro-1H-indole-2-carboxylic acid (2,5-dioxo-4-(2-fluorophenyl)imidazolidin-1-yl)amide,
5-chloro-1H-indole-2-carboxylic acid (2,5-dioxo-4-(2-thienyl)imidazolidin-1-yl)amide,
5-chloro-1H-indole-2-carboxylic acid (2,5-dioxo-4-(4-fluorophenyl)imidazolidin-1-yl)amide,
5-chloro-1H-indole-2-carboxylic acid (2,5-dioxo-4-(4-chlorophenyl)imidazolidin-1-yl)amide,
5-chloro-1H-indole-2-carboxylic acid ((4S)-2,5-dioxo-4-(4-hydroxyphenyl)imidazolidin-1-yl)amide,
5-chloro-1H-indole-2-carboxylic acid ((4S)-2,5-dioxo-4-(4-methoxyphenyl)imidazolidin-1-yl)amide,
5-chloro-1H-indole-2-carboxylic acid ((4R)-2,5-dioxo-4-(4-methoxyphenyl)imidazolidin-1-yl)amide,
5-chloro-1H-indole-2-carboxylic acid 2-(anilinocarbonyl)hydrazide,
5-chloro-1H-indole-2-carboxylic acid 2-(phenylthiocarbonyl)hydrazide,
5-chloro-1H-indole-2-carboxylic acid 2-(2-phenylacetyl)hydrazide,
5-chloro-1H-indole-2-carboxylic acid 2-(2-oxo-2-phenylacetyl)hydrazide,
5-chloro-1H-indole-2-carboxylic acid 2-((2-fluorophenyl)aminocarbonyl)hydrazide,
5-chloro-1H-indole-2-carboxylic acid 2-((3-fluorophenyl)aminocarbonyl)hydrazide,
5-chloro-1H-indole-2-carboxylic acid 2-((4-fluorophenyl)aminocarbonyl)hydrazide,
5-chloro-1H-indole-2-carboxylic acid 2-(anilinocarbonyl)-2-methylhydrazide,
5-chloro-1H-indole-2-carboxylic acid 2-((2-chloroanilino)carbonyl)hydrazide,
5-chloro-1H-indble-2-carboxylic acid 2-((3-chloroanilino)carbonyl)hydrazide,
5-chloro-1H-indole-2-carboxylic acid 2-((4-chloroanilino)carbonyl)hydrazide,
5-chloro-1H-indole-2-carboxylic acid 2-((1-phenylcyclopropane)carbonyl)hydrazide,
5-chloro-1H-indole-2-carboxylic acid 2-((1-phenylcyclopentane)carbonyl)hydrazide,
5-chloro-1H-indole-2-carboxylic acid 2-((1-phenylcyclohexane)carbonyl)hydrazide,
5-chloro-1H-indole-2-carboxylic acid 2-(2-phenylpropanoyl)hydrazide,
5-chloro-1H-indole-2-carboxylic acid 2-(3-hydroxy-2-phenylpropanoyl)hydrazide,
5-chloro-1H-indole-2-carboxylic acid 2-(2-methyl-2-phenylpropanoyl)hydrazide,
5-chloro-1H-indole-2-carboxylic acid 2-((2S)-2-amino-2-phenylacetyl)hydrazide,
N-(2-(2-(5-chloro-1H-indole-2-carbonyl)hydrazino)-2-oxo-1-phenylethyl)acetamide,
2-morpholinoethyl (2-((2-(5-chloro-1H-indole-2-carbonyl)hydrazino)carbonyl)phenyl)carbamate p-toluenesulfonate,
2-amino-4,5-difluorobenzoic acid 2-(5-methyl-lH-indole-2-carbonyl)hydrazide benzenesulfonate,
2-amino-4-fluorobenzoic acid 2-(5-chloro-1H-indole-2-carbonyl)hydrazide benzenesulfonate,
3-(dimethylamino)benzoic acid 2-(5-chloro-1H-indole-2-carbonyl)hydrazide methanesulfonate,
2-amino-4-fluorobenzoic acid 2-(5-chloro-1H-indole-2-carbonyl)hydrazide hydrochloride,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-aminobenzoic acid 2-(5-methyl-1H-indole-2-carbonyl)hydrazide p-toluenesulfonate,
2-aminobenzoic acid 2-(5-methyl-1H-indole-2-carbonyl)hydrazide benzenesulfonate,
2-amino-4-fluorobenzoic acid 2-(5-methyl-1H-indole-2-carbonyl)hydrazide benzenesulfonate,
3-aminobenzoic acid 2-(5-chloro-1H-indole-2-carbonyl)hydrazide p-toluenesulfonate,
5-chloro-1H-indole-2-carboxylic acid 2-((2-chlorophenyl)-imino-methyl)hydrazide methanesulfonate,
5-chloro-1H-indole-2-carboxylic acid 2-((2-chlorophenyl)-imino-methyl)hydrazide p-toluenesulfonate,
5-chloro-1H-indole-2-carboxylic acid 2-((2-chlorophenyl)-imino-methyl)hydrazide hydrochloride,
5-chloro-1H-indole-2-carboxylic acid 2-(imino-phenyl-methyl)hydrazide methanesulfonate,
5-chloro-1H-indole-2-carboxylic acid 2-((2-chlorophenyl)-imino-methyl)hydrazide butenedioic acid salt,
5-chloro-1H-indole-2-carboxylic acid 2-((2-fluorophenyl)-imino-methyl)hydrazide hydrochloride,
5-chloro-1H-indole-2-carboxylic acid 2-((2-fluorophenyl)-imino-methyl)hydrazide methanesulfonate,
5-chloro-1H-indole-2-carboxylic acid 2-((1-imino-2-phenylethyl)hydrazide methanesulfonate,
5-chloro-1H-indole-2-carboxylic acid 2-((3-fluorophenyl)-imino-methyl)hydrazide hydrochloride,
5-chloro-1H-indole-2-carboxylic acid 2-((3,4-difluorophenyl)-imino-methyl)hydrazide methanesulfonate,
5-chloro-1H-indole-2-carboxylic acid 2-(imino-(2-methoxyphenyl)-methyl)hydrazide methanesulfonate,
5-chloro-1H-indole-2-carboxylic acid 2-((2,6-difluorophenyl)-imino-methyl)hydrazide methanesulfonate,
5-chloro-1H-indole-2-carboxylic acid 2-((2,4-difluorophenyl)-imino-methyl)hydrazide methanesulfonate,
5-chloro-1H-indole-2-carboxylic acid 2-((1,2-dimethyl-1H-pyrrol-5-yl)-imino-methyl)hydrazide methanesulfonate,
2-aminobenzoic acid 2-(5-methyl-1H-indole-2-carbonyl)hydrazide p-toluenesulfonate,
2-aminobenzoic acid 2-(5-methyl-1H-indole-2-carbonyl)hydrazide benzenesulfonate,
2-amino-4-fluorobenzoic acid 2-(5-methyl-1H-indole-2-carbonyl)hydrazide benzenesulfonate,
2-(dimethylamino)benzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide methanesulfonate,
2-aminobenzoic acid 2-(5-bromo-lH-indole-2-carbonyl)hydrazide methanesulfonate,
2-amino-4-fluorobenzoic acid 2-(5-methyl-1H-indole-2-carbonyl)hydrazide p-toluenesulfonate, and
2-amino-4-fluorobenzoic acid 2-(5-methyl-1H-indole-2-carbonyl)hydrazide methanesulfonate,
a pharmaceutically acceptable salt thereof or a prodrug thereof.

10. The indole compound of claim 1, which is selected from the group consisting of
benzoic acid 2-(5-chloro-1H-indole-2-carbonyl)hydrazide,
2-aminobenzoic acid 2-(5-chloro-1H-indole-2-carbohyl)hydrazide,
2-hydroxybenzoic acid 2-(5-chloro-1H-indole-2-carbonyl)hydrazide,
3-(2-(2-(5-chloro-1H-indole-2-carbonyl)hydrazinocarbonyl)phenylcarbamoyloxy)-2,2-dimethylpropionic acid,
benzoic acid 2-(5-chloro-1H-indole-2-carbonyl)-1-methylhydrazide,
benzoic acid 2-(1-acetyl-5-chloro-1H-indole-2-carbonyl)hydrazide,
5-chloro-1H-indole-2-carboxylic acid 2-(imino-phenyl-methyl)hydrazide,
5-aminothiazole-4-carboxylic acid 2-(5-chloro-1H-indole-2-carbonyl)hydrazide,
benzoic acid 2-(5-fluoro-1H-indole-2-carbonyl)hydrazide,
cyclohexanecarboxylic acid 2-(5-fluoro-1H-indole-2-carbonyl)hydrazide,
thiophene-2-carboxylic acid 2-(5-fluoro-1H-indole-2-carbonyl)hydrazide,
4-nitrobenzoic acid 2-(5-chloro-1H-indole-2-carbonyl)hydrazide,
2-methylbenzoic acid 2-(5-chloro-1H-indole-2-carbonyl)hydrazide,
4-methylbenzoic acid 2-(5-chloro-1H-indole-2-carbonyl)hydrazide,
2-methoxybenzoic acid 2-(5-chloro-1H-indole-2-carbonyl)hydrazide,
3-methoxybenzoic acid 2-(5-chloro-1H-indole-2-carbonyl)hydrazide,
4-methoxybenzoic acid 2-(5-chloro-1H-indole-2-carbonyl)hydrazide,
3-methylbenzoic acid 2-(5-chloro-1H-indole-2-carbonyl)hydrazide,
2-chlorobenzoic acid 2-(5-chloro-1H-indole-2-carbonyl)hydrazide,
3-chlorobenzoic acid 2-(5-chloro-1H-indole-2-carbonyl)hydrazide,
4-chlorobenzoic acid 2-(5-chloro-1H-indole-2-carbonyl)hydrazide,
4-(2-(5-chloro-1H-indole-2-carbonyl)hydrazinocarbonyl)benzoic acid methyl ester,
cyclohexanecarboxylic acid 2-(5-chloro-1H-indole-2-carbonyl)hydrazide,
2,4-dichlorobenzoic acid 2-(5-chloro-1H-indole-2-carbonyl)hydrazide,
2,6-dichlorobenzoic acid 2-(5-chloro-1H-indole-2-carbonyl)hydrazide,
2,4-difluorobenzoic acid 2-(5-chloro-1H-indole-2-carbonyl)hydrazide,
biphenyl-2-carboxylic acid 2-(5-chloro-1H-indole-2-carbonyl)hydrazide,
3-fluorobenzoic acid 2-(5-chloro-1H-indole-2-carbonyl)hydrazide,
4-fluorobenzoic acid 2-(5-chloro-1H-indole-2-carbonyl)hydrazide,
3-trifluoromethylbenzoic acid 2-(5-chloro-1H-indole-2-carbonyl)hydrazide,
4-trifluoromethylbenzoic acid 2-(5-chloro-1H-indole-2-carbonyl)hydrazide,
2-trifluoromethylbenzoic acid 2-(5-chloro-1H-indole-2-carbonyl)hydrazide,
benzoic acid 2-(5-methyl-1H-indole-2-carbonyl)hydrazide,
benzoic acid 2-(5-chloro-3-methyl-1H-indole-2-carbonyl)hydrazide,
benzoic acid 2-(5,7-dichloro-1H-indole-2-carbonyl)hydrazide,
2-aminobenzoic acid 2-(5-isopropyl-1H-indole-2-carbonyl)hydrazide,
2-amino-4-fluorobenzoic acid 2-(5-isopropyl-1H-indole-2-carbonyl)hydrazide,
2-aminobenzoic acid 2-(5-fluoro-1H-indole-2-carbonyl)hydrazide,
2-aminobenzoic acid 2-(6-chloro-1H-indole-2-carbanyl)hydrazide,
3-amino-4-(2-(5-chloro-1H-indole-2-carbonyl)hydrazinocarbonyl)benzoic acid methyl ester,
3-aminoisonicotinic acid 2-(5-chloro-1H-indole-2-carbonyl)hydrazide,
isonicotinic acid 2-(5-chloro-1H-indole-2-carbonyl)hydrazide,
nicotinic acid 2-(5-chloro-1H-indole-2-carbonyl)hydrazide,
pyridine-2-carboxylic acid 2-(5-chloro-1H-indole-2-carbonyl)hydrazide,
3-(dimethylamino)benzoic acid 2-(5-chloro-1H-indole-2-carbonyl)hydrazide,
N-(3-(2-(5-chloro-1H-indole-2-carbonyl)hydrazinocarbonyl)phenyl)acetamide,
N-(2-(2-(5-chloro-1H-indole-2-carbonyl)hydrazinocarbonyl)phenyl)acetamide,
4-fluorobenzoic acid 2-(5-chloro-1H-indole-2-carbonyl)-2-methylhydrazide,
2-(2-(2-(5-chloro-1H-indole-2-carbonyl)hydrazinocarbonyl)phenoxy)acetic acid methyl ester,
2-(2-(2-(5-chloro-1H-indole-2-carbonyl)hydrazinocarbonyl)phenoxy)acetic acid,
2-(dimethylamino)benzoic acid 2-(5-chloro-1H-indole-2-carbonyl)hydrazide,
2-(2-(5-chloro-1H-indole-2-carbonyl)hydrazinocarbonyl)phenoxy-N,N-dimethylacetamide,
2-methylaminobenzoic acid 2-(5-chloro-1H-indole-2-carbonyl)hydrazide,
2-amino-4-chlorobenzoic acid 2-(5-chloro-1H-indole-2-carbonyl)hydrazide,
2-amino-6-chlorobenzoic acid 2-(5-chloro-1H-indole-2-carbonyl)hydrazide,
2-amino-3-chlorobenzoic acid 2-(5-chloro-1H-indole-2-carbonyl)hydrazide,
2-amino-5-chlorobenzoic acid 2-(5-chloro-1H-indole-2-carbonyl)hydrazide,
4-cyanobenzoic acid 2-(5-chloro-1H-indole-2-carbonyl)hydrazide,
4-(1H-tetrazol-5-yl)benzoic acid 2-(5-chloro-1H-indole-2-carbonyl)hydrazide,
3-(1H-tetrazol-5-yl)benzoic acid 2-(5-chloro-1H-indole-2-carbonyl)hydrazide,
2-(2-(2-(5-chloro-1H-indole-2-carbonyl)hydrazinocarbonyl)anilino)acetic acid,
2-aminobenzoic acid 2-(5-methyl-1H-indole-2-carbonyl)hydrazide,
2-amino-4-fluorobenzoic acid 2-(5-methyl-1H-indole-2-carbonyl)hydrazide,
methyl (2-(2-(5-methyl-1H-indole-2-carbonyl)hydrazinocarbonyl)phenyl)carbamate,
2-(2-(5-chloro-1H-indole-2-carbonyl)hydrazinocarbonyl)phenyl dimethylcarbamate,
2-aminobenzoic acid 2-(5-ethyl-1H-indole-2-carbonyl)hydrazide,
2-amino-4,5-difluorobenzoic acid 2-(5-methyl-1H-indole-2-carbonyl)hydrazide,
2-(2-hydroxyethoxy)benzoic acid 2-(5-chloro-1H-indole-2-carbonyl)hydrazide,
2-(3-(2-(5-chloro-1H-indole-2-carbonyl)hydrazinocarbonyl)phenoxy)acetic acid methyl ester,
2-(3-(2-(5-chloro-1H-indole-2-carbonyl)hydrazinocarbonyl)phenoxy)acetic acid,
2-(3-(2-(5-chloro-1H-indole-2-carbonyl)hydrazinocarbonyl)phenoxy)-N,N-dimethylacetamide,
2-methylthiazole-4-carboxylic acid 2-(5-chloro-1H-indole-2-carbonyl)hydrazide,
4-(2H-[1,2,4]triazol-3-yl)benzoic acid 2-(5-chloro-1H-indole-2-carbonyl)hydrazide,
3-(2H-[1,2,4]triazol-3-yl)benzoic acid 2-(5-chloro-1H-indole-2-carbonyl)hydrazide,
1,3-dihydroxy-2-propyl (2-(2-(5-chloro-1H-indole-2-carbonyl)hydrazinocarbonyl)phenyl)carbamate,
3-((2-(2-(5-chloro-1H-indole-2-carbonyl)hydrazinocarbonyl)phenyl)carbamoyloxy)-2,2-dimethylpropionic acid,
thiophene-2-carboxylic acid 2-(5-chloro-1H-indole-2-carbonyl)hydrazide,
furan-2-carboxylic acid 2-(5-chloro-1H-indole-2-carbonyl)hydrazide,
2,6-dichloronicotinic acid 2-(5-chloro-1H-indole-2-carbonyl)hydrazide,
1H-pyrrole-2-carboxylic acid 2-(5-chloro-1H-indole-2-carbonyl)hydrazide,
1H-imidazole-4-carboxylic acid 2-(5-chloro-1H-indole-2-carbonyl)hydrazide,
pyrazine-2-carboxylic acid 2-(5-chloro-1H-indole-2-carbonyl)hydrazide,
thiophene-3-carboxylic acid 2-(5-chloro-1H-indole-2-carbonyl)hydrazide,
furan-3-carboxylic acid 2-(5-chloro-1H-indole-2-carbonyl)hydrazide,
5-chlorothiophene-2-carboxylic acid 2-(5-chloro-1H-indole-2-carbonyl)hydrazide,
3-chlorothiophene-2-carboxylic acid 2-(5-chloro-1H-indole-2-carbonyl)hydrazide,
1-methyl-1H-pyrrole-2-carboxylic acid 2-(5-chloro-1H-indole-2-carbonyl)hydrazide,
5-methylthiophene-2-carboxylic acid 2-(5-chloro-1H-indole-2-carbonyl)hydrazide,
3-methylthiophene-2-carboxylic acid 2-(5-chloro-1H-indole-2-carbonyl)hydrazide,
2,6-difluorobenzoic acid 2-(5-chloro-1H-indole-2-carbonyl)hydrazide,
2,3-difluorobenzoic acid 2-(5-chloro-1H-indole-2-carbonyl)hydrazide,
5-chloro-1H-indole-2-carboxylic acid 2-(naphthalene-1-carbonyl)hydrazide,
3,4,5-trifluorobenzoic acid 2-(5-chloro-1H-indole-2-carbonyl)hydrazide,
2,3,4,5-tetrafluorobenzoic acid 2-(5-chloro-1H-indole-2-carbonyl)hydrazide,
2-amino-4-methylbenzoic acid 2-(5-chloro-1H-indole-2-carbonyl)hydrazide,
2-amino-4-fluorobenzoic acid 2-(5-chloro-1H-indole-2-carbonyl)hydrazide,
2-amino-5-methylbenzoic acid 2-(5-chloro-1H-indole-2-carbonyl)hydrazide,
2-amino-6-methylbenzoic acid 2-(5-chloro-1H-indole-2-carbonyl)hydrazide,
2-amino-3-methylbenzoic acid 2-(5-chloro-1H-indole-2-carbonyl)hydrazide,
2-amino-4,5-difluorobenzoic acid 2-(5-chloro-1H-indole-2-carbonyl)hydrazide,
3-aminothiophene-2-carboxylic acid 2-(5-chloro-1H-indole-2-carbonyl)hydrazide,
2-aminobenzoic acid 2-(5-bromo-1H-indole-2-carbonyl)hydrazide,
2-amino-4-fluorobenzoic acid 2-(5-bromo-1H-indole-2-carbonyl)hydrazide,
1H-pyrazole-4-carboxylic acid 2-(5-chloro-1H-indole-2-carbonyl)hydrazide,
methyl (2-(2-(5-fluoro-1H-indole-2-carbonyl)hydrazinocarbonyl)phenyl)carbamate,
1-methyl-1H-pyrrole-2-carboxylic acid 2-(5-methyl-1H-indole-2-carbonyl)hydrazide,
thiophene-3-carboxylic acid 2-(5-methyl-1H-indole-2-carbonyl)hydrazide,
4H-thieno[3,2-b]pyrrole-5-carboxylic acid 2-(5-chloro-1H-indole-2-carbonyl)hydrazide,
phenyl (2-(2-(5-chloro-1H-indole-2-carbonyl)hydrazinocarbonyl)phenyl)carbamate,
benzyl (2-(2-(5-chloro-1H-indole-2-carbonyl)hydrazinocarbonyl)phenyl)carbamate,
2-hydroxyethyl (2-(2-(5-chloro-1H-indole-2-carbonyl)hydrazinocarbonyl)phenyl)carbamate,
3-hydroxypropyl (2-(2-(5-chloro-1H-indole-2-carbonyl)hydrazinocarbonyl)phenyl)carbamate,
2-((2-(2-(5-chloro-1H-indole-2-carbonyl)hydrazinocarbonyl)phenyl)carbamoyloxy)acetic acid,
2-((2-(2-(5-chloro-1H-indole-2-carbonyl)hydrazinocarbonyl)phenyl)carbamoyloxymethyl)-2-methylmalonic acid,
methyl 2-(2-(5-chloro-1H-indole-2-carbonyl)hydrazinocarbonyl)phenylcarbamate,
cyclohexanecarboxylic acid 2-(5-chloro-1H-indole-2-carbonyl)-1-methylhydrazide,
thiophene-2-carboxylic acid 2-(5-chloro-1H-indole-2-carbonyl)-1-methylhydrazide,
benzoic acid 2-(1H-indole-2-carbonyl)hydrazide,
benzoic acid 2-(5-chloro-1-methyl-1H-indole-2-carbonyl)hydrazide,
benzoic acid 2-(5-methoxy-1H-indole-2-carbonyl)hydrazide,
benzoic acid 2-(5-isopropyl-1H-indole-2-carbonyl)hydrazide,
benzoic acid 2-(5-nitro-1H-indole-2-carbonyl)hydrazide,
benzoic acid 2-(5-benzyloxy-1H-indole-2-carbonyl)hydrazide,
benzoic acid 2-(6-chloro-1H-indole-2-carbonyl)hydrazide,
6H-thieno[2,3-b]pyrrole-5-carboxylic acid 2-(5-chloro-1H-indole-2-carbonyl)hydrazide,
5-chloro-1H-indole-2-carboxylic acid 2-((2-fluorophenyl)-imino-methyl)hydrazide,
5-chloro-1H-indole-2-carboxylic acid 2-((3-fluorophenyl)-imino-methyl)hydrazide,
5-chloro-1H-indole-2-carboxylic acid 2-((4-fluorophenyl)-imino-methyl)hydrazide,
5-chloro-1H-indole-2-carboxylic acid 2-(imino-(p-tolyl)-methyl)hydrazide,
5-chloro-1H-indole-2-carboxylic acid 2-((4-chlorophenyl)-imino-methyl)hydrazide,
5-chloro-1H-indole-2-carboxylic acid 2-((3-chlorophenyl)-imino-methyl)hydrazide,
5-chloro-1H-indole-2-carboxylic acid 2-((2-chlorophenyl)-imino-methyl)hydrazide,
5-chloro-1H-indole-2-carboxylic acid 2-(imino-(o-tolyl)-methyl)hydrazide,
5-chloro-1H-indole-2-carboxylic acid 2-(imino-(m-tolyl)-methyl)hydrazide,
5-chloro-1H-indole-2-carboxylic acid 2-(imino-(thiophen-2-yl)-methyl)hydrazide,
5-chloro-1H-indole-2-carboxylic acid 2-(imino-(pyridin-2-yl)-methyl)hydrazide,
5-chloro-1H-indole-2-carboxylic acid 2-((furan-2-yl)-imino-methyl)hydrazide,
5-chloro-1H-indole-2-carboxylic acid 2-((2-chloro-6-fluorophenyl)-imino-methyl)hydrazide,
5-chloro-1H-indole-2-carboxylic acid 2-(imino-(2-trifluoromethylphenyl)-methyl)hydrazide,
5-chloro-1H-indole-2-carboxylic acid 2-(imino-(pyrazin-2-yl)-methyl)hydrazide,
3-aminobenzoic acid 2-(5-chloro-1H-indole-2-carbonyl)hydrazide,
3-methoxybenzoic acid 2-(5-chloro-1H-indole-2-carbonyl)hydrazide,
5-amino-2-methylthiazole-4-carboxylic acid 2-(5-chloro-1H-indole-2-carbonyl)hydrazide,
2-morpholinoethyl (2-((2-(5-chloro-1H-indole-2-carbonyl)hydrazino)carbonyl)phenyl)carbamate p-toluenesulfonate,
2-amino-4,5-difluorobenzoic acid 2-(5-methyl-1H-indole-2-carbonyl)hydrazide benzenesulfonate,
2-amino-4-fluorobenzoic acid 2-(5-chloro-1H-indole-2-carbonyl)hydrazide benzenesulfonate,
3-(dimethylamino)benzoic acid 2-(5-chloro-1H-indole-2-carbonyl)hydrazide methanesulfonate,
2-amino-4-fluorobenzoic acid 2-(5-chloro-1H-indole-2-carbonyl)hydrazide hydrochloride,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-aminobenzoic acid 2-(5-methyl-1H-indole-2-carbonyl)hydrazide p-toluenesulfonate,
2-aminobenzoic acid 2-(5-methyl-1H-indole-2-carbonyl)hydrazide benzenesulfonate,
2-amino-4-fluorobenzoic acid 2-(5-methyl-1H-indole-2-carbonyl)hydrazide benzenesulfonate,
3-aminobenzoic acid 2-(5-chloro-1H-indole-2-carbonyl)hydrazide p-toluenesulfonate,
5-chloro-1H-indole-2-carboxylic acid 2-((2-chlorophenyl)-imino-methyl)hydrazide methanesulfonate,
5-chloro-1H-indole-2-carboxylic acid 2-((2-chlorophenyl)-imino-methyl)hydrazide p-toluenesulfonate,
5-chloro-1H-indole-2-carboxylic acid 2-((2-chlorophenyl)-imino-methyl)hydrazide hydrochloride,
5-chloro-1H-indole-2-carboxylic acid 2-(imino-phenyl-methyl)hydrazide methanesulfonate,
5-chloro-1H-indole-2-carboxylic acid 2-((2-chlorophenyl)-imino-methyl)hydrazide butenedioic acid salt,
5-chloro-1H-indole-2-carboxylic acid 2-((2-fluorophenyl)-imino-methyl)hydrazide hydrochloride,
5-chloro-1H-indole-2-carboxylic acid 2-((2-fluorophenyl)-imino-methyl)hydrazide methanesulfonate,
5-chloro-1H-indole-2-carboxylic acid 2-((1-imino-2-phenylethyl)hydrazide methanesulfonate,
5-chloro-1H-indole-2-carboxylic acid 2-((3-fluorophenyl)-imino-methyl)hydrazide hydrochloride,
5-chloro-1H-indole-2-carboxylic acid 2-((3,4-difluorophenyl)-imino-methyl)hydrazide methanesulfonate,
5-chloro-1H-indole-2-carboxylic acid 2-(imino-(2-methoxyphenyl)-methyl)hydrazide methanesulfonate,
5-chloro-1H-indole-2-carboxylic acid 2-((2,6-difluorophenyl)-imino-methyl)hydrazide methanesulfonate,
5-chloro-1H-indole-2-carboxylic acid 2-((2,4-difluorophenyl)-imino-methyl)hydrazide methanesulfonate,
5-chloro-1H-indole-2-carboxylic acid 2-((1,2-dimethyl-1H-pyrrol-5-yl)-imino-methyl)hydrazide methanesulfonate,
2-aminobenzoic acid 2-(5-methyl-1H-indole-2-carbonyl)hydrazide p-toluenesulfonate,
2-aminobenzoic acid 2-(5-methyl-1H-indole-2-carbonyl)hydrazide benzenesulfonate,
2-amino-4-fluorobenzoic acid 2-(5-methyl-1H-indole-2-carbonyl)hydrazide benzenesulfonate,
2-(dimethylamino)benzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide methanesulfonate,
2-aminobenzoic acid 2-(5-bromo-1H-indole-2-carbonyl)hydrazide methanesulfonate,
2-amino-4-fluorobenzoic acid 2-(5-methyl-1H-indole-2-carbonyl)hydrazide p-toluenesulfonate, and
2-amino-4-fluorobenzoic acid 2-(5-methyl-1H-indole-2-carbonyl)hydrazide methanesulfonate
a pharmaceutically acceptable salt thereof or a prodrug thereof.

11. The indole compound of claim 1, which is selected from the group consisting of
2-morpholinoethyl (2-((2-(5-chloro-1H-indole-2-carbonyl)hydrazino)carbonyl)phenyl)carbamate p-toluenesulfonate,
2-amino-4,5-difluorobenzoic acid 2-(5-methyl-1H-indole-2-carbonyl)hydrazide benzenesulfonate,
2-amino-4-fluorobenzoic acid 2-(5-chloro-1H-indole-2-carbonyl)hydrazide benzenesulfonate,
3-(dimethylamino)benzoic acid 2-(5-chloro-1H-indole-2-carbonyl)hydrazide methanesulfonate,
2-amino-4-fluorobenzoic acid 2-(5-chloro-1H-indole-2-carbonyl)hydrazide hydrochloride,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide p-toluenesulfonate,
2-aminobenzoic acid 2-(5-methyl-1H-indole-2-carbonyl)hydrazide p-toluenesulfonate,
2-aminobenzoic acid 2-(5-methyl-1H-indole-2-carbonyl)hydrazide benzenesulfonate,
2-amino-4-fluorobenzoic acid 2-(5-methyl-1H-indole-2-carbonyl)hydrazide benzenesulfonate,
3-aminobenzoic acid 2-(5-chloro-1H-indole-2-carbonyl)hydrazide p-toluenesulfonate,
5-chloro-1H-indole-2-carboxylic acid 2-((2-chlorophenyl)-imino-methyl)hydrazide methanesulfonate,
5-chloro-1H-indole-2-carboxylic acid 2-((2-chlorophenyl)-imino-methyl)hydrazide p-toluenesulfonate,
5-chloro-1H-indole-2-carboxylic acid 2-((2-chlorophenyl)-imino-methyl)hydrazide hydrochloride,
5-chloro-1H-indole-2-carboxylic acid 2-(imino-phenyl-methyl)hydrazide methanesulfonate,
5-chloro-1H-indole-2-carboxylic acid 2-((2-chlorophenyl)-imino-methyl)hydrazide butenedioic acid salt,
5-chloro-1H-indole-2-carboxylic acid 2-((2-fluorophenyl)-imino-methyl)hydrazide hydrochloride,
5-chloro-1H-indole-2-carboxylic acid 2-((2-fluorophenyl)-imino-methyl)hydrazide methanesulfonate,
5-chloro-1H-indole-2-carboxylic acid 2-((1-imino-2-phenylethyl)hydrazide methanesulfonate,
5-chloro-1H-indole-2-carboxylic acid 2-((3-fluorophenyl)-imino-methyl)hydrazide hydrochloride,
5-chloro-1H-indole-2-carboxylic acid 2-((3,4-difluorophenyl)-imino-methyl)hydrazide methanesulfonate,
5-chloro-1H-indole-2-carboxylic acid 2-(imino-(2-methoxyphenyl)-methyl)hydrazide methanesulfonate,
5-chloro-1H-indole-2-carboxylic acid 2-((2,6-difluorophenyl)-imino-methyl)hydrazide methanesulfonate,
5-chloro-1H-indole-2-carboxylic acid 2-((2,4-difluorophenyl)-imino-methyl)hydrazide methanesulfonate,
5-chloro-1H-indole-2-carboxylic acid 2-((1,2-dimethyl-1H-pyrrol-5-yl)-imino-methyl)hydrazide methanesulfonate,
2-aminobenzoic acid 2-(5-methyl-1H-indole-2-carbonyl)hydrazide p-toluenesulfonate,
2-aminobenzoic acid 2-(5-methyl-1H-indole-2-carbonyl)hydrazide benzenesulfonate,
2-amino-4-fluorobenzoic acid 2-(5-methyl-1H-indole-2-carbonyl)hydrazide benzenesulfonate,
2-(dimethylamino)benzoic acid 2-(5-chloro-1H-indole-2-carbonyl)hydrazide p-toluenesulfonate,
2-amino-4-fluorobenzoic acid 2-(5-chloro-1H-indole-2-carbonyl)hydrazide methanesulfonate,
2-aminobenzoic acid 2-(5-bromo-1H-indole-2-carbonyl)hydrazide methanesulfonate,
2-amino-4-fluorobenzoic acid 2-(5-methyl-1H-indole-2-carbonyl)hydrazide p-toluenesulfonate, and
2-amino-4-fluorobenzoic acid 2-(5-methyl-1H-indole-2-carbonyl)hydrazide methanesulfonate
a pharmaceutically acceptable salt thereof or a prodrug thereof.

12. A pharmaceutical composition comprising an indole compound of any of claims 1 to 11, a pharmaceutically acceptable salt thereof or a prodrug thereof, and a pharmaceutically acceptable carrier.

13. An HLGPa inhibitor comprising an indole compound of any of claims 1 to 11, a pharmaceutically acceptable salt thereof or a prodrug thereof, and a pharmaceutically acceptable carrier.

14. A therapeutic agents for diabetes, which comprises an indole compound of any of claims 1 to 11, a pharmaceutically acceptable salt thereof or a prodrug thereof, and a pharmaceutically acceptable carrier.

15. The pharmaceutical composition of claim 14, which is used together with a therapeutic agent for hyperlipidemia.

16. The pharmaceutical composition of claim 15, wherein the therapeutic agent for hyperlipidemia is a statin pharmaceutical agent.

17. The pharmaceutical composition of claim 16, wherein the statin pharmaceutical agent is lovastatin, simvastatin, pravastatin, fluvastatin, atorvastatin or cerivastatin.

18. A pharmaceutical composition for the treatment or prophylaxis of diabetes, which comprises a therapeutic agent for diabetes selected from the group consisting of insulin preparations, sulfonylurea agents, insulin secretagogues, sulfonamides, biguanides, α-glucosidase inhibitors and insulin sensitizers, and an HLGPa inhibitor in combination.

19. The pharmaceutical composition of claim 18, wherein the therapeutic agent for diabetes is selected from the group consisting of insulin, glibenclamide, torbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.

20. The therapeutic agent for diabetes of claim 18 or 19, wherein the HLGPa inhibitor is an indole compound of any of claims 1 to 11, a pharmaceutically acceptable salt thereof or a prodrug thereof.

21. A method for treating or preventing diabetes, which comprises administering an indole compound of any of claims 1 to 11, a pharmaceutically acceptable salt thereof or a prodrug thereof.

22. The method of claim 21, which comprises using a therapeutic agent for hyperlipidemia in combination.

23. The method of claim 22, wherein the therapeutic agent for hyperlipidemia is a statin pharmaceutical agent.

24. The method of claim 23, wherein the statin pharmaceutical agent is lovastatin, simvastatin, pravastatin, fluvastatin, atorvastatin or cerivastatin.

25. A method for treating or preventing diabetes, which comprises administering a pharmaceutical composition for the treatment or prophylaxis of diabetes comprising a therapeutic agent for diabetes selected from the group consisting of insulin preparations, sulfonylurea agents, insulin secretagogues, sulfonamides, biguanides, α-glucosidase inhibitors and insulin sensitizers, and an HLGPa inhibitor in combination.

26. The method of claim 25, wherein the therapeutic agent for diabetes is selected from the group consisting of insulin, glibenclamide, torbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.

27. The method of claim 25 or 26, wherein the HLGPa inhibitor is an indole compound of any of claims 1 to 11, a pharmaceutically acceptable salt thereof or a prodrug thereof.

28. Use of an indole compound of any of claims 1 to 11, a pharmaceutically acceptable salt thereof or a prodrug thereof for the production of a therapeutic agent for diabetes.

29. The use of claim 28, which comprises use of a therapeutic agent for hyperlipidemia in combination.

30. The use of claim 29, wherein the therapeutic agent for hyperlipidemia is a statin pharmaceutical agent.

31. The use of claim 30, wherein the statin pharmaceutical agent is lovastatin, simvastatin, pravastatin, fluvastatin, atorvastatin or cerivastatin.

32. Use of a therapeutic agent for diabetes selected from the group consisting of selected from the group consisting of insulin preparations, sulfonylurea agents, insulin secretagogues, sulfonamides, biguanides, α-glucosidase inhibitors and insulin sensitizers and an HLGPa inhibitor for the production of a pharmaceutical composition for the treatment or prophylaxis of diabetes.

33. The use of claim 32, wherein the therapeutic agent for diabetes is selected from the group consisting of insulin, glibenclamide, torbutamide, glyclopyramide, acetohexamide, glimepiride, tolazamide, gliclazide, nateglinide, glybuzole, metformin hydrochloride, buformin hydrochloride, voglibose, acarbose and pioglitazone hydrochloride.

34. The use of claim 32 or 33, wherein the HLGPa inhibitor is an indole compound of any of claims 1 to 11, a pharmaceutically acceptable salt thereof or a prodrug thereof.

## Amended claims

### Amended claims under Art. 19.1 PCT

1. (Amended) An indole compound represented by the formula (1) 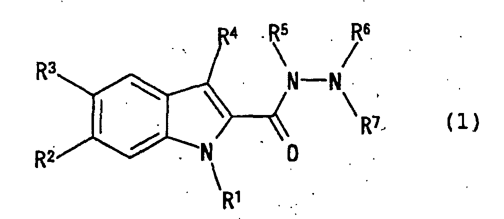 wherein
R¹ is a hydrogen atom, a C₁₋₆ alkyl group or an acyl group;
R² is a hydrogen atom or a halogen atom;
R³ is a halogen atom, a C₁₋₆ alkyl group, a C₁₋₆ alkoxy group, a nitro group, an amino group, a hydroxyl group, a cyano group, an acyl group, an aralkyloxy group or a thiazolyl group
wherein the thiazolyl group is optionally substituted by a C₁₋₆ alkyl group or an amino group;
R⁴ is a hydrogen atom or a C₁₋₆ alkyl group;
R⁵ is a hydrogen atom, a C₁₋₆ alkyl group or a C₂₋₇₋ alkoxycarbonyl group;
R⁶ is a hydrogen atom, a C₁₋₆ alkyl group or an aralkyl group
wherein the aralkyl group is optionally substituted by a halogen atom;
R⁷ is 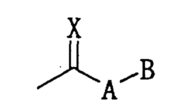 wherein X is =O or =NH;
A is -N(R⁸)- wherein R⁸ is a hydrogen atom, a C₁₋₆ alkyl group or an aryl group optionally having substituents, -C(R⁹)(R¹⁰)- wherein R⁹ and R¹⁰ are the same or different and each is independently a hydrogen atom, a hydroxyl group, an amino group, a C₁₋₆ alkyl group, a C₁₋₆ hydroxyalkyl group, a C₂₋₇ alkoxycarbonylamino group or an acylamino group, or R⁹ and R¹⁰ may form a C₃₋₇ cycloalkyl group together with the adjacent carbon atom, -(CH₂)ₘ-NH- wherein m is an integer of 1 to 4, -CO-, -S- or a single bond; and
B is substituents and R⁴⁴ is a hydrogen atom, a C₂₋₇ alkoxycarbonyl group or a C₁₋₆ alkyl group wherein the C₁₋₆ alkyl group is optionally substituted by a carboxyl group or a C₂₋₇ alkoxycarbonyl group, or -C(U)-N(R^{44'})- wherein U is =O or =S and R^{44'} is as defined for R⁴⁴ wherein an atom adjacent to the nitrogen atom on the fused ring in the formula (i) is described on the left end of each group;
R³⁸ is a hydrogen atom, an aryl group optionally having substituents or a heteroaryl group;
R³⁹ and R⁴⁰ are the same or different and each is a hydrogen atom, a C₁₋₆ alkyl group, a C₁₋₆ alkoxy group or a C₂₋₇ alkoxycarbonyl group, or R³⁹ and R⁴⁰ may form, together with the adjacent carbon atom, 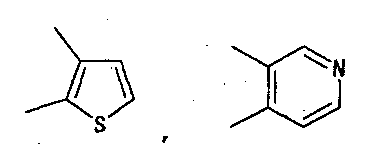 or 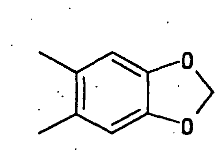
W is -CO-, -CS- or -CH₂-;
V₁ is -CO-, -CS- or -CH₂-;
V₂ is -O-, -CH₂- or -N(R⁴⁵)- wherein R⁴⁵ is a hydrogen atom, a C₁₋₆ alkyl group or an aryl group optionally having substituents; and
V₃ is -CH(R⁴⁶)- or -N(R^{46'})- wherein R⁴⁶ and R^{46'} are each a hydrogen atom, an aralkyl group, a heteroaryl group or an aryl group optionally having substituents,
provided that when A is -N(R⁸)- wherein R⁸ is as defined above and B is represented by the formula (a) 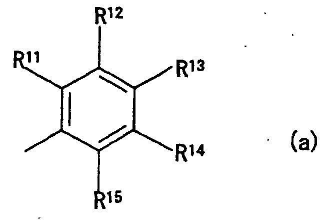 , then R¹¹, R¹², R¹³, R¹⁴ and R¹⁵ in the formula (a) are not hydrogen atoms at the same time,
a pharmaceutically acceptable salt thereof or a prodrug thereof.

2. (Amended) The indole compound of claim 1,
wherein
R⁶ is a hydrogen atom, a C₁₋₆ alkyl group or an aralkyl group wherein the aralkyl group is optionally substituted by a halogen atom;
R⁷ is 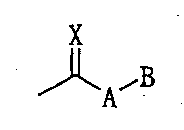 wherein X is =O or =NH;
A is -N(R^{8'})- wherein R^{8'} is a hydrogen atom, a C₁₋₆ alkyl group or a phenyl group optionally having substituents, -C(R^{9'})(R^{10'})- wherein R^{9'} and R^{10'} are the same or different and each is a hydrogen atom, a hydroxyl group, an amino group, a C₁₋₆ alkyl group, a C₁₋₆ hydroxyalkyl group, a C₂₋₇ alkoxycarbonylamino group or an acylamino group, or R^{9'} and R^{10'} may form, together with the adjacent carbon atom, a C₃₋₇ cycloalkyl group, -(CH₂)ₘ-NH- wherein m is an integer of 1 to 4, -CO-, -S- or a single bond; and
B is
Z' is -CH₂-CH₂-, -C(R⁴²)=CH-, -C(R^{42'})=N-, -N=N-, -CO-, -CO-O-, -CO-CH₂-O-, -CH₂-CO-NH-, -C(R^{42''})(R⁴³)-N(R⁴⁴)- wherein R⁴², R^{42'}, R^{42''} and R⁴³ are the same or different and each is a hydrogen atom, a C₁₋₆ alkyl group or a phenyl group optionally having substituents and R⁴⁴ are as defined in claim 1 or -C(U)-N(R^{44'})- wherein U and R^{44'} are as defined in claim 1;
R^{38'} is a hydrogen atom, a phenyl group optionally substituted by a halogen atom or a C₁₋₆ alkyl group, or a pyridyl group;
R^{39'} and R^{40'} are both hydrogen atoms, or R^{39'} and R^{40'} may form, together with the adjacent carbon atom, 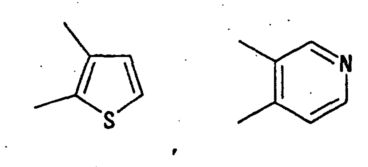 or 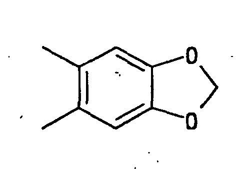
W and V₁ are as defined in claim 1;
V₂' is -O-, -CH₂- or -N(R⁴⁵)- wherein R⁴⁵ is a hydrogen atom, a C₁₋₆ alkyl group, a phenyl group optionally substituted by a halogen atom; and
V₃' is -CH(R⁴⁶)- or -N(R^{46'})- wherein R⁴⁶ and R^{46'} are each a hydrogen atom, a benzyl group, a thienyl group, or a phenyl group optionally substituted by a halogen atom, a hydroxy group or a C₁₋₆ alkoxy group;
provided that when A is -N(R^{8'})- wherein R^{8'} is as defined above and B is represented by the formula (a') 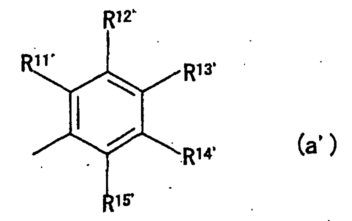 , then R^{11'}, R^{12'}, R^{13'}, R^{14'} and R^{15'} in the formula (a') are not hydrogen atoms at the same time,
a pharmaceutically acceptable salt thereof or a prodrug thereof.

3. (Amended) The indole compound of claim 1,
wherein
R⁶ is a hydrogen atom, a C₁₋₆ alkyl group or an aralkyl group wherein the aralkyl group is optionally substituted by a halogen atom;
R⁷ is 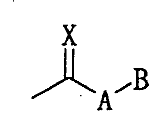 wherein X is as defined in claim 1;
A is -N(R^{8''})- wherein R^{8''} is a hydrogen atom, a C₁₋₆ alkyl group or an aryl group optionally having substituents, -C(R^{9''})(R^{10''})- wherein R^{9''} and R^{10''} are the same or different and each is a hydrogen atom, a hydroxyl group, an amino group, a C₁₋₆ alkyl group, a C₁₋₆ hydroxyalkyl group or a C₂₋₇ alkoxycarbonylamino group, or R^{9''} and R^{10''} may form, together with the adjacent carbon atom, a C₃₋₇ cycloalkyl group, -(CH₂)ₘ-NH- wherein m is as defined in claim 1, -CO- or a single bond; and
B is tetrazolyl group, a triazolyl group, -(CH₂)ₚ-CO-R¹⁹ wherein p and R¹⁹ are as defined in claim 1, -O-(CH₂)ᵣ-R²³ wherein r and R²³ are as defined in claim 1, -O-CO-R²⁷ wherein R²⁷ is as defined in claim 1 or -N(R^{28''})(R^{29''}) wherein R^{28''} and R^{29''} are the same or different and each is a hydrogen atom, a C₁₋₆ alkyl group, an aryl group optionally having substituents, -(CH₂)_{p'}-COO-R^{30''} wherein p' is as defined for p and R^{30''} is a hydrogen atom or a C₁₋₆ alkyl group wherein the C₁₋₆ alkyl group is optionally substituted by a hydroxyl group, a trifluoromethyl group or a carboxyl group, -CON(R³¹)(R³²) wherein R³¹ and R³² are as defined in claim 1, -CO-R³³ wherein R³³ is as defined in claim 1 or -CO-(CH₂)_{r'}-R³⁴ wherein r' and R³⁴ are as defined in claim 1,
R^{16b}-R¹⁶ⁿ and R^{17b}-R¹⁷ⁿ are as defined in claim 1,
R¹⁸ is as defined in claim 1,
Y'' is -S- or -N(R³⁵)- wherein R³⁵ is as defined in claim 1, and
n is as defined in claim 1, or
R⁶ and R⁷ may form, together with the adjacent nitrogen atom, 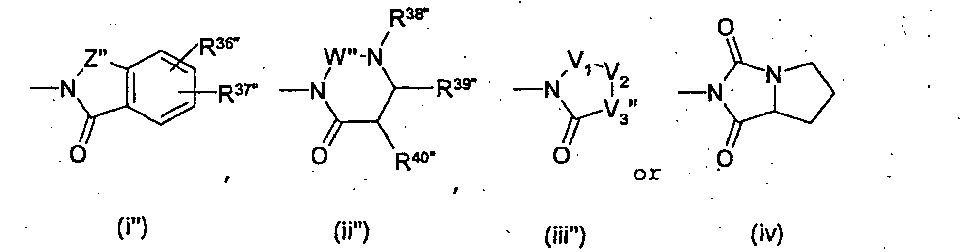 wherein R^{36''} and R^{37''} are the same or different and each is a hydrogen atom, a halogen atom, a C₁₋₆ alkyl group, a C₁₋₆ alkoxy group, an amino group, a hydroxyl group or -O-CO-R⁴¹ wherein R⁴¹ are as defined in claim 1;
Z'' is, -CH₂-CH₂-, -C(R⁴²)=CH-, -N=N-, -CO-, -CO-O-, -CO-CH₂-O-, -CH₂-CO-NH-, -C(R^{42''})(R⁴³)-N(R^{44''})- wherein R⁴², R^{42''} and R⁴³ are as defined in claim 1 and R^{44''} is a hydrogen atom, a C₁₋₆ alkyl group or a C₂₋₇ alkoxycarbonyl group or -C(U)-N(R^{44'''})- wherein U is =O or =S and R^{44'''} is as defined for R^{44''};
R^{38''} is a hydrogen atom or an aryl group optionally having substituents;
R^{39''} and R^{40''} are the same or different and each is a hydrogen atom, a C₁₋₆ alkyl group or a C₂₋₇ alkoxycarbonyl group, or R^{39''} and R^{40''} may form, together with the adjacent carbon atom, 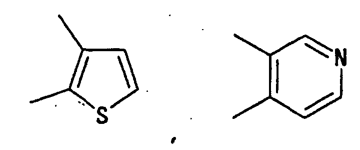 or 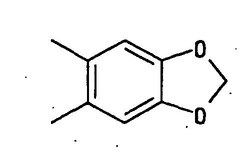
W'' is -CO- or -CH₂-;
V₁ and V₂ are as defined in claim 1; and
V₃'' is -CH(R^{46''})- or -N(R^{46'''})- wherein R^{46''} and R^{46'''} are the same or different and each is a hydrogen atom or an aryl group optionally having substituents;
provided that when A is -N(R^{8''})- wherein R^{8''} is as defined above and B is represented by the formula (a") 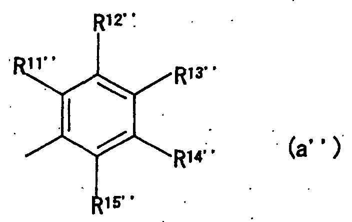 , then R^{11''}, R^{12''}, R^{13''}, R^{14''} and R^{15''} in the formula (a") are not hydrogen atoms at the same time,
a pharmaceutically acceptable salt thereof or a prodrug thereof.

4. The indole compound of claim 1, wherein R¹, R², R⁴, R⁵ and R⁶ are each a hydrogen atom, a pharmaceutically acceptable salt thereof or a prodrug thereof.

5. The indole compound of claim 4, wherein R³ is a halogen atom or a C₁₋₆ alkyl group, a pharmaceutically acceptable salt thereof or a prodrug thereof.

6. The indole compound of claim 4, wherein X=O, A is a single bond and B is 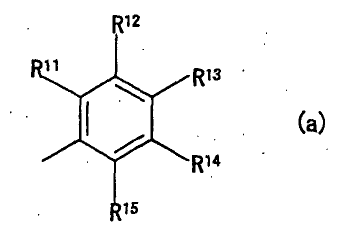 a pharmaceutically acceptable salt thereof or a prodrug thereof.

7. The indole compound of claim 4, wherein X=NH, A is a single bond and B is 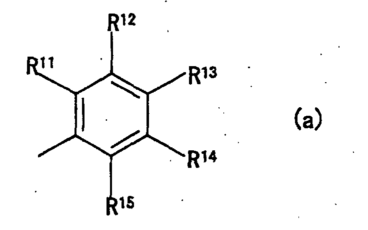 a pharmaceutically acceptable salt thereof or a prodrug thereof.

8. The indole compound of claim 1, wherein R⁶ and R⁷ may form, together with the adjacent nitrogen atom, 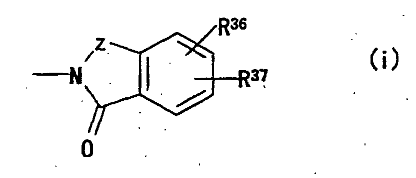 a pharmaceutically acceptable salt thereof or a prodrug thereof.

9. (Amended) The indole compound of claim 1, which is selected from the group consisting of
benzoic acid 2-(5-chloro-1H-indole-2-carbonyl)hydrazide,
2-aminobenzoic acid 2-(5-chloro-1H-indole-2-carbonyl)hydrazide,
2-hydroxybenzoic acid 2-(5-chloro-1H-indole-2-carbonyl)hydrazide,
3-(2-(2-(5-chloro-1H-indole-2-carbonyl)hydrazinocarbonyl)phenylcarbamoyloxy)-2,2-dimethylpropionic acid,
benzoic acid 2-(5-chloro-1H-indole-2-carbonyl)-1-methylhydrazide,
benzoic acid 2-(1-acetyl-5-chloro-1H-indole-2-carbonyl)hydrazide,
5-chloro-1H-indole-2-carboxylic acid 2-(imino-phenyl-methyl)hydrazide,
5-aminothiazole-4-carboxylic acid 2-(5-chloro-1H-indole-2-carbonyl)hydrazide,
benzoic acid 2-(5-fluoro-1H-indole-2-carbonyl)hydrazide,
cyclohexanecarboxylic acid 2-(5-fluoro-1H-indole-2-carbonyl)hydrazide,
hydroxyphenyl)imidazolidin-1-yl)amide,
5-chloro-1H-indole-2-carboxylic acid ((4S)-2,5-dioxo-4-(4-methoxyphenyl)imidazolidin-1-yl)amide,
5-chloro-1H-indole-2-carboxylic acid ((4R)-2,5-dioxo-4-(4-methoxyphenyl)imidazolidin-1-yl)amide,
5-chloro-1H-indole-2-carboxylic acid 2-(phenylthiocarbonyl)hydrazide,
5-chloro-1H-indole-2-carboxylic acid 2-(2-phenylacetyl)hydrazide,
5-chloro-1H-indole-2-carboxylic acid 2-(2-oxo-2-phenylacetyl)hydrazide,
5-chloro-1H-indole-2-carboxylic acid 2-((2-fluorophenyl)aminocarbonyl)hydrazide,
5-chloro-1H-indole-2-carboxylic acid 2-((3-fluorophenyl)aminocarbonyl)hydrazide,
5-chloro-1H-indole-2-carboxylic acid 2-((4-fluorophenyl)aminocarbonyl)hydrazide,
5-chloro-1H-indole-2-carboxylic acid 2-((2-chloroanilino)carbonyl)hydrazide,
5-chloro-1H-indole-2-carboxylic acid 2-((3-chloroanilino)carbonyl)hydrazide,
5-chloro-1H-indole-2-carboxylic acid 2-((4-chloroanilino)carbonyl)hydrazide,
5-chloro-1H-indole-2-carboxylic acid 2-((1-phenylcyclopropane)carbonyl)hydrazide,
5-chloro-1H-indole-2-carboxylic acid 2-((1-phenylcyclopentane)carbonyl)hydrazide,
5-chloro-1H-indole-2-carboxylic acid 2-((1-phenylcyclohexane)carbonyl)hydrazide,
5-chloro-1H-indole-2-carboxylic acid 2-(2-phenylpropanoyl)hydrazide,
5-chloro-1H-indole-2-carboxylic acid 2-(3-hydroxy-2-phenylpropanoyl)hydrazide,

Statement under Art. 19.1 PCT
In claims 1 to 3 and 9,
**1.** a compound wherein X is =S, and
**2.** a compound wherein A for R7 is -N(R⁸)-, B is a group represented by the formula (a) and R¹¹, R¹², R¹³, R¹⁴, and R¹⁵ in the formula (a) are hydrogen atoms at the same time have been deleted from the compounds of the formula (1). By this amendment, the present invention has been distinguished from the references 6 to 18 cited in the International Search Report, where the reference numbers are assigned in the order of appearance of the reference in the Report.

From the foregoing, it is concluded that the present invention hav novelty over references 6 to 18.
